# EUROPÄISCHE PATENTANMELDUNG

(11) **EP 2 017 264 A1**
(43) Veröffentlichungstag der Anmeldung: **21.01.2009**
(21) Anmeldenummer: 07013871.4
(22) Anmeldetag: 16.07.2007
(51) Int. Cl.: C07D 213/26, C07D 213/53, C07D 213/61, C07D 213/64, C07D 213/68, C07D 213/84, C07D 231/12, C07D 231/56, C07D 241/12, C07D 241/16, C07D 241/18, A01N 33/24, A01N 33/26, A01N 41/02, A01N 43/48, C07C 251/52, C07C 251/54, C07C 251/86, C07C 255/33, C07C 317/22, C07C 317/32, C07C 323/19, C07C 323/48

(54) **Substituierte Phenylpropargylverbindungen, Verfahren zu deren Herstellung und deren Verwendung als Herbizide und Pflanzenwachstumsregulatoren**

(71) Anmelder: BAYER CROPSCIENCE AG, 40789 Monheim (DE)
(72) Erfinder: Tiebes, Jörg. Dr, 60431 Frankfurt (DE); Giencke, Wolfgang. Dr, 65719 Hofheim (DE); Kehne, Heinz. Dr, 65719 Hofheim (DE); Rosinger, Christopher. Dr, 65719 Hofheim (DE); Hills, Martin, 65510 Idstein (DE)

(57) **Zusammenfassung**

Verbindungen der Formel (1) oder deren Salze, 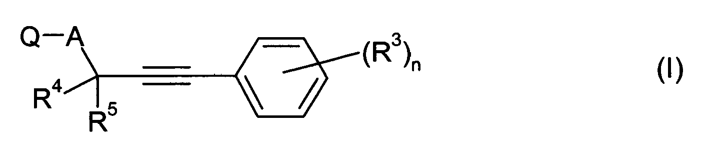 worin
Q einen (hetero)aromatischen Rest bis 6 Ringatomen, wobei im Falle des heteroaromatischen Restes der Ring 1,2,3 oder 4 Stickstoffatome oder 1 Schwefelatom oder 1 Schwefelatom und 1 Stickstoffatom als Heteroringatome aufweist, und die Reste noch wie in Anspruch 1 definert substituiert sein können, und
A eine ungesättigte divalente Gruppe der Formel (A1),(A2),(A3),(A4) oder (A5), 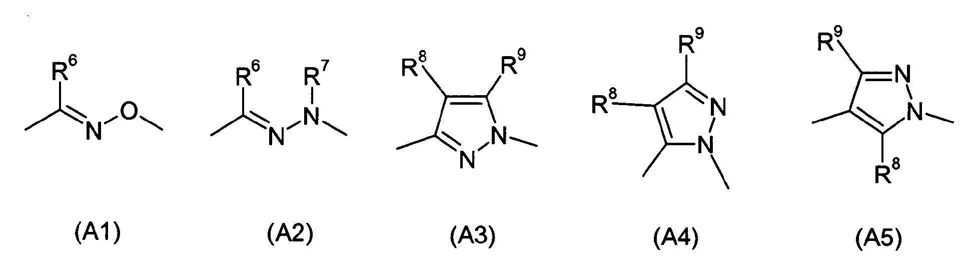 bedeuten
wobei (R³)ₙ,R⁴,R⁵,R⁶,R⁷,R⁸ und R⁹ wie in Anspruch 1 definiert sind,
eignen sich als Herbizide und Pflanzenwachstumsregulatoren. Die Verbindungen können nach Verfahren gemäß Anspruch 6 hergestellt werden.

## Beschreibung

Die Erfindung betrifft das technische Gebiet der Herbizide und Pflanzenwachstumsregulatoren, insbesondere der Herbizide zur Bekämpfung von Schadpflanzen wie Unkräutern und Ungräsern in Nutzpflanzenkulturen oder generell zur Kontrolle von unerwünschtem Pflanzenwachstum.

Aus EP-A-0374839 sind herbizid wirksame 2-(Pyrimidin-2-yloxy)-benzaldoxime bekannt, die an der Hydroxygruppe der Oximgruppe durch kurzkettiges Alkyl, Alkenyl oder Alkinyl oder auch Cycloalkyl verethert sein können, wobei die Ethergruppen gegebenenfalls durch Reste wie Halogen oder Phenyl weiter substituiert sind. Diese Verbindungen sind als Herbizide zur Bekämpfung von Schadpflanzen in Kulturpflanzen beschrieben, beispielsweise für die selektive Anwendung in breitblättrigen Kulturen wie Baumwolle, Sojabohne oder Erdnussplantagen. Die Wirkstoffe gehören zur Gruppe der Acetolactatsynthase-Inhibitoren.
Aus JP-A-11-147866 (1999) sind herbizide N-Phenyl- oder N-pyridyl-alkylurethane bekannt, die an der Phenylgruppe oder Pyridylgruppe mit einer (Hetero)arylpropargyloxygruppe oder einer (Hetero)aryl-allyloxygruppe substituiert sind.
Aus WO-A-01/55066, WO-A-02/28182, WO-A-2005/047233 sind weiterhin herbizide Wirkstoffe bekannt, welche einen mit einer gegebenenfalls substituierten und/oder verbrückten Phenylalkinyloxy-gruppe substituierten Benzolring oder 6-Ring-Heteroaromaten enthalten. Diese Herbizide können zur Bekämpfung von Schadpflanzen in unterschiedlichen mono- oder dikotylen Kulturpflanzen eingesetzt werden.

Die bekannten Pflanzenschutzmittelwirkstoffe zur selektiven Bekämpfung von Schadpflanzen in Nutzpflanzenkulturen oder Wirkstoffe zur Bekämpfung von unerwünschtem Pflanzenwuchs weisen bei ihrer Anwendung teilweise Nachteile auf.

Nachteilig ist beispielsweise, dass sie teilweise keine oder aber eine unzureichende herbizide Wirkung gegen bestimmte Schadpflanzen besitzen, dass sie ein zu geringes Spektrum der Schadpflanzen, das mit einem Wirkstoff bekämpft werden kann, aufweisen, dass sie zu geringe Selektivität in Nutzpflanzenkulturen oder ein toxikologisch ungünstiges Profil besitzen.
Derartige Wirkstoffe, die als Pflanzenwachstumsregulatoren bei einigen Nutzpflanzen eingesetzt werden können, führen bei anderen Nutzpflanzen zu unerwünscht verminderten Ernteerträgen oder sind mit der Kulturpflanze nicht oder nur in einem engen Aufwandmengenbereich verträglich.
Andere bekannte Wirkstoffe lassen sich wegen schwer zugänglicher Vorprodukte und Reagenzien im industriellen Maßstab nicht wirtschaftlich herstellen oder besitzen nur unzureichende chemische Stabilitäten. Bei anderen Wirkstoffen hängt die Wirkung zu stark von Umweltbedingungen, wie Wetter- und Bodenverhältnissen ab.

Aus den genannten Gründen besteht weiterhin ein Bedarf nach alternativen wirkungsstarken Herbiziden für die selektive Anwendung in Pflanzenkulturen oder für die Anwendung im Nichtkulturland. Auch ist es wünschenswert, alternative chemische Wirkstoffe bereitzustellen, die gegebenenfalls mit Vorteilen als Herbizide oder Pflanzenwachstumsregulatoren eingesetzt werden können.

Gegenstand der vorliegenden Erfindung sind Verbindungen der Formel (I) oder deren Salze, 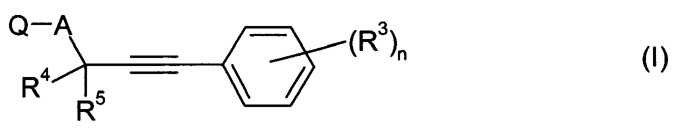 in welcher
Q einen carbocyclischen aromatischen Rest mit 6 Ringatomen oder einen heteroaromatischen Rest mit 5 bis 6 Ringatomen, wobei im Falle des heteroaromatischen Restes der Ring 1, 2, 3 oder 4 Stickstoffatome oder 1 Schwefelatom oder 1 Schwefelatom und 1 Stickstoffatom als Heteroringatome aufweist und wobei der carbocyclische aromatische Rest oder der heteroaromatische Rest an einer oder mehreren CH-Gruppen des Rings unsubstituiert oder mit einem Rest R¹ substituiert ist (CH wird zu CR¹), wobei die gegebenenfalls vorhandene Substitution der Gruppe Q durch Reste R¹ abgekürzt durch die Formel (R¹)ₘ definiert ist und der Rest oder die m Reste R¹ am Ring unabhängig voneinander wie nachstehend definiert sind, und wobei der heteroaromatische Rest, wenn er eine NH-Gruppe im Ring enthält, an der NH-Gruppe unsubstituiert oder mit einem Rest R² substituiert ist (NH wird zu NR²), wobei der Rest R² wie nachstehend definiert ist,
A eine ungesättigte divalente Gruppe der Formel (A1), (A2), (A3), (A4) oder (A5), 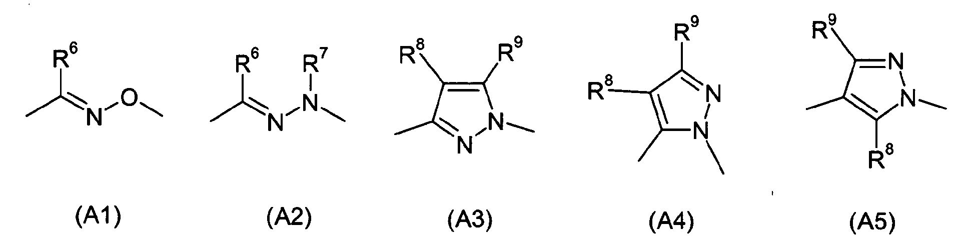 wobei der Rest Q mit der Gruppe A an dem in der jeweiligen Formel (A1) bis (A5) gezeigten ungesättigten C-Atom der Gruppe A gebunden ist (in den obigen Formeln (A1) bis (A5) jeweils links gezeichnet) und die Gruppe CR⁴R⁵ mit der Gruppe A an dem in der jeweiligen Formel (A1) bis (A5) gezeigten gesättigten Heteroatom der Gruppe A gebunden ist (in den obigen Formeln für (A1) bis (A5) jeweils rechts gezeichnet) und wobei die Doppelbindung in der Gruppe der Formel (A1) oder (A2) Z- oder E-konfiguriert ist und wobei R⁶, R⁷, R⁸ und R⁹ wie nachstehend definiert sind,
(R¹)ₘ m Substituenten R¹ bedeutet, wobei R¹, im Fall dass m = 1 ist, oder jeder der Reste R¹ jeweils unabhängig voneinander, im Fall dass m größer als 1 ist, einen Rest aus den Gruppen (R¹a) bis (R¹j) bedeutet, enthaltend die Reste (R¹a) Halogen, Cyano, Azido, SF₅, Isocyanato und Nitro, (R¹b) Reste der Formel -NR^{a}R^{b},
   worin jeder der Reste R^{a} und R^{b} unabhängig voneinander für einen Rest aus der Gruppe, welche die Reste
   (1) Wasserstoff, (C₁-C₈)Alkyl, (C₂-C₈)Alkenyl und (C₂-C₈)Alkinyl,
   (2) Phenyl und Benzyl,
      wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
   (3) [(C₁-C₈)Alkanoyl, das unsubstituiert oder durch einen oder mehrere Reste aus der Gruppe Halogen, CN, (C₁-C₆)Alkylamino, Di[(C₁-C₆)alkyl]-amino und (C₁-C₆)Alkoxy substituiert ist,
   (4) [(C₁-C₈)Alkoxy]-carbonyl, das unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist,
   (5) (C₁-C₄)Alkylsulfinyl und (C₁-C₄)Alkylsulfonyl, wobei jeder der beiden letztgenannten Reste unsubstituiert oder durch einen oder mehrere Reste aus der Gruppe Halogen, CN, (C₁-C₄)Alkoxy und (C₁-C₄)Haloalkoxy substituiert ist,
   enthält, steht
   oder
   worin einer der Reste R^{a} und R^{b} wie oben definiert ist und der andere für Hydroxy oder (C₁-C₆)Alkoxy steht
   oder
   worin R^{a} und R^{b} gemeinsam für eine (C₂-C₅)Alkylen-Kette, welche durch ein oder mehrere Heteroatome aus der Gruppe O und S unterbrochen sein kann und welche unsubstituiert oder durch einen oder mehrere (C₁-C₄)Alkylreste substituiert ist, stehen
   oder
   worin R^{a} und R^{b} gemeinsam mit dem N-Atom der Gruppe NR^{a}R^{b} für
   N-Phthalimido stehen,
(R¹c) Reste der Formel -CO₂R,
   worin R für Wasserstoff, (C₁-C₈)Alkyl, (C₂-C₈)Alkenyl oder (C₂-C₈)Alkinyl, wobei jeder der letztgenannten 3 Reste unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist, steht,
(R¹d) Reste der Formeln -CO-NR^{a}R^{b} und -CS-NR^{a}R^{b},
   worin jeder der Reste R^{a} und R^{b} in den letztgenannten beiden Formeln unabhängig voneinander für einen Rest aus der Gruppe, welche
   (1) Wasserstoff und (C₁-C₈)Alkyl, das unsubstituiert oder durch einen oder mehrere Reste aus der Gruppe Carboxy, [(C₁-C₈)Alkoxy]-carbonyl und CN substituiert ist,
   (2) (C₂-C₈)Alkenyl und (C₂-C₈)Alkinyl,
   (3) Phenyl und Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
      enthält, steht oder
   worin R^{a} und R^{b} gemeinsam für eine (C₂-C₅)Alkylen-Kette, welche durch ein oder mehrere Heteroatome aus der Gruppe O und S unterbrochen sein kann und welche unsubstituiert oder durch einen oder mehrere (C₁-C₄)Alkylreste substituiert ist, stehen,
(R¹e) Reste der Formel -CO-R,
   worin R für Wasserstoff, (C₁-C₆)Alkyl, (C₁-C₆)Haloalkyl oder (C₃-C₆)Cycloalkyl, wobei der Cycloalkylrest unsubstituiert oder durch einen oder mehrere Reste aus der Gruppe (C₁-C₄)Alkyl substituiert ist, steht,
(R¹f) Reste der Formel -C(R^{a})=N-OR^{b},
   worin
   R^{a} für Wasserstoff, (C₁-C₆)Alkyl, (C₁-C₆)Haloalkyl oder (C₃-C₆)Cycloalkyl, wobei der Cycloalkylrest unsubstituiert oder durch einen oder mehrere (C₁-C₄)Alkylreste substituiert ist, und
   R^{b} für Wasserstoff, (C₁-C₈)Alkyl, (C₁-C₈)Haloalkyl, (C₂-C₈)Alkenyl, (C₂-C₈)Haloalkenyl oder (C₂-C₈)Alkinyl stehen,
(R¹g) Reste der Formel -C(SR^{a})=NR^{b}, worin
   R^{a} für (C₁-C₆)Alkyl und
   R^{b} für Wasserstoff oder (C₁-C₆)Alkyl stehen,
(R¹h) Reste der Formel -OR, worin R
   (1) für Wasserstoff, (C₁-C₈)Alkyl, (C₂-C₈)Alkenyl oder (C₂-C₈)Alkinyl
   (2) oder für (C₁-C₈)Alkyl, das unsubstituiert oder durch einen oder mehrere Reste aus der Gruppe Halogen, CN, Hydroxy, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist,
   (3) oder für [(C₁-C₈)Alkoxy]-carbonyl, das unsubstituiert oder durch einen oder mehrere Reste aus der Gruppe Halogen, CN, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist,
   (4) oder für Phenyl oder Phenyl-(C₁-C₆)alkyl, wobei jeder der letztgenannten beiden Reste im Phenylring unsubstituiert oder durch einen oder mehrere Reste aus der Gruppe Halogen, CN, Hydroxy, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, [(C₁-C₄)Alkoxy]-carbonyl, [(C₁-C₄)Haloalkoxy]-carbonyl, R'R"N-CO-, worin jeder Rest R' und R" unabhängig voneinander H, (C₁-C₄)Alkyl oder (C₁-C₄)Haloalkyl bedeutet, (C₁-C₄)Alkylsulfinyl und (C₁-C₄)Alkylsulfonyl substituiert ist, steht,
(R¹i) Reste der Formel -SR, -S(=O)-R, -O-S(=O)-R, -S(=O)₂-R, und -O-S(=O)₂-R,
   wobei in jedem der letztgenannten 5 Formeln R jeweils unabhängig voneinander von den anderen Resten R für Wasserstoff, (C₁-C₈)Alkyl, (C₂-C₈)Alkenyl oder (C₂-C₈)Alkinyl
   oder für (C₁-C₈)Alkyl, das durch einen oder mehrere Reste aus der Gruppe Halogen, CN und (C₁-C₄)Alkoxy substituiert ist, steht,
(R¹j) (C₁-C₈)Alkyl, (C₂-C₈)Alkenyl, (C₂-C₈)Alkinyl, (C₃-C₆)Cycloalkyl, (C₅-C₆)Cycloalkenyl, Heterocyclyl mit 3 bis 6 Ringatomen und einem oder mehreren Heteroringatomen aus der Gruppe N, O und S oder Phenyl,
   wobei jeder der 7 letztgenannten Reste unsubstituiert oder durch einen oder mehrere Reste aus den Gruppen (G¹a) bis (G¹L) substituiert ist, enthaltend die Reste
   (G¹a) Halogen, Cyano, Azido, SF₅, Isocyanato und Nitro,
   (G¹b) (C₃-C₆)Cycloalkyl und (C₅-C₆)Cycloalkenyl,
      wobei jeder der letztgenannten 2 Reste unsubstituiert oder durch einen oder mehrere Reste aus der Gruppe Halogen und (C₁-C₃)Alkyl substituiert ist,
   (G¹c) Phenyl und Pyridyl,
      wobei jeder der letztgenannten 2 Reste unsubstituiert oder durch einen oder mehrere Reste aus der Gruppe enthaltend Halogen, Hydroxy, (C₁-C₄)Alkyl, (C₁-C₄)Haloalkyl, (C₁-C₄)Alkoxy, (C₁-C₄)Haloalkoxy, (C₁-C₄)Alkylthio, [(C₁-C₄)Alkoxy]-carbonyl, [(C₁-C₄)Haloalkoxy]-carbonyl, R'R"N-CO-, worin jeder Rest R' und R" unabhängig voneinander H, (C₁-C₄)Alkyl oder (C₁-C₄)Haloalkyl bedeutet oder R' und R" gemeinsam mit dem N-Atom einen gesättigten oder ungesättigten heterocyclischen Ring mit 5 oder 6 Ringatomen, der neben dem N-Atom noch ein N-Atom oder Sauerstoffatom als Heteroatom enthalten kann, bedeuten, (C₁-C₄)Alkylsulfinyl und (C₁-C₄)Alkylsulfonyl substituiert ist,
   (G¹d) Reste der Formel -NR^{a}R^{b}, worin jeder der Reste R^{a} und R^{b} unabhängig voneinander für einen Rest aus der Gruppe, welche die Reste
      (1) Wasserstoff, (C₁-C₈)Alkyl, (C₂-C₈)Alkenyl und (C₂-C₈)Alkinyl,
      (2) Phenyl und Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
      (3) [(C₁-C₈)Alkyl]-carbonyl, das unsubstituiert oder durch einen oder mehrere Reste aus der Gruppe Halogen, CN, (C₁-C₆)Alkylamino, Di[(C₁-C₆)alkyl]-amino und (C₁-C₆)Alkoxy substituiert ist,
      (4) [(C₁-C₈)Alkoxy]-carbonyl, das unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist,
      (5) (C₁-C₄)Alkylsulfinyl und (C₁-C₄)Alkylsulfonyl, wobei jeder der beiden letztgenannten Reste unsubstituiert oder durch einen oder mehrere Reste aus der Gruppe Halogen, CN, (C₁-C₄)Alkoxy und (C₁-C₄)Haloalkoxy substituiert ist,
         enthält, steht oder
         R^{a} und R^{b} gemeinsam für eine (C₂-C₅)Alkylen-kette, welche durch ein oder mehrere Heteroatome aus der Gruppe 0 und S unterbrochen sein kann und welche unsubstituiert oder durch
         einen oder mehrere (C₁-C₄)Alkylreste substituiert ist, stehen,
   (G¹e) Reste der Formel -CO₂R,
      worin R für Wasserstoff, (C₁-C₈)Alkyl, (C₂-C₈)Alkenyl oder (C₂-C₈)Alkinyl, wobei jeder der letztgenannten 3 Reste unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist, steht,
   (G¹f) Reste der Formeln -CO-NR^{a}R^{b} und -CS-NR^{a}R^{b}, worin R^{a} und R^{b} in den letztgenannten beiden Formeln jeweils unabhängig voneinander für einen Rest aus der Gruppe, welche die Reste
      (1) Wasserstoff und (C₁-C₈)Alkyl, das unsubstituiert oder durch einen oder mehrere Reste aus der Gruppe Carboxy, [(C₁-C₈)Alkoxy]-carbonyl und CN substituiert ist,
      (2) (C₂-C₈)Alkenyl und (C₂-C₈)Alkinyl,
      (3) Phenyl und Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
      enthält, stehen oder
      R^{a} und R^{b} gemeinsam für eine (C₂-C₅)Alkylen-kette, welche durch ein oder mehrere Heteroatome aus der Gruppe O und S unterbrochen sein kann und welche unsubstituiert oder durch
      einen oder mehrere (C₁-C₄)Alkylreste substituiert ist, stehen,
   (G¹g) Reste der Formel -CO-R,
      worin R für Wasserstoff, (C₁-C₆)Alkyl, (C₁-C₆)Haloalkyl oder (C₃-C₆)Cycloalkyl, wobei der Cycloalkylrest unsubstituiert oder durch einen oder mehrere (C₁-C₄)Alkylreste substituiert ist, steht,
   (G¹h) Reste der Formel -C(R^{a})=N-OR^{b}, worin
      R^{a} für Wasserstoff, (C₁-C₆)Alkyl, (C₁-C₆)Haloalkyl oder (C₃-C₆)Cycloalkyl, wobei der Cycloalkylrest unsubstituiert oder durch einen oder mehrere (C₁-C₄)Alkylreste substituiert ist, und
      R^{b} für Wasserstoff, (C₁-C₈)Alkyl, (C₁-C₈)Haloalkyl, (C₂-C₈)Alkenyl, (C₂-C₈)Haloalkenyl oder (C₂-C₈)Alkinyl
      stehen,
   (G¹i) Reste der Formel -C(SR^{a})=NR^{b}, worin
      R^{a} für (C₁-C₆)Alkyl und
      R^{b} für Wasserstoff oder (C₁-C₆)Alkyl stehen,
   (G¹j) Reste der Formel -OR,
      worin R
      (1) für Wasserstoff, (C₁-C₈)Alkyl, (C₂-C₈)Alkenyl oder (C₂-C₈)Alkinyl,
      (2) oder für (C₁-C₈)Alkyl, das unsubstituiert oder durch einen oder mehrere Reste aus der Gruppe Halogen, CN, Hydroxy, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist,
      (3) oder für [(C₁-C₈)Alkoxy]-carbonyl, das unsubstituiert oder durch einen oder mehrere Reste aus der Gruppe Halogen, CN, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist,
      (4) oder für Phenyl oder Phenyl-(C₁-C₆)alkyl, wobei jeder der letztgenannten beiden Reste im Phenylring unsubstituiert oder durch einen oder mehrere Reste aus der Gruppe Halogen, CN, Hydroxy, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, [(C₁-C₄)Alkoxy]-carbonyl, [(C₁-C₄)Haloalkoxy]-carbonyl, R'R"N-CO-, worin jeder Rest R' und R" unabhängig voneinander H, (C₁-C₄)Alkyl oder (C₁-C₄)Haloalkyl bedeutet oder R' und R" gemeinsam mit dem N-Atom einen gesättigten oder ungesättigten heterocyclischen Ring mit 5 oder 6 Ringatomen, der neben dem N-Atom noch ein N-Atom oder Sauerstoffatom als Heteroatom enthalten kann, bedeuten, (C₁-C₄)Alkylsulfinyl und (C₁-C₄)Alkylsulfonyl substituiert ist,
      steht,
   (G¹k) Reste der Formel -SR, -S(=O)-R, -O-S(=O)-R, -S(=O)₂-R und -O-S(=O)₂-R,
      wobei in jedem der letztgenannten 5 Formeln R jeweils unabhängig von den anderen Resten R für Wasserstoff, (C₁-C₈)Alkyl, (C₂-C₈)Alkenyl oder (C₂-C₈)Alkinyl
      oder für (C₁-C₈)Alkyl, das durch einen oder mehrere Reste aus der Gruppe Halogen, CN und (C₁-C₄)Alkoxy substituiert ist, steht,
   (G¹L) und, nur im Falle R¹ = (C₃-C₆)Cycloalkyl, (C₅-C₆)Cycloalkenyl, Heterocyclyl oder Phenyl als Basisgruppe, auch die Reste (C₁-C₄)Alkyl, (C₁-C₄)Haloalkyl und (C₁-C₄)Alkoxy-(C₁-C₄)alkyl,
   oder zwei benachbarte Reste R¹ gemeinsam eine (C₂-C₇)Alkylen- oder (C₃-C₇)Alkenylengruppe, welche durch 1, 2 oder 3 Heteroatome aus der Gruppe O, S und N unterbrochen sein kann und welche unsubstituiert oder durch einen oder mehrere Reste aus der Gruppe Halogen, CN, (C₁-C₄)Alkyl, (C₁-C₄)Haloalkyl und (C₁-C₄)Alkoxy substituiert ist, bedeuten,
m eine ganze Zahl von 0 bis zur Anzahl der CH-Gruppen im Ring des Grundkörpers des Restes der Formel Q bedeutet,
R² Wasserstoff, (C₁-C₆)Alkyl, (C₂-C₆)Alkenyl, (C₂-C₆)Alkinyl, (C₃-C₆)Cycloalkyl, (C₅-C₆)Cycloalkenyl, Heterocyclyl mit 3 bis 6 Ringatomen und 1, 2 oder 3 Heteroringatomen aus der Gruppe N, O und S oder Phenyl bedeutet, wobei jeder der 7 letztgenannten Reste unsubstituiert oder durch einen oder mehrere Reste aus den Gruppen (G²a) bis (G²h) substituiert ist, enthaltend die Reste
   (G²a) Halogen, Cyano, Azido, SF₅, Isocyanato und Nitro,
   (G²b) (C₃-C₆)Cycloalkyl und (C₅-C₆)Cycloalkenyl,
      wobei jeder der letztgenannten 2 Reste unsubstituiert oder durch einen oder mehrere Reste aus der Gruppe Halogen und (C₁-C₃)Alkyl substituiert ist,
   (G²c) Phenyl und Pyridyl,
      wobei jeder der letztgenannten 2 Reste unsubstituiert oder durch einen oder mehrere Reste aus der Gruppe, welche die Reste
      (1) Halogen, Hydroxy, (C₁-C₄)Alkyl, (C₁-C₄)Haloalkyl, (C₁-C₄)Alkoxy, (C₁-C₄)Haloalkoxy, (C₁-C₄)Alkylthio, [(C₁-C₄)Alkoxy]-carbony und [(C₁-C₄)Haloalkoxy]-carbonyl,
      (2) (C₁-C₄)Alkylsulfinyl und (C₁-C₄)Alkylsulfonyl enthält, substituiert ist,
   (G²d) Reste der Formel -CO₂R,
      worin R für Wasserstoff, (C₁-C₈)Alkyl, (C₂-C₈)Alkenyl oder (C₂-C₈)Alkinyl, wobei jeder der letztgenannten 3 Reste unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist, steht,
   (G²e) Reste der Formeln -CO-NR^{a}R^{b} und -CS-NR^{A}R^{b},
      worin jeder der Reste R^{a} und R^{b} in den letztgenannten beiden Formeln jeweils unabhängig voneinander für einen Rest aus der Gruppe, welche die Reste
      (1) Wasserstoff und (C₁-C₈)Alkyl, das unsubstituiert oder durch einen oder mehrere Reste aus der Gruppe Carboxy, [(C₁-C₈)Alkoxy]-carbonyl und CN substituiert ist,
      (2) (C₂-C₈)Alkenyl und (C₂-C₈)Alkinyl,
      (3) Phenyl und Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
         enthält, steht oder
         R^{a} und R^{b} gemeinsam für eine (C₂-C₅)Alkylen-kette stehen, welche durch ein oder mehrere Heteroatome aus der Gruppe O und S unterbrochen sein kann und welche unsubstituiert oder durch einen oder mehrere (C₁-C₄)Alkylreste substituiert ist,
   (G²f) Reste der Formel -OR,
      worin R für Wasserstoff, (C₁-C₈)Alkyl, (C₂-C₈)Alkenyl oder
      (C₂-C₈)Alkinyl,
      oder für (C₁-C₈)Alkyl, das unsubstituiert oder durch einen oder mehrere Reste aus der Gruppe Halogen, CN, Hydroxy, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist,
      oder für [(C₁-C₈)Alkoxy]-carbonyl, das unsubstituiert oder durch einen oder mehrere Reste aus der Gruppe Halogen, CN, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist,
      oder für Phenyl oder Phenyl-(C₁-C₆)alkyl, wobei jeder der letztgenannten beiden Reste im Phenylring unsubstituiert oder durch einen oder mehrere Reste aus der Gruppe, welche die Reste
      (1) Halogen, CN, Hydroxy, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, [(C₁-C₄)Alkoxy]-carbonyl, [(C₁-C₄)Haloalkoxy]-carbonyl,
      (2) (C₁-C₄)Alkylsulfinyl und (C₁-C₄)Alkylsulfonyl, enthält, substituiert ist,
         steht,
   (G²g) Reste der Formel -SR, -S(=O)-R, -O-S(=O)-R, -S(=O)₂-R,
      und -O-S(=O)₂-R, wobei in jedem der letztgenannten 5 Formeln R jeweils unabhängig voneinander von den anderen Resten R für Wasserstoff, (C₁-C₈)Alkyl, (C₂-C₈)Alkenyl oder (C₂-C₈)Alkinyl oder für (C₁-C₈)Alkyl, das unsubstituiert oder durch einen oder mehrere Reste aus der Gruppe Halogen, CN und (C₁-C₄)Alkoxy substituiert ist, steht,
   (G²h) und, nur für den Fall R² = (C₃-C₆)Cycloalkyl, (C₅-C₆)Cycloalkenyl, Heterocyclyl oder Phenyl als Basisgruppen, auch die Reste (C₁-C₄)Alkyl, (C₁-C₄)Haloalkyl und (C₁-C₄)Alkoxy-(C₁-C₄)alkyl,
(R³)ₙ n Substituenten R¹ bedeutet, wobei R¹, im Fall dass n = 1 ist, oder jeder der Reste R¹ jeweils unabhängig voneinander, im Fall dass n größer als 1 ist, einen Rest aus den Gruppen (R³a) bis (R³n) bedeutet, enthaltend die Reste (R³a) Halogen, Cyano, Azido, SF₅, Isocyanato und Nitro,
   (R³b) Reste der Formel -NR^{a}R^{b}, worin jeder der Reste R^{a} und R^{b} unabhängig voneinander für einen Rest aus der Gruppe, welche die Reste
      (1) Wasserstoff, (C₁-C₈)Alkyl, (C₂-C₈)Alkenyl und (C₂-C₈)Alkinyl,
      (2) Phenyl und Benzyl,
         wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
      (3) (C₁-C₈)Alkanoyl, [(C₂-C₆)Alkenyl]-carbonyl und [(C₂-C₆)Alkinyl]-carbonyl, wobei jeder der letztgenannten drei Reste unsubstituiert oder durch einen oder mehrere Reste aus der Gruppe Halogen, CN, Amino, (C₁-C₆)Alkoxy, (C₁-C₆)Alkylamino, Di[(C₁-C₆)alkyl]-amino, [(C₁-C₆)Alkoxy]-carbonyl, [(C₁-C₆)Alkoxy]-carbonylamino, Mono- und Di-[(C₁-C₆)Alkanoyl]amino und Mono- und Di-[(C₁-C₆)alkylsulfonyl]amino substituiert ist,
      (4) Phenyl-carbonyl und Benzyl-carbonyl, wobei jeder der letztgenannten beiden Reste im Phenylteil unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
      (5) [(C₁-C₈)Alkoxy]-carbonyl, [(C₂-C₆)Alkenyloxy]-carbonyl und [(C₂-C₆)Alkinyloxy]-carbonyl, wobei jeder der letztgenannten drei Reste unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist,
      (6) (C₁-C₄)Alkylsulfinyl und (C₁-C₄)Alkylsulfonyl, wobei jeder der beiden letztgenannten Reste unsubstituiert oder durch einen oder mehrere Reste aus der Gruppe Halogen, CN, (C₁-C₄)Alkoxy und (C₁-C₄)Haloalkoxy substituiert ist,
      enthält, steht
      oder
      worin einer der Reste R^{a} und R^{b} wie oben definiert ist und der andere für Hydroxy oder (C₁-C₆)Alkoxy steht
      oder
      worin R^{a} und R^{b} gemeinsam für eine (C₂-C₅)Alkylen-kette, welche durch ein oder mehrere Heteroatome aus der Gruppe O und S unterbrochen sein kann und welche unsubstituiert oder durch einen oder mehrere (C₁-C₄)Alkylreste substituiert ist, stehen
      oder
      worin R^{a} und R^{b} gemeinsam mit dem N-Atom der Gruppe NR^{a}R^{b} für
      N-Phthalimido stehen,
   (R³c) Reste der Formel -CO₂R,
      worin R für Wasserstoff, (C₁-C₈)Alkyl, (C₂-C₈)Alkenyl oder (C₂-C₈)Alkinyl, wobei jeder der letztgenannten 3 Reste unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist, steht,
   (R³d) Reste der Formeln -CO-NR^{a}R^{b}, -CS-NR^{a}R^{b} und -SO₂-NR^{a}R^{b},
      worin jeder der Reste R^{a} und R^{b} in den letztgenannten 3 Formeln unabhängig voneinander für einen Rest aus der Gruppe, welche die Reste
      (1) Wasserstoff und (C₁-C₈)Alkyl, das unsubstituiert oder durch einen oder mehrere Reste aus der Gruppe Carboxy, [(C₁-C₈)Alkoxy]-carbonyl und CN substituiert ist,
      (2) (C₂-C₈)Alkenyl und (C₂-C₈)Alkinyl,
      (3) Phenyl und Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
         enthält, steht oder
         R^{a} und R^{b} gemeinsam für eine (C₂-C₅)Alkylen-Kette, welche durch ein oder mehrere Heteroatome aus der Gruppe O und S unterbrochen sein kann und welche unsubstituiert oder durch einen oder mehrere (C₁-C₄)Alkylreste substituiert ist, stehen,
   (R³e) Reste der Formel -CO-R,
      worin R für Wasserstoff, (C₁-C₆)Alkyl, (C₁-C₆)Haloalkyl, [(C₁-C₈)Alkoxy]-carbonyl, [(C₁-C₈)Alkoxy]-oxalyl oder (C₃-C₆)Cycloalkyl, wobei der Cycloalkylrest unsubstituiert oder durch einen oder mehrere (C₁-C₄)Alkylreste substituiert ist, steht,
   (R³f) Reste der Formel -C(R^{a})=N-OR^{b}, worin
      R^{a} für Wasserstoff, (C₁-C₆)Alkyl, (C₁-C₆)Haloalkyl oder (C₃-C₆)Cycloalkyl, wobei der Cycloalkylrest unsubstituiert oder durch einen oder mehrere (C₁-C₄)Alkylreste substituiert ist, und
      R^{b} für Wasserstoff, (C₁-C₈)Alkyl, (C₁-C₈)Haloalkyl, (C₂-C₈)Alkenyl, (C₂-C₈)Haloalkenyl oder (C₂-C₈)Alkinyl stehen,
   (R³g) Reste der Formel -C(SR^{a})=NR^{b}, worin
      R^{a} für (C₁-C₆)Alkyl und
      R^{b} für Wasserstoff oder (C₁-C₆)Alkyl stehen,
   (R³h) Reste der Formel -OR, worin
      R für Wasserstoff, (C₁-C₈)Alkyl, (C₂-C₈)Alkenyl oder (C₂-C₈)Alkinyl steht oder
      R für (C₁-C₈)Alkyl, das durch einen oder mehrere Reste aus der Gruppe Halogen, CN, Hydroxy, Carboxy, Amino, (C₁-C₆)Alkoxy, (C₁-C₆)Alkylthio, (C₁-C₆)Alkylsulfinyl, (C₁-C₆)Alkylsulfonyl, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₆)Haloalkoxy, (C₁-C₄)Alkoxy-(C₁-C₄)alkoxy, (C₁-C₄)Alkylamino und Di[(C₁-C₄)alkyl]-amino substituiert ist, steht
      oder
      R für [(C₁-C₆)Alkyl]-carbonyl, [(C₁-C₆)Haloalkyl]-carbonyl steht
      oder
      R für [(C₁-C₈)Alkoxy]-carbonyl, das unsubstituiert oder durch einen oder mehrere Reste aus der Gruppe Halogen, CN, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist, steht
      oder
      R für Mono- oder Di-[(C₁-C₈)alkyl]-aminocarbonyl, Mono- oder Di-[(C₁-C₈)alkanoyl]-aminocarbonyl, Mono- oder Di-[(C₁-C₈)alkylsulfonyl]-aminocarbonyl, N-[(C₁-C₈)Alkyl]-N-[(C₁-C₈)alkanoyl]-aminocarbonyl, N-[(C₁-C₈)Alkyl]-N-[(C₁-C₈)alkylsulfonyl]-aminocarbonyl, N-[(C₁-C₈)Alkanoyl]-N-[(C₁-C₈)alkylsulfonyl]-aminocarbonyl oder für einen Rest der Formel -C(=O)NR'R', worin die R' gemeinsam eine Alkylen-kette mit 2 bis 5 C-Atomen bilden, die noch durch ein Sauerstoff oder Stickstoffatom unterbrochen sein kann, steht
      oder
      R für Mono- oder Di-[(C₁-C₈)alkyl]-aminothiocarbonyl, Mono- oder Di-[(C₁-C₈)alkanoyl]-aminothiocarbonyl, Mono- oder Di-[(C₁-C₈)alkylsulfonyl]-aminothiocarbonyl, N-[(C₁-C₈)Alkyl]-N-[(C₁-C₈)alkanoyl]-aminothiocarbonyl, N-[(C₁-C₈)Alkyl]-N-[(C₁-C₈)alkylsulfonyl]-aminothiocarbonyl, N-[(C₁-C₈)Alkanoyl]-N-[(C₁-C₈)alkylsulfonyl]-aminothiocarbonyl oder für einen Rest der Formel -C(S=)-NR'R', worin die R' gemeinsam eine Alkylen-Kette mit 2 bis 5 C-Atomen, die noch durch ein Sauerstoff oder Stickstoffatom unterbrochen sein kann, bilden, steht
      oder
      R für Phenyl oder Phenyl-(C₁-C₆)alkyl, wobei jeder der letztgenannten beiden Reste im Phenylring unsubstituiert oder durch einen oder mehrere Reste aus der Gruppe Halogen, CN, Hydroxy, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, [(C₁-C₄)Alkoxy]-carbonyl, [(C₁-C₄)Haloalkoxy]-carbonyl, R'R"N-CO-, worin jeder Rest R' und R" unabhängig voneinander H, (C₁-C₄)Alkyl oder (C₁-C₄)Haloalkyl bedeutet, (C₁-C₄)Alkylsulfinyl und (C₁-C₄)Alkylsulfonyl substituiert ist, steht,
   (R³i) Reste der Formel -SR,
      worin R
      für Wasserstoff, (C₁-C₈)Alkyl, (C₂-C₈)Alkenyl oder (C₂-C₈)Alkinyl oder für (C₁-C₈)Alkyl, das durch einen oder mehrere Reste aus der Gruppe Halogen, CN und (C₁-C₄)Alkoxy substituiert ist, steht
      oder
      R für [(C₁-C₆)Alkyl]-carbonyl oder [(C₁-C₆)Haloalkyl]-carbonyl steht
      oder
      R für [(C₁-C₈)Alkoxy]-carbonyl, das unsubstituiert oder durch einen oder mehrere Reste aus der Gruppe Halogen, CN, (C₁-C₄)Alkoxy,
      (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist, steht
      oder
      R für Mono- oder Di-[(C₁-C₈)alkyl]-aminocarbonyl, Mono- oder Di-[(C₁-C₈)alkanoyl]-aminocarbonyl, Mono- oder Di-[(C₁-C₈)alkylsulfonyl]-aminocarbonyl, N-[(C₁-C₈)Alkyl]-N-[(C₁-C₈)alkanoyl]-aminocarbonyl, N-[(C₁-C₈)Alkyl]-N-[(C₁-C₈)alkylsulfonyl]-aminocarbonyl, N-[(C₁-C₈)Alkanoyl]-N-[(C₁-C₈)alkylsulfonyl]-aminocarbonyl oder für einen Rest der Formel -C(=O)NR'R', worin die R' gemeinsam eine Alkylen-kette mit 2 bis 5 C-Atomen bilden, die noch durch ein Sauerstoff oder Stickstoffatom unterbrochen sein kann, steht,
   (R³j) Reste der Formel -S(=O)-R und -S(=O)₂-R,
      wobei in jedem der letztgenannten beiden Formeln R jeweils unabhängig voneinander für (C₁-C₈)Alkyl, (C₂-C₈)Alkenyl oder (C₂-C₈)Alkinyl oder für (C₁-C₈)Alkyl, das durch einen oder mehrere Reste aus der Gruppe Halogen, CN und (C₁-C₄)Alkoxy substituiert ist, steht,
   (R³k) Reste der Formel -O-S(=O)₂-R,
      wobei R für Wasserstoff, (C₁-C₈)Alkyl, (C₂-C₈)Alkenyl
      oder (C₂-C₈)Alkinyl oder für (C₁-C₈)Alkyl, das durch einen oder mehrere Reste aus der Gruppe Halogen, CN und (C₁-C₄)Alkoxy substituiert ist, oder für Phenyl, das durch einen oder mehrere Reste aus der Gruppe Halogen, CN, Nitro, Hydroxy, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Haloalkylsulfinyl, (C₁-C₄)Alkylsulfonyl und (C₁-C₄)Haloalkylsulfonyl substituiert ist, steht,
   (R³L) Reste der Formel -N=C(OR^{a})R^{b},
      wobei jeder der Reste R^{a} und R^{b} unabhängig voneinander für Wasserstoff oder (C₁-C₆)Alkyl stehen,
   (R³m) Reste der Formel -N(OR^{a})COR^{b}
      wobei jeder der Reste R^{a} und R^{b} unabhängig voneinander für Wasserstoff, (C₁-C₆)Alkyl oder Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, stehen,
   (R³n) (C₁-C₈)Alkyl, (C₂-C₈)Alkenyl, (C₂-C₈)Alkinyl, (C₃-C₆)Cycloalkyl, (C₅-C₆)Cycloalkenyl, Heterocyclyl mit 3 bis 6 Ringatomen und einem oder mehreren Heteroringatomen aus der Gruppe N, O und S oder Phenyl,
      wobei jeder der 7 letztgenannten Reste unsubstituiert oder durch einen oder mehrere Reste aus den Gruppen (G³a) bis (G³m) substituiert ist, enthaltend die Reste
      (G³a) Halogen, Cyano, Azido, SF₅, Isocyanato und Nitro,
      (G³b) (C₃-C₆)Cycloalkyl, (C₅-C₆)Cycloalkenyl und einen gesättigten oder ungesättigten nicht-aromatischen heterocyclischen Rest mit 3 bis 6 Ringatomen und 1, 2 oder 3 Heteroringatomen aus der Gruppe N, O und S,
         wobei jeder der letztgenannten 3 Reste unsubstituiert oder durch einen oder mehrere Reste aus der Gruppe Halogen und (C₁-C₃)Alkyl substituiert ist,
      (G³c) Phenyl und einen aromatischen heterocyclischen Rest mit 5 oder 6 Ringatomen und 1, 2 oder 3 Heteroringatomen aus der Gruppe N, O und S,
         wobei jeder der letztgenannten 2 Reste unsubstituiert oder durch einen oder mehrere Reste aus der Gruppe Halogen, Hydroxy, (C₁-C₄)Alkyl, (C₁-C₄)Haloalkyl, (C₁-C₄)Alkoxy, (C₁-C₄)Haloalkoxy, (C₁-C₄)Alkylthio, [(C₁-C₄)Alkoxy]-carbonyl, [(C₁-C₄)Haloalkoxy]-carbonyl, R'R"N-CO-, worin jeder Rest R' und R" unabhängig voneinander H, (C₁-C₄)Alkyl oder (C₁-C₄)Haloalkyl bedeutet oder R' und R" gemeinsam mit dem N-Atom einen gesättigten oder ungesättigten heterocyclischen Ring mit 5 oder 6 Ringatomen, der neben dem N-Atom noch ein N-Atom oder Sauerstoffatom als Heteroatom enthalten kann, bedeuten, (C₁-C₄)Alkylsulfinyl und (C₁-C₄)Alkylsulfonyl substituiert ist,
      (G³d) Reste der Formel -NR^{a}R^{b},
         worin jeder der Reste R^{a} und R^{b} unabhängig voneinander für einen Rest aus der Gruppe, welche die Reste
         (1) Wasserstoff, (C₁-C₈)Alkyl, (C₂-C₈)Alkenyl und (C₂-C₈)Alkinyl,
         (2) Phenyl oder Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
         (3) [(C₁-C₈)Alkanoyl, das unsubstituiert oder durch einen oder mehrere Reste aus der Gruppe Halogen, CN, (C₁-C₆)Alkylamino, Di[(C₁-C₆)alkyl]-amino und (C₁-C₆)Alkoxy substituiert ist,
         (4) Phenyl-carbonyl und Benzyl-carbonyl, wobei jeder der letztgenannten beiden Reste im Phenylteil unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
         (5) [(C₁-C₈)Alkoxy]-carbonyl, das unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist,
         (6) (C₁-C₄)Alkylsulfinyl und (C₁-C₄)Alkylsulfonyl, wobei jeder der beiden letztgenannten Reste unsubstituiert oder durch einen oder mehrere Reste aus der Gruppe Halogen, CN, (C₁-C₄)Alkoxy und (C₁-C₄)Haloalkoxy substituiert ist,
            enthält, steht
            oder
         worin einer der Reste R^{a} und R^{b} wie oben definiert ist und der andere für Hydroxy oder (C₁-C₆)Alkoxy steht
         oder
         worin R^{a} und R^{b} gemeinsam für eine (C₂-C₅)Alkylen-kette, welche durch ein oder mehrere Heteroatome aus der Gruppe O und S unterbrochen sein kann und welche unsubstituiert oder durch einen oder mehrere (C₁-C₄)Alkylreste substituiert ist, stehen
         oder
         worin R^{a} und R^{b} gemeinsam mit dem N-Atom der Gruppe NR^{a}R^{b} für N-Phthalimido stehen,
   (G³e) Reste der Formel -CO₂R,
      worin R für Wasserstoff, (C₁-C₈)Alkyl, (C₂-C₈)Alkenyl oder (C₂-C₈)Alkinyl, wobei jeder der letztgenannten 3 Reste unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist, steht,
   (G³f) Reste der Formeln -CO-NR^{a}R^{b} und -CS-NR^{a}R^{b},
      worin jeder der Reste R^{a} und R^{b} in den letztgenannten beiden Formeln jeweils unabhängig voneinander für einen Rest aus der Gruppe, welche die Reste
      (1) Wasserstoff und (C₁-C₈)Alkyl, das unsubstituiert oder durch einen oder mehrere Reste aus der Gruppe Carboxy, [(C₁-C₈)Alkoxy]-carbonyl und CN substituiert ist,
      (2) (C₂-C₈)Alkenyl und (C₂-C₈)Alkinyl,
      (3) Phenyl und Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
         enthält, steht oder
         R^{a} und R^{b} gemeinsam für eine (C₂-C₅)Alkylen-kette, welche durch ein oder mehrere Heteroatome aus der Gruppe O und S unterbrochen sein kann und welche unsubstituiert oder durch
         einen oder mehrere (C₁-C₄)Alkylreste substituiert ist, stehen,
   (G³g) Reste der Formel -CO-R,
      worin R für Wasserstoff, (C₁-C₆)Alkyl, (C₁-C₆)Haloalkyl oder (C₃-C₆)Cycloalkyl, wobei der Cycloalkylrest unsubstituiert oder durch einen oder mehrere (C₁-C₄)Alkylreste substituiert ist, steht,
   (G³h) Reste der Formel -C(R^{a})=N-OR^{b}, worin
      R^{a} für Wasserstoff, (C₁-C₆)Alkyl, (C₁-C₆)Haloalkyl oder (C₃-C₆)Cycloalkyl, wobei der Cycloalkylrest unsubstituiert oder durch einen oder mehrere (C₁-C₄)Alkylreste substituiert ist, und
      R^{b} für Wasserstoff, (C₁-C₈)Alkyl, (C₁-C₈)Haloalkyl, (C₂-C₈)Alkenyl, (C₂-C₈)Haloalkenyl oder (C₂-C₈)Alkinyl
         stehen,
   (G³i) Reste der Formel -C(SR^{a})=NR^{b}, worin
      R^{a} für (C₁-C₆)Alkyl und
      R^{b} für Wasserstoff oder (C₁-C₆)Alkyl stehen,
   (G³j) Reste der Formel -OR,
      worin R für Wasserstoff, (C₁-C₈)Alkyl, (C₂-C₈)Alkenyl oder (C₂-C₈)Alkinyl steht
      oder
      R für (C₁-C₈)Alkyl, das unsubstituiert oder durch einen oder mehrere Reste aus der Gruppe Halogen, CN, Hydroxy, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist, steht oder
      R für [(C₁-C₈)Alkoxy]-carbonyl, das unsubstituiert oder durch einen oder mehrere Reste aus der Gruppe Halogen, CN, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist, steht oder
      R für Phenyl oder Phenyl-(C₁-C₆)alkyl, wobei jeder der letztgenannten beiden Reste im Phenylring unsubstituiert oder durch einen oder mehrere Reste aus der Gruppe Halogen, CN, Hydroxy, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, [(C₁-C₄)Alkoxy]-carbonyl, [(C₁-C₄)Haloalkoxy]-carbonyl, R'R"N-CO-, worin jeder Rest R' und R" unabhängig voneinander H, (C₁-C₄)Alkyl oder (C₁-C₄)Haloalkyl bedeutet oder R' und R" gemeinsam mit dem N-Atom einen gesättigten oder ungesättigten heterocyclischen Ring mit 5 oder 6 Ringatomen, der neben dem N-Atom noch ein N-Atom oder Sauerstoffatom als Heteroatom enthalten kann, bedeuten, (C₁-C₄)Alkylsulfinyl und (C₁-C₄)Alkylsulfonyl substituiert ist, steht
      oder
      R für Si[(C₁-C₄)alkyl]₃ steht,
   (G³k) Reste der Formel -SR, -S(=O)-R, -O-S(=O)-R, -S(=O)₂-R und -O-S(=O)₂-R,
      wobei in jedem der letztgenannten 5 Formeln R jeweils unabhängig voneinander von den anderen Resten R für Wasserstoff, (C₁-C₈)Alkyl, (C₂-C₈)Alkenyl oder (C₂-C₈)Alkinyl oder für (C₁-C₈)Alkyl, das durch einen oder mehrere Reste aus der Gruppe Halogen, CN und (C₁-C₄)Alkoxy substituiert ist, steht,
   (G3L) Reste der Formeln -Si[(C₁-C₄)alkyl]₃, -S⁺[(C₁-C₄)alkyl]₂, und -N⁺[(C₁-C₄)alkyl]₃, wobei in den Fällen der kationischen Reste ein Anionäquivalent als Gegenion vorliegt,
   (G³m) und, nur für den Fall R³ = (C₃-C₆)Cycloalkyl, (C₅-C₆)Cycloalkenyl, Heterocyclyl oder Phenyl als Basisgruppe, auch die Reste (C₁-C₄)Alkyl, (C₁-C₄)Haloalkyl und (C₁-C₄)Alkoxy-(C₁-C₄)alkyl,
   oder
   zwei benachbarte Reste R³ gemeinsam eine (C₂-C₇)Alkylen- oder (C₃-C₇)Alkenylengruppe, welche durch 1, 2 oder 3 Heteroatome aus der Gruppe O, S und N unterbrochen sein kann und welche unsubstituiert oder durch einen oder mehrere Reste aus der Gruppe Halogen, CN, (C₁-C₄)Alkyl, (C₁-C₄)Haloalkyl und (C₁-C₄)Alkoxy substituiert ist, bedeuten,
n eine ganze Zahl von 0 bis 5 bedeutet,
R⁴ H, (C₁-C₄)Alkyl, (C₁-C₄)Haloalkyl, (C₃-C₆)Cycloalkyl, das unsubstituiert oder durch einen oder mehrere Reste aus der Gruppe Halogen und (C₁-C₃)Alkyl substituiert ist, bedeutet und
R⁵ H, (C₁-C₄)Alkyl, (C₁-C₄)Haloalkyl, (C₃-C₆)Cycloalkyl, das unsubstituiert oder durch einen oder mehrere Reste aus der Gruppe Halogen und (C₁-C₃)Alkyl substituiert ist, bedeutet oder
R⁴ und R⁵ gemeinsam mit dem sie verbindenden C-Atom einen 3- bis 6-gliedrigen Ring, der carbocyclisch ist oder heterocyclisch mit 1 oder 2 Heteroringatomen aus der Gruppe N, O und S ist, wobei der Ring unsubstituiert oder substituiert ist, vorzugsweise unsubstituiert oder durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist, bedeuten,
R⁶ H, (C₁-C₆)Alkyl, (C₂-C₆)Alkenyl oder (C₂-C₆)Alkinyl,
   wobei jeder der letztgenannten 3 Reste unsubstituiert oder durch einen oder mehrere Reste aus der Gruppe, welche die Reste
   (1) Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
   (2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloaikanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder
      R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
   (3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
   (4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthlo, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkylaminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
   wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
   substituiert ist,
   enthält, substituiert ist, bedeutet
   oder
R⁶ Phenyl, Pyridyl, Phenoxy, (C₃-C₆)Cycloalkyl, (C₅-C₆)Cycloalkenyl oder einen gesättigten heterocyclischen Rest mit 3 bis 6 Ringatomen und 1 oder 2 Heteroringatomen aus der Gruppe N, O und S,
   wobei jeder der letztgenannten 6 Reste unsubstituiert oder durch einen oder mehrere Reste aus der Gruppe, welche die Reste
   (1) (C₁-C₆)Alkyl, (C₂-C₆)Alkenyl, (C₂-C₆)Haloalkenyl, (C₂-C₆)Alkinyl, (C₂-C₆)Haloalkinyl, Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
   (2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder
      R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, 0 und S enthalten kann und der unsubstitutiert oder durch einen oder mehrere Reste aus der Gruppe (C₁-C₃)Alkyl und Oxo substituiert ist.
   (3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
   (4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkylaminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
      wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
   substituiert ist,
   enthält, substituiert ist, bedeutet,
R⁷ H, (C₁-C₆)Alkyl, (C₂-C₆)Alkenyl, (C₂-C₆)Alkinyl,
   wobei jeder der letztgenannten 3 Reste unsubstituiert oder durch einen oder mehrere Reste aus der Gruppe, welche die Reste
   (1) Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
   (2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder
      R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
   (3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
   (4) Phenyl, Pyridyl und Phenoxy, wobei jeder der letztgenannten 3 Reste unsubstituiert oder durch einen oder mehrere Reste aus der Gruppe
      Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkytsulfonyl, Aminocarbonyl, Mono-(C₁-C₃)alkyl-aminocarbonyl, Di(C₁-C₃)alkyl-aminocarbonyl, Mono-(C₁-C₃)haloalkylaminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
      wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
   substituiert ist,
   enthält, substituiert ist, bedeutet
   oder
R⁷ Phenyl, Pyridyl, Phenoxy, (C₃-C₆)Cycioalkyl, (C₅-C₆)Cycloalkenyl oder einen gesättigten heterocyclischen Rest mit 3 bis 6 Ringatomen und 1 oder 2 Heteroringatomen aus der Gruppe N, O und S,
   wobei jeder der letztgenannten 6 Reste unsubstituiert oder durch einen oder mehrere Reste aus der Gruppe, welche die Reste
   (1) (C₁-C₆)Alkyl, (C₂-C₆)Alkenyl, (C₂-C₆)Haloalkenyl, (C₂-C₆)Alkinyl, (C₂-C₆)Haloalkinyl, Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloaikyisulfonyl,
   (2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloaikanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder
      R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
   (3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
   (4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkylaminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
      wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
   substituiert ist,
   enthält, substituiert ist, bedeutet
   oder
R⁷ Formyl oder einen Rest der Formel CO-R, CO-OR, SO-R oder S(O)₂-R bedeutet,
   worin R jeweils für (C₁-C₆)Alkyl, (C₂-C₆)Alkenyl, (C₂-C₆)Alkinyl,
   wobei jeder der letztgenannten 3 Reste unsubstituiert oder durch einen oder mehrere Reste aus der Gruppe, welche die Reste
   (1) Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloaikanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
   (2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder
      R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
   (3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
   (4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkyl-aminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
      wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
   substituiert ist,
   enthält, substituiert ist, steht oder
   worin R für Phenyl, Pyridyl, Phenoxy, (C₃-C₆)Cycloalkyl, (C₅-C₆)Cycloalkenyl oder einen gesättigten heterocyclischen Rest mit 3 bis 6 Ringatomen und 1 oder 2 Heteroringatomen aus der Gruppe N, O und S,
   wobei jeder der letztgenannten 6 Reste unsubstituiert oder durch einen oder mehrere Reste aus der Gruppe, welche die Reste
   (1) (C₁-C₆)Alkyl, (C₂-C₆)Alkenyl, (C₂-C₆)Haloalkenyl, (C₂-C₆)Alkinyl, (C₂-C₆)Haloalkinyl, Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
   (2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloaikanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
   (3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
   (4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkylaminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
      wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
   substituiert ist,
   enthält, substituiert ist, steht,
   oder
R⁷ einen Rest der Formel CO-NR*R** bedeutet,
   worin jeder der Reste R* und R** unabhängig voneinander für Wasserstoff, (C₁-C₆)Alkyl, (C₂-C₆)Alkenyl oder (C₂-C₆)Alkinyl,
   wobei jeder der letztgenannten 3 Reste unsubstituiert oder durch einen oder mehrere Reste aus der Gruppe, welche die Reste
   (1) Halogen, Cyano, Nitro, OH, SH, (C₁-C₃)Alkoxy, (C₁-C₃)Alkylthio, (C₁-C₃)Haloalkylthlo, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
   (2) Reste der Formel CO-NR^{a}R^{b}, worin jeder der Reste R^{a} und R^{b} unabhängig voneinander für Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
   (3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
   (4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkylaminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
      wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
   substituiert ist,
   enthält, substituiert ist, oder
   für Phenyl, Pyridyl, Phenoxy, (C₃-C₆)Cycloalkyl, (C₅-C₆)Cycloalkenyl oder einen gesättigten heterocyclischen Rest mit 3 bis 6 Ringatomen und 1 oder 2 Heteroringatomen aus der Gruppe N, O und S,
   wobei jeder der letztgenannten 6 Reste unsubstituiert oder durch einen oder mehrere Reste aus der Gruppe, welche die Reste
   (1) (C₁-C₆)Alkyl, (C₂-C₆)Alkenyl, (C₂-C₆)Haloalkenyl, (C₂-C₆)Alkinyl, (C₂-C₆)Haloalkinyl, Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
   (2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, 0 und S enthalten kann und der unsubstitutiert oder durch einen oder mehrere Reste aus der Gruppe (C₁-C₃)Alkyl und Oxo substituiert ist,
   (3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
   (4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthlo, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkylaminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
      wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
   substituiert ist,
   enthält, substituiert ist, steht,
   oder worin R* und R** gemeinsam mit dem sie verbindenden N-Atom einen gesättigten oder teilweise ungesättigten (nicht aromatischen) heterocyclischen
   Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bilden, und
R⁸ und R⁹ jeweils unabhängig voneinander Reste aus den Gruppen (i), (ii), (iii), (iv), (v), (vi) und (vii) bedeuten, wobei die Gruppen (i) bis (vii) die Reste
   (i) Wasserstoff, Halogen, Cyano, Carboxy, Formyloxy, Nitro, Hydroxy und Thio,
   (ii) (C₁-C₆)Alkyl, (C₂-C₆)Alkenyl und (C₂-C₆)Alkinyl,
      wobei jeder der letztgenannten 3 Reste unsubstituiert oder durch einen oder mehrere Reste aus der Gruppe, welche die Reste
      (1) Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
      (2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl oder (C₁-C₃)Alkylsulfonyl, (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
      (3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
      (4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkyl-aminocarbonyl, Di-[(C₁-C₃)haloalkyl]-aminocarbonyl, N-(C₁-C₃)haloalkyl-N-(C₁-C₃)alkyl-aminocarbonyl und Reste der Formel CO-NR'₂,
         wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
      substituiert ist,
      enthält, substituiert ist,
   (iii) Phenyl, Pyridyl, Phenoxy, (C₃-C₆)Cycloalkyl, (C₅-C₆)Cycloalkenyl oder einen gesättigten heterocyclischen Rest mit 3 bis 6 Ringatomen und 1 oder 2 Heteroringatomen aus der Gruppe N, O und S,
      wobei jeder der letztgenannten 6 Reste unsubstituiert oder durch einen oder mehrere Reste aus der Gruppe, welche die Reste
      (1) (C₁-C₆)Alkyl, (C₂-C₆)Alkenyl, (C₂-C₆)Haloalkenyl, (C₂-C₆)Alkinyl, (C₂-C₆)Haloalkinyl, Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
      (2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
      (3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
      (4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkyl-aminocarbonyl, Di-[(C₁-C₃)haloalkyl]-aminocarbonyl, N-(C₁-C₃)haloalkyl-N-(C₁-C₃)alkyl-aminocarbonyl und Reste der Formel CO-NR'₂,
         wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
      substituiert ist, enthält, substituiert ist,
   (iv) (C₁-C₆)Alkoxy, (C₁-C₆)Alklthio und (C₂-C₆)Alkenyloxy, wobei jeder der letztgenannten beiden Reste unsubstituiert oder durch einen oder mehrere Reste aus der Gruppe Halogen, CN, OH, SH, (C₁-C₃)Alkoxy, (C₁-C₃)Haloalkoxy und (C₃-C₆)Cycloalkoxy, das unsubstituiert oder durch einen oder mehrere Reste aus der Gruppe Halogen und (C₁-C₃)Alkyl substituiert ist, substituiert ist,
   (v) (C₃-C₆)Cycloalkoxy und (C₅-C₆)Cycloalkenyloxy, wobei jeder der letztgenannten beiden Reste unsubstituiert oder durch einen oder mehrere Reste aus der Gruppe Halogen, CN, OH, SH, (C₁-C₃)Alkyl, (C₁-C₃)Haloalkyl, (C₁-C₃)Alkoxy und (C₁-C₃)Haloalkoxy substituiert ist,
   (vi) Reste der Formeln -CO-OR, -SO-R, -S(O)₂-R, -O-CO-OR, -O-SO-R und -O-S(O)₂-R,
      wobei jedes R in den letztgenannten 6 Formeln unabhängig von den anderen Resten R jeweils für (C₁-C₆)Alkyl, (C₂-C₆)Alkenyl oder
      (C₂-C₆)Alkinyl,
      wobei jeder der letztgenannten 3 Reste unsubstituiert oder durch einen oder mehrere Reste aus der Gruppe, welche die Reste
      (1) Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
      (2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
      (3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
      (4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkyl-aminocarbonyl, Di-[(C₁-C₃)haloalkyl]-aminocarbonyl, N-(C₁-C₃)haloalkyl-N-(C₁-C₃)alkyl-aminocarbonyl und Reste der Formel CO-NR'₂,
         wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
      substituiert ist,
      enthält, substituiert ist, steht
      oder
      worin jedes R unabhängig von den anderen Resten R jeweils für Phenyl, Pyridyl, Phenoxy, (C₃-C₆)Cycloalkyl, (C₅-C₆)Cycloalkenyl oder einen gesättigten heterocyclischen Rest mit 3 bis 6 Ringatomen und 1 oder 2 Heteroringatomen aus der Gruppe N, O und S,
      wobei jeder der letztgenannten 6 Reste unsubstituiert oder durch einen oder mehrere Reste aus der Gruppe, welche die Reste
      (1) (C₁-C₆)Alkyl, (C₂-C₆)Alkenyl, (C₂-C₆)Haloalkenyl, (C₂-C₆)Alkinyl, (C₂-C₆)Haloalkinyl, Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
      (2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloaikanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist bedeuten,
      (3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
      (4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkyl-aminocarbonyl, Di-[(C₁-C₃)haloalkyl]-aminocarbonyl, N-(C₁-C₃)haloalkyl-N-(C₁-C₃)alkyl-aminocarbonyl und Reste der Formel CO-NR'₂,
         wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
      substituiert ist, enthält, substituiert ist, steht,
   (vii) Reste der Formeln -CO-NR*R**, -O-CO-NR*R**, -O-CO-NR*R** und O-CO-NR*R**,
      wobei in den letztgenannten 4 Formeln jeder der Reste R* und R** unabhängig voneinander für
      Wasserstoff, (C₁-C₆)Alkyl, (C₂-C₆)Alkenyl oder (C₂-C₆)Alkinyl,
      wobei jeder der letztgenannten 3 Reste unsubstituiert oder durch einen oder mehrere Reste aus der Gruppe, welche die Reste
      (1) Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
      (2) Reste der Formel CO-NR^{a}R^{b}, worin jeder der Reste R^{a} und R^{b} unabhängig voneinander für Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
      (3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
      (4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkyl-aminocarbonyl, Di-[(C₁-C₃)haloalkyl]-aminocarbonyl, N-(C₁-C₃)haloalkyl-N-(C₁-C₃)alkyl-aminocarbonyl und Reste der Formel CO-NR'₂,
         wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
      substituiert ist,
      enthält, substituiert ist, oder
      für Phenyl, Pyridyl, Phenoxy, (C₃-C₆)Cycloalkyl, (C₅-C₆)Cycloalkenyl oder einen gesättigten heterocyclischen Rest mit 3 bis 6 Ringatomen und 1 oder 2 Heteroringatomen aus der Gruppe N, O und S,
      wobei jeder der letztgenannten 6 Reste unsubstituiert oder durch einen oder mehrere Reste aus der Gruppe, welche die Reste
      (1) (C₁-C₆)Alkyl, (C₂-C₆)Alkenyl, (C₂-C₆)Haloalkenyl, (C₂-C₆)Alkinyl, (C₂-C₆)Haloalkinyl, Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
      (2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
      (3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
      (4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkyl-aminocarbonyl, Di-[(C₁-C₃)haloalkyl]-aminocarbonyl, N-(C₁-C₃)haloalkyl-N-(C₁-C₃)alkyl-aminocarbonyl und Reste der Formel CO-NR'₂,
         wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
      substituiert ist,
      enthält, substituiert ist, steht,
      oder worin R* und R** gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bilden,
      enthalten, oder
      R⁸ und R⁹ bedeuten gemeinsam eine (C₂-C₅)Alkylen-Kette, welche durch ein oder mehrere Heteroatome aus der Gruppe N, O und S unterbrochen sein kann und welche unsubstituiert oder durch einen oder mehrere Reste aus der Gruppe Halogen und (C₁-C₄)Alkyl substituiert ist.

Wenn die Verbindungen durch Wasserstoffverschiebung Tautomere bilden können, welche strukturell formal nicht durch die Formel (I) erfasst würden, so sind diese Tautomere gleichwohl von der Definition der erfindungsgemäßen Verbindungen der Formel (I) umfasst, sofern nicht ein bestimmtes Tautomer allein Gegenstand der jeweiligen Betrachtung ist.
So können beispielsweise viele Carbonylverbindungen sowohl in der Ketoform wie auch in der Enolform vorliegen, wobei beide Formen durch die Formel (I) umfasst werden.

Die Verbindungen der allgemeinen Formel (I) können je nach Art und Verknüpfung der Substituenten als Stereoisomere vorliegen. Die durch ihre spezifische Raumform definierten möglichen Stereoisomeren, wie Enantiomere, Diastereomere, Z- und E-Isomere, geometrische Isomere und Atropisomere und deren Gemische sind alle von der Formel (I) umfaßt.
Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome (= asymmetrisch substituierte Kohlenstoffatome) vorhanden und/oder asymmetrische Schwefelatome in Form von Sulfoxiden, die in zwei enantiomeren Formen existieren können, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfasst werden, jedoch nicht mit ihrer spezifischen Stereoform angegeben sind, und deren Gemische.

Die Verbindungen der Formel (I) können Salze bilden. Salzbildung kann durch Einwirkung einer Base auf solche Verbindungen der Formel (I) erfolgen, die ein acides Wasserstoffatom tragen, z.B. im Falle daß R¹, R² oder R³ eine COOH-Gruppe oder eine Sulfonamid-Gruppe -NHSO₂- enthält. Geeignete Basen sind beispielsweise organische Amine, wie Trialkylamine, Morpholin, Piperidin oder Pyridin sowie Ammonium-, Alkali- oder Erdalkalimetallhydroxide, -carbonate und -hydrogencarbonate, insbesondere Natrium- und Kaliumhydroxid, -carbonat und -hydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R"R''']⁺, worin R bis R''' jeweils unabhängig voneinander einen organischen Rest, insbesondere Alkyl, Aryl, Aralkyl oder Alkylaryl darstellen.

Die Verbindungen der Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, wie Ameisensäure, Essigsäure, Propionsäure, Oxalsäure, Milchsäure, Salicylsäure oder Sulfonsäuren, wie zum Beispiel p-Toluolsulfonsäure, an eine basische Gruppe, wie z.B. Amino, Alkylamino, Dialkylamino, Piperidino-, Morpholino- oder Pyridino, Salze bilden. Diese Salze enthalten dann die konjugierte Base der Säure als Anion.

Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden.

Im Folgenden werden die Verbindungen der Formel (I) und ihre Salze auch kurz als erfindungsgemäß verwendete oder erfindungsgemäße "Verbindungen (I)" bezeichnet.

Die vorstehend und weiter unten verwendeten Bezeichnungen sind dem Fachmann geläufig und haben insbesondere die im Folgenden erläuterten Bedeutungen: Der Ausdruck "(C₁-C₄)Alkyl" bedeutet eine Kurzschreibweise für offenkettiges Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)Alkyl", umfassen entsprechend auch gradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen, insbesondere mit 1 bis 4 C-Atomen bzw. bei ungesättigten Gruppen mit 2 bis 6 C-Atomen, insbesondere mit 2 bis 4 C-Atomen bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, wobei mindestens eine Doppelbindung bzw. Dreifachbindung enthalten ist. Bevorzugt sind Reste mit einer Doppelbindung bzw. Dreifachbindung. Alkenyl schließt insbesondere auch geradkettige oder verweigte Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl. Alkinyl schließt insbesondere auch geradkettige oder verweigte Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl. Alkenyl bedeutet z.B. Vinyl, welches ggf. durch weitere Alkylreste substituiert sein kann, z.B. 1-Propen-1-yl, 1-Buten-1-yl, Allyl, 1-Methyl-2-propen-1-yl, 2-Methyl-2-propen-1-yl, 2-Buten-1-yl, 1-Methyl-3-butenyl und 1-Methyl-2-butenyl, 2-Methylprop-1-en-1-yl, 1-Methylprop-1-en-1-yl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl, Pentenyl, 2-Methylpentenyl oder Hexenyl, (C₂-C₆)-Alkinyl bedeutet beispielsweise Ethinyl, Propargyl, 1-Methyl-2-propinyl, 2-Butinyl, 2-Pentinyl oder 2-Hexinyl, vorzugsweise Propargyl, But-2-in-1-yl, But-3-in-1-yl oder 1-Methyl-but-3-in-1-yl.

Alkyliden, z. B. in der Form (C₁-C₁₀)Alkyliden, bedeutet den Rest eines geradkettigen oder verzweigten Alkans, der über eine Zweifachbindung gebunden ist. Als Bindungsstelle für Alkyliden kommen naturgemäß nur Positionen am Grundkörper in Frage, an denen zwei H-Atome durch die Doppelbindung des Alkylidens ersetzt werden können; Reste sind z. B. =CH₂, =CH-CH₃, =C(CH₃)-CH₃, =C(CH₃)-C₂H₅ oder =C(C₂H₅)-C₂H₅.

Cycloalkyl bedeutet ein carbocyclisches, gesättigtes Ringsystem mit vorzugsweise 3 bis 8 C-Atomen, vorzugsweise 3 bis 6 C-Atomen, z.B. Cyclopropyl, Cyclobutyl, Cyclopentyl oder Cyclohexyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind.
Im Falle von gegebenenfalls substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Bicyclo[2.2.1]hept-2-yl (Norbornyl), Adamantan-1-yl und Adamantan-2-yl.
Im Falle von gegebenenfalls substituiertem Cycloalkyl werden auch spirocyclische aliphatische Systeme umfaßt, wie beispielsweise Spiro[2.2]pent-1-yl, Spiro[2.3]hex-1-yl, Spiro[2.3]hex-4-yl, 3-Spiro[2.3]hex-5-yl.

Cycloalkenyl bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl. Im Falle von gegebenefalls substituiertem Cycloalkenyl gelten die Erläuterungen für gegebenenfalls substituiertes Cycloalkyl entsprechend.

Die Bezeichnung "Halogen" bedeutet beispielsweise Fluor, Chlor, Brom oder Iod. Haloalkyl, -alkenyl und -alkinyl bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie CH₂CH₂Cl, CH₂CH₂F, CHClCH₃, CHFCH₃, CH₂Cl, CH₂F; Perhaloalkyl wie CCl₃ oder CF₃ oder CF₂CF₃; Polyhaloalkyl wie CHF₂, CH₂F, CH₂CHFCl, CHCl₂, CF₂CF₂H, CH₂CF₃,; Haloalkoxy ist z.B. OCF₃, OCHF₂, OCH₂F, OCF₂CF₃, OCH₂CF₃ und OCH₂CH₂Cl; Entsprechendes gilt für Haloalkenyl und andere durch Halogen substituierte Reste.

Aryl bedeutet ein mono-, bi- oder polycyclisches aromatisches System mit vorzugsweise 6 bis 14, insbesondere 6 bis 12 C-Atomen, beispielsweise Phenyl, Naphthyl, Anthryl, Phenanthrenyl, und ähnliches, vorzugsweise Phenyl.

Im Falle gegebenenfalls substituiertes Aryl sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist Aryl in der Regel auch von dem Begriff "gegebenenfalls substituiertes Phenyl" umfasst.

Ein heterocyclischer Rest (Heterocyclyl) enthält mindestens einen heterocyclischen Ring, der gesättigt, ungesättigt oder heteroaromatisch ist und dabei unsubstituiert oder substituiert sein kann.

Ist der Heterocyclylrest oder heterocyclische Ring gegebenenfalls substituiert, kann er mit anderen carbocyclischen oder heterocyclischen Ringen annelliert sein. Im Falle von gegebenenfalls substituiertem Heterocyclyl werden auch mehrcyclische Systeme umfaßt, wie beispielsweise 8-Aza-bicyclo[3.2.1]octanyl oder 1-Azabicyclo[2.2.1]heptyl.
Im Falle von gegebenenfalls substituiertem Heterocyclyl werden auch spirocyclische Systeme umfaßt, wie beispielsweise 1-Oxa-5-aza-spiro[2.3]hexyl.

Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen.

"Heteroaryl" bedeutet von den vorstehend unter "Heterocyclyl" definierten Systemen jeweils eine heteroaromatische Verbindung, d. h. eine vollständig ungesättigte aromatische heterocyclische Verbindung.

Beispiele für "Heteroaryl" mit Heteroringatomen aus der Gruppe N, O und S sind:

Beispiele für "Heteroaryl" mit Heteroringatomen aus der Gruppe N, O und S sind vorzugsweise:
(Die Nomenklatur und Nummerierung der Bindungspositionen erfolgt in der Regel in Übereinstimmung mit der IUPAC-Nomenklatur.)

Mit einem Heteroatom:
Aromatische Fünfring-Heterocyclenreste wie beispielsweise Pyrrol-2-yl, Pyrrol-1-yl, Furan-2-yl, Thien-2-yl, Pyrrol-3-yl, Furan-3-yl, Thien-3-yl; aromatische Sechsring-Heterocyclenreste wie Pyridin-2-yl, Pyridin-3-yl, Pyridin-4-yl,

Mit zwei N-Atomen:
Aromatische Fünfring-Heterocyclenreste wie beispielsweise Imidazol-1-yl, Imidazol-2-yl, Imidazol-4-yl, Imidazol-5-yl, Pyrazol-1-yl, Pyrazol-3-yl, Pyrazol-4-yl, Pyrazol-5-yl; aromatische Sechsring-Heterocyclenreste wie Pyrazin-2-yl, Pyrazin-3-yl, Pyrimidin-2-yl, Pyrimidin-4-yl, Pyrimidin-5-yl, Pyridazin-3-yl, Pyridazin-4-yl;

Mit einem N- und einem O-Atom
Aromatische Fünfring-Heterocyclen wie beispielsweise
Isoxazol-3-yl, Isoxazol-4-yl, Isoxazol-5-yl, Oxazol-2-yl, Oxazol-4-yl, Oxazol-5-yl,

Mit einem N- und einem S-Atom:
Aromatische Fünfring-Heterocyclen wie beispielsweise Isothiazol-3-yl, Isothiazol-4-yl, Isothiazol-5-yl, Thiazol-2-yl, Thiazol-4-yl, Thiazol-5-yl

Mit drei N-Atomen:
Aromatische Fünfring-Heterocyclen wie beispielsweise 1,2,3-Triazol-1-yl, 1,2,3-Triazol-4-yl, 1,2,3-Triazol-5-yl, 1,2,5-Triazol-1-yl, 1,2,5-Triazol-3-yl, 1,3,4-triazol-1-yl, 1,3,4-triazol-2-yl, 1,2,4-triazol-3-yl, 1,2,4-triazol-5-yl,
aromatische Sechsring-Heterocyclenreste wie 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl,

Mit zwei N-Atomen und einem O-Atom:
Aromatische Fünfring-Heterocyclen wie beispielsweise 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl,

Mit zwei N-Atomen und einem S-Atom:
Aromatische Fünfring-Heterocyclen wie beispielsweise 1,2,4-Thiadiazol-3-yl, 1,2,4-Thiadiazol-5-yl, 1,3,4-Thiadiazol-2-yl, 1,2,3-Thiadiazol-4-yl, 1,2,3-Thiadiazol-5-yl, 1,2,5-Thiadiazol-3-yl,

Mit vier N-Atomen:
Aromatische Fünfring-Heterocyclen wie beispielsweise 1,2,3,4-Tetrazol-1-yl, 1,2,3,4-Tetrazol-5-yl, 1,2,3,5-Tetrazol-1-yl, 1,2,3,5-Tetrazol-4-yl, aromatische Sechsring-Heterocyclenreste wie 1,2,4,5-Tetrazin-3-yl;

Mit drei N-Atomen und einem O- oder S-Atom:
Aromatische Fünfring-Heterocyclen wie beispielsweise 1,2,3,4-Oxotriazol-5-yl, 1,2,3,5-Oxotriazol-4-yl, 1,2,3,5-Thiatriazol-4-yl, aromatische Sechsring-Heterocyclenreste wie beispielsweise 1,2,4,6-Thiatriazin-1-yl, 1,2,4,6-Thiatriazin-3-yl, 1,2,4,6-Thiatriazin-5-yl;

Beispiele für Heteroaryl sind auch 5- oder 6-gliedrige benzokondensierte Ringe aus der Gruppe
Benzo[b]furanyl, Dibenzofuranyl,
Benzo[c]furanyl, Benzo[b]thiophenyl, Benzo[c]thiophenyl, Dibenzothiophenyl, Indolyl, Isoindolyl, Carbazolyl,
Benzoxazolyl, Benzisoxazolyl, Benzothiazolyl, Benzisothiazolyl, Benzimidazolyl, Benzopyrazolyl,
Benzothiadiazolyl, Benzotriazolyl,
1,2,3-Benzotriazin-4-yl, 1,2,3-Benzotriazin-5-yl, 1,2,3-Benzotriazin-6-yl, 1,2,3-Benzotriazin-7-yl, 1,2,3-Benzotriazin-8-yl;
1,2,4-Benzotriazin-3-yl, 1,2,4-Benzotriazin-5-yl, 1,2,4-Benzotriazin-6-yl, 1,2,4-Benzotriazin-7-yl, 1,2,4-Benzotriazin-8-yl;
1,2,3,4-Benzotetrazin-5-yl, 1,2,3,4-Benzotetrazin-6-yl, 1,2,3,4-Benzotetrazin-7-yl, 1,2,3,4-Benzotetrazin-8-yl;
Chinolinyle, wie Chinolin-2-yl, Chinolin-3-yl, Chinolin-4-yl, Chinolin-5-yl, Chinolin-6-yl, Chinolin-7-yl, Chinolin-8-yl;
Isochinolinyle, wie Isochinolin-1-yl, Isochinolin-3-yl, Isochinolin-4-yl, Isochinolin-5-yl, Isochinolin-6-yl, Isochinolin-7-yl, Isochinolin-8-yl; Chinoxalin-2-yl,
Chinoxalinyle, wie Chinoxalin-3-yl,
Chinoxalin-5-yl, Chinoxalin-6-yl, Chinoxalin-7-yl, Chinoxalin-8-yl;
Chinazolinyle, wie Chinazolin-2-yl, Chinazolin-4-yl, Chinazolin-5-yl, Chinazolin-6-yl, Chinazolin-7-yl, Chinazolin-8-yl;
Cinnollnyle, wie Cinnolin-3-yl, Cinnolin-4-yl, Cinnolin-5-yl, Cinnolin-6-yl, Cinnolin-7-yl, Cinnolin-8-yl;
Naphthyridinyle, wie 1,5-Naphthyridin-2-yl, 1,5-Naphthyridin-3-yl, 1,5-Naphthyridin-4-yl, 1,5-Naphthyridin-6-yl, 1,5-Naphthyridin-7-yl, 1,5-Naphthyridin-8-yl; 1,6-Naphthyridin-2-yl, 1,6-Naphthyridin-3-yl, 1,6-Naphthyridin-4-yl, 1,6-Naphthyridin-5-yl, 1,6-Naphthyridin-7-yl, 1,6-Naphthyridin-8-yl; 1,7-Naphthyridin-2-yl, 1,7-Naphthyridin-3-yl, 1,7-Naphthyridin-4-yl, 1,7-Naphthyridin-5-yl, 1,7-Naphthyridin-6-yl, 1,7-Naphthyridin-8-yl; 1,8-Naphthyridin-2-yl, 1,8-Naphthyridin-3-yl, 1,8-Naphthyridin-4-yl, 1,8-Naphthyridin-5-yl, 1,8-Naphthyridin-6-yl, 1,8-Naphthyridin-7-yl; 2,6-Naphthyridin-1-yl, 2,6-Naphthyridin-3-yl, 2,6-Naphthyridin-4-yl, 2,6-Naphthyridin-5-yl, 2,6-Naphthyridin-7-yl, 2,6-Naphthyridin-8-yl; 2,7-Naphthyridin-1-yl, 2,7-Naphthyridin-3-yl, 2,7-Naphthyridin-4-yl, 2,7-Naphthyridin-5-yl, 2,7-Naphthyridin-6-yl, 2,7-Naphthyridin-8-yl;
Phthalazinyle, wie Phthalazin-1-yl, Phthalazin-4-yl, Phthalazin-5-yl, Phthalazin-6-yl, Phthalazin-7-yl, Phthalazin-8-yl;
Pyridopyrazinyle, wie Pyrido[2,3-b]pyrazin-2-yl, Pyrido[2,3-b]pyrazin-3-yl, Pyrido[2,3-b]pyrazin-6-yl, Pyrido[2,3-b]pyrazin-7-yl, Pyrido[2,3-b]pyrazin-8-yl; Pyrido[3,4-b]pyrazin-2-yl, Pyrido[3,4-b]pyrazin-3-yl, Pyrido[3,4-b]pyrazin-5-yl, Pyrido[3,4-b]pyrazin-7-yl, Pyrido[3,4-b]pyrazin-8-yl;
Pyridopyrimidinyle, wie Pyrido[3,2-d]pyrimidin-2-yl, Pyrido[3,2-d]pyrimidin-4-yl, Pyrido[3,2-d]pyrimidin-6-yl, Pyrido[3,2-d]pyrimidin-7-yl, Pyrido[3,2-d]pyrimidin-8-yl; Pyrido[4,3-d]pyrimidin-2-yl, Pyrido[4,3-d]pyrimidin-4-yl, Pyrido[4,3-d]pyrimidin-5-yl, Pyrido[4,3-d]pyrimidin-7-yl, Pyrido[4,3-d]pyrimidin-8-yl; Pyrido[3,4-d]pyrimidin-2-yl, Pyrido[3,4-d]pyrimidin-4-yl, Pyrido[3,4-d]pyrimidin-5-yl, Pyrido[3,4-d]pyrimidin-6-yl, Pyrido[3,4-d]pyrimidin-8-yl; Pyrido[2,3-d]pyrimidin-2-yl, Pyrido[2,3-d]pyrimidin-4-yl, Pyrido[2,3-d]pyrimidin-5-yl, Pyrido[2,3-d]pyrimidin-6-yl, Pyrido[2,3-d]pyrimidin-7-yl;
Pyridopyridazinyle, wie Pyrido[3,2-c]pyridazin-3-yl, Pyrido[3,2-c]pyridazin-4-yl, Pyrido[3,2-c]pyridazin-6-yl, Pyrido[3,2-c]pyridazin-7-yl, Pyrido[3,2-c]pyridazin-8-yl;
Pyrido[4,3-c]pyridazin-3-yl, Pyrido[4,3-c]pyridazin-4-yl, Pyrido[4,3-c]pyridazin-5-yl, Pyrido[4,3-c]pyridazin-7-yl, Pyrido[4,3-c]pyridazin-8-yl; Pyrido[3,4-c]pyridazin-3-yl, Pyrido[3,4-c]pyridazin-4-yl, Pyrido[3,4-c]pyridazin-5-yl, Pyrido[3,4-c]pyridazin-6-yl, Pyrido[3,4-c]pyridazin-8-yl; Pyrido[2,3-c]pyridazin-3-yl, Pyrido[2,3-c]pyridazin-4-yl, Pyrido[2,3-c]pyridazin-5-yl, Pyrido[2,3-c]pyridazin-6-yl, Pyrido[2,3-c]pyridazin-7-yl;
Pyrido[2,3-d]pyridazin-2-yl, Pyrido[2,3-d]pyridazin-3-yl, Pyrido[2,3-d]pyridazin-4-yl, Pyrido[2,3-d]pyridazin-5-yl, Pyrido[2,3-d]pyridazin-8-yl; Pyrido[3,4-d]pyridazin-1-yl, Pyrido[3,4-d]pyridazin-4-yl, Pyrido[3,4-d]pyridazin-5-yl, Pyrido[3,4-d]pyridazin-7-yl, Pyrido[3,4-d]pyridazin-8-yl;
Pyrazinopyrazinyle, wie Pyrazino[2,3-b]pyrazin-2-yl, Pyrazino[2,3-b]pyrazin-3-yl, Pyrazino[2,3-b]pyrazin-6-yl, Pyrazino[2,3-b]pyrazin-7-yl;
Pteridinyle, wie Pteridin-2-yl, Pteridin-4-yl, Pteridin-6-yl, Pteridin-7-yl;
Pyrazinopyridazinyle, wie Pyrazino[2,3-c]pyridazin-3-yl, Pyrazino[2,3-c]pyridazin-4-yl, Pyrazino[2,3-c]pyridazin-6-yl, Pyrazino[2,3-c]pyridazin-7-yl; Pyrazino[2,3-d]pyridazin-2-yl, Pyrazino[2,3-d]pyridazin-3-yl, Pyrazino[2,3-d]pyridazin-5-yl, Pyrazino[2,3-d]pyridazin-6-yl;
Pyrimidopyrimidinyle, wie Pyrimido[4,5-d]pyrimidin-2-yl, Pyrimido[4,5-d]pyrimidin-4-yl, Pyrimido[4,5-d]pyrimidin-5-yl, Pyrimido[4,5-d]pyrimidin-7-yl; Pyrimido[5,4-d]pyrimidin-2-yl, Pyrimido[5,4-d]pyrimidin-4-yl, Pyrimido[5,4-d]pyrimidin-6-yl, Pyrimido[5,4-d]pyrimidin-8-yl; Pyrimido[5,4-c]pyridazin-3-yl, Pyrimido[5,4-c]pyridazin-4-yl, Pyrimido[5,4-c]pyridazin-6-yl, Pyrimido[5,4-c]pyridazin-8-yl; Pyrimido[4,5-c]pyridazin-3-yl, Pyrimido[4,5-c]pyridazin-4-yl, Pyrimido[4,5-c]pyridazin-5-yl, Pyrimido[4,5-c]pyridazin-7-yl; Pyrimido[4,5-d]pyridazin-2-yl, Pyrimido[4,5-d]pyridazin-4-yl, Pyrimido[4,5-d]pyridazin-5-yl, Pyrimido[4,5-d]pyridazin-8-yl;
Pyridazinopyridazin-3-yle, wie Pyridazino[3,4-c]pyridazin-3-yl, Pyridazino[3,4-c]pyridazin-4-yl, Pyridazino[3,4-c]pyridazin-5-yl, Pyridazino[3,4-c]pyridazin-6-yl; Pyridazino[4,5-c]pyridazin-3-yl, Pyridazino[4,5-c]pyridazin-4-yl, Pyridazino[4,5-c]pyridazin-5-yl, Pyridazino[4,5-c]pyridazin-8-yl; Pyridazino[4,5-d]pyridazin-1-yl, Pyridazino[4,5-d]pyridazin-4-yl, Pyridazino[4,5-d]pyridazin-5-yl, Pyridazino[4,5-d]pyridazin-8-yl; Pyridazino[4,3-c]pyridazin-3-yl, Pyridazino[4,3-c]pyridazin-4-yl, Pyridazino[4,3-c]pyridazin-7-yl, Pyridazino[4,3-c]pyridazin-8-yl;
Pyridotriazinyle, wie Pyrido[3,2-e][1,2,4]triazin-3-yl, Pyrido[3,2-e][1,2,4]triazin-5-yl, Pyrido[3,2-e][1,2,4]triazin-6-yl, Pyrido[3,2-e][1,2,4]triazin-7-yl; Pyrido[4,3-e][1,2,4]triazin-3-yl, Pyrido[4,3-e][1,2,4]triazin-5-yl, Pyrido[4,3-e][1,2,4]triazin-6-yl, Pyrido[4,3-e][1,2,4]triazin-8-yl; Pyrido[3,4-e][1,2,4]triazin-3-yl, ; Pyrido[3,4-e][1,2,4]triazin-5-yl, ; Pyrido[3,4-e][1,2,4]triazin-7-yl, Pyrido[3,4-e][1,2,4]triazin-8-yl;
Pyrido[2,3-e][1,2,4]triazin-3-yl, Pyrido[2,3-e][1,2,4]triazin-6-yl, Pyrido[2,3-e][1,2,4]triazin-7-yl, Pyrido[2,3-e][1,2,4]triazin-8-yl; Pyrido[2,3-d][1,2,3]triazin-4-yl, Pyrido[2,3-d][1,2,3]triazin-5-yl, Pyrido[2,3-d][1,2,3]triazin-6-yl, Pyrido[2,3-d][1,2,3]triazin-7-yl; Pyrido[3,4-d][1,2,3]triazin-4-yl, Pyrido[3,4-d][1,2,3]triazin-5-yl, Pyrido[3,4-d][1,2,3]triazin-6-yl, Pyrido[3,4-d][1,2,3]triazin-8-yl; Pyrido[4,3-d][1,2,3]triazin-4-yl, Pyrido[4,3-d][1,2,3]triazin-5-yl, Pyrido[4,3-d][1,2,3]triazin-7-yl, Pyrido[4,3-d][1,2,3]triazin-8-yl; Pyrido[3,2-d][1,2,3]triazin-4-yl, Pyrido[3,2-d][1,2,3]triazin-6-yl, Pyrido[3,2-d][1,2,3]triazin-7-yl, Pyrido[3,2-d][1,2,3]triazin-8-yl;
Pyrazino[2,3-e][1,2,4]triazin-3-yl, Pyrazino[2,3-e][1,2,4]triazin-6-yl, Pyrazino[2,3-e][1,2,4]triazin-7-yl; Pyrazino[2,3-d][1,2,3]triazin-4-yl, Pyrazino[2,3-d][1,2,3]triazin-6-yl, Pyrazino[2,3-d][1,2,3]triazin-7-yl; Pyrimido[5,4-e][1,2,4]triazin-3-yl, Pyrimido[5,4-e][1,2,4]triazin-5-yl, Pyrimido[5,4-e][1,2,4]triazin-7-yl; Pyrimido[4,5-e][1,2,4]triazin-3-yl, Pyrimido[4,5-e][1,2,4]triazin-6-yl, Pyrimido[4,5-e][1,2,4]triazin-8-yl; Pyrimido[4,5-d][1,2,3]triazin-4-yl, Pyrimido[4,5-d][1,2,3]triazin-5-yl, Pyrimido[4,5-d][1,2,3]triazin-7-yl; Pyrimido[5,4-d][1,2,3]triazin-4-yl, Pyrimido[5,4-d][1,2,3]triazin-6-yl, Pyrimido[5,4-d][1,2,3]triazin-8-yl; Pyridazino[4,3-e][1,2,4]triazin-3-yl, Pyridazino[4,3-e][1,2,4]triazin-5-yl, Pyridazino[4,3-e][1,2,4]triazin-6-yl; Pyridazino[4,5-e][1,2,4]triazin-3-yl,
Pyridazino[4,5-e][1,2,4]triazin-5-yl, Pyridazino[4,5-e][1,2,4]triazin-8-yl; Pyridazino[3,4-e][1,2,4]triazin-3-yl, Pyridazino[3,4-e][1,2,4]triazin-7-yl, Pyridazino[3,4-e][1,2,4]triazin-8-yl; Pyridazino[3,4-d][1,2,3]triazin-4-yl, Pyridazino[3,4-d][1,2,3]triazin-5-yl,
Pyridazino[3,4-d][1,2,3]triazin-6-yl; Pyridazino[4,5-d][1,2,3]triazin-4-yl, Pyridazino[4,5-d][1,2,3]triazin-5-yl, Pyridazino[4,5-d][1,2,3]triazin-8-yl; Pyridazino[4,3-d][1,2,3]triazin-4-yl, Pyridazino[4,3-d][1,2,3]triazin-7-yl, Pyridazino[4,3-d][1,2,3]triazin-8-yl;
[1,2,4]Triazino[5,6-e][1,2,4]triazin-3-yl, [1,2,4]Triazino[5,6-e][1,2,4]triazin-6-yl;
[1,2,4]Triazino[6,5-e][1,2,4]triazin-3-yl, [1,2,4]Triazino[6,5-e][1,2,4]triazin-7-yl;
[1,2,4]Triazino[6,5-d][1,2,3]triazin-4-yl, [1,2,4]Triazino[6,5-d][1,2,3]triazin-6-yl;
[1,2,4]Triazino[5,6-d][1,2,3]triazin-4-yl, [1,2,4]Triazino[5,6-d][1,2,3]triazin-7-yl;
[1,2,3]Triazino[4,5-d][1,2,3]triazin-4-yl, [1,2,3]Triazino[4,5-d][1,2,3]triazin-5-yl;
[1,2,3]Triazino[5,4-d][1,2,3]triazin-4-yl, [1,2,3]Triazino[5,4-d][1,2,3]triazin-8-yl;
Pyridotetrazinyle, wie Pyrido[2,3-e][1,2,3,4]tetrazin-6-yl, Pyrido[2,3-e][1,2,3,4]tetrazin-7-yl, Pyrido[2,3-e][1,2,3,4]tetrazin-8-yl; Pyrido[3,4-e][1,2,3,4]tetrazin-5-yl, Pyrido[3,4-e][1,2,3,4]tetrazin-7-yl, Pyrido[3,4-e][1,2,3,4]tetrazin-8-yl; Pyrazino[2,3-e][1,2,3,4]tetrazin-6-yl, Pyrazino[2,3-e][1,2,3,4]tetrazin-7-yl; Pyrimido[4,5-e][1,2,3,4]tetrazin-6-yl, Pyrimido[4,5-e][1,2,3,4]tetrazin-8-yl; Pyridazino[3,4-e][1,2,3,4]tetrazin-7-yl, Pyridazino[3,4-e][1,2,3,4]tetrazin-8-yl; Pyridazino[4,5-e][1,2,3,4]tetrazin-5-yl, Pyridazino[4,5-e][1,2,3,4]tetrazin-8-yl; [1,2,4]Triazino[5,6-e][1,2,3,4]tetrazin-7-yl; [1,2,3]Triazino[4,5-e][1,2,3,4]tetrazin-8-yl;
Phenazinyl, Phenanthrolinyl, Anthyridinyl, Triazaphenanthrenyl

Heterocyclyl umfasst neben den genannten heteroaromatischen Resten auch gesättigte oder ungesättigte, insbesondere gesättigte Heterocyclylreste mit 3 bis 7, insbesondere 3 bis 6 Ringatomen.
Heterocyclyl enthält vorzugsweise Heteroringatome aus der Gruppe N, O und S.

Beispiele für "Heterocyclyl" sind ein partiell oder vollständig hydrierter heterocyclischer Rest mit einem Heteroatom aus der Gruppe N, O und S, beispielsweise Oxiranyl, 2- oder 3-Oxetanyl, 2- oder 3-Oxolanyl (= 2- oder 3-Tetrahydrofuranyl), 2- oder 3- oder 4-Oxanyl (= 2- oder 3- oder 4-Tetrahydropyranyl), 2- oder 3-Pyrrolinyl, 2- oder 3-Pyrrolidinyl oder auch 2- oder 3- oder 4-Piperidinyl. [

Weitere Beispiele für "Heterocyclyl" sind ein partiell oder vollständig hydrierter heterocyclischer Rest mit 2 Heteroatomen aus der Gruppe N, O und S, beispielsweise Imidazolinyl, Oxazolinyl, Thiazolinyl, Piperazinyl, 1,3-Dioxolanyl, 1,3- und 1,4-Dioxanyl, 1,3- und 1,4-Dithianyl, Oxazolinyl, Isoxazolinyl, Oxazolidinyl, Isoxazolidinyl, Thiazolidinyl und Morpholinyl.

Handelt es sich es sich um einen teilweise oder vollständig gesättigten StickstoffHeterocyclus, so kann dieser sowohl über Kohlenstoff als auch über den Stickstoff mit dem Rest des Moleküls verknüpft sein.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Als Substituenten für einen substituierten heterocyclischen Rest kommen die weiter unten genannten Substituenten in Frage, zusätzlich auch Oxo und Thioxo.
Die Oxogruppe als Substituent an einem Ring-C-Atom bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen -N(O)-, -S(O)- (auch kurz SO) und -S(O)₂- (auch kurz SO₂) im heterocyclischen Ring. Im Fall von -N(O)- und - S(O)-Gruppen sind jeweils beide Enantiomere umfasst.

Andere Substituenten als die Oxogruppe können an einem heterocyclischen Ring auch an einem Heteroatom gebunden sein, beispielsweise an einem Stickstoffatom, wenn dabei ein Wasserstoffatom am Stickstoffatom des Grundkörpers ersetzt wird. Im Falle des Stickstoffatoms und auch anderer Heteroatome wie z. B. des Schwefelatoms, kommt auch eine weitere Substitution unter Bildung von quartären Ammoniumverbindungen oder Sulfoniumverbindungen in Frage.

Als Substituenten für einen substituierten heterocyclischen Rest kommen die weiter unten genannten Substituenten in Frage, zusätzlich auch Oxo und Thioxo.
Die Oxogruppe als Substituent an einem Ring-C-Atom bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen -N(O)-, -S(O)- (auch kurz SO) und -S(O)₂- (auch kurz SO₂) im heterocyclischen Ring. Im Fall von -N(O)- und -S(O)-Gruppen sind jeweils beide Enantiomere umfasst.

Andere Substituenten als die Oxogruppe können an einem heterocyclischen Ring auch an einem Heteroatom gebunden sein, beispielsweise an einem Stickstoffatom, wenn dabei ein Wasserstoffatom am Stickstoffatom des Grundkörpers ersetzt wird. Im Falle des Stickstoffatoms und auch anderer Heteroatome wie z. B. des Schwefelatoms, kommt auch eine weitere Substitution unter Bildung von quartären Ammoniumverbindungen oder Sulfoniumverbindungen in Frage.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Substituierte Reste, wie ein substituierter Alkyl-, Alkenyl-, Alkinyl-, Cycloalkyl-, Cycloalkenyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, Trialkylsilyl und gegebenenfalls substituiertes Cycloalkyl, gegebenenfalls substituiertes Aryl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, wobei beide Enantiomere der Alkylsulfonylgruppe umfasst sind, Alkylsulfonyl Alkylphosphinyl, Alkylphosphonyl und, im Falle cyclischer Reste (= "cyclischer Grundkörper"), auch Alkyl, Haloalkyl, Alkylthio-alkyl, Alkoxy-alkyl, gegebenfalls substituiertes Mono- und Dialkyl-aminoalkyl und Hydroxy-alkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden oder einer Oxogruppe, Iminogruppe oder substituierten Iminogruppe substituiert sind.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bevorzugte Substituenten für die Substituentenebenen sind beispielsweise Amino, Hydroxy, Halogen, Nitro, Cyano, Isocyano, Mercapto, Isothiocyanato, Carboxy, Carbonamid, SF₅, Aminosulfonyl, Alkyl, Cycloalkyl, Alkenyl, Cycloalkenyl, Alkinyl, Monoalkyl-amino, Dialkyl-amino, N-Alkanoyl-amino, Alkoxy, Alkenyloxy, Alkinyloxy, Cycloalkoxy, Cycloalkenyloxy, Alkoxy-carbonyl, Alkenyloxy-carbonyl, Alkinyloxy-carbonyl, Aryloxycarbonyl, Alkanoyl, Alkenyl-carbonyl, Alkinyl-carbonyl, Aryl-carbonyl, Alkylthio, Cycloalkylthio, Alkenylthio, Cycloalkenylthio, Alkinylthio, Alkylsulfenyl, Alkylsulfinyl, wobei beide Enantiomere der Alkylsulfinylgruppe umfasst sind, Alkylsulfonyl, Monoalkyl-aminosulfonyl, Dialkyl-aminosulfonyl, Alkylphosphinyl, Alkylphosphonyl, wobei für Alkylphosphinyl bzw. Alkylphosphonyl beide Enantiomere umfasst sind, N-Alkyl-aminocarbonyl, N,N-Dialkyl-aminocarbonyl, N-Alkanoyl-aminocarbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl und Trialkylsilyl.

Substituenten, die aus mehreren Substituentenebenen zusammengesetzt sind, sind bevorzugt Haloalkoxyalkoxy, Haloalkoxyalkylthio, Haloalkoxyalkanoyl, Haloalkoxyalkyl .

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Mono- und Dialkyl-amino, Mono- und Diarylamino, Acylamino, N-Alkyl-N-arylamino, N-Alkyl-N-acylamino sowie gesättigte N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino. Substituiertes Amino schließt auch quartäre Ammoniumverbindungen (Salze) mit vier organischen Substituenten am Stickstoffatom ein.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy, (C₁-C₄)Alkylthio, (C₁-C₄)Halogenalkylthio, Cyano und Nitro substituiert ist, z.B. o-, m- und p-Tolyl, Dimethylphenyle, 2-, 3- und 4-Chlorphenyl, 2-, 3- und 4-Fluorphenyl, 2-, 3- und 4-Trifluormethyl- und -Trichlormethylphenyl, 2,4-, 3,5-, 2,5- und 2,3-Dichlorphenyl, o-, m- und p-Methoxyphenyl.

Gegebenenfalls substituiertes Cycloalkyl ist vorzugsweise Cycloalkyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Halogenalkyl und (C₁-C₄)Halogenalkoxy substituiert ist, insbesondere durch einen oder zwei (C₁-C₄)Alkylreste substituiert ist.

Gegebenenfalls substituiertes Heterocyclyl ist vorzugsweise Heterocyclyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Halogenalkyl, (C₁-C₄)Halogenalkoxy, Nitro und Oxo substituiert ist, insbesondere ein- oder mehrfach durch Reste aus der Gruppe Halogen, (C₁-C₄)Alkyl, (C₁-C₄)Alkoxy, (C₁-C₄)Halogenalkyl und Oxo, ganz besonders durch einen oder zwei (C₁-C₄)Alkylreste substituiert ist.

Ein "organischer Säurerest", wie Acyl, bedeutet einen Rest einer Oxosäure oder Thiosäure der allgemeinen Formel

R*ₖ*E(=Q)*ₚ*(OH)(QR')*ₙ*

wobei
R ein organischer Rest ist,
E ein Atom aus der Gruppe C, S, P bedeutet,
Q unabhängig voneinander ein Atom oder ein Molekülfragment aus der Gruppe O, S, NR' bedeutet und
R' unabhängig voneinander ein Wasserstoffatom, Alkyl, Haloalkyl, Alkoxyalkyl oder Aryl bedeutet,
k, p natürliche Zahlen sind,
n eine natürliche Zahl oder Null ist.

Der organische Säurerest entspricht der nachstehenden Formel und entsteht formal durch Abtrennen einer Hydroxygruppe an der Säurefunktion der Oxosäure, wobei der organische Rest R in der Säure auch über ein oder mehrere Heteroatome mit der Säurefunktion verbunden sein kann:

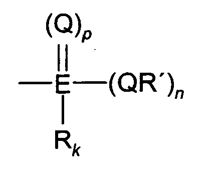

Für Oxosäuren des Kohlenstoffs ist dies im IUPAC Compendium of Chemical Terminology (1997) beschrieben.

Beispiele für organische Säurereste, die von den Oxosäuren bzw. Thiosäuren des Schwefels abgeleitet sind (E=S), sind S(O)OCH₃, SO₂OH, SO₂OCH₃ oder SO₂NHR (N-substituierte Sulfonamidsäuren).

Im Falle von *k*=1 sind auch Alkylsulfonyl- und Alkylsulfinyl-Reste, wie z.B. (H₃C)S(O)₂, (F₃C)S(O)₂, p-TolylS(O)₂, (H₃C)S(O)(NH-n-C₄H₉), (C₆H₅)S(S)(O) oder (C₆H₅)S(O) mit umfasst.

Beispiele für organische Säurereste, die von den Oxosäuren bzw. Thiosäuren des Phosphors abgeleitet sind (E=P), sind von der Phosphinsäure und der Phosphonsäure abgeleitete Reste, wobei diese Reste weiter verestert sein können, z.B. -PO(OCH₃)₂, (C₂H₅O)P(O)OH, (C₂H₅O)P(O)(SC₆H₅), (H₃CO)P(O)NH(C₆H₅) oder -PO(NMe₂)₂.

Im Falle von *k*=1 sind auch Alkylphosphinyl- und Alkylphosphonyl-Reste, wie z.B. (H₃C)₂ P(O), (C₆H₅)₂P(O), (H₃C)(C₆H₅)P(O); (H₃C)P(O)OCH₃, (H₅C₂)P(O)(OC₂H₅). (C₆H₅)P(O)(OC₂H₅), (C₂H₅)P(O)(SC₆H₅), (H₃C)P(O)NH(C₆H₅), (H₃C)P(S)(NH-i-C₃H₇), (C₆H₅)P(S)(OC₂H₅) oder (C₆H₅)P(S)(SC₂H₅) mit umfasst.

Organische Säurereste, die von den Oxosäuren des Kohlenstoffs abgeleitet sind (E=C, Q=O), werden im engeren Sinne auch mit dem Begriff "Acyl" bezeichnet.

Beispiele für Acyl sind der Rest -CO-R einer Carbonsäure HO-CO-R und Reste davon abgeleiteter Säuren oder der Rest von Kohlensäuremonoestern oder N-substituierter Carbaminsäuren sowie Carbonate und deren Ester.

Acyl bedeutet beispielsweise Formyl, Oxalyl(ester), Alkylcarbonyl wie [(C₁-C₄)Alkyl]-carbonyl, Haloalkylcarbonyl, Phenylcarbonyl, Alkyloxycarbonyl, speziell tert.-Butyloxycarbonyl, Phenyloxycarbonyl, Benzyloxycarbonyl, Fluorenyloxycarbonyl, N-Alkyl-1-iminoalkyl, N-Alkyl- und N,N-Dialkylcarbamoyl. Dabei können die Reste jeweils im Alkyl- oder Phenylteil noch weiter substituiert sein, beispielsweise im Alkylteil durch ein oder mehrere Reste aus der Gruppe Halogen, Cyano, Alkoxy, Phenyl und Phenoxy; Beispiele für Substituenten im Phenylteil sind die bereits weiter oben allgemein für substituiertes Phenyl erwähnten Substituenten.

Acyl bedeutet vorzugsweise einen Acylrest im engeren Sinne, d. h. einen Rest einer organischen Säure, bei der die Säuregruppe direkt mit dem C-Atom eines organischen Restes verbunden ist, beispielsweise Alkanoyl, wie Formyl und Acetyl, Aroyl wie Phenylcarbonyl, und andere Reste von gesättigten oder ungesättigten organischen Säuren.

"Aroyl" bedeutet einen wie vorstehend definierter Arylrest, der über eine CarbonylGruppe gebunden ist, z.B. die Benzoyl-Gruppe.

Wenn ein allgemeiner Rest als "Wasserstoff" definiert ist, bedeutet dies ein Wasserstoffatom.

Mit "yl-Position" eines Restes ist dessen Bindungstelle bezeichnet.

Der "Grundkörper" oder "Basiskörper" eines substituierten Restes bezeichnet den ansonsten unsubstituierten Rest, d. h. der Rest weist neben der Bindungsstelle des Restes keine weitere Substitution auf. Der Grundkörper eines Restes wie Alkyl, Alkenyl, Phenyl, Aryl, Heteroaryl enthält jeweils das Kohlenstoffgerüst bzw. das Kohlenstoffgerüst mit den Heteroatomen, wobei die Zahl der übrigen Bindungen am Gerüst gemäß dem jeweils definierten Sättigungsgrad bestimmt ist und die Bindungen mit Wasserstoffatomen besetzt sind. Im substituierten Grundkörper sind einzelne Wasserstoffatome durch andere Reste als Wasserstoff besetzt.

Vor allem aus den Gründen der höheren herbiziden Wirkung, besseren Selektivität und/oder besseren Herstellbarkeit sind erfindungsgemäße Verbindungen der genannten Formel (I) oder deren Salze bzw. deren erfindungsgemäße Verwendung von besonderem Interesse, worin einzelne Reste eine der bereits genannten oder im folgenden genannten bevorzugten Bedeutungen haben, oder insbesondere solche, worin eine oder mehrere der bereits genannten oder im Folgenden genannten bevorzugten Bedeutungen kombiniert auftreten.

Unabhängig von den jeweils anderen Resten aus der Gruppe Q, A, R³, R⁴ und R⁵ und der den allgemeinen Resten entsprechenden Unterbedeutungen, und vorzugsweise in Kombination mit bevorzugten Bedeutungen von einem oder mehreren dieser Reste, sind erfindungsgemäße Verbindungen bzw. erfindungsgemäße Verwendungen von Verbindungen mit nachfolgend aufgeführten bevorzugten Bedeutungen der betreffenden Reste von besonderem Interesse.

A bedeutet vorzugweise eine Gruppe der genannten Formel (A1), (A2), (A3) und (A4), insbesondere (A1), (A2) und (A3), weiter bevorzugt (A1), wobei die Reste R⁶, R⁷, R⁸ und R⁹ vorzugsweise die weiter unten genannten bevorzugten Bedeutungen haben.

Q bedeutet vorzugsweise einen Phenylrest oder einen heteroaromatischen Rest mit 6 Ringatomen, der 1, 2, 3 oder 4 Stickstoffatome, vorzugsweise 1 oder 2 Stickstoffatome, aufweist, oder einen heteroaromatischen Rest mit 5 Ringatomen, der 1, 2 oder 3 Stickstoffatome oder 1 Schwefelatom oder 1 Schwefelatom und 1 Stickstoffatom als Heteroringatome aufweist und wobei der Phenylrest oder der heteroaromatische Rest an einer oder mehreren CH-Gruppen des Rings unsubstituiert oder mit einem Rest R¹ substituiert ist (CH wird zu CR¹), wobei die gegebenenfalls vorhandene Substitution der Gruppe Q durch Reste R¹ abgekürzt durch die Formel (R¹)ₘ definiert ist und der Rest oder die m Reste R¹ am Ring unabhängig voneinander wie nachstehend definiert sind, und wobei der heteroaromatische Rest mit 5-Ringatomen, wenn er eine NH-Gruppe enthält, an der NH-Gruppe unsubstituiert oder mit einem Rest R² substituiert ist (NH wird zu NR²),
wobei R¹, m und Rest R² die genannten Bedeutungen oder die im Folgenden bevorzugt genannten Bedeutungen haben. m bedeutet eine ganze Zahl von 0 bis zur Anzahl der CH-Gruppen im Ring des Grundkörpers des Restes der Formel Q, vorzugsweise 0 oder wenn möglich 1 oder 2 oder 3, insbesondere 0 oder, wenn möglich, 1 oder 2.
Der Fall m = 0 bedeutet dabei keine Substitution des Grundkörpers, d. h. der Grundkörper weist im Fall m = 0 an den Positionen, welche für R¹ möglich sind, jeweils eine CH-Gruppe auf.

Der heteroaromatische Rest Q kann im Falle eines Fünfrings, der ein gesättigtes N-Atom im Ring enthält, mit diesem N-Atom am Rest A gebunden sein. Vorzugsweise ist der Rest Q mit einem C-Atom des jeweiligen Rings am Rest A gebunden.

Q bedeutet weiter bevorzugt einen Rest der Formeln (Q1) bis (Q12): 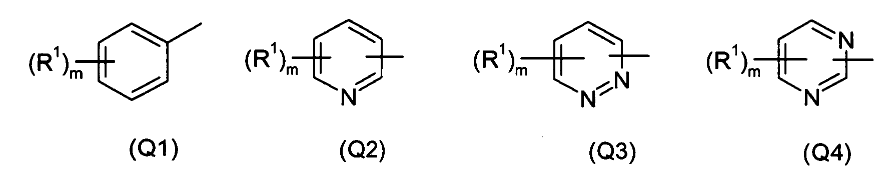 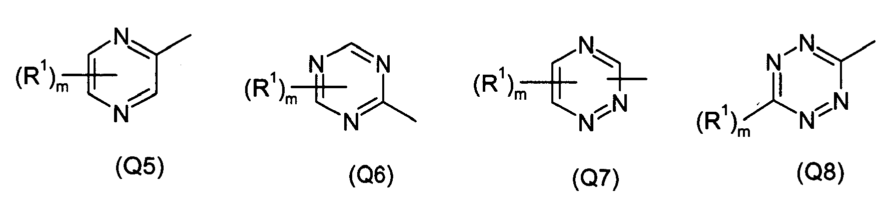 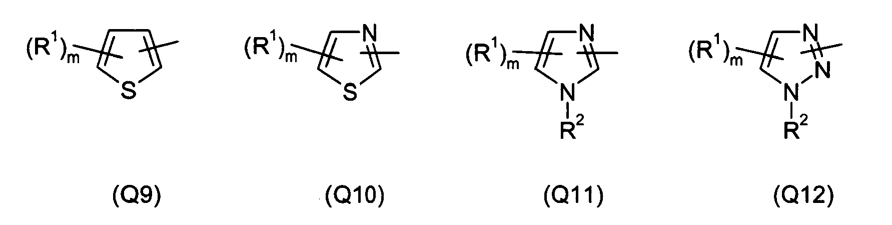 worin
(R¹)ₘ die im Zusammenhang mit Formel (I) genannte Bedeutung, insbesondere die weiter unten genannte bevorzugte Bedeutung hat.

Im Fall Q = (Q1) bedeutet m = 0, 1, 2, 3, 4 oder 5, vorzugsweise 0, 1, 2, 3 oder 4, weiter bevorzugt, 0, 1, 2 oder 3, insbesondere 0, 1 oder 2.

Im Fall Q = (Q2) bedeutet m = 0, 1, 2, 3 oder 4, vorzugsweise 0, 1, 2 oder 3, weiter bevorzugt 0, 1 oder 2, insbesondere 0 oder 1

Im Fall Q = (Q3), (Q4), (Q5) oder (Q9) bedeutet m = 0, 1, 2 oder 3, vorzugsweise 0, 1 oder 2, weiter bevorzugt 0 oder 1, insbesondere 0.

Im Fall Q = (Q6), (Q7), (Q10) oder (Q11) bedeutet m = 0, 1 oder 2, vorzugsweise 0 oder 1, insbesondere 0.

Im Fall Q = (Q8) oder (Q12) bedeutet m = 0 oder 1, vorzugsweise 0.

Der Fall m = 0 bedeutet dabei jeweils keine Substitution des Grundkörpers, d. h. der Grundkörper weist im Fall m = 0 an den Positionen, welche für R¹ möglich sind, jeweils eine CH-Gruppe auf.

n bedeutet entsprechend 0, 1, 2, 3, 4 oder 5, vorzugsweise 0, 1, 2, 3 oder 4, weiter bevorzugt, 0, 1, 2 oder 3, insbesondere 0, 1 oder 2. Der Fall n = 0 bedeutet keine Substitution des Grundkörpers, d. h. der Grundkörper weist im Fall n = 0 an den Positionen, welche für R³ möglich sind, jeweils eine CH-Gruppe auf.

Weiter bevorzugt bedeutet Q den Rest (Q1), worin (R¹)ₘ die genannte Bedeutung hat, vorzugsweise die weiter unten genannte bevorzugte Bedeutung hat.
Weiter bevorzugt bedeutet Q auch den Rest (Q2), insbesondere einen 2-oder 3-Pyridylrest, worin (R¹)ₘ die genannte Bedeutung hat, vorzugsweise die weiter unten genannte bevorzugte Bedeutung hat.
Weiter bevorzugt bedeutet Q auch den Rest (Q5), worin (R¹)ₘ die genannte Bedeutung hat, vorzugsweise die weiter unten genannte bevorzugte Bedeutung hat.

Weiter bevorzugt sind Verbindungen der Formel (I) oder deren Salze, worin
(R¹)ₘ m Substituenten R¹ bedeutet, wobei R¹, im Fall dass m = 1 ist, oder jeder der Reste R¹ jeweils unabhängig voneinander, im Fall dass m größer als 1 ist, vorzugweise einen Rest aus den Gruppen (R¹a) bis (R¹j) bedeutet, enthaltend die Reste
(R¹a) Halogen, Cyano, Isocyanato und Nitro, vorzugsweise Halogen, wie
Fluor, Chlor, Brom und Iod, und Cyano, insbesondere Fluor und Chlor, (R¹b) Reste der Formel -NR^{a}R^{b},
   worin jeder der Reste R^{a} und R^{b} unabhängig voneinander für einen Rest aus der Gruppe, welche die Reste
   (1) Wasserstoff, (C₁-C₆)Alkyl, (C₂-C₆)Alkenyl und (C₂-C₆)Alkinyl, vorzugsweise (C₁-C₃)Alkyl,
   (2) Phenyl und Benzyl,
      wobei jeder der letztgenannten beiden Reste im Ring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy und (C₁-C₄)Haloalkoxy, vorzugsweise durch Reste aus der Gruppe (C₁-C₄)Alkyl, (C₁-C₄)Haloalkyl und (C₁-C₄)Alkoxy substituiert ist,
   (3) [(C₁-C₆)Alkanoyl und [(C₁-C₆)Haloalkanoyl,
   (4) [(C₁-C₆)Alkoxy]-carbonyl und [(C₁-C₆)Haloalkyloxy]-carbonyl,
   (5) (C₁-C₄)Alkylsulfinyl, (C₁-C₄)Haloalkylsulfinyl, (C₁-C₄)Alkylsulfonyl und (C₁-C₄)Haloalkylsulfonyl,
   enthält, steht
   oder
   worin einer der Reste R^{a} und R^{b} wie oben definiert ist und der andere für Hydroxy oder (C₁-C₆)Alkoxy steht
   oder
   worin R^{a} und R^{b} gemeinsam für eine (C₂-C₅)Alkylen-kette, welche durch ein Heteroatom aus der Gruppe 0 und S unterbrochen sein kann und welche unsubstituiert oder durch einen oder mehrere (C₁-C₄)Alkylreste substituiert ist, stehen,
(R¹c) Reste der Formel -CO₂R,
   worin R für Wasserstoff, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, (C₁-C₄)Alkoxy und Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist, substituiert ist, vorzugsweise R für (C₁-C₃)Alkyl, (C₁-C₃)Haloalkyl oder Benzyl, insbesondere für Methyl oder Ethyl steht,
(R¹d) Reste der Formeln -CO-NR^{a}R^{b} und -CS-NR^{a}R^{b},
   worin jeder der Reste R^{a} und R^{b} in den letztgenannten beiden Formeln unabhängig voneinander für einen Rest aus der Gruppe, welche
   (1) Wasserstoff und (C₁-C₄)Alkyl, das unsubstituiert oder durch einen oder mehrere Reste aus der Gruppe (C₁-C₆)Alkoxy]-carbonyl und CN substituiert ist, vorzugsweise Wasserstoff oder (C₁-C₄)Alkyl,
   (2) (C₂-C₆)Alkenyl und (C₂-C₆)Alkinyl, vorzugsweise (C₃-C₆)Alkenyl und (C₃-C₆)Alkinyl, insbesondere Allyl oder Propargyl,
   (3) Phenyl und Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl, vorzugsweise aus der Gruppe Halogen, (C₁-C₄)Alkyl, (C₁-C₄)Haloalkyl und (C₁-C₄)Alkoxy substituiert ist,
      enthält, steht oder
      worin R^{a} und R^{b} gemeinsam für eine (C₂-C₅)Alkylen-kette, welche durch ein oder mehrere Heteroatome aus der Gruppe O und S unterbrochen sein kann und welche unsubstituiert oder durch einen oder mehrere (C₁-C₄)Alkylreste substituiert ist, stehen,
(R¹e) Reste der Formel -CO-R,
   worin R für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, vorzugsweise für Wasserstoff oder (C₁-C₃)Alkyl steht,
(R¹f) Reste der Formel -C(R^{a})=N-OR^{b}, worin
   R^{a} für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, vorzugsweise für Wasserstoff oder (C₁-C₃)Alkyl und
   R^{b} für Wasserstoff, (C₁-C₆)Alkyl, (C₁-C₆)Haloalkyl, (C₂-C₆)Alkenyl, (C₂-C₆)Haloalkenyl oder (C₂-C₆)Alkinyl, vorzugsweise für Wasserstoff oder (C₁-C₄)Alkyl stehen,
(R¹g) Reste der Formel -C(SR^{a})=NR^{b}, worin
   R^{a} für (C₁-C₄)Alkyl und
   R^{b} für Wasserstoff oder (C₁-C₄)Alkyl stehen,
(R¹h) Reste der Formel -OR, worin R
   (1) für Wasserstoff, (C₁-C₆)Alkyl, (C₂-C₆)Alkenyl oder (C₂-C₆)Alkinyl
   (2) oder für (C₁-C₆)Alkyl, das unsubstituiert oder durch einen oder mehrere Reste aus der Gruppe Halogen, CN, (C₁-C₄)Alkoxy, (C₁-C₄)Haloalkoxy und (C₁-C₄)Alkoxy-(C₁-C₄)alkoxy, vorzugsweise aus der Gruppe Halogen, (C₁-C₄)Alkoxy und (C₁-C₄)Haloalkoxy substituiert ist,
   (3) oder für [(C₁-C₆)Alkoxy]-carbonyl, das unsubstituiert oder durch einen oder mehrere Reste aus der Gruppe Halogen, (C₁-C₄)Alkoxy, (C₁-C₄)Haloalkoxy und (C₁-C₄)Alkoxy-(C₁-C₄)alkoxy, vorzugsweise aus der Gruppe Halogen und (C₁-C₄)Alkoxy substituiert ist,
   (4) oder für Phenyl oder Phenyl-(C₁-C₄)alkyl, wobei jeder der letztgenannten beiden Reste im Phenylring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy und (C₁-C₄)Alkylthio, vorzugsweise aus der Gruppe Halogen, (C₁-C₄)Alkyl, (C₁-C₄)Haloalkyl, (C₁-C₄)Alkoxy und (C₁-C₄)Haloalkoxy, substituiert ist,
   steht,
   vorzugsweise die Reste (C₁-C₄)Alkoxy und (C₁-C₄)Haloalkoxy,
(R¹i) Reste der Formel -SR, -S(=O)-R und -S(=O)₂-R,
   wobei in jedem der letztgenannten 3 Formeln R jeweils unabhängig voneinander von den anderen Resten R für Wasserstoff, (C₁-C₆)Alkyl, (C₂-C₆)Alkenyl oder (C₂-C₆)Alkinyl oder für (C₁-C₆)Alkyl, das durch einen oder mehrere Reste aus der Gruppe Halogen, CN und (C₁-C₄)Alkoxy substituiert ist, steht,
   vorzugsweise die Reste (C₁-C₄)Alkylthio, (C₁-C₄)Alkylsulfonyl und (C₁-C₄)Haloalkylsulfonyl,
(R¹j) (C₁-C₆)Alkyl, (C₂-C₆)Alkenyl, (C₂-C₆)Alkinyl, (C₃-C₆)Cycloalkyl, (C₅-C₆)Cycloalkenyl, Heterocyclyl mit 3 bis 6 Ringatomen und einem bis 3 Heteroringatomen aus der Gruppe N, O und S oder Phenyl, wobei jeder der 7 letztgenannten Reste unsubstituiert oder durch einen oder mehrere Reste aus den Gruppen (G¹a) bis (G¹L) substituiert ist, enthaltend die Reste
   (G¹a) Halogen und Cyano, vorzugsweise Halogen, wie Fluor oder Chlor,
   (G¹b) (C₃-C₆)Cycloalkyl, das unsubstituiert oder durch einen oder mehrere (C₁-C₃)Alkylreste substituiert ist,
   (G¹c) Phenyl und Pyridyl,
      wobei jeder der letztgenannten 2 Reste unsubstituiert oder durch einen oder mehrere Reste aus der Gruppe enthaltend Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, vorzugsweise Phenyl,
   (G¹d) Reste der Formel -NR^{a}R^{b}, worin jeder der Reste R^{a} und R^{b} unabhängig voneinander für einen Rest aus der Gruppe, welche die Reste
      (1) Wasserstoff, (C₁-C₆)Alkyl, (C₂-C₆)Alkenyl und (C₂-C₆)Alkinyl, vorzugsweise (C₁-C₃)Alkyl,
      (2) Phenyl und Benzyl,
         wobei jeder der letztgenannten beiden Reste im Ring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy und (C₁-C₄)Haloalkoxy, vorzugsweise durch Reste aus der Gruppe (C₁-C₄)Alkyl, (C₁-C₄)Haloalkyl und (C₁-C₄)Alkoxy substituiert ist,
      (3) [(C₁-C₆)Alkanoyl und [(C₁-C₆)Haloalkanoyl,
      (4) [(C₁-C₆)Alkoxy]-carbonyl und [(C₁-C₆)Haloalkyloxy]-carbonyl,
      (5) (C₁-C₄)Alkylsulfinyl, (C₁-C₄)Haloalkylsulfinyl, (C₁-C₄)Alkylsulfonyl und (C₁-C₄)Haloalkylsulfonyl
         enthält, steht oder
         R^{a} und R^{b} gemeinsam für eine (C₂-C₅)Alkylen-kette, welche ein Heteroatom aus der Gruppe O und S unterbrochen sein kann und welche unsubstituiert oder durch einen oder mehrere (C₁-C₃)Alkylreste substituiert ist, stehen,
   (G¹e) Reste der Formel -CO₂R,
      worin R für Wasserstoff, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, (C₁-C₄)Alkoxy und Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist, substituiert ist, steht,
      vorzugsweise R für (C₁-C₃)Alkyl, (C₁-C₃)Haloalkyl oder Benzyl, insbesondere für Methyl oder Ethyl steht,
   (G¹f) Reste der Formeln -CO-NR^{a}R^{b} und -CS-NR^{a}R^{b},
      worin jeder der Reste R^{a} und R^{b} unabhängig voneinander für einen Rest aus der Gruppe, welche die Reste
      (1) Wasserstoff und (C₁-C₄)Alkyl, das unsubstituiert oder durch einen oder mehrere Reste aus der Gruppe (C₁-C₆)Alkoxy]-carbonyl und CN substituiert ist, vorzugsweise Wasserstoff oder (C₁-C₄)Alkyl,
      (2) (C₂-C₆)Alkenyl und (C₂-C₆)Alkinyl, vorzugsweise (C₃-C₆)Alkenyl und (C₃-C₆)Alkinyl, insbesondere Allyl oder Propargyl,
      (3) Phenyl und Benzyl, wobei jeder der letztgenannten beiden letztgenannten Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl, vorzugsweise aus der Gruppe Halogen, (C₁-C₄)Alkyl, (C₁-C₄)Haloalkyl und (C₁-C₄)Alkoxy substituiert ist,
      enthält, steht oder
      R^{a} und R^{b} gemeinsam für eine (C₂-C₅)Alkylen-kette, welche durch ein Heteroatom aus der Gruppe O und S unterbrochen sein kann und welche unsubstituiert oder durch einen oder mehrere (C₁-C₃)Alkylreste substituiert ist, stehen,
   (G¹g) Reste der Formel -CO-R,
      worin R für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, vorzugsweise für Wasserstoff oder (C₁-C₃)Alkyl steht,
   (G¹h) Reste der Formel -C(R^{a})=N-OR^{b}, worin R^{a} für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, vorzugsweise für Wasserstoff oder (C₁-C₃)Alkyl und R^{b} für Wasserstoff, (C₁-C₆)Alkyl, (C₁-C₆)Haloalkyl, (C₂-C₆)Alkenyl, (C₂-C₆)Haloalkenyl oder (C₂-C₆)Alkinyl, vorzugsweise für Wasserstoff oder (C₁-C₄)Alkyl stehen,
   (G¹i) Reste der Formel -C(SR^{a})=NR^{b}, worin
      R^{a} für (C₁-C₄)Alkyl und
      R^{b} für Wasserstoff oder (C₁-C₄)Alkyl stehen,
   (G¹j) Reste der Formel -OR, worin R
      (1) für Wasserstoff, (C₁-C₆)Alkyl, (C₂-C₆)Alkenyl oder (C₂-C₆)Alkinyl
      (2) oder für (C₁-C₆)Alkyl, das unsubstituiert oder durch einen oder mehrere Reste aus der Gruppe Halogen, CN, (C₁-C₄)Alkoxy, (C₁-C₄)Haloalkoxy und (C₁-C₄)Alkoxy-(C₁-C₄)alkoxy, vorzugsweise aus der Gruppe Halogen, (C₁-C₄)Alkoxy und (C₁-C₄)Haloalkoxy substituiert ist,
      (3) oder für [(C₁-C₆)Alkoxy]-carbonyl, das unsubstituiert oder durch einen oder mehrere Reste aus der Gruppe Halogen, (C₁-C₄)Alkoxy, (C₁-C₄)Haloalkoxy und (C₁-C₄)Alkoxy-(C₁-C₄)alkoxy, vorzugsweise aus der Gruppe Halogen und (C₁-C₄)Alkoxy substituiert ist,
      (4) oder für Phenyl oder Phenyl-(C₁-C₄)alkyl, wobei jeder der letztgenannten beiden Reste im Phenylring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy und (C₁-C₄)Alkylthio, vorzugsweise aus der Gruppe Halogen, (C₁-C₄)Alkyl, (C₁-C₄)Haloalkyl, (C₁-C₄)Alkoxy und (C₁-C₄)Haloalkoxy substituiert ist,
      steht,
      vorzugsweise die Reste (C₁-C₄)Alkoxy und (C₁-C₄)Haloalkoxy,
   (G¹k) Reste der Formel -SR, -S(=O)-R und -S(=O)₂-R,
      wobei in jedem der letztgenannten 3 Formeln R jeweils unabhängig voneinander von den anderen Resten R für Wasserstoff, (C₁-C₆)Alkyl, (C₂-C₆)Alkenyl oder (C₂-C₆)Alkinyl oder für (C₁-C₆)Alkyl, das durch einen oder mehrere Reste aus der Gruppe Halogen, CN und (C₁-C₄)Alkoxy substituiert ist, steht,
      vorzugsweise die Reste (C₁-C₄)Alkylthlo, (C₁-C₄)Alkylsulfonyl und (C₁-C₄)Haloalkylsulfonyl,
   (G¹L) und, nur für den Fall R¹ = (C₃-C₆)Cycloalkyl, (C₅-C₆)Cycloalkenyl, Heterocyclyl oder Phenyl als Basisgruppe, auch die Reste (C₁-C₄)Alkyl, (C₁-C₄)Haloalkyl und (C₁-C₄)Alkoxy-(C₁-C₄)alkyl,
   oder
   zwei benachbarte Reste R¹ gemeinsam eine (C₂-C₇)Alkylen- oder (C₃-C₇)Alkenylengruppe, welche durch 1 oder 2 Heteroatome aus der Gruppe O, S und N unterbrochen sein kann und welche unsubstituiert oder durch einen oder mehrere Reste aus der Gruppe Halogen, CN, (C₁-C₃)Alkyl, (C₁-C₃)Haloalkyl und (C₁-C₃)Alkoxy substituiert ist, bedeuten, und
m eine ganze Zahl von 0 bis zur Anzahl der CH-Gruppen im Ring des Restes der Formel Q.
   Weiter bevorzugt bedeutet dabei
   (R¹)ₘ m Substituenten R¹, wobei R¹, im Fall dass m = 1 ist, oder jeder der Reste R¹ jeweils unabhängig voneinander, im Fall dass m größer als 1 ist,
   Halogen, wie Fluor, Chlor, Brom oder Iod, vorzugweise Fluor oder Chlor, Cyano, (C₁-C₆)Alkyl, das unsubstituiert oder durch einen oder mehrere Reste aus der Gruppe Halogen, wie Fluor oder Chlor, CN, (C₁-C₄)Alkoxy, (C₁-C₄)Haloalkoxy und (C₁-C₄)Alkoxy-(C₁-C₄)alkoxy, vorzugsweise aus der Gruppe Halogen, (C₁-C₄)Alkoxy und (C₁-C₄)Haloalkoxy substituiert ist, oder
   (C₁-C₆)Alkoxy, das unsubstituiert oder durch einen oder mehrere Reste aus der Gruppe Halogen, wie Fluor oder Chlor, CN, (C₁-C₄)Alkoxy, (C₁-C₄)Haloalkoxy und (C₁-C₄)Alkoxy-(C₁-C₄)alkoxy, vorzugsweise aus der Gruppe Halogen, (C₁-C₄)Alkoxy und (C₁-C₄)Haloalkoxy substituiert ist,
   oder Phenoxy, das unsubstituiert oder durch einen oder mehrere Reste aus der
   Gruppe Halogen, wie Fluor, Chlor, Brom oder Iod, oder CN, (C₁-C₄)Alkyl,
   (C₁-C₄)Haloalkyl, (C₁-C₄)Alkoxy, (C₁-C₄)Haloalkoxy, (C₁-C₄)Alkoxy-
   (C₁-C₄)alkoxy, (C₁-C₄)Alkylthio, vorzugsweise aus der Gruppe Halogen,
   (C₁-C₄)Alkyl, (C₁-C₄)Alkoxy und (C₁-C₄)Haloalkoxy substituiert ist,
   oder [(C₁-C₄)Alkoxyl-carbonyl, Mono- oder Di-[(C₁-C₄)Alkyl]-amino, Mono- oder Di-[(C₁-C₄)Alkyl]-aminocarbonyl, Mono- oder Di-[(C₁-C₄)Alkanoyl]-amino bedeutet.

Weiter bevorzugt bedeutet dabei
(R¹)ₘ m Substituenten R¹, wobei R¹, im Fall dass m = 1 ist, oder jeder der Reste R¹ jeweils unabhängig voneinander, im Fall dass m größer als 1 ist,
Halogen, wie Fluor, Chlor, Brom oder Iod, vorzugweise Fluor oder Chlor, oder Cyano, (C₁-C₄)Alkyl, (C₁-C₄)Haloalkyl, (C₁-C₄)Alkoxy oder (C₁-C₄)Haloalkoxy, bedeutet.

Weiter bevorzugt sind die oben genannten Verbindungen der Formel (I) oder die jeweils bezüglich der Reste Q, A und R¹ bevorzugt genannten Verbindungen der Formel (I) oder deren Salze, worin
R² Wasserstoff, (C₁-C₄)Alkyl, (C₂-C₄)Alkenyl, (C₂-C₄)Alkinyl, (C₃-C₆)Cycloalkyl, (C₅-C₆)Cycloalkenyl, Heterocyclyl mit 3 bis 6 Ringatomen und 1, 2 oder 3 Heteroringatome aus der Gruppe N, O und S oder Phenyl bedeutet,
   wobei jeder der 7 letztgenannten Reste unsubstituiert oder durch einen oder mehrere Reste aus den Gruppen (G²a) bis (G²h) substituiert ist, enthaltend die Reste
   (G²a) Halogen und Cyano, vorzugsweise Halogen, wie Fluor oder Chlor,
   (G²b) (C₃-C₆)Cycloalkyl, das unsubstituiert oder durch einen oder mehrere (C₁-C₃)Alkylreste substituiert ist,
   (G²c) Phenyl und Pyridyl,
      wobei jeder der letztgenannten 2 Reste unsubstituiert oder durch einen oder mehrere Reste aus der Gruppe enthaltend Halogen, (C₁-C₄)Alkyl, (C₁-C₄)Haloalkyl, (C₁-C₄)Alkoxy, (C₁-C₄)Haloalkoxy, (C₁-C₄)Alkylthlo, (C₁-C₄)Alkylsulfinyl und (C₁-C₄)Alkylsulfonyl substituiert ist, vorzugsweise Phenyl,
   (G²d) Reste der Formel -CO₂R,
      worin R für Wasserstoff, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, (C₁-C₄)Alkoxy und Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist, substituiert ist, vorzugsweise R für (C₁-C₃)Alkyl, (C₁-C₃)Haloalkyl oder Benzyl, insbesondere für Methyl oder Ethyl steht,
   (G²e) Reste der Formeln -CO-NR^{a}R^{b} und -CS-NR^{a}R^{b},
      worin jeder der Reste R^{a} und R^{b} unabhängig voneinander für einen Rest aus der Gruppe, welche die Reste
      (1) Wasserstoff und (C₁-C₄)Alkyl, das unsubstituiert oder durch einen oder mehrere Reste aus der Gruppe (C₁-C₆)Alkoxy]-carbonyl und CN substituiert ist, vorzugsweise Wasserstoff oder (C₁-C₄)Alkyl,
      (2) (C₂-C₆)Alkenyl und (C₂-C₆)Alkinyl, vorzugsweise (C₃-C₆)Alkenyl und (C₃-C₆)Alkinyl, insbesondere Allyl oder Propargyl,
      (3) Phenyl und Benzyl, wobei jeder der beiden letztgenannten Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl, vorzugsweise aus der Gruppe Halogen, (C₁-C₄)Alkyl, (C₁-C₄)Haloalkyl und (C₁-C₄)Alkoxy substituiert ist,
      enthält, steht oder
      R^{a} und R^{b} gemeinsam für eine (C₂-C₅)Alkylen-kette, welche durch ein Heteroatom aus der Gruppe 0 und S unterbrochen sein kann und welche unsubstituiert oder durch einen oder mehrere (C₁-C₃)Alkylreste substituiert ist, stehen,
   (G²f) Reste der Formel -OR, worin R
      (1) für Wasserstoff, (C₁-C₆)Alkyl, (C₂-C₆)Alkenyl oder (C₂-C₆)Alkinyl
      (2) oder für (C₁-C₆)Alkyl, das unsubstituiert oder durch einen oder mehrere Reste aus der Gruppe Halogen, CN, (C₁-C₄)Alkoxy, (C₁-C₄)Haloalkoxy und (C₁-C₄)Alkoxy-(C₁-C₄)alkoxy, vorzugsweise aus der Gruppe Halogen, (C₁-C₄)Alkoxy und (C₁-C₄)Haloalkoxy substituiert ist,
      (3) oder für [(C₁-C₆)Alkoxy]-carbonyl, das unsubstituiert oder durch einen oder mehrere Reste aus der Gruppe Halogen, (C₁-C₄)Alkoxy, (C₁-C₄)Haloalkoxy und (C₁-C₄)Alkoxy-(C₁-C₄)alkoxy, vorzugsweise aus der Gruppe Halogen und (C₁-C₄)Alkoxy substituiert ist,
      (4) oder für Phenyl oder Phenyl-(C₁-C₄)alkyl, wobei jeder der letztgenannten beiden Reste im Phenylring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy und (C₁-C₄)Alkylthio, vorzugsweise aus der Gruppe Halogen, (C₁-C₄)Alkyl, (C₁-C₄)Haloalkyl, (C₁-C₄)Alkoxy und (C₁-C₄)Haloalkoxy, substituiert ist, steht,
      vorzugsweise die Reste (C₁-C₄)Alkoxy und (C₁-C₄)Haloalkoxy,
   (G²g) Reste der Formel -SR, -S(=O)-R und -S(=O)₂-R,
      wobei in jedem der letztgenannten 3 Formeln R jeweils unabhängig voneinander von den anderen Resten R für Wasserstoff, (C₁-C₆)Alkyl, (C₂-C₆)Alkenyl oder (C₂-C₆)Alkinyl oder für (C₁-C₆)Alkyl, das durch einen oder mehrere Reste aus der Gruppe Halogen, CN und (C₁-C₄)Alkoxy substituiert ist, steht,
      vorzugsweise die Reste (C₁-C₄)Alkylthio, (C₁-C₄)Alkylsulfonyl und (C₁-C₄)Haloalkylsulfonyl,
   (G²h) und, nur für den Fall R² = (C₃-C₆)Cycloalkyl, (C₅-C₆)Cycloalkenyl, Heterocyclyl oder Phenyl als Basisgruppen, auch die Reste (C₁-C₄)Alkyl, (C₁-C₄)Haloalkyl und (C₁-C₄)Alkoxy-(C₁-C₄)alkyl.

Weiter bevorzugt sind dabei die genannten Verbindungen (I) oder deren Salze, worin R² Wasserstoff oder (C₁-C₄)Alkyl, das unsubstituiert oder durch einen oder mehrere Reste aus der Gruppe Halogen, wie Fluor oder Chlor, CN, (C₁-C₄)Alkoxy, (C₁-C₄)Haloalkoxy und (C₁-C₄)Alkoxy-(C₁-C₄)alkoxy, vorzugsweise aus der Gruppe Halogen, (C₁-C₄)Alkoxy und (C₁-C₄)Haloalkoxy substituiert ist, oder
Formyl, (C₁-C₄)Alkanoyl, (C₁-C₄)Haloalkanoyl, [(C₁-C₃)Alkoxy]-carbonyl, Phenoxy-carbonyl, das im Phenylteil unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
oder (C₁-C₄)Alkylsulfinyl, (C₁-C₄)Haloalkylsulfinyl, (C₁-C₄)Alkylsulfonyl oder (C₁-C₄)Haloalkylsulfonyl bedeutet.
Weiter bevorzugt sind dabei die genannten Verbindungen (I) oder deren Salze, worin R² Wasserstoff oder (C₁-C₃)Alkyl, insbesondere Wasserstoff bedeutet.

Weiter bevorzugt sind die oben genannten Verbindungen der Formel (I) oder die jeweils bezüglich der Reste Q, A, R¹ und R² und deren Kombinationen bevorzugt genannten Verbindungen der Formel (I) oder deren Salze, worin
(R³)ₙ n Substituenten R¹ bedeutet, wobei R¹, im Fall dass n = 1 ist, oder jeder der Reste R¹ jeweils unabhängig voneinander, im Fall dass n größer als 1 ist, einen Rest aus den Gruppen (R³a) bis (R³n) bedeutet, enthaltend die Reste
   (R³a) Halogen, Cyano, Isocyanato und Nitro, vorzugsweise Halogen, wie Fluor, Chlor, Brom und Iod, insbesondere Fluor und Chlor,
   (R³b) Reste der Formel -NR^{a}R^{b}, worin jeder der Reste R^{a} und R^{b} unabhängig voneinander für einen Rest aus der Gruppe, welche die Reste
      (1) Wasserstoff, (C₁-C₆)Alkyl, (C₂-C₆)Alkenyl und (C₂-C₆)Alkinyl, vorzugsweise (C₁-C₃)Alkyl,
      (2) Phenyl und Benzyl,
         wobei jeder der letztgenannten beiden Reste im Ring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy und (C₁-C₄)Haloalkoxy, vorzugsweise durch Reste aus der Gruppe (C₁-C₄)Alkyl, (C₁-C₄)Haloalkyl und (C₁-C₄)Alkoxy substituiert ist,
         vorzugsweise der Rest Phenyl,
      (3) [(C₁-C₆)Alkanoyl und [(C₁-C₆)Haloalkanoyl,
      (4) Phenyl-carbonyl und Benzyl-carbonyl, wobei jeder der letztgenannten beiden Reste im Phenylteil unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist,
      (5) [(C₁-C₆)Alkoxy]-carbonyl, das unsubstituiert oder durch einen oder mehrere Reste aus der Gruppe Halogen, (C₁-C₄)Alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist, substituiert ist,
      (6) (C₁-C₄)Alkylsulfinyl, (C₁-C₄)Haloalkylsulfinyl, (C₁-C₄)Alkylsulfonyl und (C₁-C₄)Haloalkylsulfonyl
         enthält, steht
         oder
      worin einer der Reste R^{a} und R^{b} wie oben definiert ist und der andere für Hydroxy oder (C₁-C₄)Alkoxy steht
      oder
      worin R^{a} und R^{b} gemeinsam für eine (C₂-C₅)Alkylen-kette, welche durch ein oder zwei Heteroatome aus der Gruppe O und S unterbrochen sein kann und welche unsubstituiert oder durch einen oder mehrere
      (C₁-C₃)Alkylreste substituiert ist, stehen
      oder
      worin R^{a} und R^{b} gemeinsam mit dem N-Atom der Gruppe NR^{a}R^{b} für N-Phthalimido stehen,
   (R³c) Reste der Formel -CO₂R,
      worin R für Wasserstoff, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, (C₁-C₄)Alkoxy und Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist, substituiert ist, vorzugsweise R für (C₁-C₃)Alkyl, (C₁-C₃)Haloalkyl oder Benzyl, insbesondere für Methyl oder Ethyl steht,
   (R³d) Reste der Formeln -CO-NR^{a}R^{b}, -CS-NR^{a}R^{b} und -SO₂NR^{a}R^{b},
      worin jeder der Reste R^{a} und R^{b} in den letztgenannten 3 Formeln unabhängig voneinander für einen Rest aus der Gruppe, welche die Reste
      (1) Wasserstoff und (C₁-C₄)Alkyl, das unsubstituiert oder durch einen oder mehrere Reste aus der Gruppe (C₁-C₆)Alkoxy]-carbonyl und CN substituiert ist, vorzugsweise Wasserstoff oder (C₁-C₄)Alkyl,
      (2) (C₂-C₆)Alkenyl und (C₂-C₆)Alkinyl, vorzugsweise (C₃-C₆)Alkenyl und (C₃-C₆)Alkinyl, insbesondere Allyl oder Propargyl,
      (3) Phenyl und Benzyl, wobei jeder der letztgenannten beiden letztgenannten Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl, vorzugsweise aus der Gruppe Halogen, (C₁-C₄)Alkyl, (C₁-C₄)Haloalkyl und (C₁-C₄)Alkoxy substituiert ist,
         enthält, steht oder
      worin R^{a} und R^{b} gemeinsam für eine (C₂-C₅)Alkylen-kette, welche durch ein oder zwei Heteroatome aus der Gruppe O und S unterbrochen sein kann und welche unsubstituiert oder durch einen oder mehrere (C₁-C₄)Alkylreste substituiert ist, stehen,
   (R³e) Reste der Formel -CO-R,
      worin R für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, vorzugsweise für Wasserstoff oder (C₁-C₃)Alkyl steht,
   (R³f) Reste der Formel -C(R^{a})=N-OR^{b}, worin
      R^{a} für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, vorzugsweise für Wasserstoff oder (C₁-C₃)Alkyl und
      R^{b} für Wasserstoff, (C₁-C₆)Alkyl, (C₁-C₆)Haloalkyl, (C₂-C₆)Alkenyl, (C₂-C₆)Haloalkenyl oder (C₂-C₆)Alkinyl, vorzugsweise für Wasserstoff oder (C₁-C₄)Alkyl stehen,
   (R³g) Reste der Formel -C(SR^{a})=NR^{b}, worin
      R^{a} für (C₁-C₄)Alkyl und
      R^{b} für Wasserstoff oder (C₁-C₄)Alkyl stehen,
   (R³h) Reste der Formel -OR, worin
      (1) für Wasserstoff, (C₁-C₆)Alkyl, (C₂-C₆)Alkenyl oder (C₂-C₆)Alkinyl
      (2) oder für (C₁-C₆)Alkyl, das unsubstituiert oder durch einen oder mehrere Reste aus der Gruppe Halogen, CN, (C₁-C₄)Alkoxy, (C₁-C₄)Haloalkoxy und (C₁-C₄)Alkoxy-(C₁-C₄)alkoxy, vorzugsweise aus der Gruppe Halogen, (C₁-C₄)Alkoxy und (C₁-C₄)Haloalkoxy substituiert ist,
      (3) oder für [(C₁-C₆)Alkoxy]-carbonyl, das unsubstituiert oder durch einen oder mehrere Reste aus der Gruppe Halogen, (C₁-C₄)Alkoxy, (C₁-C₄)Haloalkoxy und (C₁-C₄)Alkoxy-(C₁-C₄)alkoxy, vorzugsweise aus der Gruppe Halogen und (C₁-C₄)Alkoxy substituiert ist,
      (4) oder für Phenyl oder Phenyl-(C₁-C₄)alkyl, wobei jeder der letztgenannten beiden Reste im Phenylring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy und (C₁-C₄)Alkylthio, vorzugsweise aus der Gruppe Halogen, (C₁-C₄)Alkyl, (C₁-C₄)Haloalkyl, (C₁-C₄)Alkoxy und (C₁-C₄)Haloalkoxy, substituiert ist,
         steht, vorzugsweise die Reste (C₁-C₄)Alkoxy und (C₁-C₄)Haloalkoxy,
   (R³i) Reste der Formel -SR, worin R
      für Wasserstoff, (C₁-C₆)Alkyl, (C₂-C₆)Alkenyl oder für (C₁-C₆)Alkyl, das durch einen oder mehrere Reste aus der Gruppe Halogen, CN und (C₁-C₄)Alkoxy substituiert ist, steht
      oder
      R für [(C₁-C₄)Alkyl]-carbonyl oder [(C₁-C₄)Haloalkyl]-carbonyl steht
      oder
      R für [(C₁-C₆)Alkoxyl-carbonyl, das unsubstituiert oder durch einen oder mehrere Reste aus der Gruppe Halogen, CN, (C₁-C₄)Alkoxy und (C₁-C₄)Haloalkoxy, substituiert ist, steht
      oder
      R für Mono- oder Di-[(C₁-C₆)alkyl]-aminocarbonyl, Mono- oder Di-[(C₁-C₆)alkanoyl]-aminocarbonyl, Mono- oder Di-[(C₁-C₆)alkylsulfonyl]-aminocarbonyl, N-[(C₁-C₆)Alkyl]-N-[(C₁-C₆)alkanoyl]-aminocarbonyl, N-[(C₁-C₆)Alkyl]-N-[(C₁-C₆)alkylsulfonyl]-aminocarbonyl, N-[(C₁-C₆)Alkanoyl]-N-[(C₁-C₆)alkylsulfonyl]-aminocarbonyl oder für einen Rest der Formel -C(=O)NR'R', worin die R' gemeinsam eine Alkylen-kette mit 2 bis 5 C-Atomen bilden, die noch durch ein Sauerstoff oder Stickstoffatom unterbrochen sein kann, steht,
      vorzugsweise der Rest (C₁-C₆)Alkylthio,
   (R³j) Reste der Formel -S(=O)-R und -S(=O)₂-R,
      wobei in jedem der letztgenannten beiden Formeln R jeweils unabhängig voneinander für (C₁-C₆)Alkyl, (C₂-C₆)Alkenyl oder (C₂-C₆)Alkinyl oder für (C₁-C₆)Alkyl, das durch einen oder mehrere Reste aus der Gruppe Halogen, CN und (C₁-C₄)Alkoxy substituiert ist, steht,
      vorzugsweise die Reste (C₁-C₄)Alkylsulfinyl, (C₁-C₄)Haloalkylsulfinyl, (C₁-C₄)Alkylsulfonyl und (C₁-C₄)Haloalkylsulfonyl,
   (R³k) Reste der Formel -O-S(=O)₂-R,
      wobei R für Wasserstoff, (C₁-C₆)Alkyl, (C₂-C₆)Alkenyl oder (C₂-C₆)Alkinyl oder für (C₁-C₆)Alkyl, das durch einen oder mehrere Reste aus der Gruppe Halogen, CN und (C₁-C₄)Alkoxy substituiert ist, oder für Phenyl, das durch einen oder mehrere Reste aus der Gruppe Halogen, CN, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Haloalkylsulfinyl, (C₁-C₄)Alkylsulfonyl und (C₁-C₄)Haloalkylsulfonyl substituiert ist, steht,
   (R³L) Reste der Formel -N=C(OR^{a})R^{b},
      wobei jeder der Reste R^{a} und R^{b} unabhängig voneinander für Wasserstoff oder (C₁-C₄)Alkyl stehen,
   (R³m) Reste der Formel -N(OR^{a})COR^{b}
      wobei jeder der Reste R^{a} und R^{b} unabhängig voneinander für Wasserstoff, (C₁-C₄)Alkyl oder Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, stehen,
   (R³n) (C₁-C₆)Alkyl, (C₂-C₆)Alkenyl, (C₂-C₆)Alkinyl, (C₃-C₆)Cycloalkyl,
      (C₅-C₆)Cycloalkenyl, Heterocyclyl mit 3 bis 6 Ringatomen und einem bis 3 Heteroringatome aus der Gruppe N, O und S oder Phenyl, wobei jeder der 7 letztgenannten Reste unsubstituiert oder durch einen oder mehrere Reste aus den Gruppen (G³a) bis (G³m) substituiert ist, enthaltend die Reste
      (G³a) Halogen, Cyano und Isocyanato,
      (G³b) (C₃-C₆)Cycloalkyl und einen gesättigten oder ungesättigten nicht-aromatischen heterocyclischen Rest mit 3 bis 6 Ringatomen und 1, 2 oder 3 Heteroringatomen aus der Gruppe N, O und S,
         wobei jeder der letztgenannten 2 Reste unsubstituiert oder durch einen oder mehrere Reste aus der Gruppe Halogen und (C₁-C₃)Alkyl substituiert ist,
      (G³c) Phenyl und einen aromatischen heterocyclischen Rest mit 5 oder 6 Ringatomen und 1, 2 oder 3 Heteroringatomen aus der Gruppe N, O und S,
         wobei jeder der letztgenannten 2 Reste unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
         vorzugsweise der Rest Phenyl,
      (G³d) Reste der Formel -NR^{a}R^{b},
         worin jeder der Reste R^{a} und R^{b} unabhängig voneinander für einen Rest aus der Gruppe, welche die Reste
         (1) Wasserstoff, (C₁-C₆)Alkyl, (C₂-C₆)Alkenyl und (C₂-C₆)Alkinyl, vorzugsweise (C₁-C₃)Alkyl,
         (2) Phenyl und Benzyl,
            wobei jeder der letztgenannten beiden Reste im Ring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy und (C₁-C₄)Haloalkoxy, vorzugsweise durch Reste aus der Gruppe (C₁-C₄)Alkyl, (C₁-C₄)Haloalkyl und (C₁-C₄)Alkoxy substituiert ist, vorzugsweise der Rest Phenyl,
         (3) [(C₁-C₆)Alkanoyl und [(C₁-C₆)Haloalkanoyl,
         (4) Phenyl-carbonyl und Benzyl-carbonyl, wobei jeder der letztgenannten beiden Reste im Phenylteil unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist,
         (5) [(C₁-C₆)Alkoxy]-carbonyl, das unsubstituiert oder durch einen oder mehrere Reste aus der Gruppe Halogen, (C₁-C₄)Alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist, substituiert ist,
         (6) (C₁-C₄)Alkylsulfinyl, (C₁-C₄)Haloalkylsulfinyl, (C₁-C₄)Alkylsulfonyl und (C₁-C₄)Haloalkylsulfonyl enthält, steht oder
         worin R^{a} und R^{b} gemeinsam für eine (C₂-C₅)Alkylen-kette, welche ein Heteroatom aus der Gruppe O und S unterbrochen sein kann und welche unsubstituiert oder durch einen oder mehrere (C₁-C₃)Alkylreste substituiert ist, stehen oder
         worin einer der Reste R^{a} und R^{b} wie oben definiert ist und der andere für Hydroxy oder (C₁-C₄)Alkoxy steht
         oder
         worin R^{a} und R^{b} gemeinsam für eine (C₂-C₅)Alkylen-kette, welche durch ein oder zwei Heteroatome aus der Gruppe O und S unterbrochen sein kann und welche unsubstituiert oder durch einen oder mehrere (C₁-C₃)Alkylreste substituiert ist, stehen, oder
      (G³e) Reste der Formel -CO₂R,
         worin R für Wasserstoff, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, (C₁-C₄)Alkoxy und Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist, substituiert ist,
         vorzugsweise R für (C₁-C₃)Alkyl, (C₁-C₃)Haloalkyl oder Benzyl, insbesondere für Methyl oder Ethyl steht,
      (G³f) Reste der Formeln -CO-NR^{a}R^{b} und -CS-NR^{a}R^{b},
         worin jeder der Reste R^{a} und R^{b} in den letztgenannten 2 Formeln unabhängig voneinander für einen Rest aus der Gruppe, welche die Reste
         (1) Wasserstoff und (C₁-C₄)Alkyl, das unsubstituiert oder durch einen oder mehrere Reste aus der Gruppe (C₁-C₆)Alkoxy]-carbonyl und CN substituiert ist, vorzugsweise Wasserstoff oder (C₁-C₄)Alkyl,
         (2) (C₂-C₆)Alkenyl und (C₂-C₆)Alkinyl, vorzugsweise (C₃-C₆)Alkenyl und (C₃-C₆)Alkinyl, insbesondere Allyl oder Propargyl,
         (3) Phenyl und Benzyl, wobei jeder der letztgenannten beiden letztgenannten Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl, vorzugsweise aus der Gruppe Halogen, (C₁-C₄)Alkyl, (C₁-C₄)Haloalkyl und (C₁-C₄)Alkoxy substituiert ist,
            enthält, steht oder
         worin R^{a} und R^{b} gemeinsam für eine (C₂-C₅)Alkylen-kette, welche durch ein oder zwei Heteroatome aus der Gruppe O und S unterbrochen sein kann und welche unsubstituiert oder durch einen oder mehrere (C₁-C₄)Alkylreste substituiert ist, stehen,
      (G³g) Reste der Formel -CO-R,
         worin R für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, vorzugsweise für Wasserstoff oder (C₁-C₃)Alkyl steht,
      (G³h) Reste der Formel -C(R^{a})=N-OR^{b}, worin
         R^{a} für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, vorzugsweise für Wasserstoff oder (C₁-C₃)Alkyl und
         R^{b} für Wasserstoff, (C₁-C₆)Alkyl, (C₁-C₆)Haloalkyl, (C₂-C₆)Alkenyl, (C₂-C₆)Haloalkenyl oder (C₂-C₆)Alkinyl, vorzugsweise für Wasserstoff oder (C₁-C₄)Alkyl stehen,
      (G³i) Reste der Formel -C(SR^{a})=NR^{b}, worin
         R^{a} für (C₁-C₆)Alkyl und
         R^{b} für Wasserstoff oder (C₁-C₄)Alkyl stehen,
      (G³j) Reste der Formel -OR, worin R für
         (1) für Wasserstoff, (C₁-C₆)Alkyl, (C₂-C₆)Alkenyl oder (C₂-C₆)Alkinyl
         (2) oder für (C₁-C₆)Alkyl, das unsubstituiert oder durch einen oder mehrere Reste aus der Gruppe Halogen, CN, (C₁-C₄)Alkoxy, (C₁-C₄)Haloalkoxy und (C₁-C₄)Alkoxy-(C₁-C₄)alkoxy, vorzugsweise aus der Gruppe Halogen, (C₁-C₄)Alkoxy und (C₁-C₄)Haloalkoxy substituiert ist,
         (3) oder für [(C₁-C₆)Alkoxy]-carbonyl, das unsubstituiert oder durch einen oder mehrere Reste aus der Gruppe Halogen, (C₁-C₄)Alkoxy, (C₁-C₄)Haloalkoxy und (C₁-C₄)Alkoxy-(C₁-C₄)alkoxy, vorzugsweise aus der Gruppe Halogen und (C₁-C₄)Alkoxy substituiert ist,
         (4) oder für Phenyl oder Phenyl-(C₁-C₄)alkyl, wobei jeder der letztgenannten beiden Reste im Phenylring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy und (C₁-C₄)Alkylthio, vorzugsweise aus der Gruppe Halogen, (C₁-C₄)Alkyl, (C₁-C₄)Haloalkyl, (C₁-C₄)Alkoxy und (C₁-C₄)Haloalkoxy, substituiert ist,
         steht,
         vorzugsweise die Reste (C₁-C₄)Alkoxy und (C₁-C₄)Haloalkoxy,
      (G³k) Reste der Formel -SR, -S(=O)-R und -S(=O)₂-R,
         wobei in jedem der letztgenannten 3 Formeln R jeweils unabhängig voneinander von den anderen Resten R für Wasserstoff, (C₁-C₆)Alkyl, (C₂-C₆)Alkenyl oder (C₂-C₆)Alkinyl oder für (C₁-C₆)Alkyl, das durch einen oder mehrere Reste aus der Gruppe Halogen, CN und (C₁-C₄)Alkoxy substituiert ist, steht,
         vorzugsweise die Reste (C₁-C₄)Alkylthio, (C₁-C₄)Alkylsulfinyl, (C₁-C₄)Haloalkylsulfinyl, (C₁-C₄)Alkylsulfonyl und (C₁-C₄)Haloalkylsulfonyl,
      (G³L) Reste der Formeln -Si[(C₁-C₃)alkyl]₃, -S⁺(C₁-C₃)alkyl]₂, und -N⁺[(C₁-C₃)alkyl]₃, wobei in den Fällen der kationischen Reste ein Anionäquivalent als Gegenion vorliegt,
      (G³m) und, nur für den Fall R³ = (C₃-C₆)Cycloalkyl, (C₅-C₆)Cycloalkenyl, Heterocyclyl oder Phenyl als Basisgruppe, auch die Reste (C₁-C₄)Alkyl, (C₁-C₄)Haloalkyl und (C₁-C₄)Alkoxy-(C₁-C₄)alkyl, oder
         zwei benachbarte Reste R³ gemeinsam eine (C₂-C₅)Alkylen- oder (C₃-C₅)Alkenylengruppe, welche durch 1, 2 oder 3 Heteroatome aus der Gruppe O, S und N unterbrochen sein kann und welche unsubstituiert oder durch einen oder mehrere Reste aus der Gruppe CN und (C₁-C₄)Alkyl substituiert ist, bedeuten und
      n eine ganze Zahl von 0 bis 5, vorzugsweise 0, 1, 2 oder 3 bedeutet,

Weiter bevorzugt sind die oben genannten Verbindungen der Formel (I) oder die jeweils bezüglich der Reste Q, A, R¹ und R² und deren Kombinationen bevorzugt genannten Verbindungen der Formel (I) oder deren Salze, worin
(R³)ₙ n Substituenten R³ bedeutet, wobei R³, im Fall dass n = 1 ist, oder jeder der Reste R³ jeweils unabhängig voneinander, im Fall dass n größer als 1 ist,
Halogen, wie Fluor, Chlor, Brom oder Iod, vorzugweise Fluor oder Chlor, Cyano, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, wie Fluor oder Chlor, CN, (C₁-C₄)Alkoxy, (C₁-C₄)Haloalkoxy, (C₁-C₄)Alkoxy-(C₁-C₄)alkoxy und Mono- und Di-[(C₁-C₄)Alkyl]-amino, vorzugsweise aus der Gruppe Halogen, (C₁-C₄)Alkoxy und (C₁-C₄)Haloalkoxy substituiert ist, oder
(C₃-C₆)Cycloalkyl, wie Cyclopropyl, Cyclobutyl, Cyclopentyl, Cyclohexyl, oder
(C₁-C₆)Alkoxy, (C₂-C₆)Alkenyloxy oder (C₂-C₆)Alkinyloxy, wobei jeder der letztgenannten 3 Reste unsubstituiert oder durch einen oder mehrere Reste aus der Gruppe Halogen, wie Fluor oder Chlor, CN, (C₁-C₄)Alkoxy, (C₁-C₄)Haloalkoxy und (C₁-C₄)Alkoxy-(C₁-C₄)alkoxy, vorzugsweise aus der Gruppe Halogen, (C₁-C₄)Alkoxy und (C₁-C₄)Haloalkoxy substituiert ist, oder (C₁-C₆)Alkylthio, (C₂-C₆)Alkenylthio, (C₁-C₄)Alkylsulfinyl,
(C₁-C₄)Haloalkylsulfinyl, (C₁-C₄)Alkylsulfonyl, (C₁-C₄)Haloalkylsulfonyl, Aminosulfonyl, Mono- oder Di-[(C₁-C₄)Alkyl]-amino, Mono- oder Di-[(C₁-C₄)Alkyl]-aminosulfonyl, Mono- oder Di-[(C₁-C₄)Alkyl]-sulfonylamino, Mono- oder Di-[(C₁-C₄)Alkanoyl]-amino bedeutet oder weiter bevorzugt
Halogen, wie Fluor, Chlor, Brom oder Iod, vorzugweise Fluor oder Chlor, oder Cyano, (C₁-C₄)Alkyl, (C₁-C₄)Haloalkyl, (C₁-C₄)Alkoxy oder (C₁-C₄)Haloalkoxy, (C₁-C₆)Alkylthio, (C₁-C₄)Alkylsulfinyl, (C₁-C₄)Haloalkylsulfinyl, (C₁-C₄)Alkylsulfonyl oder (C₁-C₄)Haloalkylsulfonyl bedeutet.

Weiter bevorzugt sind die oben genannten Verbindungen der Formel (I) oder die jeweils bezüglich der Reste Q, A, R¹, R² und R³ und deren Kombinationen bevorzugt genannten Verbindungen der Formel (I) oder deren Salze, worin
R⁴ H, (C₁-C₃)Alkyl, (C₁-C₃)Haloalkyl, (C₃-C₆)Cycloalkyl, das unsubstituiert oder durch einen oder mehrere Reste aus der Gruppe Halogen und (C₁-C₃)Alkyl substituiert ist, bedeutet und
R⁵ H oder (C₁-C₃)Alkyl bedeutet oder
R⁴ und R⁵ gemeinsam mit dem sie verbindenden C-Atom einen 3- bis 6-gliedrigen Ring, der carbocyclisch ist, wobei der Ring unsubstituiert oder durch einen oder mehrere Reste aus der Gruppe Halogen, wie Fluor oder Chlor, und (C₁-C₃)Alkyl substituiert ist, bedeuten.

Weiter bevorzugt sind die oben genannten Verbindungen der Formel (I) oder die jeweils bezüglich der Reste Q, R¹, R², R³, R⁴ und R⁵ und deren Kombinationen bevorzugt genannten Verbindungen der Formel (I) oder deren Salze, worin
A eine Gruppe der Formel (A1) bedeutet und
R⁶ H, (C₁-C₄)Alkyl, (C₃-C₄)Alkenyl oder (C₃-C₄)Alkinyl,
   wobei jeder der letztgenannten 3 Reste unsubstituiert oder durch einen oder mehrere Reste aus der Gruppe, welche die Reste
   (1) Halogen, Cyano, (C₁-C₃)Alkoxy, (C₁-C₃)Alkylthio, (C₁-C₃)Haloalkoxy, (C₁-C₄)Alkanoyl, (C₁-C₄)Haloalkanoyl, [(C₁-C₄)Alkoxy]-carbonyl, [(C₁-C₄)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
   (2) Reste der Formel CO-NR^{a}R^{b}, worin jeder der Reste R^{a} und R^{b} unabhängig voneinander Wasserstoff, (C₁-C₃)Alkyl und (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten heterocyclischen Rest mit 3 bis 6 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
   (3) (C₃-C₆)Cycloalkyl, das unsubstituiert oder durch einen oder mehrere Reste aus der Gruppe Halogen und (C₁-C₃)Alkyl substituiert ist,
   (4) Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, substituiert ist,
      enthält, substituiert ist, bedeutet
      oder
R⁶ Phenyl, (C₃-C₆)Cycloalkyl oder einen gesättigten heterocyclischen Rest mit 3 bis 6 Ringatomen und 1 oder 2 Heteroringatomen aus der Gruppe N, O und S, wobei jeder der letztgenannten 3 Reste unsubstituiert oder durch einen oder mehrere Reste aus der Gruppe, welche die Reste
   (1) (C₁-C₄)Alkyl, Halogen, Cyano, (C₁-C₃)Alkoxy, (C₁-C₃)Alkylthlo, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
   (2) Reste der Formel CO-NR^{a}R^{b}, worin jeder der Reste R^{a} und R^{b} unabhängig voneinander Wasserstoff, (C₁-C₃)Alkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet,
   (3) (C₃-C₆)Cycloalkyl, das unsubstituiert oder durch einen oder mehrere Reste aus der Gruppe Halogen und (C₁-C₃)Alkyl substituiert ist,
   (4) Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl substituiert ist,
      enthält, substituiert ist, bedeutet.

Weiter bevorzugt bedeutet dabei R⁶ Wasserstoff, (C₁-C₄)Alkyl oder Cyclopropyl, insbesondere Wasserstoff oder Methyl.

Weiter bevorzugt sind die oben genannten Verbindungen der Formel (I) oder die jeweils bezüglich der Reste Q, R¹, R², R³, R⁴ und R⁵ und deren Kombinationen bevorzugt genannten Verbindungen der Formel (I) oder deren Salze, worin
A eine Gruppe der Formel (A2) bedeutet und
R⁶ wie oben für R⁶ für die Gruppe A = (A1) definiert ist und
R⁷ H, (C₁-C₄)Alkyl, (C₃-C₄)Alkenyl oder (C₃-C₄)Alkinyl,
   wobei jeder der letztgenannten 3 Reste unsubstituiert oder durch einen oder mehrere Reste aus der Gruppe, welche die Reste
   (1) Halogen, Cyano, (C₁-C₃)Alkoxy, (C₁-C₃)Alkylthio, (C₁-C₃)Haloalkoxy, (C₁-C₄)Alkanoyl, (C₁-C₄)Haloalkanoyl, [(C₁-C₄)Alkoxy]-carbonyl, [(C₁-C₄)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
   (2) Reste der Formel CO-NR^{a}R^{b}, worin jeder der Reste R^{a} und R^{b} unabhängig voneinander Wasserstoff, (C₁-C₃)Alkyl und (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder
      R^{a} und R^{b} gemeinsam mit dem sie verbindenden N-Atom einen gesättigten heterocyclischen Rest mit 3 bis 6 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
   (3) (C₃-C₆)Cycloalkyl, das unsubstituiert oder durch einen oder mehrere Reste aus der Gruppe Halogen und (C₁-C₃)Alkyl substituiert ist,
   (4) Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl substituiert ist,
      enthält, substituiert ist, bedeutet
      oder
R⁷ Phenyl, (C₃-C₆)Cycloalkyl oder einen gesättigten heterocyclischen Rest mit 3 bis 6 Ringatomen und 1 oder 2 Heteroringatomen aus der Gruppe N, O und S, wobei jeder der letztgenannten 3 Reste unsubstituiert oder durch einen oder mehrere Reste aus der Gruppe, welche die Reste
   (1) (C₁-C₄)Alkyl, Halogen, Cyano, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
   (2) Reste der Formel CO-NR^{a}R^{b}, worin jeder der Reste R^{a} und R^{b} unabhängig voneinander Wasserstoff, (C₁-C₃)Alkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet,
   (3) (C₃-C₆)Cycloalkyl, das unsubstituiert oder durch einen oder mehrere Reste aus der Gruppe Halogen und (C₁-C₃)Alkyl substituiert ist,
   (4) Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, CN, [(C₁-C₆)Alkoxyl-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl substituiert ist,
      enthält, substituiert ist, bedeutet,
      oder
R⁷ Formyl oder einen Rest der Formel CO-R, CO-OR, SO-R oder S(O)₂-R bedeutet,
   worin R jeweils für (C₁-C₄)Alkyl, (C₃-C₄)Alkenyl, (C₃-C₄)Alkinyl,
   wobei jeder der letztgenannten 3 Reste unsubstituiert oder durch einen oder mehrere Reste aus der Gruppe, welche die Reste
   (1) Halogen, Cyano, (C₁-C₃)Alkoxy, (C₁-C₃)Alkylthio, (C₁-C₃)Haloalkoxy, (C₁-C₄)Alkanoyl, (C₁-C₄)Haloalkanoyl, [(C₁-C₄)Alkoxy]-carbonyl, [(C₁-C₄)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
   (2) Reste der Formel CO-NR^{a}R^{b}, worin jeder der Reste R^{a} und R^{b} unabhängig voneinander Wasserstoff, (C₁-C₃)Alkyl und (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten heterocyclischen Rest mit 3 bis 6 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
   (3) (C₃-C₆)Cycloalkyl, das unsubstituiert oder durch einen oder mehrere Reste aus der Gruppe Halogen und (C₁-C₃)Alkyl substituiert ist,
   (4) Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl substituiert ist,
      enthält, substituiert ist, steht
      oder
   worin R für Phenyl oder (C₃-C₆)Cycloalkyl, wobei jeder der letztgenannten 2 Reste unsubstituiert oder durch einen oder mehrere Reste aus der Gruppe, welche die Reste
   (1) (C₁-C₄)Alkyl, Halogen, Cyano, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
   (2) Reste der Formel CO-NR^{a}R^{b}, worin jeder der Reste R^{a} und R^{b} unabhängig voneinander Wasserstoff, (C₁-C₃)Alkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet,
   (3) (C₃-C₆)Cycloalkyl, das unsubstituiert oder durch einen oder mehrere Reste aus der Gruppe Halogen und (C₁-C₃)Alkyl substituiert ist,
   (4) Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl substituiert ist,
      enthält, substituiert ist, steht,
      oder
R⁷ einen Rest der Formel CO-NR*R** bedeutet,
   worin jeder der Reste R* und R** unabhängig voneinander für
   Wasserstoff, (C₁-C₆)Alkyl, (C₂-C₆)Alkenyl oder (C₂-C₆)Alkinyl,
   wobei jeder der letztgenannten 3 Reste unsubstituiert oder durch einen oder mehrere Reste aus der Gruppe, welche die Reste
   (1) Halogen, Cyano, (C₁-C₃)Alkoxy, (C₁-C₃)Alkylthio, (C₁-C₃)Haloalkoxy, (C₁-C₄)Alkanoyl, (C₁-C₄)Haloalkanoyl, [(C₁-C₄)Alkoxyl-carbonyl, [(C₁-C₄)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
   (2) Reste der Formel CO-NR^{a}R^{b}, worin jeder der Reste R^{a} und R^{b} unabhängig voneinander Wasserstoff, (C₁-C₃)Alkyl und (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten heterocyclischen Rest mit 3 bis 6 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
   (3) (C₃-C₆)Cycloalkyl, das unsubstituiert oder durch einen oder mehrere Reste aus der Gruppe Halogen und (C₁-C₃)Alkyl substituiert ist,
   (4) Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl substituiert ist,
      enthält, substituiert ist, oder
   worin R für Phenyl, (C₃-C₆)Cycloalkyl, wobei jeder der letztgenannten 2 Reste unsubstituiert oder durch einen oder mehrere Reste aus der Gruppe, welche die Reste
   (1) (C₁-C₄)Alkyl, Halogen, Cyano, (C₁-C₃)Alkoxy, (C₁-C₃)Alkylthlo, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
   (2) Reste der Formel CO-NR^{a}R^{b}, worin jeder der Reste R^{a} und R^{b} unabhängig voneinander Wasserstoff, (C₁-C₃)Alkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet,
   (3) (C₃-C₆)Cycloalkyl, das unsubstituiert oder durch einen oder mehrere Reste aus der Gruppe Halogen und (C₁-C₃)Alkyl substituiert ist,
   (4) Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl substituiert ist,
      enthält, substituiert ist, steht,
      oder worin R* und R** gemeinsam mit dem sie verbindenden N-Atom einen gesättigten oder teilweise ungesättigten (nicht aromatischen) heterocyclischen
      Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bilden.

Weiter bevorzugt bedeutet dabei R⁶ Wasserstoff, (C₁-C₄)Alkyl oder Cyclopropyl, insbesondere Wasserstoff oder Methyl, und
bedeutet R⁷ vorzugsweise Wasserstoff, (C₁-C₄)Alkyl, (C₁-C₄)Alkanoyl oder [(C₁-C₄)Alkoxy]-carbonyl, insbesondere Wasserstoff, Methyl oder Ethyl.

Weiter bevorzugt sind die oben genannten Verbindungen der Formel (I) oder die jeweils bezüglich der Reste Q, R¹, R², R³, R⁴ und R⁵ und deren Kombinationen bevorzugt genannten Verbindungen der Formel (I) oder deren Salze, worin
A eine Gruppe der Formel (A3), (A4) oder (A5) bedeutet und
R⁸ und R⁹ jeweils unabhängig voneinander Reste aus den Gruppen (i), (ii), (iii), (iv), (v), (vi) und (vii) bedeuten, wobei die Gruppen (i) bis (vii) die Reste
   (i) Wasserstoff, Halogen, Cyano und Nitro
   (ii) (C₁-C₄)Alkyl, (C₂-C₄)Alkenyl und (C₂-C₄)Alkinyl,
      wobei jeder der letztgenannten 3 Reste unsubstituiert oder durch einen oder mehrere Reste aus der Gruppe, welche die Reste
      (1) Halogen, Cyano, (C₁-C₃)Alkoxy, (C₁-C₃)Alkylthio, (C₁-C₃)Haloalkoxy, (C₁-C₄)Alkanoyl, (C₁-C₄)Haloalkanoyl, [(C₁-C₄)Alkoxy]-carbonyl, [(C₁-C₄)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
      (2) Reste der Formel CO-NR^{a}R^{b}, worin jeder der Reste R^{a} und R^{b} unabhängig voneinander Wasserstoff, (C₁-C₃)Alkyl und (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten heterocyclischen Rest mit 3 bis 6 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
      (3) (C₃-C₆)Cycloalkyl, das unsubstituiert oder durch einen oder mehrere Reste aus der Gruppe Halogen und (C₁-C₃)Alkyl substituiert ist,
      (4) Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl substituiert ist, enthält, substituiert ist,
   (iii) Phenyl, (C₃-C₆)Cycloalkyl, wobei jeder der letztgenannten 2 Reste unsubstituiert oder durch einen oder mehrere Reste aus der Gruppe, welche die Reste
      (1) (C₁-C₄)Alkyl, Halogen, Cyano, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
      (2) Reste der Formel CO-NR^{a}R^{b}, worin jeder der Reste R^{a} und R^{b} unabhängig voneinander Wasserstoff, (C₁-C₃)Alkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet,
      (3) (C₃-C₆)Cycloalkyl, das unsubstituiert oder durch einen oder mehrere Reste aus der Gruppe Halogen und (C₁-C₃)Alkyl substituiert ist,
      (4) Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl substituiert ist, enthält, substituiert ist,
   (iv) (C₁-C₄)Alkoxy und (C₁-C₄)Alkthio, wobei jeder der letztgenannten beiden Reste unsubstituiert oder durch einen oder mehrere Reste aus der Gruppe Halogen, CN und (C₁-C₃)Alkoxy substituiert ist,
   (v) (C₃-C₆)Cycloalkoxy, das unsubstituiert oder durch einen oder mehrere Reste aus der Gruppe Halogen, CN, (C₁-C₃)Alkyl, (C₁-C₃)Haloalkyl und (C₁-C₃)Alkoxy substituiert ist,
   (vi) [(C₁-C₆)Alkoxy]-carbonyl, [(C₁-C₆)Haloalkoxy]-carbonyl, (C₁-C₄)Alkylsulfinyl, (C₁-C₄)Haloalkylsulfinyl, (C₁-C₄)Alkylsulfonyl und (C₁-C₄)Haloalkylsulfonyl,
   (vii) Reste der Formeln -CO-NR*R**, -O-CO-NR*R**, -O-CO-NR*R** und O-CO-NR*R**,
      wobei in den letztgenannten 4 Formeln jeder der Reste R* und R** unabhängig voneinander für
      Wasserstoff, (C₁-C₄)Alkyl, (C₂-C₄)Alkenyl oder (C₂-C₄)Alkinyl,
      wobei jeder der letztgenannten 3 Reste unsubstituiert oder durch einen oder mehrere Reste aus der Gruppe, welche die Reste
      (1) Halogen, Cyano, (C₁-C₃)Alkoxy, (C₁-C₃)Alkylthio, (C₁-C₃)Haloalkoxy, (C₁-C₄)Alkanoyl, (C₁-C₄)Haloalkanoyl, [(C₁-C₄)Alkoxy]-carbonyl, [(C₁-C₄)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
      (2) Reste der Formel CO-NR^{a}R^{b}, worin jeder der Reste R^{a} und R^{b} unabhängig voneinander Wasserstoff, (C₁-C₃)Alkyl und (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkytsulfonyl bedeutet oder R^{a} und R^{b} gemeinsam mit dem sie verbindenden N-Atom einen gesättigten heterocyclischen Rest mit 3 bis 6 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
      (3) (C₃-C₆)Cycloalkyl, das unsubstituiert oder durch einen oder mehrere Reste aus der Gruppe Halogen und (C₁-C₃)Alkyl substituiert ist,
      (4) Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl substituiert ist,
         enthält, substituiert ist, oder für Phenyl oder (C₃-C₆)Cycloalkyl, wobei jeder der letztgenannten 2 Reste unsubstituiert oder durch einen oder mehrere Reste aus der Gruppe, welche die Reste

      (1) (C₁-C₄)Alkyl, Halogen, Cyano, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloaikanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
      (2) Reste der Formel CO-NR^{a}R^{b}, worin jeder der Reste R^{a} und R^{b} unabhängig voneinander Wasserstoff, (C₁-C₃)Alkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet,
      (3) (C₃-C₆)Cycloalkyl, das unsubstituiert oder durch einen oder mehrere Reste aus der Gruppe Halogen und (C₁-C₃)Alkyl substituiert ist,
      (4) Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl substituiert ist,
         enthält, substituiert ist, steht,
         oder worin R* und R** gemeinsam mit dem sie verbindenden N-Atom einen gesättigten oder teilweise ungesättigten (nicht aromatischen) heterocyclischen Rest mit 3 bis 6 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bilden,
         enthalten, oder
         R⁸ und R⁹ bedeuten gemeinsam eine (C₂-C₅)Alkylen-Kette, welche durch ein oder mehrere Heteroatome aus der Gruppe N, O und S unterbrochen sein kann und welche unsubstituiert oder durch einen oder mehrere Reste aus der Gruppe Halogen und (C₁-C₄)Alkyl substituiert ist.

Weiter bevorzugt bedeuten dabei unabhängig voneinander
R⁸ Wasserstoff, Halogen, (C₁-C₄)Alkyl, insbesondere Wasserstoff, Chlor oder Methyl, R⁹ Wasserstoff, Halogen, (C₁-C₄)Alkyl, insbesondere Wasserstoff, Chlor oder Methyl, oder
R⁸ und R⁹ bedeuten vorzugsweise gemeinsam eine (C₂-C₅)Alkylen-Kette, welche durch ein oder mehrere Heteroatome aus der Gruppe N, O und S unterbrochen sein kann und welche unsubstituiert oder durch einen oder mehrere Reste aus der Gruppe Halogen und (C₁-C₄)Alkyl substituiert ist, insbesondere bedeuten gemeinsam eine unsubstituierte (C₂-C₅)Alkylen-Kette, wie beispielsweise -CH₂CH₂-, -CH₂CH₂CH₂-, -CH₂CH₂CH₂CH₂- oder -CH₂CH₂CH₂CH₂CH₂-.

Bevorzugt sind auch die Verbindungen der Formel (I) oder deren Salze, worin die Restebedeutungen gemäß zwei oder mehreren der genannten bevorzugten Bedeutungen ausgewählt worden sind. Dabei sind besonders die Verbindungen der Formeln (I) oder deren Salze bevorzugt, in welchen die Reste R¹ bis R⁹, A und Q die in den Beispieltabellen für einzelen Verbindungen der Formel (I) verwendeten Restebedeutungen und deren entsprechenden generischen Restebedeutungen aufweisen.

Gegenstand der Erfindung sind auch Verfahren zur Herstellung der neuen Verbindungen der Formel (I) und deren Salze, dadurch gekennzeichnet, dass man
(a) eine Verbindung der Formel (II),

   A-A-H (II)

   worin Q und A wie in der herzustellenden Verbindung der Formel (I) definiert sind, mit einer Verbindung der Formel (III), 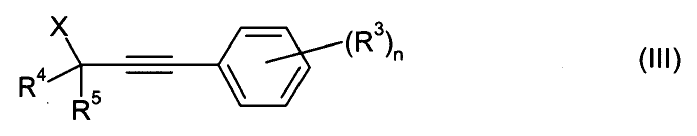 worin (R³)ₙ, R⁴ und R⁵ wie in der herzustellenden Verbindung der Formel (I) definiert sind und X eine Abgangsgruppe bedeutet, zur Verbindung der Formel (I) oder deren Salz umsetzt
   oder
(b) eine Verbindung der Formel (IV), 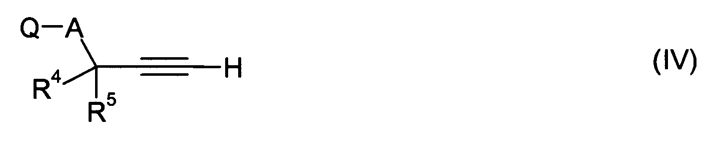 worin Q, A, R⁴ und R⁵ wie in der herzustellenden Verbindung der Formel (I) definiert sind, mit einer Verbindung der Formel (V), 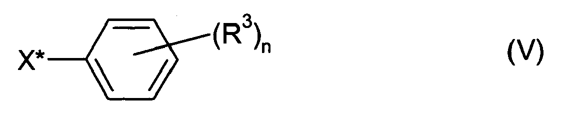 worin (R³)ₙ wie in der herzustellenden Verbindung der Formel (I) definiert ist und X* eine Abgangsgruppe bedeutet, zur Verbindung der Formel (I) oder deren Salz umsetzt
   oder
(c) für den Fall, dass A eine Gruppe der Formel (A1) bedeutet, eine Verbindung der Formel (VI), 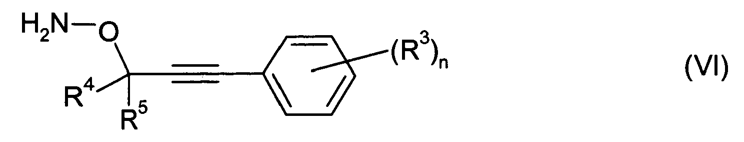 worin (R³)ₙ, R⁴ und R⁵ wie in der herzustellenden Verbindung der Formel (I) definiert sind, mit einer Verbindung der Formel (VII)

   Q-CO-R⁶ (VII)

   worin Q und R⁶ wie in der herzustellenden Verbindung der Formel (I) definiert sind, zur Verbindung der Formel (I) oder deren Salz, worin A eine Gruppe der Formel (A1) 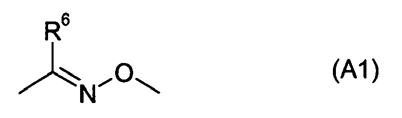 bedeutet, umsetzt
   oder
(d) für den Fall, dass A eine Gruppe der Formel (A2) bedeutet, eine Verbindung der Formel (VIII), 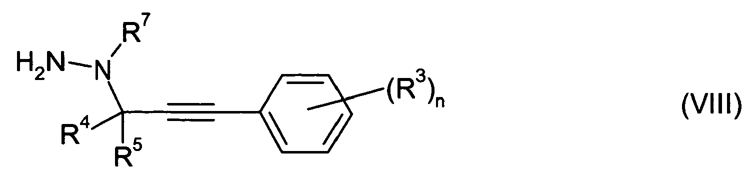 worin (R³)ₙ, R⁴, R⁵ und R⁷ wie in der herzustellenden Verbindung der Formel (I) definiert sind, mit einer Verbindung der Formel (VII)

   Q-CO-R⁶ (VII)

   worin Q und R⁶ wie in der herzustellenden Verbindung der Formel (I) definiert sind, zur Verbindung der Formel (I) oder deren Salz, worin A eine Gruppe der Formel (A2) 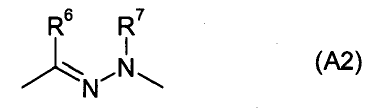 bedeutet, umsetzt
   oder
(e) für den Fall, dass A eine Gruppe der Formel A = (A3), (A4) oder (A5) bedeutet, eine Verbindung der Formel (IX), 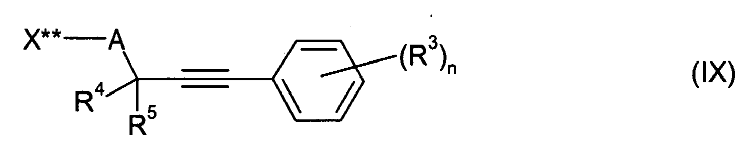 worin A, (R³)ₙ, R⁴ und R⁵ wie in der herzustellenden Verbindung der Formel (I) definiert sind und X** eine Abgangsgruppe bedeutet, mit einer Verbindung der Formel (X)

   Q-B(OH)₂ (X)

   unter Bedingungen einer Kreuzkupplung zur Verbindung der Formel (I) oder deren Salz, worin A die Gruppe der Formel (A3), (A4) oder (A5) bedeutet, 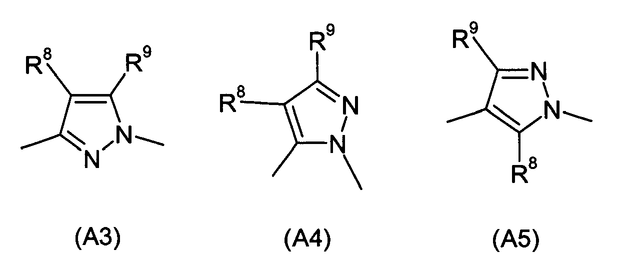 wobei R⁸ und R⁹ wie in der herzustellenden Verbindung der Formel (I) definiert sind, umsetzt oder
(f) für den Fall, dass A eine Gruppe der Formel A = (A3) oder (A4) bedeutet, eine Verbindung der Formel (VIII'), 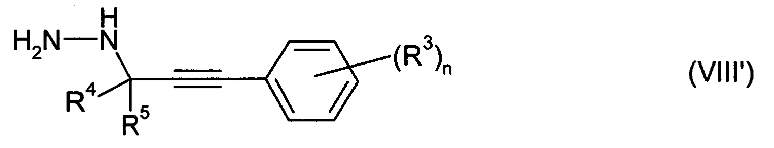 worin (R³)ₙ, R⁴ und R⁵ wie in der herzustellenden Verbindung der Formel (I) definiert sind, mit einer Verbindung der Formel (XI), 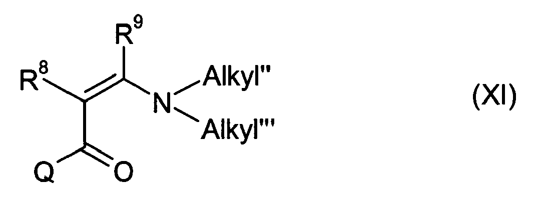 worin Q, R⁸ und R⁹ wie in der herzustellenden Verbindung der Formel (I) definiert sind und Alkyl" und Alkyl"' Alkylreste mit 1 bis 10 C-Atomen bedeuten, zur Verbindung der Formel (I) oder deren Salz, worin A die Gruppe der Formel (A3) oder (A4) bedeutet, 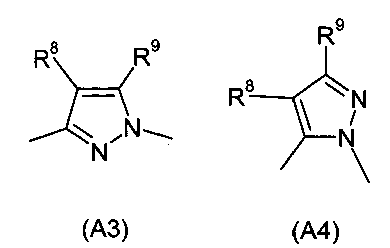 wobei R⁸ und R⁹ wie in der herzustellenden Verbindung der Formel (I) definiert sind, umsetzt oder
(g) für den Fall, dass A eine Gruppe der Formel A = (A5) bedeutet, eine Verbindung der Formel (VIII'), 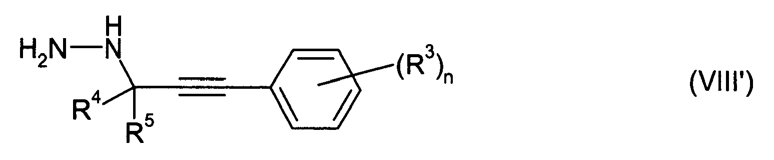 worin (R³)ₙ, R⁴ und R⁵ wie in der herzustellenden Verbindung der Formel (I) definiert sind, mit einer Verbindung der Formel (XII), 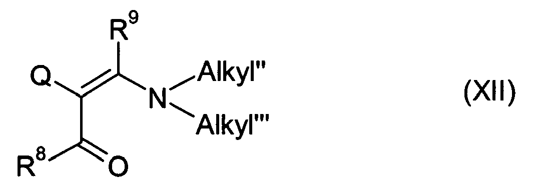 worin Q, R⁸ und R⁹ wie in der herzustellenden Verbindung der Formel (I) definiert sind und Alkyl" und Alkyl"' Alkylreste mit 1 bis 10 C-Atomen bedeuten, zur Verbindung der Formel (I) oder deren Salz, worin A die Gruppe der Formel (A5) bedeutet, 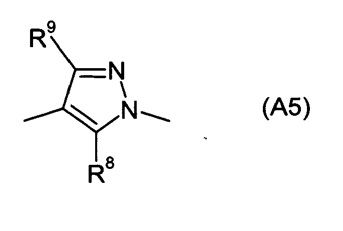 wobei R⁸ und R⁹ wie in der herzustellenden Verbindung der Formel (I) definiert sind, umsetzt.

Die Ausgangsverbindungen der Formeln (II), (III), (IV), (V), (VI), (VII), (VIII), (VIII'), (IX), (X), (XI) und (XII) sind in der Regel bekannt oder können analog bekannten Verfahren aus bekannten Ausgangsmaterialien hergestellt werden.

Die im Nachfolgenden beschriebenen Synthesewege benutzen Reaktionsdurchführungen sowie Aufarbeitungs- und Reinigungsmethoden wie sie üblicherweise in der organisch-chemischen Literatur anzutreffen sind. Reaktionen finden im Allgemeinen in Lösung statt, wobei beispielsweise alkoholische Lösungsmittel wie Methanol, Ethanol etc., Kohlenwasserstoffe wie Hexan, Benzol, Toluol etc., Ether wie Diethylether, Tetrahydrofuran etc, Chlorkohlenwasserstoffe wie Dichlormethan, Chloroform etc., amidhaltige Lösungsmittel wie N,N-Dimethylformamid etc. oder schwefelhaltige Lösungsmittel wie Dimethylsulfoxid, Sulfolan etc. zum Einsatz kommen können. Im Einzelfall können Reaktionen auch in einer Suspension, Emulsion oder in fester Phase stattfinden. Als Reaktionstemperatur kann ein Bereich zwischen -200°C und 250°C in Frage kommen, im Allgemeinen liegt die Reaktionstemperatur jedoch zwischen -78°C und 150°C. Zur Aufarbeitung wird üblicherweise eine Extraktion mit zwei oder mehreren nicht miteinander mischbaren Lösungsmitteln vorgenommen. Alternativ können unter anderem Festphasenextraktionen oder das Ausfällen der Produkte in einem geeigneten Lösungsmittel bzw. Lösungsmittelgemisch zur Anwendung kommen. Die Reinigung der Rohprodukte erfolgt üblicherweise durch chromatographische Verfahren wie Säulenchromatographie, HPLC etc., durch Destillation oder Kristallisation aus geeigneten Lösungsmitteln. Andere Verfahren sind jedoch nicht ausgeschlossen.

Gemäß der Verfahrensvariante (a) werden eine Verbindung der Formel (II) (= Verbindung (II)) und eine Phenol-propargyl-verbindung der Formel (III) (= Verbindung (III)), welche eine Abgangsgruppe X trägt, miteinander umgesetzt.

Als Abgangsgruppe kommen beispielsweise Halogenide in Betracht, insbesondere Chlorid oder Bromid, oder auch eine mit Organosulfonsäuren veresterte Hydroxygruppe, wie zum Beispiel eine Methylsulfonatgruppe oder eine Tolylsulfonatgruppe. Denkbar ist zudem der Einsatz anderer Estergruppen organischer wie anorganischer Säuren als Abgangsgruppe. Beispielsweise können Phosphate verwendet werden, wie analog unter anderem in Organic Letters 2004, 6(24), 4631-4634 beschrieben. In einigen Fällen können die Verbindungen (III), worin X = Hydroxy bedeutet, (= Verbindungen (III')) eingesetzt werden, wenn übliche Kondensationshilfsmittel verwendbar sind.

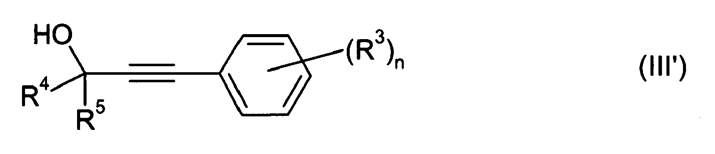

So kommen in Abhängigkeit von der Natur der Gruppe A in Verbindung (II) Umsetzungen in Frage, bei denen eine noch reaktivere Abgangsgruppe intermediär aus der Hydroxygruppe gebildet wird. So können beispielsweise die Verbindungen (II) und (III') direkt miteinander zu Verbindungen (I) unter Zuhilfenahme von Dialkylazodicarboxylaten und Triphenylphosphin umgesetzt werden, wobei - wie in Synthesis 1981, 1-28 beschrieben - als reaktive Zwischenstufe aus dem Phenylpropargylalkohol ein quaternäres Alkoxytriphenylphosphonium-Salz gebildet wird.

Die Verbindungen (III), worin X = Halogen oder eine reaktive Estergruppe bedeutet, kann ausgehend von den Phenylpropargylalkoholen (III') (= Verbindung der Formel (III), worin X = OH) durch Umsetzung mit PBr₃ (analog Journal of the Chemical Society 1952, 2005; Abgangsgruppe X = Br), mit PPh₃, CCl₄ (analog Journal of the American Chemical Society 1974, 96, 3684; Abgangsgruppe X = Cl) oder mit p-Tolylsulfonylchlorid (Abgangsgruppe X = Tosylat) hergestellt werden.

Zur Synthese geeigneter Phenylpropargylverbindungen (III') können Reaktionen zum Einsatz kommen, die jeweils eine Bildung der Einfachbindungen neben der CC-Dreifachbindung bewirken So lassen sich Propargylalkohole (XIII) mit den Phenylderivaten (V), 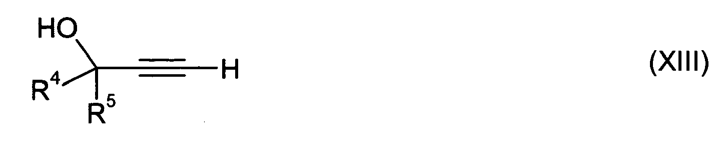 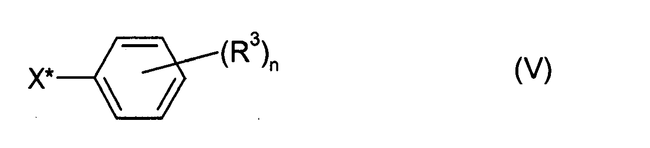 worin X* eine Abgangsgruppe bedeutet, nach bekannten Verfahren, wie beispielsweise in K. Sonogashira, Comprehensive Organic Synthesis 1991, Band 3, Seite 521 beschrieben, umsetzen. Die Abgangsgruppe X* kann dabei beispielsweise ein Halogenid, vorzugsweise Iodid oder Bromid, oder ein Sulfonat sein kann. Zur Durchführung dieser Umsetzung wird ein Übergangsmetallkatalysator, in der Regel ein Palladiumkomplex, sowie eine Base verwendet. Als Base können diverse Amine oder Phosphine zum Einsatz kommen. Reaktionen, die insbesondere unter Sonogashira-Bedingungen ablaufen, können in Anwesenheit zusätzlicher Übergangsmetallsalze wie zum Beispiel Kupferiodid ablaufen. Die für die hier beschriebenen Umsetzungen benötigten Phenylverbindungen (V) sind in großer Zahl kommerziell erhältlich oder können durch übliche Grundreaktionen der präparativen organischen Chemie hergestellt werden. Die Einführung eines lodsubstituenten an einen Aromaten kann beispielsweise durch direkte elektrophile lodierung oder ausgehend von wohlfeilen Anilinen über Diazotierung und Umsetzung des Diazoniumsalzes mit Kaliumiodid erfolgen.

Zur Darstellung der Phenylpropargylalkohole (III') kann man alternativ auch Phenylalkine (XIV) mit Ketonen bzw. Aldehyden (XV) umsetzen.

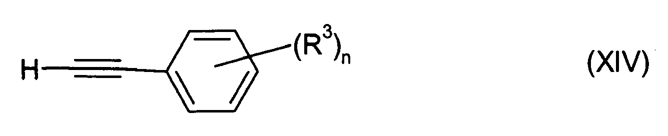

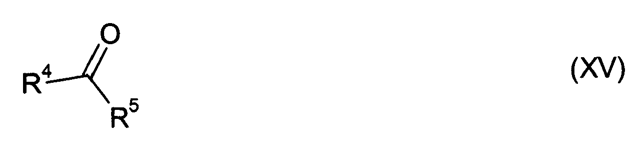

Das Phenylalkin (XIV) wird dabei in der Regel mit einer starken Base deprotoniert werden, wobei beispielsweise Grignard- oder Organolithiumverbindungen geeignet sind. Aber auch die Verwendung von Alkalimetall-Amid-Basen, wie zum Beispiel Lithiumdiisopropylamid oder Lithiumhexamethyldisilazid, Alkalimetallalkoholaten, wie zum Beispiel Kalium-t-butylat, oder anderen starken organischen wie anorganischen Basen ist denkbar. Umsetzungen von Alkinen mit Ketonen bzw. Aldehyden unter basischen Bedingungen sind an zahlreichen Stellen in der Literatur beschrieben, z.B.: Tetrahedron 2005, 61(15), 3759-3769; Advanced Synthesis & Catalysis 2004, 346(9+10), 1090-1092, Ultrasonics Sonochemistry 2005, 12(3), 161-163. Darüberhinaus sind durch Übergangsmetalle katalysierte Prozesse bekannt: Organic Letters 2006, 8(1), 67-69.
Analog zur Darstellung der Verbindungen (III') können Phenylalkine (XIV) durch Reaktion von Phenylverbindungen (V) mit Acetylen unter Sonogashira-Bedingungen erhalten werden. Bei der Umsetzung kann als Acetylenäquivalent auch ein Ethinylsilan zum Einsatz kommen, das einem mono-geschützten Acetylen entspricht und nach der Umsetzung mit Verbindung (V) wie in Th. W. Greene, P. G. M. Wuts "Protective Groups in Organic Synthesis", John Wiley & Sons, 3. Auflage, 1999, Seite 654-660 beschrieben entschützt werden kann. Zusätzlich kommen Reaktionssequenzen zur Synthese von Phenylalkinen in Frage, die den Aufbau der Dreifachbindung zum Ziel haben. Solche Reaktionen sind in großer Zahl in R. C. Larock "Comprehensive Organic Transformations" VCH Verlagsgesellschaft, 1. Auflage, 1989, Seite 298-296 referiert.

Je nach Art der Reste R⁴ und R⁵ sind Reaktionssequenzen zum Aufbau der Verbindungen (III') möglich, die diese Reste gleichzeitig oder stufenweise als Nukleophile durch Addition an die CO-Doppelbindung von beispielsweise Phenylpropargylestem oder äquivalenten Substraten der Formel (XVI) einführen, 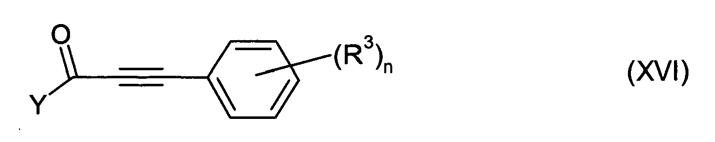 worin Y eine Abgangsgruppe darstellt. So können Verbindungen (XVI) mit Organometallverbindungen der Formel M⁺(R⁴)⁻ zunächst unter Austausch der Abgangsgruppe Y durch den Rest R⁴ zur Verbindungen (XVII) 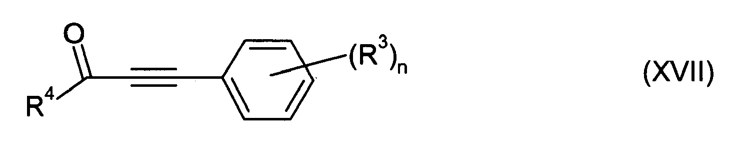 als Zwischenstufe umgesetzt und anschließend die Zwischenstufe mit Organometallverbindungen der Formel M⁺(R⁵)⁻ zu den Propargylalkoholen (III') weiter umgesetzt werden.

Für den Fall, dass die Reste R⁴ und R⁵ gleich sind, können sie in einem Schritt eingeführt werden, beispielsweise durch Einwirken von mindestens zwei Moläquivalenten einer Organometallverbindung M⁺(R⁴)⁻ auf einen entsprechenden Propargylester (XVI), wobei Alkylester (Y = Alkoxy), Haloalkylester (Y = Haloalkoxy), Arylester (Y = Aryloxy) oder jeder andere Ester zum Einsatz kommen kann.

Das Metallkation M⁺ der Organometallverbindung kann je nach den gewählten Reaktionsbedingungen unterschiedlich sein, bevorzugt sind aber Kationen von Alkalimetallen, wie Natrium und Lithium, oder Kationen von Erdalkalimetallen, wie Magnesium, jeweils in ihren stabilsten Oxidationsstufen.
Für den Fall, dass die Reste R⁴ und R⁵ verschieden sind, sollte das Keton bzw. der Aldehyd (XVII) als Zwischenstufe des Prozesses isolierbar sein. Dies gelingt bei richtiger Wahl der Reaktionsbedingungen, des Restes Y sowie der Organometallspezies M⁺(R⁴)⁻ gegebenenfalls unter Verwendung eines geeigneten Katalysators.
Aus der Literatur stehen mehrere Optionen für derartige Umsetzungen zur Verfügung. So können zum Beispiel nach EP-A-461483 Säurechloride (Y = Cl) mit Alkylzink-Verbindungen (M = Zn; R⁴ = Alkyl) zur Reaktion gebracht werden, wobei ein Palladiumkomplex als Katalysator eingesetzt wird.
Alternativ können auch N-Methoxy-N-methylamide (XVI; Y = -N(OMe)Me) mit Organometallverbindungen wie Grignardverbindungen oder Hydridreagenzien wie Lithiumaluminiumhydrid umgesetzt werden, um zur Zwischenstufe (XVII) zu gelangen (siehe J. für Praktische Chem / Chemiker-Zeitung 1997, 339(6), 517-524). Die Umsetzung der Zwischenstufe (XVII) zur gewünschten Phenylpropargylverbindung (III') erfolgt analog der beschriebenen einstufigen Umsetzung von (XVI) nach (XVII) mit dem Unterschied, dass eine geringere Menge an Organometallverbindung verwendet werden kann.

Die Herstellverfahren für die nach Herstellvariante (a) benötigten Verbindungen der Formel (II) unterscheiden sich je nach Art der Gruppe A. Die Natur der Gruppe Q sowie die mit ihr verbundenen Reste R¹ beeinflussen die Reaktionen zur Herstellung der Verbindungen (II) in der Regel nicht.
Im Fall von A gleich (A1) kann zur Herstellung der Verbindung (II) (= Verbindung der Formel (IIa)) ein Aldehyd oder Keton der Formel (VII) mit Hydroxylamin oder einem seiner Salze, z. B.: Hydroxylaminhydrochlorid, umgesetzt werden (siehe Schema 1)

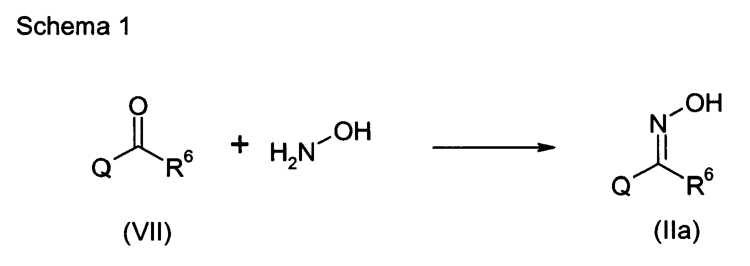

Die Reaktion kann prinzipiell in den üblichen protischen wie auch aprotischen Lösemitteln sowohl unter Basen- als auch Säurekatalyse durchgeführt werden, wobei die Reaktionstemperatur vorzugsweise zwischen 20 °C und 150 °C liegt. Bevorzugt sind dabei Bedingungen mit kurzkettigen aliphatischen Alkoholen, wie beispielsweise Ethanol oder Propanol als Lösungsmittel, Natriumacetat als Katalysator sowie Rückflusstemperatur des jeweiligen Lösungsmittels als Reaktionstemperatur.

Ist die Gruppe A gleich (A2) wird wie in Schema 2 dargestellt der Aldehyd- bzw. Ketonbaustein (VII) mit einem Hydrazin (XVIII) zur Verbindung (IIb) umgesetzt, 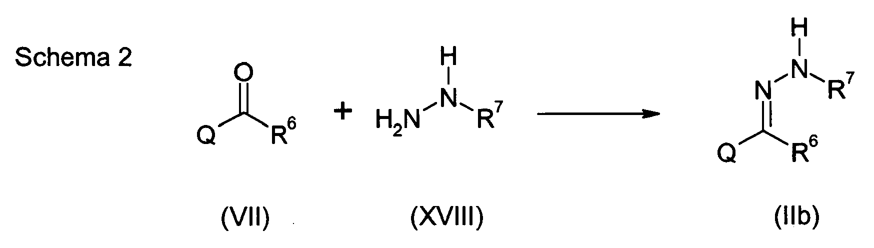 wobei die Reste Q, R⁶ und R⁷ wie in Formel (I) definiert sind. Soll im Endprodukt der Formel (I) der Rest R⁷ gleich Wasserstoff sein, kann es sinnvoll sein, während der Umsetzung gemäß Schema 2 für R⁷ eine Gruppe zu wählen, die als Schutzgruppe fungieren und auf einer späteren Stufe abgespalten werden kann. Damit könnte vermieden werden, dass bei der Reaktion der Verbindung (IIb) mit einer Verbindung (III) für den Fall von R⁷ gleich Wasserstoff Produkte aus einer doppelten Alkylierung am freien Hydrazonstickstoff auftreten. Gruppen, die dem hier beschriebenen Sinne als Schutzgruppen verwendet werden können, sind in großer Zahl in Th. W. Greene, P. G. M. Wuts "Protective Groups in Organic Synthesis", John Wiley & Sons, 3. Auflage, 1999, Seite 494-615 beschrieben und umfassen beispielsweise einen 2-Trimethylsilylethoxycarbonyl-Rest, einen tert-Butyloxycarbonyl-Rest, einen 9-Fluorenylmethoxycarbonyl-Rest oder andere. Die Umsetzungsbedingungen für eine Reaktion in Schema 2 sind stark abhängig vom sterischen Anspruch des Restes R⁶. Sie können demzufolge in einem weiten Bereich variieren. So kann es beispielsweise ausreichend sein, die Edukte (VII) und (XVIII) bei Temperaturen von -20°C bis 25°C mit oder ohne Lösemittel zu vermischen, um eine ausreichende Umsetzung zum Produkt (IIb) zu bewirken. Demgegenüber kann aber auch das Erhitzen in einem hochsiedenden Lösemittel bei Temperaturen von 150°C bis 200°C unter gleichzeitiger azeotroper Entfernung von Wasser notwendig sein. In vielen Fällen ist die Anwesenheit einer Säure als Katalysator hilfreich. Beispiele für solche Säuren sind unter anderen: p-Toluolsulfonsäure, Camphersulfonsäure, Pyridinium p-Tolylsulfonat.

Für den Fall, dass die Gruppe A gleich (A3) oder (A4) ist, kann die Synthese des Bausteins (II) von Ketonen der Formel (XIX) ausgehen. Diese Ketone können mit Estern oder anderen Carbonsäurederivaten in 1,3-Diketone oder äquivalente, von 1,3-Diketonen abgeleitete Strukturen überführt werden. Eine Übersicht hierzu liefert beispielsweise Organic Reactions 1954, 8, Seite 59ff. So können beispielsweise Ketone der Formel (XIX) leicht mit N,N-Dialkylamid-Acetalen (XX) umgesetzt werden, wobei ein Aminoalkylidenketon der Formel (XI) ensteht. Deren Kondensation mit Hydrazin liefert die Pyrazolbausteine der Formel (IIc) (siehe Schema 3).

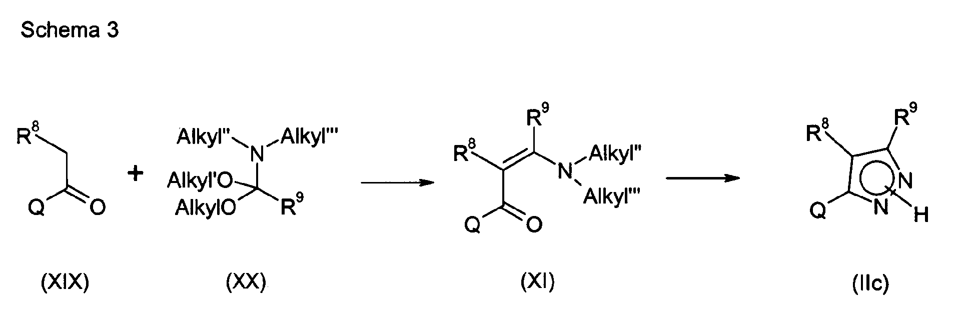

Die hier beschriebene Synthese der Bausteine (IIc), die ein Ausgangsmaterial für eine weitere Umsetzung nach Herstellvariante (a) darstellen, gehört zu den Standardverfahren der organischen Heterozyklenchemie und ist neben anderen Verfahren ausführlich beispielsweise von K. Kirschke in Houben-Weyl, Methoden der Organischen Chemie, Band E8b, Hetarene III/Teil2, Georg Thieme Verlag Stuttgart, 1994, Seite 399ff beschrieben.
Die Umsetzung Der Pyrazole (IIc) gemäß Herstellvariante (a) liefert je nach sterischer wie elektronischer Natur der Reste Q, R⁸ und R⁹ Gemische bestehend aus erfindungsgemäßen Verbindungen der Formel (I), in denen die Gruppe A zum einen gleich (A3) und zum anderen gleich (A4) ist. Diese Gemische können im Allgemeinen durch übliche Trennverfahren der präparativen organischen Chemie, wie zum Beispiel Chromatographie, in ihre Einzelbestandteile aufgetrennt werden, wodurch diese rein erhalten werden können.

Für den Fall, dass die Gruppe A der erfindungsgemäßen Verbindungen der allgemeinen Formel (I) gleich (A5) ist, können die für die Synthese der Verbindungen (I) nach Herstellvariante (a) benötigten Verbindungen der Formel (IId) in analoger Weise zu den Verbindungen (IIc) hergestellt werden. Schema 4 verdeutlicht dies.

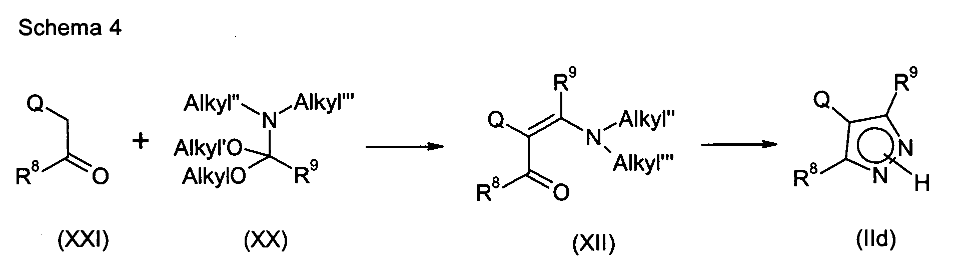

Der Unterschied im Vergleich zu Schema 3 ist dabei das Keton der Formel (XXI) als Ausgangsverbindung, welche mit demselben Reagenz (XX) zum regioisomeren Zwischenprodukt (XII) und dem regioisomeren Produkt (IId) führt.
Auch hier können bei der weiteren Umsetzung gemäß Herstellung von Verbindung (I) nach Variante (a) bei Ungleichheit von R⁸ und R⁹ Gemische auftreten, die im Allgemeinen durch übliche Trennverfahren aufzutrennen sind.

Alternativ zu den in Schema 3 und Schema 4 beschriebenen Verfahren, die auf einem Aufbau des Pyrazolringsystems basieren, sind auch Synthesestrategien möglich, bei denen die aromatischen Ringverbindungen Q-H nach Überführung in Borsäurederivate (X) mit den Pyrazolen der Gruppen (A3) bis (A5) im Sinne einer übergangsmetallkatalysierten Kreuzkupplungsreaktion verknüpft werden. Beispielhaft ist dies in Analogie zu literaturbeschriebenen Reaktionen - siehe zum Beispiel: Angew. Chem. (Int. Ed.) 2004, 43(14), 1871-1876 - in Schema 5 für die Gruppe (A5) verdeutlicht. 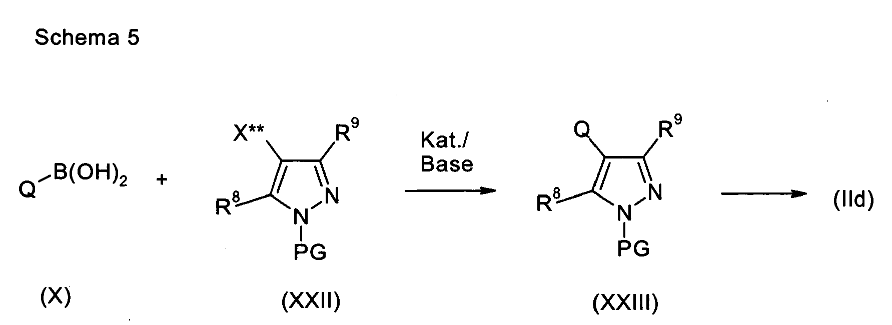 X** ist hierbei eine Abgangsgruppe, insbesondere Brom oder Iod.

Sinnvollerweise ist der Pyrazolring vor der Kreuzkupplung mit einer Schutzgruppe (PG) zu versehen, die nach der Kreuzkupplung wieder abgespalten werden kann. Als Schutzgruppe eignet sich unter anderen eine Benzylgruppe, deren Abspaltung zum Beispiel hydrogenolytisch erfolgen kann.

Die hier beschriebenen Verfahren zur Synthese der erfindungsgemäßen Verbindungen der Formel (I) bauen die Zielmoleküle schrittweise aus einzelnen Bausteinen auf, wobei der letzte Schritt jeweils in der Knüpfung der propargylischen Bindung an der Gruppe A besteht (Herstellvariante (a)). Alternativ sind allerdings auch Syntheseverfahren möglich, in denen die Reihenfolge der Schritte, das heißt, die Reihenfolge der Bindungsbildung zwischen den einzelnen Bausteinen ausgetauscht ist. So kann als letzter Schritt beispielsweise auch die Bindung zwischen Dreifachbindung und Phenylring in Formel (I) geknüpft werden (Herstellvariante (b)).

Gemäß der Verfahrensvariante (b) werden ein Propargylderivat der Formel (IV) (= Verbindung (IV)) und ein Phenylderivat der Formel (V) (= Verbindung (V)), welches eine Abgangsgruppe X* trägt, miteinander umgesetzt. Als Abgangsgruppe kommt beispielsweise ein Halogenid, vorzugsweise Iodid oder Bromid, oder ein Sulfonat in Betracht. Die Reaktion der Verbindungen (IV) und (V) zur Verbindung (I), bei der die Knüpfung der Bindung zwischen dem endständigen C-Atom der Dreifachbindung und der Phenyleinheit erfolgt, kann unter den Bedingungen der Sonogashira-Reaktion oder einer analogen Umsetzung durchgeführt werden, wie es bereits oben für die analoge Umsetzung der Verbindungen (V) und (XI) beschrieben ist.
Der für die Synthesesequenz notwendige Baustein, die Verbindung der Formel (IV), kann ausgehend von der Verbindung (II) durch Umsetzung mit einem geeigneten Propargylbaustein (XXIV) hergestellt werden (siehe Schema 6), 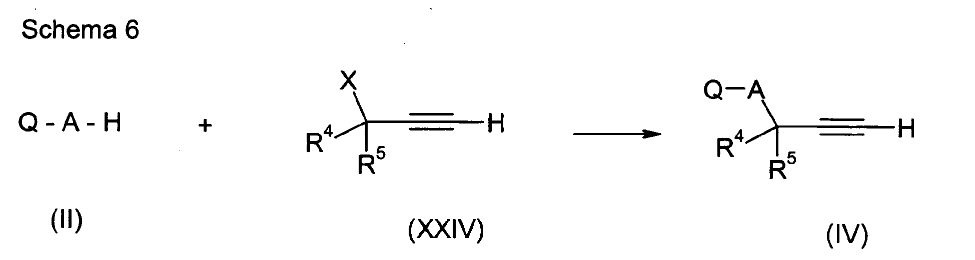 wobei in den Formeln Q, A, R⁴ und R⁵ wie in Formel (I) definiert sind und X eine Abgangsgruppe darstellt, beispielsweise ein Halogenid, vorzugsweise Iodid oder Bromid, oder ein Sulfonat. Wenn R⁴ und R⁵ jeweils H bedeuten, kommt als Verbindung (XXIV) beispielsweise Propargylbromid in Betracht.

Alternativ können für bestimmten Gruppen A = (A1) bis (A5) unterschiedliche Synthesewege zweckmäßig sein. Beispielsweise kann im Falle A = (A1) die Herstellung von Verbindung (IV), worin A = (A1), (= Verbindung (IVa)) ausgehend von dem Keton bzw. Aldehyd (VII) durch Kondensation mit einem geeigneten O-Propargyl-hydroxylamin (XXV) erfolgen (siehe Schema 7).

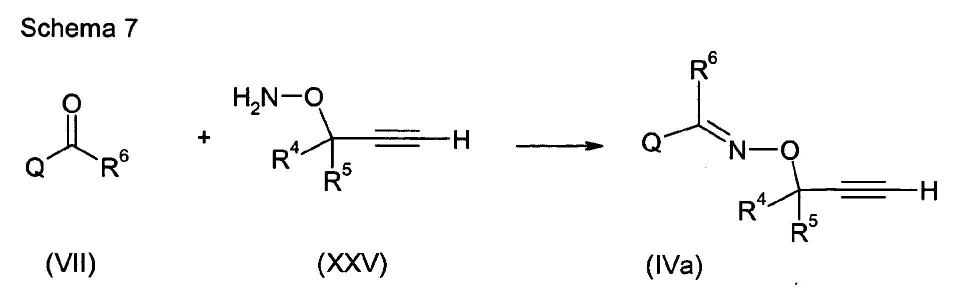

Hydroxylamine vom Typ (XXV) sind zahlreich in der Literatur beschrieben, zum Beispiel in Bioorg. & Med. Chem. 2001, 10(1), 207-213 oder EP-A-1266887.

In Analogie zu der in Schema 7 dargestellten Synthesesequenz kann auch im Fall von A gleich (A2) verfahren werden. Statt der Hydroxylamineinheit wird hierbei jedoch jeweils eine Hydrazineinheit der Formel (XXVI) verwendet. Der zusätzliche Substituent R⁷ stört die Umsetzungen dabei nicht (siehe Schema 8).

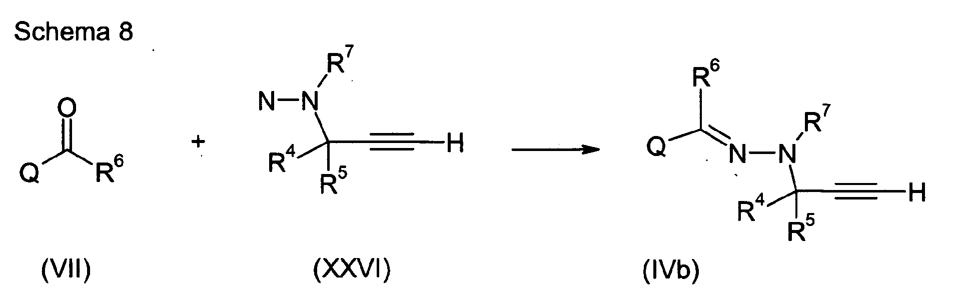

Gemäß der Verfahrensvariante (c) werden Verbindungen (VI) und (VII) zur Verbindung (I), in der A eine Gruppe der Formel (A1) (= Verbindung (Ia)) bedeutet, umgesetzt. Bei dieser Variante wird somit im letzten Syntheseschritt die Struktureinheit A gebildet. Die für den Syntheseschritt benötigten Verbindungen (VI) 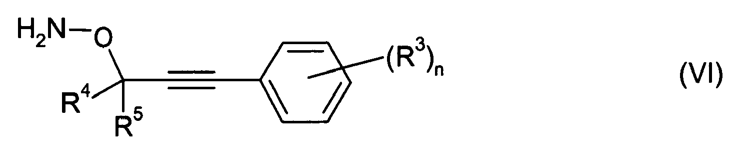 worin (R³)ₙ, R⁴ und R⁵ wie in der herzustellenden Verbindung der Formel (I) definiert sind, sind beispielsweise in Journal of Medicinal Chemistry (1998), 41(14), 2524 bis 2536 beschrieben oder können in analoger Weise hergestellt werden. Die Umsetzung zur Verbindung (la) kann in der Regel unter den Standardbedingungen für die Herstellung von Ketoximen oder Aldoximen durchgeführt werden.

Ebenso kann die Verfahrensvariante (d) analog Variante (c) zur Herstellung von Verbindungen (I), worin A gleich (A2) ist, (= Verbindung (Ib)) genutzt werden. Dabei wird die Keto- oder Aldehyd-Verbindung (VI) mit dem Hydrazinderivat (VIII) umgesetzt.

Die für die Herstellvariante (b) benötigten Zwischenprodukte der Formel (IV) stellen für die Fälle, wenn A gleich (A3) bis (A5) bedeutet, die Verbindungen (IVc), (IVd) bzw. (IVe) dar. Letztere können gemäß dem allgemeinen Schema 6 aus den Pyrazolen (IIc) oder (IId) durch Reaktion mit einem geeigneten Propargylbaustein (XXIV) hergestellt werden (siehe Schema 9 und Schema 10).

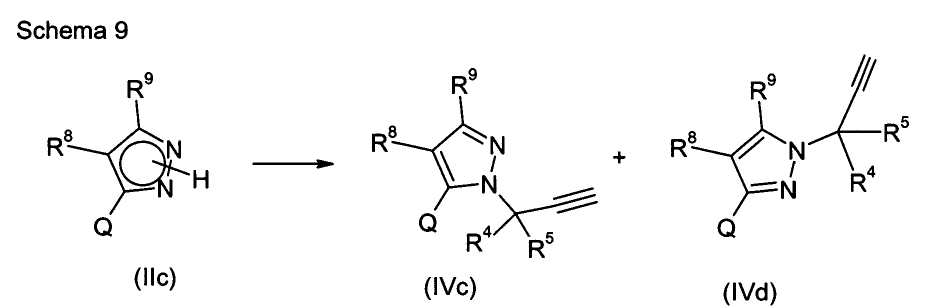

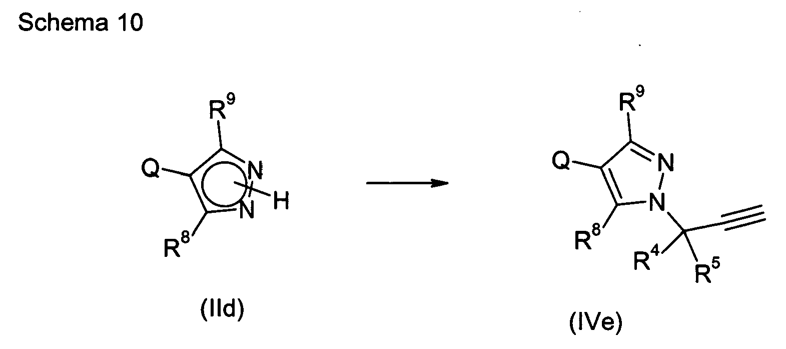

Zur Herstellung der Verbindungen (I), d.h. speziell (Ic), (Id) und (Ie), in denen A die (A3), (A4) bzw. (A5) bedeutet, kann die Knüpfung der Bindung zwischen Dreifachbindung und Phenyleinheit beispielsweise durch Sonogashira-Reaktion oder durch eine analoge Umsetzung erfolgen.

Gemäß der Verfahrensvariante (e) werden Verbindungen (IX) und (X) zur Verbindung (I), in der A eine Gruppe der Formel (A3, (A4) oder (A5) bedeutet, (= Verbindung (Ic), (Id) bzw. (Ie)) umgesetzt. Als Abgangsgruppe X** in Verbindung (IX) eignet sich in der Regel ein Halogen, wie Brom oder Iod. Die Verbindung (IX) kann im Sinne einer übergangsmetallkatalysierten Kreuzkupplungsreaktion mit dem Borsäurederivat der Formel (X) in Analogie zu Schema 5 in die Zielverbindungen der Formel (I) überführt werden. Zur Herstellung der Phenylpropargylverbindungen (IX) kann man eine Reaktion von Verbindungen (III) mit Pyrazolen der Formel X**-A-H durchführen, wobei ein Austausch des aciden Wasserstoffatoms des Pyrazolrings durch den Rest der Verbindung (III) erfolgt.

Gemäß den Verfahrensvarianten (f) und (g) werden Verbindungen (VIII') und (XI) bzw. (XII) zur Verbindung (I), in der A eine Gruppe der Formel (A3) und/oder (A4) bzw. (A5) bedeutet, (= Verbindung (Ic) und/oder (Id) bzw. (Ie)) unter Aufbau des Pyrazolrings umgesetzt.
Geeignete Phenylpropargylhydrazine (VIII') sind beispielsweise in J. Am. Chem. Soc. 1990, 112(26), 9641-9643 beschrieben. Die Herstellung geeigneter Ausgangsverbindungen (XI) ist bereits im Zusammenhang mit Schema 3 bzw. Schema 4 erwähnt worden.

Die hier beschriebenen Syntheseverfahren werden im Allgemeinen durch die Reste R¹ oder R³ nicht beeinträchtigt. Gegebenenfalls ist es jedoch sinnvoll in Abhängigkeit der konkret durchzuführenden Reaktion sowie der genauen Natur des Restes R¹ bzw. R³, diese mit geeigneten Schutzgruppen zu versehen. Schutzgruppen, ihre Einführung als auch ihre Abspaltung sind hinreichend beschrieben in: Th. W. Greene, P. G. M. Wuts "Protective Groups in Organic Synthesis", John Wiley & Sons, 3. Auflage, 1999.
Darüberhinaus ist es möglich, die Reste R¹ oder R³ nach Abschluss der hier beschriebenen Synthesesequenzen durch weitere Reaktionen zu modifizieren, wodurch neue, ebenfalls erfindungsgemäße Verbindungen der allgemeinen Formel (I) erhalten werden.

Für die Herstellung von Enantiomeren der Verbindungen (I) kommen auch übliche Racemattrennungsmethoden in Frage (vgl. Handbücher der Stereochemie), z. B. im Anschluss an Verfahren zur Trennung von Gemischen in Diastereomere, z. B. physikalische Verfahren wie Kristallisation, Chromatographieverfahren, vor allem Säulenchromatographie und Hochdruckflüssigchromatographie, Destillation, gegebenenfalls unter reduziertem Druck , Extraktion und andere Verfahren, können verbleibende Gemische von Enantiomeren in der Regel durch chromatographische Trennung an chiralen Festphasen getrennt werden. Für präparative Mengen oder im industriellen Maßstab kommen Verfahren wie die Kristallisation diastereomerer Salze, die aus den Verbindungen (I) mit optisch aktiven Säuren und gegebenenfalls bei vorhandenen sauren Gruppen mit optisch aktiven Basen erhalten werden können, in Frage.

Zur Racemattrennung durch Kristallisation diastereomerer Salze kommen als optisch aktive Säure z. B. Camphersulfonsäure, Camphersäure, Bromcamphersulfonsäure, Chinasäure, Weinsäure, Dibenzoylweinsäure und andere analoge Säure in Betracht; als optisch aktive Basen kommen z. B. Chinin, Cinchonin, Chinidin, Brucin, 1-Phenylethylamin und andere analoge Basen in Frage. Die Kristallisationen werden dann meist in wässrigen oder wässrig-organischen Lösungsmittel durchgeführt, wobei das Diastereomer mit der geringeren Löslichkeit gegebenenfalls nach Animpfen zunächst ausfällt. Das eine Enantiomer der Verbindung der Formel (I) wird danach aus dem ausgefällten Salz oder das andere aus dem Kristallisat durch Ansäuern bzw. mit Base freigesetzt.

Zur Herstellung der Säureadditionssalze der Verbindungen der Formel (I) kommen folgende Säuren in Frage: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphtalindisulfonsäure. Die Säureadditionsverbindungen der Formel (I) können in einfacher Weise nach den üblichen Salzbildungsmethoden, z.B. durch Lösen einer Verbindung der Formel (I) in einem geeigneten organischen Lösungsmittel wie z.B. Methanol, Aceton, Methylenchlorid oder Benzol und Hinzufügen der Säure bei Temperaturen von 0 bis 100 °C erhalten werden und in bekannter Weise, z.B. durch Abfiltrieren, isoliert und gegebenfalls durch Waschen mit einem inerten organischen Lösemittel gereinigt werden.

Die Basenadditionssalze der Verbindungen der Formel (I) werden vorzugsweise in inerten polaren Lösungsmitteln wie z.B. Wasser, Methanol oder Aceton bei Temperaturen von 0 bis 100 °C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, Alkali- und Erdalkalihydride, z.B. NaH, Alkali- und Erdalalkoholate, z.B. Natriummethanolat oder Kalium-tert.-butylat, oder Ammoniak, Ethanolamin oder quartäres Ammoniumhydroxid der Formel [NRR'R"R"']⁺ OH⁻.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Eine Kollektion aus Verbindungen der Formel (I), die nach den obengenannten Verfahren synthetisiert werden können, können zusätzlich in parallelisierter Weise hergestellt werden, wobei dies in manueller, teilweise automatisierter oder vollständig automatisierter Weise geschehen kann. Dabei ist es möglich, sowohl die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch S. H. DeWitt in "Annual Reports in Combinatorial Chemistry and Molecular Diversity: Automated Synthesis", Band 1, Verlag Escom, 1997, Seite 69 bis 77 beschrieben wird.

Zur parallelisierten Reaktionsdurchführung und Aufarbeitung können eine Reihe von im Handel erhältlichen Geräten verwendet werden wie sie beispielsweise von den Firmen Stem Corporation, Woodrolfe Road, Tollesbury, Essex, CM9 8SE, England oder H + P Labortechnik GmbH, Bruckmannring 28, 85764 Oberschleißheim, Deutschland angeboten werden. Für die parallelisierte Aufreinigung von Verbindungen (I) oder von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA. Die aufgeführten Apparaturen ermöglichen eine modulare Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise von Roboter bedient werden. Derartige Automationssysteme können zum Beispiel von der Firma Zymark Corporation, Zymark Center, Hopkinton, MA 01748, USA bezogen werden.

Neben den beschriebenen Methoden kann die Herstellung von Verbindungen der Formel (I) vollständig oder partiell durch festphasen-unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen unterstützte Synthesemethoden sind in der Fachliteratur hinreichend beschrieben, z. B.: Barry A. Bunin in "The Combinatorial Index", Verlag Academic Press, 1998. Die Verwendung von festphasen-unterstützten Synthesemethoden erlaubt eine Reihe von literaturbekannten Protokollen, die wiederum manuell oder automatisiert ausgeführt werden können. Zum Beispiel kann die "Teebeutelmethode" (Houghten, US 4,631,211; Houghten et al., Proc. Natl. Acad. Sci., 1985, 82, 5131 - 5135) mit Produkten der Firma IRORI, 11149 North Torrey Pines Road, La Jolla, CA 92037, USA teilweise automatisiert werden. Die Automatisierung von Festphasen unterstützter Parallelsynthese gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß den hier beschriebenen Verfahren liefert Verbindungen der Formel (I) in Form von Substanzkollektionen oder -bibliotheken. Gegenstand der vorliegenden Erfindung sind daher auch Bibliotheken der Verbindungen der Formel (I), die mindestens zwei Verbindungen der Formel (I) enthalten, und deren Vorprodukte.

Die erfindungsgemäßen Verbindungen der Formel (I) und deren Salze, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im Einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll. Auf der Seite der monokotylen Unkrautarten werden z.B. Agrostis, Alopecurus, Apera, Avena, Brachicaria, Bromus, Dactyloctenium, Digitaria, Echinochloa, Eleocharis, Eleusine, Festuca, Fimbristylis, Ischaemum, Lolium, Monochoria, Panicum, Paspalum, Phalaris, Phleum, Poa, Sagittaria, Scirpus, Setaria, Sphenoclea sowie Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.
Herbizide Wirkung wird auch erreicht bei dikotylen Schadpflanzen wie Ambrosia, Anthemis, Carduus, Centaurea, Chenopodium, Cirsium, Convolvulus, Datura, Emex, Galeopsis, Galinsoga, Kochia, Lepidium, Lindernia, Papaver, Portlaca, Polygonum, Ranunculus, Rorippa, Rotala, Seneceio, Sesbania, Solanum, Sonchus, Taraxacum, Trifolium, Urtica und Xanthium.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Soja, insbesondere Plantagenkulturen wie Ölpalme, Olive, Kokosnuss, Gummibaum, Zitrus, Ananas, Apfel, Birne, Kirsche, Baumwolle, Kaffee, Kakau, Wein und andere vergleichbare Obst und Plantagenkulturen nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen inklusive Zierpflanzungen. Auch eignen sich die Wirkstoffe, gegebenenfalls in Kombination mit anderen Wirkstoffen für die Anwendung im Nichtkulturland, wie auf Wegen, Plätzen, Beeten, Rasenflächen, Bahndämmen, Industrieanlagen zur Bekämpfung von unerwünschtem Pflanzenwuchs.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Emteguts bekannt.

Bevorzugt ist die Anwendung der erfindungsgemäßen Verbindungen der Formel (I) oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.
Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Herbizide aus der Gruppe der Imidazolinone, Sulfonylharnstoffe, Glufosinate-ammonium oder Glyphosateisopropylammonium und analoge Wirkstoffe resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen (I) als Herbizide zur Bekämpfung von Schadpflanzen in transgenen Kulturpflanzen.

Die erfindungsgemäße Verwendung zur Bekämpfung von Schadpflanzen oder zur Wachstumsregulierung von Pflanzen schließt auch den Fall ein, bei dem der Wirkstoff der Formel (I) oder dessen Salz erst nach der Ausbringung auf der Pflanze, in der Pflanze oder im Boden aus einer Vorläufersubstanz ("Prodrug") gebildet wird.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.
Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formullerungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden. Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I). In Spritzputvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Die Verbindungen der Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.
Als Kombinationspartner für die erfindungsgemäßen Wirkstoffe in Mischungsformulierungen oder im Tank-Mix sind beispielsweise bekannte, vorzugsweise herbizide Wirkstoffe, die auf einer Inhibition von beispielsweise Acetolactat-Synthase, Acetyl-Coenzym-A-Carboxylase, PS I, PS II, HPPDO, Phytoene-Desaturase, Protoporphyrinogen-Oxidase, Glutamine-Synthetase, Cellulosebiosynthese, 5-Enolpyruvylshikimat-3-phosphat-Synthetase beruhen, einsetzbar. Solche Verbindungen und auch andere einsetzbare Verbindungen mit teilweise unbekanntem oder anderem Wirkungsmechanismus sind z.B. in Weed Research 26, 441-445 (1986), oder in dem Handbuch "The Pesticide Manual", 12. Auflage 2000, oder 13. Auflage 2003 oder 14. Auflage 2006/2007, oder in dem entsprechenden "e-Pesticide Manual", Version 4 (2006), jeweils herausgegeben vom British Crop Protection Council, (im Folgenden auch kurz "PM"), und dort zitierter Literatur beschrieben. Listen von "Common names" sind auch in "The Compendium of Pesticide Common Names" im Internet verfügbar. Als literaturbekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acibenzolar-S-methyl; acifluorfen(-sodium); aclonifen; AD-67; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim(-sodium); ametryn; amicarbazone, amidochlor, amidosulfuron; aminopyralid; amitrol; AMS, d.h. Ammoniumsulfamat; ancimidol; anilofos; asulam; atrazin; aviglycine; azafenidin, azimsulfuron (DPX-A8947); aziprotryn; barban; BAS 516 H, d.h. 5-Fluor-2-phenyl-4H-3,1-benzoxazin-4-on; beflubutamid (UBH-509), benazolin(-ethyl); bencarbazone; benfluralin; benfuresate; benoxacor; bensulfuron(-methyl); bensulide; bentazone; benzfendizone; benzobicyclon, benzofenap; benzofluor; benzoylprop(-ethyl); benzthiazuron; bialaphos; bifenox; bispyribac(-sodium) (KIH-2023); borax; bromacil; bromobutide; bromofenoxim; bromoxynil; bromuron; buminafos; busoxinone; butachlor; butafenacil, butamifos; butenachlor (KH-218); buthidazole; butralin; butroxydim, butylate; cafenstrole (CH-900); carbetamide; carfentrazone(-ethyl); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlorbromuron; chlorbufam; chlorfenac; chlorfenprop; chlorflurecol(-methyl); chlorflurenol(-methyl); chloridazon; chlorimuron(-ethyl); chlormequat(-chloride); chlornitrofen; chlorophthalim (MK-616); chlorotoluron; chloroxuron; chlorpropham; chlorsulfuron; chlorthal-dimethyl; chlorthiamid; chlortoluron, cinidon(-methyl und -ethyl), cinmethylin; cinosulfuron; clefoxydim, clethodim; clodinafop und dessen Esterderivate (z.B. clodinafoppropargyl); clofencet; clomazone; clomeprop; cloprop; cloproxydim; clopyralid; clopyrasulfuron(-methyl), cloquintocet(-mexyl); cloransulam(-methyl), cumyluron (JC 940); cyanamide; cyanazine; cycloate; cyclosulfamuron (AC 104); cycloxydim; cycluron; cyhalofop und dessen Esterderivate (z.B. Butylester, DEH-112); cyperquat; cyprazine; cyprazole; cyprosulfamide; daimuron; 2,4-D, 2,4-DB; dalapon; daminozide; dazomet; n-decanol; desmedipham; desmetryn; di-allate; dicamba; dichlobenil; dichlormid; dichlorprop(-P)-salze; diclofop und dessen Ester wie diclofop-methyl; diclofop-P(-methyl); diclosulam, diethatyl(-ethyl); difenoxuron; difenzoquat(-metilsulfate); diflufenican; diflufenzopyr(-sodium); dimefuron; dimepiperate, dimethachlor; dimethametryn; dimethazone; dimethenamid (SAN-582H); dimethenamide-P; dimethylarsinic acid; dimethipin; dimetrasulfuron, dinitramine; dinoseb; dinoterb; diphenamid; dipropetryn; diquat-salze; dithiopyr; diuron; DNOC; eglinazine-ethyl; EL 77, d.h. 5-Cyano-1-(1,1-dimethylethyl)-N-methyl-1 H-pyrazole-4-carboxamid; endothal; epoprodan, EPTC; esprocarb; ethalfluralin; ethametsulfuron-methyl; ethephon; ethidimuron; ethiozin; ethofumesate; ethoxyfen und dessen Ester (z.B. Ethylester, HN-252); ethoxysulfuron, etobenzanid (HW 52); F5231, d.h. N-[2-Chlor-4-fluor-5-[4-(3-fluorpropyl)-4,5-dihydro-5-oxo-1 H-tetrazol-1-yl]-phenyl]-ethansulfonamid; fenchlorazole(-ethyl); fenclorim; fenoprop; fenoxan, fenoxaprop und fenoxaprop-P sowie deren Ester, z.B. fenoxaprop-P-ethyl und fenoxaprop-ethyl; fenoxydim; fentrazamide, fenuron; ferrous sulfate; flamprop(-methyl oder -isopropyl oder -isopropyl-L); flamprop-M(-methyl oder - isopropyl); flazasulfuron; florasulam, fluazifop und fluazifop-P und deren Ester, z.B. fluazifop-butyl und fluazifop-P-butyl; fluazolate, flucarbazone(-sodium), flucetosulfuron; fluchloralin; flufenacet; flufenpyr(-ethyl); flumetralin; flumetsulam; flumeturon; flumiclorac(-pentyl), flumioxazin (S-482); flumipropyn; fluometuron, fluorochloridone, fluorodifen; fluoroglycofen(-ethyl); flupoxam (KNW-739); flupropacil (UBIC-4243); flupropanoate; flupyrsulfuron(-methyl)(-sodium); flurazole; flurenol(-butyl); fluridone; flurochloridone; fluroxypyr(-meptyl); flurprimidol, flurtamone; fluthiacet(-methyl) (KIH-9201); fluthiamide, fluxofenim; fomesafen; foramsulfuron, forchlorfenuron; fosamine; furilazole; furyloxyfen; gibberillic acid; glufosinate(-ammonium); glyphosate(-isopropylammonium); halosafen; halosulfuron(-methyl); haloxyfop und dessen Ester; haloxyfop-P (= R-haloxyfop) und dessen Ester; HC-252; hexazinone; imazamethabenz(-methyl); imazamethapyr, imazamox, imazapic, imazapyr; imazaquin und Salze wie das Ammoniumsalz; imazethamethapyr; imazethapyr; imazosulfuron; inabenfide; indanofan; indol-3-acetic acid; 4-indol-3-ylbutyric acid; iodosulfuron-methyl(-sodium); ioxynil; isocarbamid; isopropalin; isoproturon; isouron; isoxaben; isoxachlortole, isoxadifen(-ethyl); isoxaflutole, isoxapyrifop; karbutilate; lactofen; lenacil; linuron; Maleinsäurehydrazid (MH), MCPA; MCPB; mecoprop(-P); mefenacet; mefenpyr(-diethyl); mefluidid; mepiquat(-chloride); mesosulfuron(-methyl); mesotrione, metam; metamifop; metamitron; metazachlor; methabenzthiazuron; metham; methazole; methoxyphenone; methylarsonic acid; methyl-cyclopropene; methyldymron; methylisothiocyanate; methabenzthiazuron; metobenzuron; metobromuron; (alpha-)metolachlor; metosulam (XRD 511); metoxuron; metribuzin; metsulfuron-methyl; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclofen; nitralin; nitrofen; nitrophenolate mixture; nitrofluorfen; nonanoic acid; norflurazon; orbencarb; orthasulfamuron; oxabetrinil; oryzalin; oxadiargyl (RP-020630); oxadiazon; oxasulfuron, oxaziclomefone, oxyfluorfen; paclobutrazol; paraquat(-dichloride); pebulate; pelargonic acid, pendimethalin; penoxulam; pentachlorophenol; pentanochlor; pentoxazone, perfluidone; pethoxamid; phenisopham; phenmedipham; picloram; picolinafen, pinoxaden, piperophos; piributicarb; pirifenop-butyl; pretilachlor; primisulfuron(-methyl); probenazole; procarbazone-(sodium), procyazine; prodiamine; profluralin; profoxydim; prohexadione(-calcium); prohydrojasmon; proglinazine(-ethyl); prometon; prometryn; propachlor; propanil; propaquizafop; propazine; propham; propisochlor; propoxycarbazone(-sodium) (MKH-6561); n-propyl-dihydrojasmonate; propyzamide; prosulfalin; prosulfocarb; prosulfuron (CGA-152005); prynachlor; pyraclonil; pyraflufen(-ethyl) (ET-751); pyrasulfotole; pyrazolynate; pyrazon; pyrazosulfuron(-ethyl); pyrazoxyfen; pyribenzoxim, pyributicarb, pyridafol, pyridate; pyriftalid; pyriminobac(-methyl) (KIH-6127); pyrimisulfan (KIH-5996); pyrithiobac(-sodium) (KIH-2031); pyroxasulfone (KIH-485); pyroxofop und dessen Ester (z.B. Propargylester); pyroxulam; quinclorac; quinmerac; quinoclamine, quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; sintofen; SN 106279, d.h. 2-[[7-[2-Chlor-4-(trifluor-methyl)-phenoxy]-2-naphthalenyl]-oxy]-propansäure und -methylester; sulcotrione, sulfentrazone (FMC-97285, F-6285); sulfazuron; sulfometuron(-methyl); sulfosate (ICI-A0224); sulfosulfuron, TCA; tebutam (GCP-5544); tebuthiuron; tecnacene; tembotrione; tefuryltrione; tepraloxydim, terbacil; terbucarb; terbuchlor; terbumeton; terbuthylazine; terbutryn; TFH 450, d.h. N,N-Diethyl-3-[(2-ethyl-6-methytphenyl)-sulfonyl]-1H-1,2,4-triazol-1-carboxamid; thenylchlor (NSK-850); thiafluamide, thiazafluron; thiazopyr (Mon-13200); thidiazimin (SN-24085); thidiazuron; thiencarbazone; thifensulfuron(-methyl); thiobencarb; Ti 35; tiocarbazil; topramezone; tralkoxydim; tri-allate; triasulfuron; triaziflam, triazofenamide; tribenuron(-methyl); triclopyr; tridiphane; trietazine; trifloxysulfuron; trifluralin; triflusulfuron und Ester (z.B. Methylester, DPX-66037); trimeturon; trinexapac; tritosulfuron, tsitodef; uniconazole; vernolate; WL 110547, d.h. 5-Phenoxy-1-[3-(trifluormethyl)-phenyl]-1H-tetrazol; D-489; LS 82-556; KPP-300; NC-324; NC-330; DPX-N8189; SC-0774; DOWCO-535; DK-8910; V-53482; PP-600 und MBH-001.

Von besonderem Interesse ist die selektive Bekämpfung von Schadpflanzen in Kulturen von Nutz- und Zierpflanzen. Obgleich die erfindungsgemäßen Verbindungen (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen (I) von besonderem Interesse, welche die Verbindungen (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide. Folgende Gruppen von Verbindungen kommen beispielsweise als Safener für die Verbindungen (I) und deren Kombinationen mit weiteren Pestiziden in Frage:
a) Verbindungen vom Typ der Dichlorphenylpyrazolin-3-carbonsäure, vorzugsweise Verbindungen wie 1-(2,4-Dichlorphenyl)-5-(ethoxycarbonyl)-5-methyl-2-pyrazolin-3-carbonsäureethylester (S1-1) ("Mefenpyr-diethyl", PM), und verwandte Verbindungen, wie sie in der WO 91/07874 beschrieben sind,
b) Derivate der Dichlorphenylpyrazolcarbonsäure, vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4), 1-(2,4-Dichlorphenyl)-5-phenyl-pyrazol-3-carbonsäureethylester (S1-5) und verwandte Verbindungen, wie sie in EP-A-333 131 und EP-A-269 806 beschrieben sind.
c) Verbindungen vom Typ der Triazolcarbonsäuren, vorzugsweise Verbindungen wie Fenchlorazol(-ethylester), d.h. 1-(2,4-Dichlorphenyl)-5-trichlormethyl-(1H)-1,2,4-triazol-3-
   carbonsäureethylester (S1-6), und verwandte Verbindungen EP-A-174 562 und EP-A-346 620);
d) Verbindungen vom Typ der 5-Benzyl- oder 5-Phenyl-2-isoxazolin-3- carbonsäure, oder der 5,5-Diphenyl-2-isoxazolin-3-carbonsäure vorzugsweise Verbindungen wie
   5-(2,4-Dichlorbenzyl)-2-isoxazolin-3-carbonsäureethylester (S1-7) oder 5-Phenyl-2-isoxazolin-3-carbonsäureethylester (S1-8) und verwandte Verbindungen, wie sie in WO 91/08202 beschrieben sind, bzw. der 5,5-Diphenyl-2-isoxazolin-carbonsäureethylester (S1-9) ("Isoxadifen-ethyl") oder - n-propylester (S1-10) oder der 5-(4-Fluorphenyl)-5-phenyl-2-isoxazolin-3-carbonsäureethylester (S1-11), wie sie in der deutschen Patentanmeldung (WO-A-95/07897) beschrieben sind.
e) Verbindungen vom Typ der 8-Chinolinoxyessigsäure (S2), vorzugsweise (5-Chlor-8-chinolinoxy)-essigsäure-(1-methyl-hex-1-yl)-ester (Common name "Cloquintocet-mexyl" (S2-1) (siehe PM)
   (5-Chlor-8-chinolinoxy)-essigsäure-(1,3-dimethyl-but-1-yl)-ester (S2-2), (5-Chlor-8-chinolinoxy)-essigsäure-4-allyl-oxy-butylester (S2-3), (5-Chlor-8-chinolinoxy)-essigsäure-1-allyloxy-prop-2-ylester (S2-4), (5-Chlor-8-chinolinoxy)-essigsäureethylester (S2-5), (5-Chlor-8-chinolinoxy)-essigsäuremethylester (S2-6), (5-Chlor-8-chinolinoxy)-essigsäureallylester (S2-7), (5-Chlor-8-chinolinoxy)-essigsäure-2-(2-propyliden-iminoxy)-1-ethylester (S2-8),
   (5-Chlor-8-chinolinoxy)-essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind.
f) Verbindungen vom Typ der (5-Chlor-8-chinolinoxy)-malonsäure, vorzugsweise Verbindungen wie (5-Chlor-8-chinolinoxy)-malonsäure-diethylester, (5-Chlor-8-chinolinoxy)-malonsäurediallylester,
   (5-Chlor-8-chinolinoxy)-malonsäure-methyl-ethylester und verwandte Verbindungen, wie sie in EP-A-0 582 198 beschrieben sind.
g) Wirkstoffe vom Typ der Phenoxyessig- bzw. -propionsäurederivate bzw. der aromatischen Carbonsäuren, wie z.B. 2,4-Dichlorphenoxyessigsäure(ester) (2,4-D), 4-Chlor-2-methyl-phenoxy-propionester (Mecoprop), MCPA oder 3,6-Dichlor-2-methoxy-benzoesäure(ester) (Dicamba).
h) Wirkstoffe vom Typ der Pyrimidine, die als bodenwirksame Safener in Reis angewendet werden, wie z. B. "Fenclorim" (PM) (= 4,6-Dichlor-2-phenylpyrimidin), das als Safener für Pretilachlor in gesätem Reis bekannt ist,
i) Wirkstoffe vom Typ der Dichloracetamide, die häufig als Vorauflaufsafener (bodenwirksame Safener) angewendet werden, wie z. B. "Dichlormid" (PM) (= N,N-Diallyl-2,2-dichloracetamid), "R-29148" (= 3-Dichloracetyl-2,2,5-trimethyl-1,3-oxazolidin von der Firma Stauffer),
   "Benoxacor" (PM) (= 4-Dichloracetyl-3,4-dihydro-3-methyl-2H-1,4-benzoxazin).
   "PPG-1292" (= N-Allyl-N-[(1,3-dioxolan-2-yl)-methyl]-dichloracetamid von der Firma PPG Industries),
   "DK-24" (= N-Allyl-N-[(allylaminocarbonyl)-methyl]-dichloracetamid von der Firma Sagro-Chem),
   "AD-67" oder "MON 4660" (= 3-Dichloracetyl-1-oxa-3-aza-spiro[4,5]decan von der Firma Nitrokemia bzw. Monsanto),
   "Diclonon" oder "BAS145138" oder "LAB145138" (= (= 3-Dichloracetyl-2,5,5-trimethyl-1,3-diazabicyclo[4.3.0]nonan von der Firma BASF) und "Furilazol" oder "MON 13900" (siehe PM) (= (RS)-3-Dichloracetyl-5-(2-furyl)-2,2-dimethyloxazolidin)
j) Wirkstoffe vom Typ der Dichloracetonderivate, wie z. B. "MG 191" (CAS-Reg. Nr. 96420-72-3) (= 2-Dichlormethyl-2-methyl-1,3-dioxolan von der Firma Nitrokemia), das als Safener für Mais bekannt ist,
k) Wirkstoffe vom Typ der Oxyimino-Verbindungen, die als Saatbeizmittel bekannt sind, wie z. B. "Oxabetrinil" (PM) (= (Z)-1,3-Dioxolan-2-ylmethoxyimino(phenyl)acetonitril), das als Saatbeiz-Safener für Hirse gegen Schäden von Metolachlor bekannt ist,
   "Fluxofenim" (PM) (= 1-(4-Chlorphenyl)-2,2,2-trifluor-1-ethanon-O-(1,3-dioxolan-2-ylmethyl)-oxim, das als Saatbeiz-Safener für Hirse gegen Schäden von Metolachlor bekannt ist, und
   "Cyometrinil" oder "-CGA-43089" (PM) (= (Z)-
   Cyanomethoxyimino(phenyl)acetonitril), das als Saatbeiz-Safener für Hirse gegen Schäden von Metolachlor bekannt ist,
l) Wirkstoffe vom Typ der Thiazolcarbonsäureester, die als Saatbeizmittel bekannt sind, wie z. B.
   "Flurazol" (PM) (= 2-Chlor-4-trifluormethyl-1,3-thiazol-5-carbonsäurebenzylester), das als Saatbeiz-Safener für Hirse gegen Schäden von Alachlor und Metolachlor bekannt ist,
m) Wirkstoffe vom Typ der Naphthalindicarbonsäurederivate, die als Saatbeizmittel bekannt sind, wie z. B.
   "Naphthalic anhydrid" (PM) (= 1,8-Naphthalindicarbonsäureanhydrid), das als Saatbeiz-Safener für Mais gegen Schäden von Thiocarbamatherbiziden bekannt ist,
n) Wirkstoffe vom Typ Chromanessigsäurederivate, wie z. B. "CL 304415" (CAS-Reg. Nr. 31541-57-8) (= 2-(4-Carboxy-chroman-4-yl)-essigsäure von der Firma American Cyanamid), das als Safener für Mais gegen Schäden von Imidazolinonen bekannt ist,
o) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (PM) (= Piperidin-1-thiocarbonsäure-S-1-methyl-1-phenylethylester), das als Safener für Reis gegen Schäden des Herbizids Molinate bekannt ist,
   "Daimuron" oder "SK 23" (PM) (= 1-(1-Methyl-1-phenylethyl)-3-p-tolylharnstoff), das als Safener für Reis gegen Schäden des Herbizids Imazosulfuron bekannt ist,
   "Cumyluron" = "JC-940" (= 3-(2-Chlorphenylmethyl)-1-(1-methyl-1-phenylethyl)-harnstoff, siehe JP-A-60087254), das als Safener für Reis gegen Schäden einiger Herbizide bekannt ist,
   "Methoxyphenon" oder "NK 049" (= 3,3'-Dimethyl-4-methoxy-benzophenon), das als Safener für Reis gegen Schäden einiger Herbizide bekannt ist, "CSB" (= 1-Brom-4-(chlormethylsulfonyl)-benzol) (CAS-Reg. Nr. 54091-06-4 von Kumiai), das als Safener gegen Schäden einiger Herbizide in Reis bekannt ist,
p) N-Acylsulfonamide der Formel (S3) und ihre Salze, 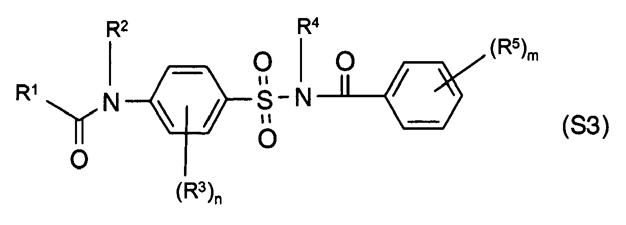 wie sie in WO-A-97/45016 beschrieben sind,
q) Acylsulfamoylbenzoesäureamide der allgemeinen Formel (S4), gegebenenfalls auch in Salzform, 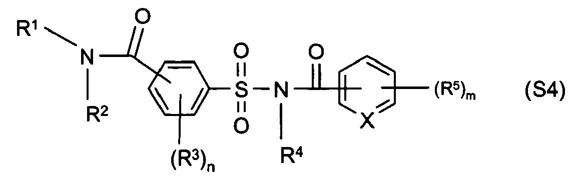 wie sie in der Internationalen Anmeldung Nr. PCT/EP98/06097 beschrieben sind, beispielsweise "cyprosulfamide" (S4-1) und
r) Verbindungen der Formel (S5), 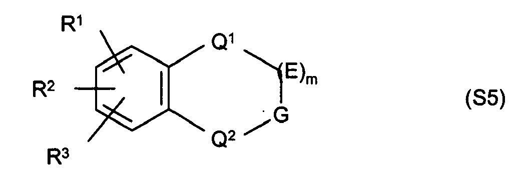 wie sie in der WO-A 98/13 361 beschrieben sind, einschließlich der Stereoisomeren und den in der Landwirtschaft gebräuchlichen Salzen.

Von besonderem Interesse sind unter den genannten Safenern sind (S1-1), (S1-9), (S2-1) und (S4-1). Einige der Safener sind bereits als Herbizide bekannt und entfalten somit neben der Herbizidwirkung bei Schadpflanzen zugleich auch Schutzwirkung bei den Kulturpflanzen.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

In den folgenden Beispielen beziehen sich Mengenangaben (auch Prozentangaben) auf das Gewicht, sofern nichts anderes speziell angegeben ist. Wenn im Rahmen der Beschreibung und der Beispiele Bezeichnungen "R" und "S" für die absolute Konfiguration an einem Chiralitätszentrum der Stereoisomeren der Formel (I) angegeben ist, folgt diese der RS-Nomenklatur nach der Cahn-Ingold- Prelog-Regel, wenn nichts anderes näher definiert ist.

Nachfolgend werden einige Beispiele für Herstellungen von Verbindungen der allgemeinen Formel (I) näher beschrieben.

In den nachfolgenden und auch bereits genannten chemischen Formeln werden die üblichen chemischen Symbole verwendet. Aus Gründen der Übersichtlichkeit sind die Formeln teilweise in der üblichen chemischen Kurzschreibweise angegeben, d. h. die C-Atome des Grundgerüstes in den aromatischen Ringen und teilweise in den aliphatischen Teilen sind nicht mit dem Symbol "C" angegeben, sondern nur als Endpunkte der gezeichneten Einfach-, Doppel- und Dreifachbindungen erkennbar. Weiterhin sind die Wasserstoffatome und die C-H-Bindungen am Grundgerüst der aromatischen Ringe nicht extra angeben.

### A) Synthesebeispiele:

### A1) 4-Fluorbenzaldehyd O-{3-[3-(Methylthio)phenyl]-2-propin-1-yl}oxim

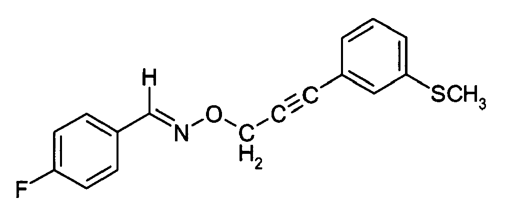

### a) 1-lod-3-(methylthlo)benzol

3 g 3-(Methylthio)anilin wurden zu 15 ml 6 normaler Salzsäure gegeben und auf 0°C abgekühlt. Zu der entstandenen pastösen Masse wurden langsam 1,64 g Natriumnitrit gelöst in 5 ml Wasser gegeben, wobei die Temperatur nicht über 3°C stieg, und die Reaktionslösung 2 Stunden lang bei 0°C nachgerührt. Anschließend wurden langsam 7,87 g Kaliumiodid, gelöst in 4 ml Wasser, zugetropft, wobei die Temperatur nicht über 5°C stieg. Nach beendeter Zugabe ließ man die Reaktionslösung auf Raumtemperatur kommen und rührte noch 2 Stunden bei dieser Temperatur nach. Der Ansatz wurde in Eiswasser gegeben und dreimal mit Essigsäureethylester extrahiert. Die vereinigten organischen Extrakte wurden zur Entfärbung zweimal mit gesättigter Natriumthiosulfatlösung gewaschen, anschließend mit Magnesiumsulfat getrocknet und am Rotationsverdampfer eingeengt. Das so erhaltene Rohprodukt wurde säulenchromatographisch gereinigt (Kieselgel, Eluent: Heptan / Essigsäureethylester = 9 / 1).
Ausbeute an 1-lod-3-(methylthio)benzol: 3,67 g. ¹H-NMR (CDCl₃): δ (ppm) = 2,45 (s, 3H); 7,0 (t, 1 H); 7,2 (m, 1 H), 7,43 (m, 1 H), 7,58 (m, 1 H).

### b) 3-[3-(Methylthio)phenyl]-2-propin-1-ol

92 mg Tetrakis(triphenylphosphin)palladium(0) und 30 mg Cul wurden unter Argonatmosphäre in einem Reaktionsgefäß vorgelegt und anschließend nacheinander 2 g 1-lod-3-(methylthio)benzol in 15 ml Dimethylformamid und 0,45 g Propargylalkohol in 5 ml Dimethylformamid zugegeben. Die entstandene Lösung wurde daraufhin mit 1,09 g Diisopropylethylamin versetzt und 20 Stunden lang bei Raumtemperatur gerührt. Nach beendeter Reaktion wurde mit Wasser versetzt und das Produkt dreimal mit Essigsäureethylester extrahiert. Die vereinigten organischen Extrakte wurden mit Wasser gewaschen, getrocknet und am Rotationsverdampfer eingeengt. Durch Säulenchromatographie (Kieselgel, Eluent: Heptan / Essigsäureethylester = 9 / 1) wurden 1,08 g 3-[3-(Methylthio)phenyl]-2-propin-1-ol erhalten. ¹H-NMR (CDCl₃): δ = 1,75 (t, 1 H); 2,45 (s, 3H); 4,5 (d, 2H); 7,0 (m, 3H); 7,32 (m, 1 H).

### c) 1-(3-Brom-1-propin-1-yl)-3-(methylthio)benzol

1 g 3-[3-(Methylthio)phenyl]-2-propin-1-ol wurde in 10 ml Diethylether gelöst und mit 67 mg Pyridin versetzt. Nach Abkühlen auf 0°C wurde 0,61 g Phosphortribromid, das zuvor in 5 ml Diethylether gelöst wurde, langsam bei 0 bis 5°C zugetropft. Nach beendeter Zugabe wurde die Kühlung entfernt und noch einige Stunden bei Raumtemperatur nachgerührt. Anschließend wurde mit Wasser versetzt und das Produkt mit Diethylether mehrmals extrahiert. Die vereinigten Extrakte wurden über Magnesiumsulfat getrocknet, am Rotationsverdampfer eingengt und das so erhaltene Rohprodukt durch Säulenchromatographie (Kieselgel, Eluent: Heptan / Essigsäureethylester = 9 / 1) gereinigt.
Ausbeute: 1,32 g 1-(3-Brom-1-propin-1-yl)-3-(methylthio)benzol
¹H-NMR (CDCl₃): δ = 2,43 (s, 3H); 4,18 (s, 2H); 7,0 (m, 3H); 7,32 (m, 1 H).

### d) 4-Fluorbenzaldehyd O-{3-[3-(Methylthio)phenyl]-2-propin-1-yl}oxim

0,26 g 1-(3-Brom-1-propin-1-yl)-3-(methylthio)benzol wurden in 5 ml Acetonitril gelöst und nacheinander mit 0,15 g 4-Fluorbenzaldoxim und 0,35 g Cäsiumcarbonat versetzt. Bei der Zugabe von Cäsiumcarbonat entstand eine leichte Gasentwicklung. Es wurde 24 Stunden lang bei Raumtemperatur gerührt, anschließend der Ansatz filtriert, am Rotationsverdampfer eingeengt und säulenchromatographisch gereinigt (Kieselgel, Eluent: Heptan / Essigsäureethylester = 9 / 1).
Ausbeute: 0,21 g 4-Fluorbenzaldehyd *O*-{3-[3-(Methylthio)phenyl]-2-propin-1-yl}oxim ¹H-NMR (CDCl₃): δ = 2,42 (s, 3H); 4,98 (s, 2H); 7,05 (m, 2H); 7,2 (m, 3H); 7,35 (m, 1 H); 7,6 (m, 1 H); 8,13 (s, 1 H).

### A2) 2-Methoxybenzaldehyd O-{3-[3-(Methylsulfinyl)phenyl]-2-propin-1-yl}oxim und 2-Methoxybenzaldehyd O-{3-[3-(Methylsulfonyl)phenyl]-2-propin-1-yl}oxim

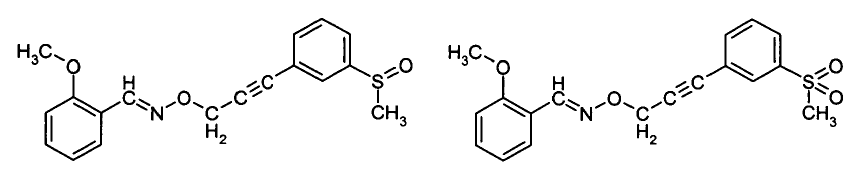

a) 2-Methoxybenzaldehyd *O*-{3-[3-(Methylthio)phenyl]-2-propin-1-yl}oxim 0,53 g 1-(3-Brom-1-propin-1-yl)-3-(methylthio)benzol werden in 5 ml Acetonitril gelöst und nacheinander mit 0,3 g 2-Methoxybenzaldoxim und 0,65 g Cäsiumcarbonat versetzt. Bei der Zugabe von Cäsiumcarbonat entstand eine leichte Gasentwicklung. Nach Rühren über 14 Stunden bei 60°C wurde der Ansatz filtriert, am Rotationsverdampfer eingeengt und säulenchromatographisch gereinigt (Kieselgel, Eluent: Heptan / Essigsäureethylester = 9 / 1). Ausbeute: 0,36 g 2-Methoxybenzaldehyd *O*-{3-[3-(Methylthio)phenyl]-2-propin-1-yl}oxim. ¹H-NMR (CDCl₃): δ (ppm) = 2,43 (s, 3H); 3,83 (s, 3H); 4,98 (s, 2H); 6,88 (d, 1 H); 6,96 (t, 1 H); 7,2 (m, 3H); 7,37 (m, 2H); 7,81 (m, 1 H); 8,58 (s, 1 H).
b) 2-Methoxybenzaldehyd *O*-{3-[3-(Methylsulfinyl)phenyl]-2-propin-1-yl}oxim und 2-Methoxybenzaldehyd *O*-{3-[3-(Methylsulfonyl)phenyl]-2-propin-1-yl}oxim

0,2 g 2-Methoxybenzaldehyd *O*-{3-[3-(Methylthio)phenyl]-2-propin-1-yl}oxim wurden in 5 ml Dichlormethan gelöst und auf 0°C abgekühlt. Nach Zugabe von 0,66 g m-Chlorperbenzoesäure in einer Portion ließ man auf Raumtemperatur kommen und rührte 15 Stunden lang bei dieser Temperatur. Anschließend wurden weitere 55 mg m-Chlorperbenzoesäure zugegeben und wiederum 5 Stunden lang bei Raumtemperatur gerührt. Währenddessen wurde der Fortgang der Reaktion stetig durch Dünnschichtchromatographie verfolgt. Schließlich wurde der Ansatz filtriert, am Rotationsverdampfer eingeengt und durch Säulenchromatographie (Kieselgel, Eluent: Heptan / Essigsäureethylester = 4 / 1) aufgereinigt.
Ausbeute: 102 mg 2-Methoxybenzaldehyd *O*-{3-[3-(Methylsulfinyl)phenyl]-2-propin-1-yl}oxim [¹H-NMR (CDCl₃): δ (ppm) = 2,75 (s, 3H); 3,83 (s, 3H); 5,0 (s, 2H); 6,9 (d, 1 H); 6,96 (t, 1 H); 7,38 (m, 1 H); 7,5 (m, 1 H); 7,6 (m, 2H); 7,72 (s, 1 H); 7,81 (d, 1 H); 8,58 (s, 1 H)] und 97 mg 2-Methoxybenzaldehyd *O*-{3-[3-(Methylsulfonyl)phenyl]-2-propin-1-yl}oxim ¹H-NMR (CDCl₃): δ (ppm) = 3,05 (s, 3H); 3,83 (s, 3H); 5,0 (s, 2H); 6,9 (d, 1 H); 6,98 (t, 1 H); 7,38 (m, 1H); 7,53 (m, 1 H); 7,72 (m, 1H); 7,81 (m, 1 H); 7,88 (m, 1 H); 8,04 (s, 1 H); 8,58 (s, 1 H).

### A3) 3-(3-{[(4-chlorbenzyliden)amino]oxy}-1-propin-1-yl)-phenylacetonitril

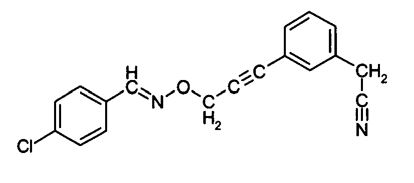

### a) 3-(3-Hydroxy-1-propin-1-yl)phenylacetonitril

47 mg Tetrakis(triphenylphosphin)palladium(0) und 15 mg Cul wurden unter Argonatmosphäre in einem Reaktionsgefäß vorgelegt und anschließend nacheinander 1 g 3-lodphenylacetonitril in 8 ml Dimethylformamid und 0,23 g Propargylalkohol in 2 ml Dimethylformamid zugegeben. Die entstandene Lösung wurde mit 0,55 g Diisopropylethylamin versetzt und 20 Stunden lang bei Raumtemperatur gerührt. Nach beendeter Reaktion wurde mit Wasser versetzt und das Produkt dreimal mit Essigsäureethylester extrahiert. Die vereinigten organischen Extrakte wurden mit Wasser gewaschen, getrocknet und am Rotationsverdampfer eingeengt. Durch Säulenchromatographie (Kieselgel, Eluent: Heptan / Essigsäureethylester = 9 / 1) wurde 0,58 g 3-(3-Hydroxy-1-propin-1-yl)phenylacetonitril erhalten. ¹H-NMR (CDCl₃): δ (ppm) = 2,0 (br t, 1 H); 3,74 (s, 2H); 4,5 (d, 2H); 7,25 - 7,4 (m, 4H).

### b) 3-(3-Brom-1-propin-1-yl)phenylacetonitril

0,58 g 3-(3-Hydroxy-1-propin-1-yl)phenylacetonitril wurden in 7 ml Diethylether gelöst und mit 40 mg Pyridin versetzt. Nach Abkühlen auf 0°C wurde 0,37 g Phosphortribromid, das zuvor in 3 ml Diethylether gelöst wurde, so langsam zugetropft, dass die Temperatur der Reaktionslösung nicht über 5°C steigt. Nach beendeter Zugabe wurde die Kühlung entfernt und noch einige Stunden bei Raumtemperatur nachgerührt. Anschließend wurde mit Wasser versetzt und das Produkt mit Diethylether mehrmals extrahiert. Die vereinigten Extrakte wurden über Magnesiumsulfat getrocknet, am Rotationsverdampfer eingengt und das so erhaltene Rohprodukt durch Säulenchromatographie (Kieselgel, Eluent: Heptan / Essigsäureethylester = 9 / 1) gereinigt. Ausbeute: 0,65 g 3-(3-Brom-1-propin-1-yl)phenylacetonitril; ¹H-NMR (CDCl₃): δ (ppm) = 3,77 (s, 2H); 4,15 (s, 2H); 7,3 - 7,42 (m, 4H).

### c) 3-(3-{[(4-chlorbenzyliden)amino]oxyl-1-propin-1-yl)phenylacetonitril

0,2 g 3-(3-Brom-1-propin-1-yl)phenylacetonitril wurden in 5 ml Acetonitril gelöst und nacheinander mit 0,17 g 4-Chlorbenzaldehydoxim und 0,28 g Cäsiumcarbonat versetzt. Nach 15 Stunden Rühren bei 60°C wurde der Ansatz filtriert, am Rotationsverdampfer eingeengt und säulenchromatographisch gereinigt (Kieselgel, Eluent: Heptan / Essigsäureethylester = 9 / 1). Ausbeute: 0,19 g 3-(3-{[(4-chlorbenzyliden)amino]oxy}-1-propin-1-yl)phenylacetonitril; ¹H-NMR (CDCl₃): δ (ppm) = 3,76 (s, 2H); 4,99 (s, 2H); 7,28 - 7,45 (m, 6H); 7,58 (m, 2H); 8,12 (s, 1 H).

### A4) 5-(2-Chlorphenyl)-1-{3-[3-(methylthio)phenyl]-2-propin-1-yl}-1 H-pyrazol und 3-(2-Chlorphenyl)-1-{3-[3-(methylthio)phenyl]-2-propin-1-yl}-1H-pyrazol

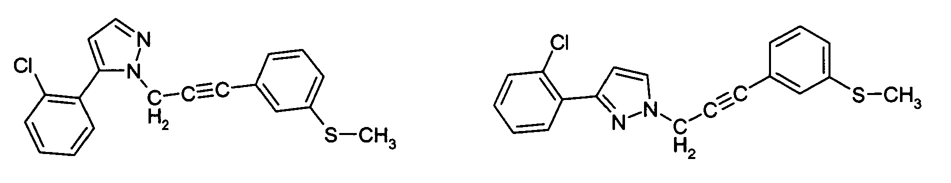

0,2 g 3-(2-Chlorphenyl)-1 H-pyrazol wurden in 6 ml Acetonitril gelöst und zu 0,3 g 1-(3-Brom-1-propin-1-yl)-3-(methylthio)benzol gegeben. Die Lösung wurde unter leichter Gasentwicklung mit 0,37 g Cäsiumcarbonat versetzt und 14 Stunden bei 40°C gerührt. Anschließend wurde der Ansatz filtriert, am Rotationsverdampfer eingeengt und per HPLC gereinigt (Kieselgel RP-18, Eluent: 20% bis 80% Acetonitril in 0,05%iger wässeriger Trifluoressigsäure). Ausbeute: 53 mg 5-(2-Chlorphenyl)-1-{3-[3-(methylthio)phenyl]-2-propin-1-yl}-1H-pyrazol [¹H-NMR (CDCl₃): δ (ppm) = 2,45 (s, 3H); 5,01 (s, 2H); 6,38 (d, 1 H); 7,08 (m, 1 H); 7,19 (m, 3H); 7,35 - 7,45 (m, 3H); 7,53 (m, 1 H); 7,62 (d, 1 H)] und 139 mg 3-(2-Chlorphenyl)-1-{3-[3-(methylthio)phenyl]-2-propin-1-yl}-1H-pyrazol [¹H-NMR (CDCl₃): δ (ppm) = 2,49 (s, 3H); 5,22 (s, 2H); 6,82 (d, 1 H); 7,2 - 7,35 (m, 6H); 7,45 (m, 1 H); 7,76 (d, 1 H); 7,8 (m, 1 H)].

### A5) 5-(2,4-Difluorphenyl)-1-{3-[3-(methoxy)phenyl]-2-propin-1-yl}-1H-pyrazol und 3-(2,4-Difluorphenyl)-1-{3-[3-(methoxy)phenyl]-2-propin-1-yl}-1H-pyrazol

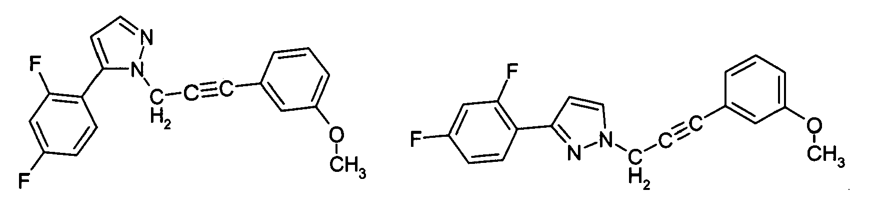

### a) 1-(3-Brom-1-propin-1-yl)-3-(methoxy)benzol

2 g 3-Methoxyphenylpropargylalkohol wurden in 20 ml Diethylether gelöst und mit 140 mg Pyridin versetzt. Nach Abkühlen auf 0°C wurde 1,3 g Phosphortribromid, gelöst in 5 ml Diethylether, langsam zugetropft, so dass die Temperatur in der Reaktionslösung nicht über 5°C stieg. Nach beendeter Zugabe wurde die Kühlung entfernt und über Nacht bei Raumtemperatur nachgerührt. Anschließend wurde mit Wasser versetzt und das Produkt mit Diethylether mehrmals extrahiert. Die vereinigten Extrakte wurden über Magnesiumsulfat getrocknet, am Rotationsverdampfer eingengt und das so erhaltene Rohprodukt durch Säulenchromatographie (Kieselgel, Eluent: Heptan / Essigsäureethylester = 9 / 1) gereinigt. Ausbeute: 2,51 g 1-(3-Brom-1-propin-1-yl)-3-(methoxy)benzol;
¹H-NMR (CDCl₃): δ (ppm) = 3,8 (s, 3H); 4,17 (s, 2H); 6,87 (m, 1 H); 6,98 (m, 1 H); 7,02 (m, 1 H); 7,21 (m, 1 H).

b) 0,2 g 3-(2,4-Difluorphenyl)-1 H-pyrazol wurden in 5 ml Acetonitril gelöst und zu 0,27 g 1-(3-Brom-1-propin-1-yl)-3-(methoxy)benzol gegeben. Die Lösung wurde mit 0,36 g Cäsiumcarbonat versetzt, wobei eine leichte Gasentwicklung entstand. Nach 16 Stunden Rühren bei 60°C wurde der Ansatz filtriert, am Rotationsverdampfer eingeengt und per HPLC gereinigt (Kieselgel RP-18, Eluent: 20% bis 100% Acetonitril in 0,05%iger wässeriger Trifluoressigsäure).
Ausbeute: 46 mg 5-(2,4-Difluorphenyl)-1-{3-[3-(methoxy)phenyl]-2-propin-1-yl}-1H-pyrazol [¹H-NMR (CDCl₃): δ (ppm) = 3,77 (s, 3H); 5,08 (s, 2H); 6,39 (d, 1 H); 6,82 bis 7,02 (m, 5H); 7,08 (m, 1 H); 7,47 (m, 1 H); 7,61 (d, 1 H)] und
226 mg 3-(2,4-Difluorphenyl)-1-{3-[3-(methoxy)phenyl]-2-propin-1-yl}-1 H-pyrazol ¹H-NMR (CDCl₃): δ (ppm) = 3,8 (s, 3H); 5,21 (s, 2H); 6,71 (d, 1 H); 6,85 - 6,95 (m, 3H); 7,0 (m, 1 H); 7,07 (m, 1 H); 7,21 (m, 1 H); 7,75 (d, 1 H); 7,99 (m, 1H)].

Die in den nachfolgenden Tabellen beschriebenen Verbindungen wurden gemäß den oder analog zu den oben beschriebenen Beispielen A1 bis A5 erhalten.

In den nachfolgenden Tabellen werden die üblichen chemischen Symbole verwendet. Aus Gründen der Übersichtlichkeit sind die Formeln vor jeder Tabelle in der üblichen chemischen Kurzschreibweise angegeben, d. h. die C-Atome des Grundgerüstes in den aromatischen Ringen und in der aliphatischen Kette sind nicht mit dem Symbol "C" angegeben, sondern nur als Endpunkte der gezeichneten Einfach-, Doppel- und Dreifachbindungen erkennbar. Weiterhin sind die Wasserstoffatome und die C-H-Bindungen am Grundgerüst der aromatischen Ringe nicht extra angeben.

Außerdem werden folgende Abkürzungen in den Tabellen verwendet:
- c-Pr =: Cyclopropyl
- c-Pent =: Cyclopentyl
- (R¹)ₘ oder (R³)ₙ =: H bedeutet unsubstituierter Cyclus (n = 0)

In der Spalte zu physikalische Daten ("Daten") der Tabellen bedeuten:
- "NMR" =: Daten gemäß ¹H-NMR-Spektum (¹H-Kernresonanz-Daten) sind am Ende der Tabellen 1-7 in der Tabelle 8 angegeben.
- "LogP" =: Verteilungskoeffizient in einem Zweiphasengemisch aus n-Octanol und Wasser. Daten sind am Ende der Tabellen 1-7 in der Tabelle 9 angegeben.

**Tabelle 1: Verbindungen der Formel (Ia-Q1)**

| | | | | | | |
|---|---|---|---|---|---|---|
| 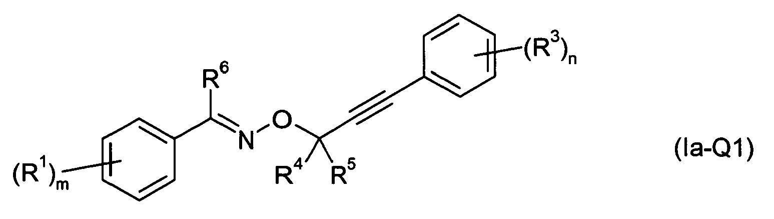 | | | | | | |

| Nr. | (R¹)ₘ | (R³)ₙ | R⁴ | R⁵ | R⁶ | Daten |
|---|---|---|---|---|---|---|
| 1.001 | H | H | H | H | H | NMR |
| 1.002 | H | 3-CF₃ | H | H | H | NMR |
| 1.003 | H | 3-CHF₂ | H | H | H | |
| 1.004 | H | 3-SCH₃ | H | H | H | NMR |
| 1.005 | H | 3-S(O)CH₃ | H | H | H | |
| 1.006 | H | 3-S(O)₂CH₃ | H | H | H | NMR |
| 1.007 | H | 3-SCF₃ | H | H | H | NMR |
| 1.008 | H | 3-S(O)CF₃ | H | H | H | NMR |
| 1.009 | H | 3-S(O)₂CF₃ | H | H | H | NMR |
| 1.010 | H | 3-CH₂CN | H | H | H | NMR |
| 1.011 | H | 4-F-3-SCH₃ | H | H | H | |
| 1.012 | H | 4-F-3-S(O) CH₃ | H | H | H | |
| 1.013 | H | 4-F-3-S(O)₂CH₃ | H | H | H | |
| 1.014 | H | 3-SCH₂CH₃ | H | H | H | |
| 1.015 | H | 3-S(O)CH₂CH₃ | H | H | H | |
| 1.016 | H | 3-S(O)₂CH₂CH₃ | H | H | H | |
| 1.017 | H | 3-OCH₃ | H | H | H | NMR |
| 1.018 | H | 3,5-(CF₃)₂ | H | H | H | NMR |
| 1.019 | 2-I | H | H | H | H | |
| 1.020 | 2-I | 3-CF₃ | H | H | H | |
| 1.021 | 2-I | 3-CHF₂ | H | H | H | |
| 1.022 | 2-I | 3-SCH₃ | H | H | H | NMR |
| 1.023 | 2-I | 3-S(O)CH₃ | H | H | H | NMR |
| 1.024 | 2-I | 3-S(O)₂CH₃ | H | H | H | NMR |
| 1.025 | 2-I | 3-CH₂CN | H | H | H | |
| 1.026 | 2-I | 4-F-3-SCH₃ | H | H | H | |
| 1.027 | 2-I | 4-F-3-S(O) CH₃ | H | H | H | |
| 1.028 | 2-I | 4-F-3-S(O)₂CH₃ | H | H | H | |
| 1.029 | 2-I | 3-SCH₂CH₃ | H | H | H | |
| 1.030 | 2-1 | 3-S(O)CH₂CH₃ | H | H | H | |
| 1.031 | 2-I | 3-S(O)₂CH₂CH₃ | H | H | H | |
| 1.032 | 2-CN | H | H | H | H | |
| 1.033 | 2-CN | 3-CF₃ | H | H | H | |
| 1.034 | 2-CN | 3-CHF₂ | H | H | H | |
| 1.035 | 2-CN | 3-SCH₃ | H | H | H | NMR |
| 1.036 | 2-CN | 3-S(O)CH₃ | H | H | H | NMR |
| 1.037 | 2-CN | 3-S(O)₂CH₃ | H | H | H | NMR |
| 1.038 | 2-CN | 3-CH₂CN | H | H | H | NMR |
| 1.039 | 2-CN | 4-F-3-SCH₃ | H | H | H | |
| 1.040 | 2-CN | 4-F-3-S(O) CH₃ | H | H | H | |
| 1.041 | 2-CN | 4-F-3-S(O)₂CH₃ | H | H | H | |
| 1.042 | 2-CN | 3-SCH₂CH₃ | H | H | H | |
| 1.043 | 2-CN | 3-S(O)CH₂CH₃ | H | H | H | |
| 1.044 | 2-CN | 3-S(O)₂CH₂CH₃ | H | H | H | |
| 1.045 | 2-CN | 3-OCH₃ | H | H | H | |
| 1.046 | 2-OCH₃ | H | H | H | H | |
| 1.047 | 2-OCH₃ | 3-CF₃ | H | H | H | |
| 1.048 | 2-OCH₃ | 3-CHF₂ | H | H | H | |
| 1.049 | 2-OCH₃ | 3-SCH₃ | H | H | H | NMR |
| 1.050 | 2-OCH₃ | 3-S(O)CH₃ | H | H | H | NMR |
| 1.051 | 2-OCH₃ | 3-S(O)₂CH₃ | H | H | H | NMR |
| 1.052 | 2-OCH₃ | 3-CH₂CN | H | H | H | |
| 1.053 | 2-OCH₃ | 4-F-3-SCH₃ | H | H | H | LogP |
| 1.054 | 2-OCH₃ | 4-F-3-S(O) CH₃ | H | H | H | |
| 1.055 | 2-OCH₃ | 4-F-3-S(O)₂CH₃ | H | H | H | |
| 1.056 | 2-OCH₃ | 3-SCH₂CH₃ | H | H | H | LogP |
| 1.057 | 2-OCH₃ | 3-S(O)CH₂CH₃ | H | H | H | |
| 1.058 | 2-OCH₃ | 3-S(O)₂CH₂CH₃ | H | H | H | |
| 1.059 | 4-F-2-OCH₃ | H | H | H | H | |
| 1.060 | 4-F-2-OCH₃ | 3-CF₃ | H | H | H | |
| 1.061 | 4-F-2-OCH₃ | 3-CHF₂ | H | H | H | |
| 1.062 | 4-F-2-OCH₃ | 3-SCH₃ | H | H | H | NMR |
| 1.063 | 4-F-2-OCH₃ | 3-S(O)CH₃ | H | H | H | NMR |
| 1.064 | 4-F-2-OCH₃ | 3-S(O)₂CH₃ | H | H | H | NMR |
| 1.065 | 4-F-2-OCH₃ | 3-CH₂CN | H | H | H | NMR |
| 1.066 | 4-F-2-OCH₃ | 4-F-3-SCH₃ | H | H | H | LogP |
| 1.067 | 4-F-2-OCH₃ | 4-F-3-S(O) CH₃ | H | H | H | |
| 1.068 | 4-F-2-OCH₃ | 4-F-3-S(O)₂CH₃ | H | H | H | |
| 1.069 | 4-F-2-OCH₃ | 3-SCH₂CH₃ | H | H | H | LogP |
| 1.070 | 4-F-2-OCH₃ | 3-S(O)CH₂CH₃ | H | H | H | |
| 1.071 | 4-F-2-OCH₃ | 3-S(O)₂CH₂CH₃ | H | H | H | |
| 1.072 | 2-Cl | H | H | H | H | |
| 1.073 | 2-Cl | 3-CF₃ | H | H | H | |
| 1.074 | 2-Cl | 3-CHF₂ | H | H | H | |
| 1.075 | 2-Cl | 3-SCH₃ | H | H | H | |
| 1.076 | 2-Cl | 3-S(O)CH₃ | H | H | H | |
| 1.077 | 2-Cl | 3-S(O)₂CH₃ | H | H | H | |
| 1.078 | 2-Cl | 3-CH₂CN | H | H | H | |
| 1.079 | 2-Cl | 4-F-3-SCH₃ | H | H | H | |
| 1.080 | 2-Cl | 4-F-3-S(O) CH₃ | H | H | H | |
| 1.081 | 2-Cl | 4-F-3-S(O)₂CH₃ | H | H | H | |
| 1.082 | 2-Cl | 3-SCH₂CH₃ | H | H | H | |
| 1.083 | 2-Cl | 3-S(O)CH₂CH₃ | H | H | H | |
| 1.084 | 2-Cl | 3-S(O)₂CH₂CH₃ | H | H | H | |
| 1.085 | 2,4-Cl₂ | H | H | H | H | |
| 1.086 | 2,4-Cl₂ | 3-CF₃ | H | H | H | |
| 1.087 | 2,4-Cl₂ | 3-CHF₂ | H | H | H | |
| 1.088 | 2,4-Cl₂ | 3-SCH₃ | H | H | H | |
| 1.089 | 2,4-Cl₂ | 3-S(O)CH₃ | H | H | H | |
| 1.090 | 2,4-Cl₂ | 3-S(O)₂CH₃ | H | H | H | |
| 1.091 | 2,4-Cl₂ | 3-CH₂CN | H | H | H | |
| 1.092 | 2,4-Cl₂ | 4-F-3-SCH₃ | H | H | H | |
| 1.093 | 2,4-Cl₂ | 4-F-3-S(O) CH₃ | H | H | H | |
| 1.094 | 2,4-Cl₂ | 4-F-3-S(O)₂CH₃ | H | H | H | |
| 1.095 | 2,4-Cl₂ | 3-SCH₂CH₃ | H | H | H | |
| 1.096 | 2,4-Cl₂ | 3-S(O)CH₂CH₃ | H | H | H | |
| 1.097 | 2,4-Cl₂ | 3-S(O)₂CH₂CH₃ | H | H | H | |
| 1.098 | 2,4-F₂ | H | H | H | H | |
| 1.099 | 2,4-F₂ | 3-CF₃ | H | H | H | |
| 1.100 | 2,4-F₂ | 3-CHF₂ | H | H | H | |
| 1.101 | 2,4-F₂ | 3-SCH₃ | H | H | H | |
| 1.102 | 2,4-F₂ | 3-S(O)CH₃ | H | H | H | |
| 1.103 | 2,4-F₂ | 3-S(O)₂CH₃ | H | H | H | |
| 1.104 | 2,4-F₂ | 3-CH₂CN | H | H | H | |
| 1.105 | 2,4-F₂ | 4-F-3-SCH₃ | H | H | H | |
| 1.106 | 2,4-F₂ | 4-F-3-S(O) CH₃ | H | H | H | |
| 1.107 | 2,4-F₂ | 4-F-3-S(O)₂CH₃ | H | H | H | |
| 1.108 | 2,4-F₂ | 3-SCH₂CH₃ | H | H | H | |
| 1.109 | 2,4-F₂ | 3-S(O)CH₂CH₃ | H | H | H | |
| 1.110 | 2,4-F₂ | 3-S(O)₂CH₂CH₃ | H | H | H | |
| 1.111 | 4-CF₃-2-OCH₃ | H | H | H | H | |
| 1.112 | 4-CF₃-2-OCH₃ | 3-CF₃ | H | H | H | |
| 1.113 | 4-CF₃-2-OCH₃ | 3-CHF₂ | H | H | H | |
| 1.114 | 4-CF₃-2-OCH₃ | 3-SCH₃ | H | H | H | NMR |
| 1.115 | 4-CF₃-2-OCH₃ | 3-S(O)CH₃ | H | H | H | NMR |
| 1.116 | 4-CF₃-2-OCH₃ | 3-S(O)₂CH₃ | H | H | H | NMR |
| 1.117 | 4-CF₃-2-OCH₃ | 3-CH₂CN | H | H | H | NMR |
| 1.118 | 4-CF₃-2-OCH₃ | 4-F-3-SCH₃ | H | H | H | |
| 1.119 | 4-CF₃-2-OCH₃ | 4-F-3-S(O) CH₃ | H | H | H | |
| 1.120 | 4-CF₃-2-OCH₃ | 4-F-3-S(O)₂CH₃ | H | H | H | |
| 1.121 | 4-CF₃-2-OCH₃ | 3-SCH₂CH₃ | H | H | H | |
| 1.122 | 4-CF₃-2-OCH₃ | 3-S(O)CH₂CH₃ | H | H | H | |
| 1.123 | 4-CF₃-2-OCH₃ | 3-S(O)₂CH₂CH₃ | H | H | H | |
| 1.124 | 4-F | H | H | H | H | |
| 1.125 | 4-F | 3-CF₃ | H | H | H | |
| 1.126 | 4-F | 3-CHF₂ | H | H | H | |
| 1.127 | 4-F | 3-OCH₃ | H | H | H | NMR |
| 1.128 | 4-F | 3-SCH₃ | H | H | H | NMR |
| 1.129 | 4-F | 3-S(O)CH₃ | H | H | H | |
| 1.130 | 4-F | 3-S(O)₂CH₃ | H | H | H | |
| 1.131 | 4-F | 3-CH₂CN | H | H | H | NMR |
| 1.132 | 4-F | 4-F-3-SCH₃ | H | H | H | |
| 1.133 | 4-F | 4-F-3-S(O) CH₃ | H | H | H | |
| 1.134 | 4-F | 4-F-3-S(O)₂CH₃ | H | H | H | |
| 1.135 | 4-F | 3-SCH₂CH₃ | H | H | H | |
| 1.136 | 4-F | 3-S(O)CH₂CH₃ | H | H | H | |
| 1.137 | 4-F | 3-S(O)₂CH₂CH₃ | H | H | H | |
| 1.138 | 4-F | 3,5-(CF₃)₂ | H | H | H | NMR |
| 1.139 | 4-CH₃ | H | H | H | H | |
| 1.140 | 4-CH₃ | 3-CF₃ | H | H | H | |
| 1.141 | 4-CH₃ | 3-CHF₂ | H | H | H | |
| 1.142 | 4-CH₃ | 3-OCH₃ | H | H | H | |
| 1.143 | 4-CH₃ | 3-SCH₃ | H | H | H | |
| 1.144 | 4-CH₃ | 3-S(O)CH₃ | H | H | H | |
| 1.145 | 4-CH₃ | 3-S(O)₂CH₃ | H | H | H | |
| 1.146 | 4-CH₃ | 3-CH₂CN | H | H | H | |
| 1.147 | 4-CH₃ | 4-F-3-SCH₃ | H | H | H | |
| 1.148 | 4-CH₃ | 4-F-3-S(O) CH₃ | H | H | H | |
| 1.149 | 4-CH₃ | 4-F-3-S(O)₂CH₃ | H | H | H | |
| 1.150 | 4-CH₃ | 3-SCH₂CH₃ | H | H | H | |
| 1.151 | 4-CH₃ | 3-S(O)CH₂CH₃ | H | H | H | |
| 1.152 | 4-CH₃ | 3-S(O)₂CH₂CH₃ | H | H | H | |
| 1.153 | 4-Cl | H | H | H | H | |
| 1.154 | 4-Cl | 3-CF₃ | H | H | H | |
| 1.155 | 4-Cl | 3-CHF₂ | H | H | H | |
| 1.156 | 4-Cl | 3-OCH₃ | H | H | H | NMR |
| 1.157 | 4-Cl | 3-SCH₃ | H | H | H | NMR |
| 1.158 | 4-Cl | 3-S(O)CH₃ | H | H | H | |
| 1.159 | 4-Cl | 3-S(O)₂CH₃ | H | H | H | |
| 1.160 | 4-Cl | 3-CH₂CN | H | H | H | NMR |
| 1.161 | 4-Cl | 4-F-3-SCH₃ | H | H | H | |
| 1.162 | 4-Cl | 4-F-3-S(O) CH₃ | H | H | H | |
| 1.163 | 4-Cl | 4-F-3-S(O)₂CH₃ | H | H | H | |
| 1.164 | 4-Cl | 3-SCH₂CH₃ | H | H | H | |
| 1.165 | 4-Cl | 3-S(O)CH₂CH₃ | H | H | H | |
| 1.166 | 4-Cl | 3-S(O)₂CH₂CH₃ | H | H | H | |
| 1.167 | 4-OCF₃ | H | H | H | H | NMR |
| 1.168 | 4-OCF₃ | 3-CF₃ | H | H | H | |
| 1.169 | 4-OCF₃ | 3-CHF₂ | H | H | H | |
| 1.170 | 4-OCF₃ | 3-OCH₃ | H | H | H | NMR |
| 1.171 | 4-OCF₃ | 3-SCH₃ | H | H | H | |
| 1.172 | 4-OCF₃ | 3-S(O)CH₃ | H | H | H | |
| 1.173 | 4-OCF₃ | 3-S(O)₂CH₃ | H | H | H | |
| 1.174 | 4-OCF₃ | 3-CH₂CN | H | H | H | |
| 1.175 | 4-OCF₃ | 4-F-3-SCH₃ | H | H | H | |
| 1.176 | 4-OCF₃ | 4-F-3-S(O) CH₃ | H | H | H | |
| 1.177 | 4-OCF₃ | 4-F-3-S(O)₂CH₃ | H | H | H | |
| 1.178 | 4-OCF₃ | 3-SCH₂CH₃ | H | H | H | |
| 1.179 | 4-OCF₃ | 3-S(O)CH₂CH₃ | H | H | H | |
| 1.180 | 4-OCF₃ | 3-S(O)₂CH₂CH₃ | H | H | H | |
| 1.181 | H | H | H | H | CH₃ | |
| 1.182 | H | 3-CH₃ | H | H | CH₃ | NMR |
| 1.183 | H | 3-CF₃ | H | H | CH₃ | NMR |
| 1.184 | H | 3-CHF₂ | H | H | CH₃ | |
| 1.185 | H | 3-SCH₃ | H | H | CH₃ | |
| 1.186 | H | 3-S(O)CH₃ | H | H | CH₃ | |
| 1.187 | H | 3-S(O)₂CH₃ | H | H | CH₃ | |
| 1.188 | H | 3-SCF₃ | H | H | CH₃ | |
| 1.189 | H | 3-CH₂CN | H | H | CH₃ | |
| 1.190 | H | 4-F-3-SCH₃ | H | H | CH₃ | |
| 1.191 | H | 3-OCH₃ | H | H | CH₃ | |
| 1.192 | 2-Cl | H | H | H | CH₃ | |
| 1.193 | 2-Cl | 3-CF₃ | H | H | CH₃ | |
| 1.194 | 2-Cl | 3-CHF₂ | H | H | CH₃ | |
| 1.195 | 2-Cl | 3-SCH₃ | H | H | CH₃ | NMR |
| 1.196 | 2-Cl | 3-S(O)CH₃ | H | H | CH₃ | NMR |
| 1.197 | 2-Cl | 3-S(O)₂CH₃ | H | H | CH₃ | NMR |
| 1.198 | 2-Cl | 3-SCF₃ | H | H | CH₃ | |
| 1.199 | 2-Cl | 3-CH₂CN | H | H | CH₃ | |
| 1.200 | 2-Cl | 4-F-3-SCH₃ | H | H | CH₃ | |
| 1.201 | 2-Cl | 3-OCH₃ | H | H | CH₃ | |
| 1.202 | 4-Cl | H | H | H | CH₃ | |
| 1.203 | 4-Cl | 3-CF₃ | H | H | CH₃ | |
| 1.204 | 4-Cl | 3-CHF₂ | H | H | CH₃ | |
| 1.205 | 4-Cl | 3-SCH₃ | H | H | CH₃ | |
| 1.206 | 4-Cl | 3-S(O)CH₃ | H | H | CH₃ | |
| 1.207 | 4-Cl | 3-S(O)₂CH₃ | H | H | CH₃ | |
| 1.208 | 4-Cl | 3-SCF₃ | H | H | CH₃ | |
| 1.209 | 4-Cl | 3-CH₂CN | H | H | CH₃ | |
| 1.210 | 4-Cl | 4-F-3-SCH₃ | H | H | CH₃ | |
| 1.211 | 4-Cl | 3-OCH₃ | H | H | CH₃ | |
| 1.212 | 2-OCH₃ | H | H | H | CH₃ | |
| 1.213 | 2-OCH₃ | 3-CF₃ | H | H | CH₃ | |
| 1.214 | 2-OCH₃ | 3-CHF₂ | H | H | CH₃ | |
| 1.215 | 2-OCH₃ | 3-SCH₃ | H | H | CH₃ | NMR |
| 1.216 | 2-OCH₃ | 3-S(O)CH₃ | H | H | CH₃ | NMR |
| 1.217 | 2-OCH₃ | 3-S(O)₂CH₃ | H | H | CH₃ | NMR |
| 1.218 | 2-OCH₃ | 3-SCF₃ | H | H | CH₃ | |
| 1.219 | 2-OCH₃ | 3-CH₂CN | H | H | CH₃ | |
| 1.220 | 2-OCH₃ | 4-F-3-SCH₃ | H | H | CH₃ | |
| 1.221 | 2-OCH₃ | 3-OCH₃ | H | H | CH₃ | |
| 1.222 | 4-F-2-OCH₃ | H | H | H | CH₃ | |
| 1.223 | 4-F-2-OCH₃ | 3-CF₃ | H | H | CH₃ | |
| 1.224 | 4-F-2-OCH₃ | 3-CHF₂ | H | H | CH₃ | |
| 1.225 | 4-F-2-OCH₃ | 3-SCH₃ | H | H | CH₃ | |
| 1.226 | 4-F-2-OCH₃ | 3-S(O)CH₃ | H | H | CH₃ | |
| 1.227 | 4-F-2-OCH₃ | 3-S(O)₂CH₃ | H | H | CH₃ | |
| 1.228 | 4-F-2-OCH₃ | 3-SCF₃ | H | H | CH₃ | |
| 1.229 | 4-F-2-OCH₃ | 3-CH₂CN | H | H | CH₃ | |
| 1.230 | 4-F-2-OCH₃ | 4-F-3-SCH₃ | H | H | CH₃ | |
| 1.231 | 4-F-2-OCH₃ | 3-OCH₃ | H | H | CH₃ | |
| 1.232 | H | H | H | H | c-Pr | |
| 1.233 | H | 3-CF₃ | H | H | c-Pr | |
| 1.234 | H | 3-CHF₂ | H | H | c-Pr | |
| 1.235 | H | 3-SCH₃ | H | H | c-Pr | |
| 1.236 | H | 3-S(O)CH₃ | H | H | c-Pr | |
| 1.237 | H | 3-S(O)₂CH₃ | H | H | c-Pr | |
| 1.238 | H | 3-SCF₃ | H | H | c-Pr | |
| 1.239 | H | 3-CH₂CN | H | H | c-Pr | |
| 1.240 | H | 4-F-3-SCH₃ | H | H | c-Pr | |
| 1.241 | H | 3-OCH₃ | H | H | c-Pr | |
| 1.242 | 2-Cl | H | H | H | c-Pr | |
| 1.243 | 2-Cl | 3-CF₃ | H | H | c-Pr | |
| 1.244 | 2-Cl | 3-CHF₂ | H | H | c-Pr | |
| 1.245 | 2-Cl | 3-SCH₃ | H | H | c-Pr | |
| 1.246 | 2-Cl | 3-S(O)CH₃ | H | H | c-Pr | |
| 1.247 | 2-Cl | 3-S(O)₂CH₃ | H | H | c-Pr | |
| 1.248 | 2-Cl | 3-SCF₃ | H | H | c-Pr | |
| 1.249 | 2-Cl | 3-CH₂CN | H | H | c-Pr | |
| 1.250 | 2-Cl | 4-F-3-SCH₃ | H | H | c-Pr | |
| 1.251 | 2-Cl | 3-OCH₃ | H | H | c-Pr | NMR |
| 1.252 | 2-OCH₃ | H | H | H | c-Pr | |
| 1.253 | 2-OCH₃ | 3-CF₃ | H | H | c-Pr | |
| 1.254 | 2-OCH₃ | 3-CHF₂ | H | H | c-Pr | |
| 1.255 | 2-OCH₃ | 3-SCH₃ | H | H | c-Pr | |
| 1.256 | 2-OCH₃ | 3-S(O)CH₃ | H | H | c-Pr | |
| 1.257 | 2-OCH₃ | 3-S(O)₂CH₃ | H | H | c-Pr | |
| 1.258 | 2-OCH₃ | 3-SCF₃ | H | H | c-Pr | |
| 1.259 | 2-OCH₃ | 3-CH₂CN | H | H | c-Pr | |
| 1.260 | 2-OCH₃ | 4-F-3-SCH₃ | H | H | c-Pr | |
| 1.261 | 2-OCH₃ | 3-OCH₃ | H | H | c-Pr | |
| 1.262 | 4-F-2-OCH₃ | H | H | H | c-Pr | |
| 1.263 | 4-F-2-OCH₃ | 3-CF₃ | H | H | c-Pr | |
| 1.264 | 4-F-2-OCH₃ | 3-CHF₂ | H | H | c-Pr | |
| 1.265 | 4-F-2-OCH₃ | 3-SCH₃ | H | H | c-Pr | |
| 1.266 | 4-F-2-OCH₃ | 3-S(O)CH₃ | H | H | c-Pr | |
| 1.267 | 4-F-2-OCH₃ | 3-S(O)₂CH₃ | H | H | c-Pr | |
| 1.268 | 4-F-2-OCH₃ | 3-SCF₃ | H | H | c-Pr | |
| 1.269 | 4-F-2-OCH₃ | 3-CH₂CN | H | H | c-Pr | |
| 1.270 | 4-F-2-OCH₃ | 4-F-3-SCH₃ | H | H | c-Pr | |
| 1.271 | 4-F-2-OCH₃ | 3-OCH₃ | H | H | c-Pr | |
| 1.272 | 4-Cl | H | H | H | c-Pr | |
| 1.273 | 4-Cl | 3-CF₃ | H | H | c-Pr | |
| 1.274 | 4-Cl | 3-CHF₂ | H | H | c-Pr | |
| 1.275 | 4-Cl | 3-SCH₃ | H | H | c-Pr | |
| 1.276 | 4-Cl | 3-S(O)CH₃ | H | H | c-Pr | |
| 1.277 | 4-Cl | 3-S(O)₂CH₃ | H | H | c-Pr | |
| 1.278 | 4-Cl | 3-SCF₃ | H | H | c-Pr | |
| 1.279 | 4-Cl | 3-CH₂CN | H | H | c-Pr | |
| 1.280 | 4-Cl | 4-F-3-SCH₃ | H | H | c-Pr | |
| 1.281 | 4-Cl | 3-OCH₃ | H | H | c-Pr | |
| 1.282 | H | H | CH₃ | H | H | |
| 1.283 | H | 3-CF₃ | CH₃ | H | H | |
| 1.284 | H | 3-CHF₂ | CH₃ | H | H | |
| 1.285 | H | 3-SCH₃ | CH₃ | H | H | |
| 1.286 | H | 3-S(O)CH₃ | CH₃ | H | H | |
| 1.287 | H | 3-S(O)₂CH₃ | CH₃ | H | H | |
| 1.288 | H | 3-CH₂CN | CH₃ | H | H | |
| 1.289 | H | 3-OCH₃ | CH₃ | H | H | |
| 1.290 | 2-Cl | H | CH₃ | H | H | |
| 1.291 | 2-Cl | 3-CF₃ | CH₃ | H | H | |
| 1.292 | 2-Cl | 3-CHF₂ | CH₃ | H | H | |
| 1.293 | 2-Cl | 3-SCH₃ | CH₃ | H | H | |
| 1.294 | 2-Cl | 3-S(O)CH₃ | CH₃ | H | H | |
| 1.295 | 2-Cl | 3-S(O)₂CH₃ | CH₃ | H | H | |
| 1.296 | 2-Cl | 3-CH₂CN | CH₃ | H | H | |
| 1.297 | 2-Cl | 3-OCH₃ | CH₃ | H | H | |
| 1.298 | 2-OCH₃ | H | CH₃ | H | H | |
| 1.299 | 2-OCH₃ | 3-CF₃ | CH₃ | H | H | |
| 1.300 | 2-OCH₃ | 3-CHF₂ | CH₃ | H | H | |
| 1.301 | 2-OCH₃ | 3-SCH₃ | CH₃ | H | H | LogP |
| 1.302 | 2-OCH₃ | 3-S(O)CH₃ | CH₃ | H | H | |
| 1.303 | 2-OCH₃ | 3-S(O)₂CH₃ | CH₃ | H | H | |
| 1.304 | 2-OCH₃ | 3-CH₂CN | CH₃ | H | H | |
| 1.305 | 2-OCH₃ | 3-OCH₃ | CH₃ | H | H | |
| 1.306 | 4-F-2-OCH₃ | H | CH₃ | H | H | |
| 1.307 | 4-F-2-OCH₃ | 3-CF₃ | CH₃ | H | H | |
| 1.308 | 4-F-2-OCH₃ | 3-CHF₂ | CH₃ | H | H | |
| 1.309 | 4-F-2-OCH₃ | 3-SCH₃ | CH₃ | H | H | |
| 1.310 | 4-F-2-OCH₃ | 3-S(O)CH₃ | CH₃ | H | H | |
| 1.311 | 4-F-2-OCH₃ | 3-S(O)₂CH₃ | CH₃ | H | H | |
| 1.312 | 4-F-2-OCH₃ | 3-CH₂CN | CH₃ | H | H | |
| 1.313 | 4-F-2-OCH₃ | 3-OCH₃ | CH₃ | H | H | |
| 1.314 | 4-Cl | H | CH₃ | H | H | |
| 1.315 | 4-Cl | 3-CF₃ | CH₃ | H | H | |
| 1.316 | 4-Cl | 3-CHF₂ | CH₃ | H | H | |
| 1.317 | 4-Cl | 3-SCH₃ | CH₃ | H | H | |
| 1.318 | 4-Cl | 3-S(O)CH₃ | CH₃ | H | H | |
| 1.319 | 4-Cl | 3-S(O)₂CH₃ | CH₃ | H | H | |
| 1.320 | 4-Cl | 3-CH₂CN | CH₃ | H | H | |
| 1.321 | 4-Cl | 3-OCH₃ | CH₃ | H | H | |
| 1.322 | H | H | -(CH₂)₄- | | H | |
| 1.323 | H | 3-CF₃ | -(CH₂)₄- | | H | |
| 1.324 | H | 3-CHF₂ | -(CH₂)₄- | | H | |
| 1.325 | H | 3-SCH₃ | -(CH₂)₄- | | H | |
| 1.326 | H | 3-S(O)CH₃ | -(CH₂)₄- | | H | |
| 1.327 | H | 3-S(O)₂CH₃ | -(CH₂)₄- | | H | |
| 1.328 | H | 3-CH₂CN | -(CH₂)₄- | | H | |
| 1.329 | H | 3-OCH₃ | -(CH₂)₄- | | H | |
| 1.330 | 2-Cl | H | -(CH₂)₄- | | H | |
| 1.331 | 2-Cl | 3-CF₃ | -(CH₂)₄- | | H | |
| 1.332 | 2-Cl | 3-CHF₂ | -(CH₂)₄- | | H | |
| 1.333 | 2-Cl | 3-SCH₃ | -(CH₂)₄- | | H | |
| 1.334 | 2-Cl | 3-S(O)CH₃ | -(CH₂)₄- | | H | |
| 1.335 | 2-Cl | 3-S(O)₂CH₃ | -(CH₂)₄- | | H | |
| 1.336 | 2-Cl | 3-CH₂CN | -(CH₂)₄- | | H | |
| 1.337 | 2-Cl | 3-OCH₃ | -(CH₂)₄- | | H | |
| 1.338 | 2-OCH₃ | H | -(CH₂)₄- | | H | |
| 1.339 | 2-OCH₃ | 3-CF₃ | -(CH₂)₄- | | H | |
| 1.340 | 2-OCH₃ | 3-CHF₂ | -(CH₂)₄- | | H | |
| 1.341 | 2-OCH₃ | 3-SCH₃ | -(CH₂)₄- | | H | |
| 1.342 | 2-OCH₃ | 3-S(O)CH₃ | -(CH₂)₄- | | H | |
| 1.343 | 2-OCH₃ | 3-S(O)₂CH₃ | -(CH₂)₄- | | H | |
| 1.344 | 2-OCH₃ | 3-CH₂CN | -(CH₂)₄- | | H | |
| 1.345 | 2-OCH₃ | 3-OCH₃ | -(CH₂)₄- | | H | |
| 1.346 | 4-F-2-OCH₃ | H | -(CH₂)₄- | | H | |
| 1.347 | 4-F-2-OCH₃ | 3-CF₃ | -(CH₂)₄- | | H | |
| 1.348 | 4-F-2-OCH₃ | 3-CHF₂ | -(CH₂)₄- | | H | |
| 1.349 | 4-F-2-OCH₃ | 3-SCH₃ | -(CH₂)₄- | | H | |
| 1.350 | 4-F-2-OCH₃ | 3-S(O)CH₃ | -(CH₂)₄- | | H | |
| 1.351 | 4-F-2-OCH₃ | 3-S(O)₂CH₃ | -(CH₂)₄- | | H | |
| 1.352 | 4-F-2-OCH₃ | 3-CH₂CN | -(CH₂)₄- | | H | |
| 1.353 | 4-F-2-OCH₃ | 3-OCH₃ | -(CH₂)₄- | | H | |
| 1.354 | 4-Cl | H | -(CH₂)₄- | | H | |
| 1.355 | 4-Cl | 3-CF₃ | -(CH₂)₄- | | H | |
| 1.356 | 4-Cl | 3-CHF₂ | -(CH₂)₄- | | H | |
| 1.357 | 4-Cl | 3-SCH₃ | -(CH₂)₄- | | H | |
| 1.358 | 4-Cl | 3-S(O)CH₃ | -(CH₂)₄- | | H | |
| 1.359 | 4-Cl | 3-S(O)₂CH₃ | -(CH₂)₄- | | H | |
| 1.360 | 4-Cl | 3-CH₂CN | -(CH₂)₄- | | H | |
| 1.361 | 4-Cl | 3-OCH₃ | -(CH₂)₄- | | H | |
| 1.362 | 3-CF₃ | SCH₃ | H | H | H | |
| 1.363 | 4-N(CH₂CH₃)₂ | SCH₃ | H | H | H | |
| 1.364 | 3-CF₃-4-Cl | H | H | H | CH₃ | |
| 1.365 | 3,4-OCH₂O- | 3-SCH₃ | H | H | H | NMR |
| 1.366 | 2-CH₃ | 3-CH₃ | H | H | H | |
| 1.367 | 4-N(CH₂CH₃)₂ | 3-CH₃ | H | H | H | |
| 1.368 | 3-CH₃ | 3,4-OCH₂O- | H | H | H | |
| 1.369 | 3,4-Cl₂ | 3-CH₃ | H | H | H | NMR |
| 1.370 | 3-CF₃ | 3-CF₃ | H | H | H | |
| 1.371 | 3,4-Cl₂ | 3-CF₃ | H | H | H | NMR |
| 1.372 | 2-(4-Cl-phenoxy) | 3-SCH₃ | H | H | H | NMR |
| 1.373 | 2-(4-Cl-phenoxy) | 3-CH₂CN | H | H | H | NMR |
| 1.374 | 4-F-2-OCH₃ | 3-OCH₃ | H | H | H | NMR |
| 1.375 | 4-CF₃-2-OCH₃ | 3-OCH₃ | H | H | H | NMR |
| 1.376 | 2-(4-Cl-phenoxy) | 3-OCH₃ | H | H | H | NMR |
| 1.377 | 2-Cl | 4-CH₃-3-SCH₃ | H | H | CH₃ | NMR |
| 1.378 | 4-CF₃-2-OCH₃ | 4-CH₃-3-S(O)CH₃ | H | H | H | NMR |
| 1.379 | 2-(4-Cl-phenoxy) | 4-CH₃-3-S(O)CH₃ | H | H | H | LogP |
| 1.380 | 2-OCH₃ | 3-S(O)CF₃ | H | H | H | LogP |
| 1.381 | 2,3-(OCH₃)₂ | 3-S(O)CH₃ | H | H | H | NMR |
| 1.382 | 2-OCH₃ | 4-CH₃-3-SCH₃ | H | H | CH₃ | NMR |
| 1.383 | 3,4-Cl₂ | 3-OCH₃ | H | H | CH₂CH₃ | NMR |
| 1.384 | 3,4-Cl₂ | 3-CF₃ | H | H | CH₂CH₃ | NMR |
| 1.385 | 3,4-Cl₂ | 3-SCH₃ | H | H | CH₂CH₃ | NMR |
| 1.386 | 2-OCH₃ | 2-SCH₃ | H | H | H | LogP |
| 1.387 | 2,4-(OCH₃)₂ | 3-CH₂CN | H | H | H | LogP |
| 1.388 | 2,4-(OCH₃)₂ | 3-SCH₃ | H | H | H | LogP |
| 1.389 | 2,4-(OCH₃)₂ | 3-S(O)CH₃ | H | H | H | LogP |
| 1.390 | 2,4-(OCH₃)₂ | NHC(=O)CH₃ | H | H | H | |
| 1.391 | 2,4-(OCH₃)₂ | 4-S(O)₂NH₂ | H | H | H | |
| 1.392 | 2,4-(OCH₃)₂ | CH=CH-N(CH₃)₂ | H | H | CH₃ | |
| 1.393 | 2,6-(OCH₃)₂ | 3-SCH₃ | H | H | H | LogP |
| 1.394 | 2-OCH₃ | 4-CN-3-SCH₃ | H | H | H | LogP |
| 1.395 | 4-F-2-OCH₃ | 4-CN-3-SCH₃ | H | H | H | LogP |
| 1.396 | 2,6-(OCH₃)₂ | 3-S(O)CH₃ | H | H | H | LogP |
| 1.397 | 2,6-(OCH₃)₂ | 3-CH₂CN | H | H | H | LogP |
| 1.398 | 2-OCH₃ | 4-OCH₃-3-SCH₂CH₃ | H | H | H | LogP |
| 1.399 | 4-F-2-OCH₃ | 4-OCH₃-3-SCH₂CH₃ | H | H | H | LogP |
| 1.400 | 2-C(=O)N(CH₃)₂ | 4-OCH₃-3-SCH₂CH₃ | CH₃ | H | H | |
| 1.401 | 2-OCH₃ | 4-OCH₃-3-S(O)CH₂CH₃ | H | H | H | LogP |
| 1.402 | 4-F-2-OCH₃ | 4-OCH₃-3-S(O)CH₂CH₃ | H | H | H | LogP |
| 1.403 | H | 3-OCH₃ | H | H | CH₂CH₃ | LogP |
| 1.404 | H | 3-CF₃ | H | H | CH₂CH₃ | LogP |
| 1.405 | H | 3-SCH₃ | H | H | CH₂CH₃ | LogP |
| 1.406 | H | 3-CH₂CN | H | H | CH₂CH₃ | LogP |
| 1.407 | 4-CH₃ | 3-OCH₃ | H | H | CH₂CH₃ | LogP |
| 1.408 | 4-CH₃ | 3-CF₃ | H | H | CH₂CH₃ | LogP |
| 1.409 | 4-CH₃ | 3-SCH₃ | H | H | CH₂CH₃ | LogP |
| 1.410 | 4-CH₃ | 3-CH₂CN | H | H | CH₂CH₃ | LogP |
| 1.411 | 4-F | 3-OCH₃ | H | H | CH₂CH₃ | LogP |
| 1.412 | 4-F | 3-CF₃ | H | H | CH₂CH₃ | LogP |
| 1.413 | 4-F | 3-SCH₃ | H | H | CH₂CH₃ | LogP |
| 1.414 | 4-F | 3-CH₂CN | H | H | CH₂CH₃ | LogP |
| 1.415 | 2-F | 3-OCH₃ | H | H | CH₂CH₃ | LogP |
| 1.416 | 2-F | 3-CF₃ | H | H | CH₂CH₃ | LogP |
| 1.417 | 2-F | 3-SCH₃ | H | H | CH₂CH₃ | LogP |
| 1.418 | 2-F | 3-CH₂CN | H | H | CH₂CH₃ | LogP |
| 1.419 | 2,3-Cl₂ | 3-CH₂CN | H | H | CH₂CH₃ | LogP |
| 1.420 | H | 3-S(O)CH₃ | H | H | CH₂CH₃ | LogP |
| 1.421 | 4-CH₃ | 3-S(O)CH₃ | H | H | CH₂CH₃ | LogP |
| 1.422 | 2,3-Cl₂ | 3-S(O)CH₃ | H | H | CH₂CH₃ | LogP |
| 1.423 | 4-F | 3-S(O)CH₃ | H | H | CH₂CH₃ | LogP |
| 1.424 | 2-OCH₃ | 4-CH₃-3-SCH₂CH₃ | H | H | H | LogP |
| 1.425 | 4-F-2-OCH₃ | 4-CH₃-3-SCH₂CH₃ | H | H | H | LogP |
| 1.426 | 2-OCH₃ | 4-CH₃-3-S(O)CH₂CH₃ | H | H | H | LogP |
| 1.427 | 4-F-2-OCH₃ | 4-CH₃-3-S(O)CH₂CH₃ | H | H | H | LogP |
| 1.428 | 2-OCH₃ | 3-OCF₃ | H | H | H | LogP |
| 1.429 | 4-F-2-OCH₃ | 3-OCF₃ | H | H | H | LogP |
| 1.430 | 2-OCH₃ | 3-SCH(CH₃)₂ | H | H | H | LogP |
| 1.431 | 4-F-2-OCH₃ | 3-SCH(CH₃)₂ | H | H | H | LogP |
| 1.432 | 4-F-2-OCH₃ | 3-SCH₂CH=CH₂ | H | H | H | LogP |
| 1.433 | 4-F-2-OCH₃ | 4-OCH₃-3-SCH₃ | H | H | H | LogP |
| 1.434 | 2-OCH₃ | 4-OCH₃-3-SCH₃ | H | H | H | LogP |
| 1.435 | 4-F-2-OCH₃ | 2,4-F₂-3-SCH₃ | H | H | H | LogP |
| 1.436 | 2-OCH₃ | 2,4-F₂-3-SCH₃ | H | H | H | LogP |
| 1.437 | 2-OCH₃ | 2,4-F₂-3-S(O)CH₃ | H | H | H | LogP |
| 1.438 | 4-F-2-OCH₃ | 2,4-F₂-3-S(O)CH₃ | H | H | H | LogP |
| 1.439 | 2-OCH₃ | 2,3-F₂ | H | H | H | LogP |
| 1.440 | 2-OCH₃ | 4-F-3-CH₃-5-SCH₃ | H | H | H | LogP |
| 1.441 | 4-F-2-OCH₃ | 4-F-3-CH₃-5-SCH₃ | H | H | H | LogP |
| 1.442 | 4-F-2-OCH₃ | 2-SCH₃ | H | H | H | LogP |
| 1.443 | 4-F-2-OCH₃ | 4-F-3-CH₃-5-S(O)CH₃ | H | H | H | LogP |
| 1.444 | 2-OCH₃ | 4-F-3-CH₃-5-S(O)CH₃ | H | H | H | LogP |
| 1.445 | 2-OCH₃ | 2-S(O)CH₃ | H | H | H | LogP |
| 1.446 | 4-F-2-OCH₃ | 2-S(O)CH₃ | H | H | H | LogP |
| 1.447 | 4-F-2-OCH₃ | 3,4-F₂-2-SCH₃ | H | H | H | LogP |
| 1.448 | 4-F-2-OCH₃ | 3,4-F₂-2-S(O)CH₃ | H | H | H | LogP |
| 1.449 | 4-F-2-OCH₃ | 4-F-3,5-(SCH₃)₂ | H | H | H | LogP |
| 1.450 | 2-OCH₃ | 4-F-3,5-(SCH₃)₂ | H | H | H | LogP |
| 1.451 | 4-CH₃O(O=)C-2-OCH₃ | 4-F-3,5-(SCH₃)₂ | H | H | H | |
| 1.452 | 3-CH₃C(=O)NH | 3-SCH₃ | H | H | H | |
| 1.453 | 3,5-(CN)₂ | 4-F | H | H | H | |
| 1.454 | 3-CH₃C(=O)NH | 4-C(=O)OCH₃ | H | H | H | |
| 1.455 | 3,5-(CN)₂ | 4-C(=O)OCH₃ | H | H | H | |
| 1.456 | 3,5-Br₂ | 3-CF₃ | H | H | H | |
| 1.457 | 3,5-Br₂ | 4-F | H | H | H | |
| 1.458 | 3,5-Br₂ | 3-SCH₃ | H | H | H | |
| 1.459 | 3,5-Br₂ | 3-CH₂CN | H | H | H | |
| 1.460 | 2-Cl | 2-CN | H | H | H | |
| 1.461 | 2-Cl | 4-NH-S(O)₂CH₃ | H | H | H | |
| 1.462 | 4-Cl | 4-NH-S(O)₂CH₃ | H | H | H | |

**Tabelle 2: Verbindungen der Formel (Ib-Q1)**

| | | | | | | | |
|---|---|---|---|---|---|---|---|
| 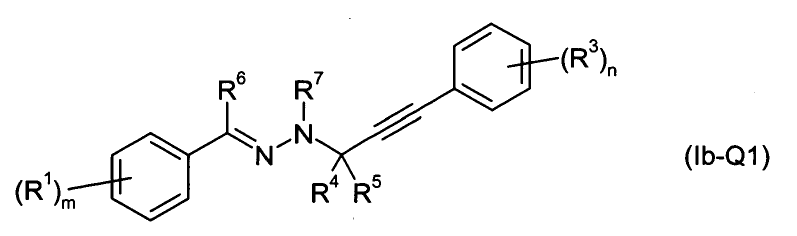 | | | | | | | |

| Nr. | (R¹)ₘ | (R³)ₙ | R⁴ | R⁵ | R⁶ | R⁷ | Daten |
|---|---|---|---|---|---|---|---|
| 2.001 | H | H | H | H | H | CH₃ | |
| 2.002 | H | 3-CF₃ | H | H | H | CH₃ | |
| 2.003 | H | 3-CHF₂ | H | H | H | CH₃ | |
| 2.004 | H | 3-SCH₃ | H | H | H | CH₃ | |
| 2.005 | H | 3-S(O)CH₃ | H | H | H | CH₃ | |
| 2.006 | H | 3-S(O)₂CH₃ | H | H | H | CH₃ | |
| 2.007 | H | 3-SCF₃ | H | H | H | CH₃ | |
| 2.008 | H | 3-S(O)CF₃ | H | H | H | CH₃ | |
| 2.009 | H | 3-S(O)₂CF₃ | H | H | H | CH₃ | |
| 2.010 | H | 3-CH₂CN | H | H | H | CH₃ | |
| 2.011 | H | 4-F-3-SCH₃ | H | H | H | CH₃ | |
| 2.012 | H | 4-F-3-S(O) CH₃ | H | H | H | CH₃ | |
| 2.013 | H | 4-F-3-S(O)₂CH₃ | H | H | H | CH₃ | |
| 2.014 | H | 3-SCH₂CH₃ | H | H | H | CH₃ | |
| 2.015 | H | 3-S(O)CH₂CH₃ | H | H | H | CH₃ | |
| 2.016 | H | 3-S(O)₂CH₂CH₃ | H | H | H | CH₃ | |
| 2.017 | H | 3-OCH₃ | H | H | H | CH₃ | |
| 2.018 | H | 3,5-(CF₃)₂ | H | H | H | CH₃ | |
| 2.019 | 2-CN | H | H | H | H | CH₃ | |
| 2.020 | 2-CN | 3-CF₃ | H | H | H | CH₃ | |
| 2.021 | 2-CN | 3-CHF₂ | H | H | H | CH₃ | |
| 2.022 | 2-CN | 3-SCH₃ | H | H | H | CH₃ | |
| 2.023 | 2-CN | 3-S(O)CH₃ | H | H | H | CH₃ | |
| 2.024 | 2-CN | 3-S(O)₂CH₃ | H | H | H | CH₃ | |
| 2.025 | 2-CN | 3-CH₂CN | H | H | H | CH₃ | |
| 2.026 | 2-CN | 4-F-3-SCH₃ | H | H | H | CH₃ | |
| 2.027 | 2-CN | 4-F-3-S(O) CH₃ | H | H | H | CH₃ | |
| 2.028 | 2-CN | 4-F-3-S(O)₂CH₃ | H | H | H | CH₃ | |
| 2.029 | 2-CN | 3-SCH₂CH₃ | H | H | H | CH₃ | |
| 2.030 | 2-CN | 3-S(O)CH₂CH₃ | H | H | H | CH₃ | |
| 2.031 | 2-CN | 3-S(O)₂CH₂CH₃ | H | H | H | CH₃ | |
| 2.032 | 2-CN | 3-OCH₃ | H | H | H | CH₃ | |
| 2.033 | 2-OCH₃ | H | H | H | H | CH₃ | |
| 2.034 | 2-OCH₃ | 3-CF₃ | H | H | H | CH₃ | |
| 2.035 | 2-OCH₃ | 3-CHF₂ | H | H | H | CH₃ | |
| 2.036 | 2-OCH₃ | 3-SCH₃ | H | H | H | CH₃ | |
| 2.037 | 2-OCH₃ | 3-S(O)CH₃ | H | H | H | CH₃ | |
| 2.038 | 2-OCH₃ | 3-S(O)₂CH₃ | H | H | H | CH₃ | |
| 2.039 | 2-OCH₃ | 3-CH₂CN | H | H | H | CH₃ | |
| 2.040 | 2-OCH₃ | 4-F-3-SCH₃ | H | H | H | CH₃ | |
| 2.041 | 2-OCH₃ | 4-F-3-S(O) CH₃ | H | H | H | CH₃ | |
| 2.042 | 2-OCH₃ | 4-F-3-S(O)₂CH₃ | H | H | H | CH₃ | |
| 2.043 | 2-OCH₃ | 3-SCH₂CH₃ | H | H | H | CH₃ | |
| 2.044 | 2-OCH₃ | 3-S(O)CH₂CH₃ | H | H | H | CH₃ | |
| 2.045 | 2-OCH₃ | 3-S(O)₂CH₂CH₃ | H | H | H | CH₃ | |
| 2.046 | 4-F-2-OCH₃ | H | H | H | H | CH₃ | |
| 2.047 | 4-F-2-OCH₃ | 3-CF₃ | H | H | H | CH₃ | |
| 2.048 | 4-F-2-OCH₃ | 3-CHF₂ | H | H | H | CH₃ | |
| 2.049 | 4-F-2-OCH₃ | 3-SCH₃ | H | H | H | CH₃ | NMR |
| 2.050 | 4-F-2-OCH₃ | 3-S(O)CH₃ | H | H | H | CH₃ | |
| 2.051 | 4-F-2-OCH₃ | 3-S(O)₂CH₃ | H | H | H | CH₃ | |
| 2.052 | 4-F-2-OCH₃ | 3-CH₂CN | H | H | H | CH₃ | |
| 2.053 | 4-F-2-OCH₃ | 4-F-3-SCH₃ | H | H | H | CH₃ | |
| 2.054 | 4-F-2-OCH₃ | 4-F-3-S(O) CH₃ | H | H | H | CH₃ | |
| 2.055 | 4-F-2-OCH₃ | 4-F-3-S(O)₂CH₃ | H | H | H | CH₃ | |
| 2.056 | 4-F-2-OCH₃ | 3-SCH₂CH₃ | H | H | H | CH₃ | |
| 2.057 | 4-F-2-OCH₃ | 3-S(O)CH₂CH₃ | H | H | H | CH₃ | |
| 2.058 | 4-F-2-OCH₃ | 3-S(O)₂CH₂CH₃ | H | H | H | CH₃ | |
| 2.059 | 2-Cl | H | H | H | H | CH₃ | |
| 2.060 | 2-Cl | 3-CF₃ | H | H | H | CH₃ | |
| 2.061 | 2-Cl | 3-CHF₂ | H | H | H | CH₃ | |
| 2.062 | 2-Cl | 3-SCH₃ | H | H | H | CH₃ | |
| 2.063 | 2-Cl | 3-S(O)CH₃ | H | H | H | CH₃ | |
| 2.064 | 2-Cl | 3-S(O)₂CH₃ | H | H | H | CH₃ | |
| 2.065 | 2-Cl | 3-CH₂CN | H | H | H | CH₃ | |
| 2.066 | 2-Cl | 4-F-3-SCH₃ | H | H | H | CH₃ | |
| 2.067 | 2-Cl | 4-F-3-S(O) CH₃ | H | H | H | CH₃ | |
| 2.068 | 2-Cl | 4-F-3-S(O)₂CH₃ | H | H | H | CH₃ | |
| 2.069 | 2-Cl | 3-SCH₂CH₃ | H | H | H | CH₃ | |
| 2.070 | 2-Cl | 3-S(O)CH₂CH₃ | H | H | H | CH₃ | |
| 2.071 | 2-Cl | 3-S(O)₂CH₂CH₃ | H | H | H | CH₃ | |
| 2.072 | 2,4-Cl₂ | H | H | H | H | CH₃ | |
| 2.073 | 2,4-Cl₂ | 3-CF₃ | H | H | H | CH₃ | |
| 2.074 | 2,4-Cl₂ | 3-CHF₂ | H | H | H | CH₃ | |
| 2.075 | 2,4-Cl₂ | 3-SCH₃ | H | H | H | CH₃ | |
| 2.076 | 2,4-Cl₂ | 3-S(O)CH₃ | H | H | H | CH₃ | |
| 2.077 | 2,4-Cl₂ | 3-S(O)₂CH₃ | H | H | H | CH₃ | |
| 2.078 | 2,4-Cl₂ | 3-CH₂CN | H | H | H | CH₃ | |
| 2.079 | 2,4-Cl₂ | 4-F-3-SCH₃ | H | H | H | CH₃ | |
| 2.080 | 2,4-Cl₂ | 4-F-3-S(O) CH₃ | H | H | H | CH₃ | |
| 2.081 | 2,4-Cl₂ | 4-F-3-S(O)₂CH₃ | H | H | H | CH₃ | |
| 2.082 | 2,4-Cl₂ | 3-SCH₂CH₃ | H | H | H | CH₃ | |
| 2.083 | 2,4-Cl₂ | 3-S(O)CH₂CH₃ | H | H | H | CH₃ | |
| 2.084 | 2,4-Cl₂ | 3-S(O)₂CH₂CH₃ | H | H | H | CH₃ | |
| 2.085 | 2,4-F₂ | H | H | H | H | CH₃ | |
| 2.086 | 2,4-F₂ | 3-CF₃ | H | H | H | CH₃ | |
| 2.087 | 2,4-F₂ | 3-CHF₂ | H | H | H | CH₃ | |
| 2.088 | 2,4-F₂ | 3-SCH₃ | H | H | H | CH₃ | |
| 2.089 | 2,4-F₂ | 3-S(O)CH₃ | H | H | H | CH₃ | |
| 2.090 | 2,4-F₂ | 3-S(O)₂CH₃ | H | H | H | CH₃ | |
| 2.091 | 2,4-F₂ | 3-CH₂CN | H | H | H | CH₃ | |
| 2.092 | 2,4-F₂ | 4-F-3-SCH₃ | H | H | H | CH₃ | |
| 2.093 | 2,4-F₂ | 4-F-3-S(O) CH₃ | H | H | H | CH₃ | |
| 2.094 | 2,4-F₂ | 4-F-3-S(O)₂CH₃ | H | H | H | CH₃ | |
| 2.095 | 2,4-F₂ | 3-SCH₂CH₃ | H | H | H | CH₃ | |
| 2.096 | 2,4-F₂ | 3-S(O)CH₂CH₃ | H | H | H | CH₃ | |
| 2.097 | 2,4-F₂ | 3-S(O)₂CH₂CH₃ | H | H | H | CH₃ | |
| 2.098 | 2-Cl | H | H | H | CH₃ | CH₃ | |
| 2.099 | 2-Cl | 3-CF₃ | H | H | CH₃ | CH₃ | |
| 2.100 | 2-Cl | 3-SCH₃ | H | H | CH₃ | CH₃ | |
| 2.101 | 2-Cl | 3-SCH₃ | H | H | H | H | |
| 2.102 | 2-Cl | 3-SCH₃ | H | H | cPr | CH₃ | |
| 2.103 | 2-Cl | 3-S(O)CH₃ | H | H | CH₃ | CH₃ | |
| 2.104 | 2-Cl | 3-S(O)₂CH₃ | H | H | CH₃ | CH₃ | |
| 2.105 | 2-Cl | 3-CH₂CN | H | H | CH₃ | CH₃ | |
| 2.106 | 2-Cl | 3-CH₂CN | H | H | H | H | |
| 2.107 | 2-Cl | 3-CH₂CN | H | H | cPr | CH₃ | |
| 2.108 | 2-Cl | 4-F-3-SCH₃ | H | H | CH₃ | CH₃ | |
| 2.109 | 2-Cl | 3-SCH₂CH₃ | H | H | CH₃ | CH₃ | |
| 2.110 | 2-Cl | 3-S(O)CH₂CH₃ | H | H | CH₃ | CH₃ | |
| 2.111 | 2-Cl | 3-S(O)₂CH₂CH₃ | H | H | CH₃ | CH₃ | |
| 2.112 | 4-F-2-OCH₃ | H | H | H | CH₃ | CH₃ | |
| 2.113 | 4-F-2-OCH₃ | 3-CF₃ | H | H | CH₃ | CH₃ | |
| 2.114 | 4-F-2-OCH₃ | 3-SCH₃ | H | H | CH₃ | CH₃ | |
| 2.115 | 4-F-2-OCH₃ | 3-SCH₃ | H | H | H | H | |
| 2.116 | 4-F-2-OCH₃ | 3-SCH₃ | H | H | cPr | CH₃ | |
| 2.117 | 4-F-2-OCH₃ | 3-S(O)CH₃ | H | H | CH₃ | CH₃ | |
| 2.118 | 4-F-2-OCH₃ | 3-S(O)₂CH₃ | H | H | CH₃ | CH₃ | |
| 2.119 | 4-F-2-OCH₃ | 3-CH₂CN | H | H | CH₃ | CH₃ | |
| 2.120 | 4-F-2-OCH₃ | 3-CH₂CN | H | H | H | H | |
| 2.121 | 4-F-2-OCH₃ | 3-CH₂CN | H | H | cPr | CH₃ | |
| 2.122 | 4-F-2-OCH₃ | 4-F-3-SCH₃ | H | H | CH₃ | CH₃ | |
| 2.123 | 4-F-2-OCH₃ | 3-SCH₂CH₃ | H | H | CH₃ | CH₃ | |
| 2.124 | 4-F-2-OCH₃ | 3-S(O)CH₂CH₃ | H | H | CH₃ | CH₃ | |
| 2.125 | 4-F-2-OCH₃ | 3-S(O)₂CH₂CH₃ | H | H | CH₃ | CH₃ | |
| 2.126 | 2,4-Cl₂ | H | H | H | CH₃ | CH₃ | |
| 2.127 | 2,4-Cl₂ | 3-CF₃ | H | H | CH₃ | CH₃ | |
| 2.128 | 2,4-Cl₂ | 3-SCH₃ | H | H | CH₃ | CH₃ | |
| 2.129 | 2,4-Cl₂ | 3-SCH₃ | H | H | H | H | |
| 2.130 | 2,4-Cl₂ | 3-SCH₃ | H | H | cPr | CH₃ | |
| 2.131 | 2,4-Cl₂ | 3-S(O)CH₃ | H | H | CH₃ | CH₃ | |
| 2.132 | 2,4-Cl₂ | 3-S(O)₂CH₃ | H | H | CH₃ | CH₃ | |
| 2.133 | 2,4-Cl₂ | 3-CH₂CN | H | H | CH₃ | CH₃ | |
| 2.134 | 2,4-Cl₂ | 3-CH₂CN | H | H | H | H | |
| 2.135 | 2,4-Cl₂ | 3-CH₂CN | H | H | cPr | CH₃ | |
| 2.136 | 2,4-Cl₂ | 4-F-3-SCH₃ | H | H | CH₃ | CH₃ | |
| 2.137 | 2,4-Cl₂ | 3-SCH₂CH₃ | H | H | CH₃ | CH₃ | |
| 2.138 | 2,4-Cl₂ | 3-S(O)CH₂CH₃ | H | H | CH₃ | CH₃ | |
| 2.139 | 2,4-Cl₂ | 3-S(O)₂CH₂CH₃ | H | H | CH₃ | CH₃ | |
| 2.140 | 2-CN | H | H | H | CH₃ | CH₃ | |
| 2.141 | 2-CN | 3-CF₃ | H | H | CH₃ | CH₃ | |
| 2.142 | 2-CN | 3-SCH₃ | H | H | CH₃ | CH₃ | |
| 2.143 | 2-CN | 3-SCH₃ | H | H | H | H | |
| 2.144 | 2-CN | 3-SCH₃ | H | H | cPr | CH₃ | |
| 2.145 | 2-CN | 3-S(O)CH₃ | H | H | CH₃ | CH₃ | |
| 2.146 | 2-CN | 3-S(O)₂CH₃ | H | H | CH₃ | CH₃ | |
| 2.147 | 2-CN | 3-CH₂CN | H | H | CH₃ | CH₃ | |
| 2.148 | 2-CN | 3-CH₂CN | H | H | H | H | |
| 2.149 | 2-CN | 3-CH₂CN | H | H | cPr | CH₃ | |
| 2.150 | 2-CN | 4-F-3-SCH₃ | H | H | CH₃ | CH₃ | |
| 2.151 | 2-CN | 3-SCH₂CH₃ | H | H | CH₃ | CH₃ | |
| 2.152 | 2-CN | 3-S(O)CH₂CH₃ | H | H | CH₃ | CH₃ | |
| 2.153 | 2-CN | 3-S(O)₂CH₂CH₃ | H | H | CH₃ | CH₃ | |

**Tabelle 3: Verbindungen der Formel (Ia-Q2-1)**

| | | | | | | |
|---|---|---|---|---|---|---|
| 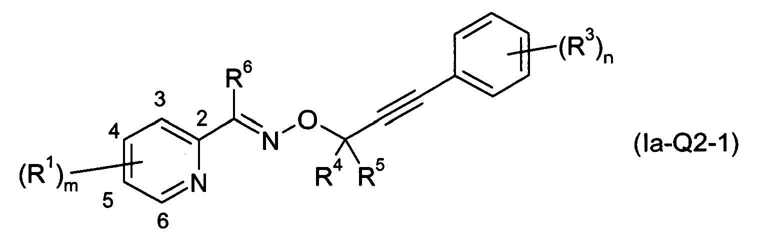 | | | | | | |

| Nr. | (R¹)ₘ | (R³)ₙ | R⁴ | R⁵ | R⁶ | Daten |
|---|---|---|---|---|---|---|
| 3.001 | H | H | H | H | H | |
| 3.002 | H | 3-CF₃ | H | H | H | |
| 3.003 | H | 3-CHF₂ | H | H | H | |
| 3.004 | H | 3-SCH₃ | H | H | H | |
| 3.005 | H | 3-S(O)CH₃ | H | H | H | |
| 3.006 | H | 3-S(O)₂CH₃ | H | H | H | |
| 3.007 | H | 3-SCF₃ | H | H | H | |
| 3.008 | H | 3-S(O)CF₃ | H | H | H | |
| 3.009 | H | 3-S(O)₂CF₃ | H | H | H | |
| 3.010 | H | 3-CH₂CN | H | H | H | |
| 3.011 | H | 4-F-3-SCH₃ | H | H | H | |
| 3.012 | H | 4-F-3-S(O) CH₃ | H | H | H | |
| 3.013 | H | 4-F-3-S(O)₂CH₃ | H | H | H | |
| 3.014 | H | 3-SCH₂CH₃ | H | H | H | |
| 3.015 | H | 3-S(O)CH₂CH₃ | H | H | H | |
| 3.016 | H | 3-S(O)₂CH₂CH₃ | H | H | H | |
| 3.017 | H | 3-OCH₃ | H | H | H | |
| 3.018 | H | 3,5-(CF₃)₂ | H | H | H | |
| 3.019 | 5-Br | H | H | H | H | |
| 3.020 | 5-Br | 3-CF₃ | H | H | H | |
| 3.021 | 5-Br | 3-CHF₂ | H | H | H | |
| 3.022 | 5-Br | 3-SCH₃ | H | H | H | NMR |
| 3.023 | 5-Br | 3-S(O)CH₃ | H | H | H | NMR |
| 3.024 | 5-Br | 3-S(O)₂CH₃ | H | H | H | NMR |
| 3.025 | 5-Br | 3-SCF₃ | H | H | H | |
| 3.026 | 5-Br | 3-CH₂CN | H | H | H | |
| 3.027 | 5-Br | 4-F-3-SCH₃ | H | H | H | |
| 3.028 | 5-Br | 3-SCH₂CH₃ | H | H | H | |
| 3.029 | 5-Br | 3-S(O)CH₂CH₃ | H | H | H | |
| 3.030 | 5-Br | 3-S(O)₂CH₂CH₃ | H | H | H | |
| 3.031 | 5-Br | 3-OCH₃ | H | H | H | |
| 3.032 | 4-Cl-3-CH₃ | H | H | H | H | |
| 3.033 | 4-Cl-3-CH₃ | 3-CF₃ | H | H | H | |
| 3.034 | 4-Cl-3-CH₃ | 3-CHF₂ | H | H | H | |
| 3.035 | 4-Cl-3-CH₃ | 3-SCH₃ | H | H | H | |
| 3.036 | 4-Cl-3-CH₃ | 3-S(O)CH₃ | H | H | H | |
| 3.037 | 4-Cl-3-CH₃ | 3-S(O)₂CH₃ | H | H | H | |
| 3.038 | 4-Cl-3-CH₃ | 3-SCF₃ | H | H | H | |
| 3.039 | 4-Cl-3-CH₃ | 3-CH₂CN | H | H | H | |
| 3.040 | 4-Cl-3-CH₃ | 4-F-3-SCH₃ | H | H | H | |
| 3.041 | 4-Cl-3-CH₃ | 3-SCH₂CH₃ | H | H | H | |
| 3.042 | 4-Cl-3-CH₃ | 3-S(O)CH₂CH₃ | H | H | H | |
| 3.043 | 4-Cl-3-CH₃ | 3-S(O)₂CH₂CH₃ | H | H | H | |
| 3.044 | 4-Cl-3-CH₃ | 3-OCH₃ | H | H | H | |
| 3.045 | 3-CH₃-4-OCH₃ | H | H | H | H | |
| 3.046 | 3-CH₃-4-OCH₃ | 3-CF₃ | H | H | H | |
| 3.047 | 3-CH₃-4-OCH₃ | 3-CHF₂ | H | H | H | |
| 3.048 | 3-CH₃-4-OCH₃ | 3-SCH₃ | H | H | H | |
| 3.049 | 3-CH₃-4-OCH₃ | 3-S(O)CH₃ | H | H | H | |
| 3.050 | 3-CH₃-4-OCH₃ | 3-S(O)₂CH₃ | H | H | H | |
| 3.051 | 3-CH₃-4-OCH₃ | 3-SCF₃ | H | H | H | |
| 3.052 | 3-CH₃-4-OCH₃ | 3-CH₂CN | H | H | H | |
| 3.053 | 3-CH₃-4-OCH₃ | 4-F-3-SCH₃ | H | H | H | |
| 3.054 | 3-CH₃-4-OCH₃ | 3-SCH₂CH₃ | H | H | H | |
| 3.055 | 3-CH₃-4-OCH₃ | 3-S(O)CH₂CH₃ | H | H | H | |
| 3.056 | 3-CH₃-4-OCH₃ | 3-S(O)₂CH₂CH₃ | H | H | H | |
| 3.057 | 3-CH₃-4-OCH₃ | 3-OCH₃ | H | H | H | |
| 3.058 | 3-CF₃ | H | H | H | H | |
| 3.059 | 3-CF₃ | 3-CF₃ | H | H | H | |
| 3.060 | 3-CF₃ | 3-CHF₂ | H | H | H | |
| 3.061 | 3-CF₃ | 3-SCH₃ | H | H | H | NMR |
| 3.062 | 3-CF₃ | 3-S(O)CH₃ | H | H | H | NMR |
| 3.063 | 3-CF₃ | 3-S(O)₂CH₃ | H | H | H | NMR |
| 3.064 | 3-CF₃ | 3-SCF₃ | H | H | H | |
| 3.065 | 3-CF₃ | 3-CH₂CN | H | H | H | |
| 3.066 | 3-CF₃ | 4-F-3-SCH₃ | H | H | H | |
| 3.067 | 3-CF₃ | 3-SCH₂CH₃ | H | H | H | |
| 3.068 | 3-CF₃ | 3-S(O)CH₂CH₃ | H | H | H | |
| 3.069 | 3-CF₃ | 3-S(O)₂CH₂CH₃ | H | H | H | |
| 3.070 | 3-CF₃ | 3-OCH₃ | H | H | H | |
| 3.071 | 6-CH₃ | H | H | H | H | |
| 3.072 | 6-CH₃ | 3-CF₃ | H | H | H | |
| 3.073 | 6-CH₃ | 3-CHF₂ | H | H | H | |
| 3.074 | 6-CH₃ | 3-SCH₃ | H | H | H | NMR |
| 3.075 | 6-CH₃ | 3-S(O)CH₃ | H | H | H | |
| 3.076 | 6-CH₃ | 3-S(O)₂CH₃ | H | H | H | |
| 3.077 | 6-CH₃ | 3-SCF₃ | H | H | H | |
| 3.078 | 6-CH₃ | 3-CH₂CN | H | H | H | |
| 3.079 | 6-CH₃ | 4-F-3-SCH₃ | H | H | H | |
| 3.080 | 6-CH₃ | 3-SCH₂CH₃ | H | H | H | |
| 3.081 | 6-CH₃ | 3-S(O)CH₂CH₃ | H | H | H | |
| 3.082 | 6-CH₃ | 3-S(O)₂CH₂CH₃ | H | H | H | |
| 3.083 | 6-CH₃ | 3-OCH₃ | H | H | H | |
| 3.084 | 6-CH₃ | H | CH₃ | H | H | |
| 3.085 | 6-CH₃ | 3-SCH₃ | CH₃ | H | H | LogP |
| 3.086 | 6-CH₃ | 3-S(O)CH₃ | CH₃ | H | H | LogP |
| 3.087 | 6-CH₃ | 3-S(O)₂CH₃ | CH₃ | H | H | |
| 3.088 | 6-CH₃ | 3-CH₂CN | CH₃ | H | H | |
| 3.089 | 6-CH₃ | 3-CH₂CN | CH₃ | CH₃ | H | |
| 3.090 | 6-CH₃ | 3-SCH₂CH₃ | CH₃ | H | H | |
| 3.091 | 6-CH₃ | 3-SCH₃ | H | H | CH₃ | LogP |
| 3.092 | 6-CH₃ | 3-S(O)CH₃ | H | H | CH₃ | LogP |
| 3.093 | 6-CH₃ | 3-S(O)₂CH₃ | H | H | CH₃ | LogP |
| 3.094 | 6-CH₃ | 3-CH₂CN | H | H | CH₃ | LogP |
| 3.095 | 6-CH₃ | 3-SCH₃ | H | H | CH₂CH₃ | LogP |
| 3.096 | 6-CH₃ | 3-S(O)CH₃ | H | H | CH₂CH₃ | LogP |
| 3.097 | 6-CH₃ | 3-S(O)₂CH₃ | H | H | CH₂CH₃ | LogP |
| 3.098 | 6-CH₃ | 3-CH₂CN | H | H | CH₂CH₃ | LogP |
| 3.099 | H | H | CH₃ | H | H | |
| 3.100 | H | 3-SCH₃ | CH₃ | H | H | |
| 3.101 | H | 3-SCH₃ | CH₂CH₃ | H | H | |
| 3.102 | H | 3-S(O)CH₃ | CH₃ | H | H | |
| 3.103 | H | 3-S(O)₂CH₃ | CH₃ | H | H | |
| 3.104 | H | 3-CH₂CN | CH₃ | H | H | |
| 3.105 | H | 3-CH₂CN | CH₃ | CH₃ | H | |
| 3.106 | H | 3-SCH₂CH₃ | CH₃ | H | H | |
| 3.107 | H | H | CH₃ | H | CH₃ | |
| 3.108 | H | 3-SCH₃ | CH₃ | H | CH₃ | |
| 3.109 | H | 3-SCH₃ | CH₂CH₃ | H | CH₃ | |
| 3.110 | H | 3-S(O)CH₃ | CH₃ | H | CH₃ | |
| 3.111 | H | 3-S(O)₂CH₃ | CH₃ | H | CH₃ | |
| 3.112 | H | 3-CH₂CN | CH₃ | H | CH₃ | |
| 3.113 | H | 3-CH₂CN | CH₃ | CH₃ | CH₃ | |
| 3.114 | H | 3-SCH₂CH₃ | CH₃ | H | CH₃ | |
| 3.115 | H | H | CH₂OCH₃ | H | H | |
| 3.116 | H | 3-SCH₃ | CH₂OCH₃ | H | H | |
| 3.117 | H | 3-S(O)CH₃ | CH₂OCH₃ | H | H | |
| 3.118 | H | 3-S(O)₂CH₃ | CH₂OCH₃ | H | H | |
| 3.119 | H | CH₂CN | CH₂OCH | H | H | |
| 3.120 | 5-Cl-4-CF₃ | 3-SCH₃ | H | H | H | LogP |
| 3.121 | 5-Cl-4-CF₃ | 3-S(O)CH₃ | H | H | H | LogP |
| 3.122 | 5-Cl-4-CF₃ | 4-F-3,5-(SCH₃)₂ | H | H | H | NMR |
| 3.123 | 5-Cl-4-CF₃ | 4-CF₃ | H | H | H | |
| 3.124 | 5-Cl-4-CF₃ | 2-C(=O)OCH₃ | H | H | H | |
| 3.125 | 5-F-6-OCH₂CH₃ | 3-SCH₃ | H | H | H | LogP |
| 3.126 | 5-F-6-OCH₂CH₃ | 3-S(O)CH₃ | H | H | H | LogP |
| 3.127 | 5-F-6-OCH₂CH₃ | 2-C(=O)OCH₃ | H | H | H | |

**Tabelle 4: Verbindungen der Formel (Ia-Q5)**

| | | | | | | |
|---|---|---|---|---|---|---|
| 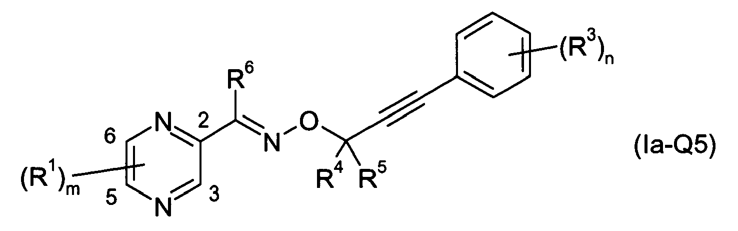 | | | | | | |

| Nr. | (R¹)ₘ | (R³)ₙ | R⁴ | R⁵ | R⁶ | Daten |
|---|---|---|---|---|---|---|
| 4.001 | H | H | H | H | H | |
| 4.002 | H | 3-CF₃ | H | H | H | |
| 4.003 | H | 3-CHF₂ | H | H | H | |
| 4.004 | H | 3-SCH₃ | H | H | H | NMR |
| 4.005 | H | 3-S(O)CH₃ | H | H | H | NMR |
| 4.006 | H | 3-S(O)₂CH₃ | H | H | H | NMR |
| 4.007 | H | 3-SCF₃ | H | H | H | |
| 4.008 | H | 3-S(O)CF₃ | H | H | H | |
| 4.009 | H | 3-S(O)₂CF₃ | H | H | H | |
| 4.010 | H | 3-CH₂CN | H | H | H | NMR |
| 4.011 | H | 4-F-3-SCH₃ | H | H | H | |
| 4.012 | H | 4-F-3-S(O) CH₃ | H | H | H | |
| 4.013 | H | 4-F-3-S(O)₂CH₃ | H | H | H | |
| 4.014 | H | 3-SCH₂CH₃ | H | H | H | |
| 4.015 | H | 3-S(O)CH₂CH₃ | H | H | H | |
| 4.016 | H | 3-S(O)₂CH₂CH₃ | H | H | H | |
| 4.017 | H | 3-OCH₃ | H | H | H | NMR |
| 4.018 | H | 3,5-(CF₃)₂ | H | H | H | |
| 4.019 | 3-Cl | H | H | H | H | |
| 4.020 | 3-Cl | 3-CF₃ | H | H | H | |
| 4.021 | 3-Cl | 3-CHF₂ | H | H | H | |
| 4.022 | 3-Cl | 3-SCH₃ | H | H | H | |
| 4.023 | 3-Cl | 3-S(O)CH₃ | H | H | H | |
| 4.024 | 3-Cl | 3-S(O)₂CH₃ | H | H | H | |
| 4.025 | 3-Cl | 3-SCF₃ | H | H | H | |
| 4.026 | 3-Cl | 3-S(O)CF₃ | H | H | H | |
| 4.027 | 3-Cl | 3-S(O)₂CF₃ | H | H | H | |
| 4.028 | 3-Cl | 3-CH₂CN | H | H | H | |
| 4.029 | 3-Cl | 4-F-3-SCH₃ | H | H | H | |
| 4.030 | 3-Cl | 4-F-3-S(O) CH₃ | H | H | H | |
| 4.031 | 3-Cl | 4-F-3-S(O)₂CH₃ | H | H | H | |
| 4.032 | 3-Cl | 3-SCH₂CH₃ | H | H | H | |
| 4.033 | 3-Cl | 3-S(O)CH₂CH₃ | H | H | H | |
| 4.034 | 3-Cl | 3-S(O)₂CH₂CH₃ | H | H | H | |
| 4.035 | 3-Cl | 3-OCH₃ | H | H | H | |
| 4.036 | 3-Cl | 3,5-(CF₃)₂ | H | H | H | |
| 4.037 | H | H | CH₃ | H | H | |
| 4.038 | H | 3-CF₃ | CH₃ | H | H | |
| 4.039 | H | 3-CHF₂ | CH₃ | H | H | |
| 4.040 | H | 3-SCH₃ | CH₃ | H | H | |
| 4.041 | H | 3-S(O)CH₃ | CH₃ | H | H | |
| 4.042 | H | 3-S(O)₂CH₃ | CH₃ | H | H | |
| 4.043 | H | 3-SCF₃ | CH₃ | H | H | |
| 4.044 | H | 3-CH₂CN | CH₃ | H | H | |
| 4.045 | H | 4-F-3-SCH₃ | CH₃ | H | H | |
| 4.046 | H | 3-SCH₂CH₃ | CH₃ | H | H | |
| 4.047 | H | 3-S(O)CH₂CH₃ | CH₃ | H | H | |
| 4.048 | H | 3-S(O)₂CH₂CH₃ | CH₃ | H | H | |
| 4.049 | H | 3-OCH₃ | CH₃ | H | H | |
| 4.050 | 3-Cl | H | CH₃ | H | H | |
| 4.051 | 3-Cl | 3-CF₃ | CH₃ | H | H | |
| 4.052 | 3-Cl | 3-CHF₂ | CH₃ | H | H | |
| 4.053 | 3-Cl | 3-SCH₃ | CH₃ | H | H | |
| 4.054 | 3-Cl | 3-S(O)CH₃ | CH₃ | H | H | |
| 4.055 | 3-Cl | 3-S(O)₂CH₃ | CH₃ | H | H | |
| 4.056 | 3-Cl | 3-SCF₃ | CH₃ | H | H | |
| 4.057 | 3-Cl | 3-S(O)CF₃ | CH₃ | H | H | |
| 4.058 | 3-Cl | 3-S(O)₂CF₃ | CH₃ | H | H | |
| 4.059 | 3-Cl | 3-CH₂CN | CH₃ | H | H | |
| 4.060 | 3-Cl | 4-F-3-SCH₃ | CH₃ | H | H | |
| 4.061 | 3-Cl | 3-SCH₂CH₃ | CH₃ | H | H | |
| 4.062 | 3-Cl | 3-OCH₃ | CH₃ | H | H | |
| 4.063 | H | H | -(CH₂)₄- | | H | |
| 4.064 | H | 3-CHF₂ | -(CH₂)₄- | | H | |
| 4.065 | H | 3-SCH₃ | -(CH₂)₄- | | H | |
| 4.066 | H | 3-S(O)CH₃ | -(CH₂)₄- | | H | |
| 4.067 | H | 3-S(O)₂CH₃ | -(CH₂)₄- | | H | |
| 4.068 | H | 3-CH₂CN | -(CH₂)₄- | | H | |
| 4.069 | H | 3-OCH₃ | -(CH₂)₄- | | H | |
| 4.070 | 3-Cl | H | -(CH₂)₄- | | H | |
| 4.071 | 3-Cl | 3-CHF₂ | -(CH₂)₄- | | H | |
| 4.072 | 3-Cl | 3-SCH₃ | -(CH₂)₄- | | H | |
| 4.073 | 3-Cl | 3-S(O)CH₃ | -(CH₂)₄- | | H | |
| 4.074 | 3-Cl | 3-S(O)₂CH₃ | -(CH₂)₄- | | H | |
| 4.075 | 3-Cl | 3-CH₂CN | -(CH₂)₄- | | H | |
| 4.076 | 3-Cl | 3-OCH₃ | -(CH₂)₄- | | H | |
| 4.077 | H | 4-CH₃-3-SCH₃ | H | H | H | NMR |
| 4.078 | 3-SCH₃ | 3-SCH₃ | H | H | H | LogP |
| 4.079 | 3-SCH₃ | 4-OCH₃ | H | H | H | |
| 4.080 | 3-SCH₃ | 3-CH₂CN | H | H | H | LogP |
| 4.081 | 3-SCH₃ | 2-F | H | H | H | |
| 4.082 | 3-SCH₃ | 2-CF₃ | H | H | H | |
| 4.083 | 3-SCH₃ | 4-F-3-SCH₃ | H | H | H | |
| 4.084 | 3-OCH₃ | 3-SCH₃ | H | H | H | LogP |
| 4.085 | 3-OCH₃ | 4-OCH₃ | H | H | H | |
| 4.086 | 3-OCH₃ | 3-CH₂CN | H | H | H | LogP |
| 4.087 | 3-OCH₃ | 2-F | H | H | H | |
| 4.088 | 3-OCH₃ | 2-CF₃ | H | H | H | |
| 4.089 | 3-OCH₃ | 4-F-3-SCH₃ | H | H | H | |
| 4.090 | 3-OCH₃ | 3-S(O)CH₃ | H | H | H | LogP |
| 4.091 | 3-OCH₃ | 3-S(O)₂CH₃ | H | H | H | LogP |
| 4.092 | 6-Cl-3-OCH₃ | 3-SCH₃ | H | H | H | LogP |
| 4.093 | 6-Cl-3-OCH₃ | 4-OCH₃ | H | H | H | |
| 4.094 | 6-Cl-3-OCH₃ | 3-CH₂CN | H | H | H | LogP |
| 4.095 | 6-Cl-3-OCH₃ | 2-F | H | H | H | |
| 4.096 | 6-Cl-3-OCH₃ | 2-CF₃ | H | H | H | |
| 4.097 | 6-Cl-3-OCH₃ | 4-F-3-SCH₃ | H | H | H | |
| 4.098 | 6-Cl-3-OCH₃ | 3-S(O)CH₃ | H | H | H | LogP |
| 4.099 | 6-Cl-3-OCH₃ | 3-S(O)₂CH₃ | H | H | H | |
| 4.100 | 5-Cl-3-OCH₃ | 3-SCH₃ | H | H | H | LogP |
| 4.101 | 5-Cl-3-OCH₃ | 4-OCH₃ | H | H | H | |
| 4.102 | 5-Cl-3-OCH₃ | 3-CH₂CN | H | H | H | |
| 4.103 | 5-Cl-3-OCH₃ | 2-F | H | H | H | |
| 4.104 | 5-Cl-3-OCH₃ | 2-CF₃ | H | H | H | |
| 4.105 | 5-Cl-3-OCH₃ | 4-F-3-SCH₃ | H | H | H | |
| 4.106 | 5-Cl-3-OCH₃ | 3-S(O)CH₃ | H | H | H | LogP |
| 4.107 | 5-Cl-3-OCH₃ | 3-S(O)₂CH₃ | H | H | H | |

**Tabelle 5: Verbindungen der Formel (la-Q2-2)**

| | | | | | | |
|---|---|---|---|---|---|---|
| 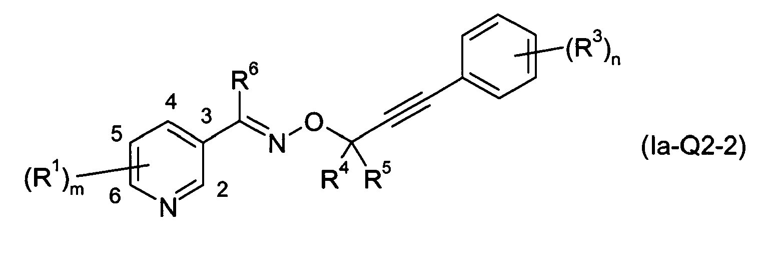 | | | | | | |

| Nr. | (R¹)ₘ | (R³)ₙ | R⁴ | R⁵ | R⁶ | Daten |
|---|---|---|---|---|---|---|
| 5.001 | H | H | H | H | H | |
| 5.002 | H | 3-CF₃ | H | H | H | |
| 5.003 | H | 3-CHF₂ | H | H | H | |
| 5.004 | H | 3-SCH₃ | H | H | H | NMR |
| 5.005 | H | 3-S(O)CH₃ | H | H | H | |
| 5.006 | H | 3-S(O)₂CH₃ | H | H | H | NMR |
| 5.007 | H | 3-SCF₃ | H | H | H | |
| 5.008 | H | 3-S(O)CF₃ | H | H | H | |
| 5.009 | H | 3-S(O)₂CF₃ | H | H | H | |
| 5.010 | H | 3-CH₂CN | H | H | H | NMR |
| 5.011 | H | 4-F-3-SCH₃ | H | H | H | |
| 5.012 | H | 4-F-3-S(O) CH₃ | H | H | H | |
| 5.013 | H | 4-F-3-S(O)₂CH₃ | H | H | H | |
| 5.014 | H | 3-SCH₂CH₃ | H | H | H | |
| 5.015 | H | 3-S(O)CH₂CH₃ | H | H | H | |
| 5.016 | H | 3-S(O)₂CH₂CH₃ | H | H | H | |
| 5.017 | H | 3-OCH₃ | H | H | H | NMR |
| 5.018 | H | 3,5-(CF₃)₂ | H | H | H | |
| 5.019 | 2-OCH₃ | H | H | H | H | |
| 5.020 | 2-OCH₃ | 3-CF₃ | H | H | H | |
| 5.021 | 2-OCH₃ | 3-CHF₂ | H | H | H | |
| 5.022 | 2-OCH₃ | 3-SCH₃ | H | H | H | NMR |
| 5.023 | 2-OCH₃ | 3-S(O)CH₃ | H | H | H | |
| 5.024 | 2-OCH₃ | 3-S(O)₂CH₃ | H | H | H | NMR |
| 5.025 | 2-OCH₃ | 3-SCF₃ | H | H | H | |
| 5.026 | 2-OCH₃ | 3-CH₂CN | H | H | H | NMR |
| 5.027 | 2-OCH₃ | 4-F-3-SCH₃ | H | H | H | |
| 5.028 | 2-OCH₃ | 3-SCH₂CH₃ | H | H | H | |
| 5.029 | 2-OCH₃ | 3-S(O)CH₂CH₃ | H | H | H | |
| 5.030 | 2-OCH₃ | 3-S(O)₂CH₂CH₃ | H | H | H | |
| 5.031 | 2-OCH₃ | 3-OCH₃ | H | H | H | NMR |
| 5.032 | 2-OCH₂CH₃ | 3-SCH₃ | H | H | H | NMR |
| 5.033 | 2-OCH₂CH₃ | 3-S(O)CH₃ | H | H | H | NMR |
| 5.034 | 2-OCH₂CH₃ | 3-S(O)₂CH₃ | H | H | H | NMR |
| 5.035 | 2-OCH₂CH₃ | 3-SCF₃ | H | H | H | |
| 5.036 | 2-OCH₂CH₃ | 3-CH₂CN | H | H | H | NMR |
| 5.037 | 5-F-6-OCH₃ | H | H | H | H | |
| 5.038 | 5-F-6-OCH₃ | 3-CF₃ | H | H | H | |
| 5.039 | 5-F-6-OCH₃ | 3-CHF₂ | H | H | H | |
| 5.040 | 5-F-6-OCH₃ | 3-SCH₃ | H | H | H | LogP |
| 5.041 | 5-F-6-OCH₃ | 3-S(O)CH₃ | H | H | H | LogP |
| 5.042 | 5-F-6-OCH₃ | 3-S(O)₂CH₃ | H | H | H | |
| 5.043 | 5-F-6-OCH₃ | 3-SCF₃ | H | H | H | |
| 5.044 | 5-F-6-OCH₃ | 3-CH₂CN | H | H | H | LogP |
| 5.045 | 5-F-6-OCH₃ | 4-F-3-SCH₃ | H | H | H | |
| 5.046 | 5-F-6-OCH₃ | 3-SCH₂CH₃ | H | H | H | |
| 5.047 | 5-F-6-OCH₃ | 3-S(O)CH₂CH₃ | H | H | H | |
| 5.048 | 5-F-6-OCH₃ | 3-S(O)₂CH₂CH₃ | H | H | H | |
| 5.049 | 5-F-6-OCH₃ | 3-OCH₃ | H | H | H | |
| 5.050 | 6-OCH₂CF₃ | H | H | H | H | |
| 5.051 | 6-OCH₂CF₃ | 3-CF₃ | H | H | H | |
| 5.052 | 6-OCH₂CF₃ | 3-CHF₂ | H | H | H | |
| 5.053 | 6-OCH₂CF₃ | 3-SCH₃ | H | H | H | NMR |
| 5.054 | 6-OCH₂CF₃ | 3-S(O)CH₃ | H | H | H | |
| 5.055 | 6-OCH₂CF₃ | 3-S(O)₂CH₃ | H | H | H | |
| 5.056 | 6-OCH₂CF₃ | 3-SCF₃ | H | H | H | |
| 5.057 | 6-OCH₂CF₃ | 3-CH₂CN | H | H | H | |
| 5.058 | 6-OCH₂CF₃ | 4-F-3-SCH₃ | H | H | H | |
| 5.059 | 6-OCH₂CF₃ | 3-SCH₂CH₃ | H | H | H | |
| 5.060 | 6-OCH₂CF₃ | 3-S(O)CH₂CH₃ | H | H | H | |
| 5.061 | 6-OCH₂CF₃ | 3-S(O)₂CH₂CH₃ | H | H | H | |
| 5.062 | 6-OCH₂CF₃ | 3-OCH₃ | H | H | H | |
| 5.063 | 4-CF₃ | H | H | H | H | |
| 5.064 | 4-CF₃ | 3-CF₃ | H | H | H | |
| 5.065 | 4-CF₃ | 3-CHF₂ | H | H | H | |
| 5.066 | 4-CF₃ | 3-SCH₃ | H | H | H | NMR |
| 5.067 | 4-CF₃ | 3-S(O)CH₃ | H | H | H | NMR |
| 5.068 | 4-CF₃ | 3-S(O)₂CH₃ | H | H | H | NMR |
| 5.069 | 4-CF₃ | 3-SCF₃ | H | H | H | |
| 5.070 | 4-CF₃ | 3-CH₂CN | H | H | H | NMR |
| 5.071 | 4-CF₃ | 4-F-3-SCH₃ | H | H | H | |
| 5.072 | 4-CF₃ | 3-SCH₂CH₃ | H | H | H | |
| 5.073 | 4-CF₃ | 3-S(O)CH₂CH₃ | H | H | H | |
| 5.074 | 4-CF₃ | 3-S(O)₂CH₂CH₃ | H | H | H | |
| 5.075 | 4-CF₃ | 3-OCH₃ | H | H | H | NMR |
| 5.076 | 5-CN | H | H | H | H | |
| 5.077 | 5-CN | 3-CF₃ | H | H | H | |
| 5.078 | 5-CN | 3-CHF₂ | H | H | H | |
| 5.079 | 5-CN | 3-SCH₃ | H | H | H | |
| 5.080 | 5-CN | 3-S(O)CH₃ | H | H | H | |
| 5.081 | 5-CN | 3-S(O)₂CH₃ | H | H | H | |
| 5.082 | 5-CN | 3-SCF₃ | H | H | H | |
| 5.083 | 5-CN | 3-CH₂CN | H | H | H | |
| 5.084 | 5-CN | 4-F-3-SCH₃ | H | H | H | |
| 5.085 | 5-CN | 3-SCH₂CH₃ | H | H | H | |
| 5.086 | 5-CN | 3-S(O)CH₂CH₃ | H | H | H | |
| 5.087 | 5-CN | 3-S(O)₂CH₂CH₃ | H | H | H | |
| 5.088 | 5-CN | 3-OCH₃ | H | H | H | |
| 5.089 | 5-CN | H | CH₃ | H | H | |
| 5.090 | 5-CN | 3-SCH₃ | CH₃ | H | H | |
| 5.091 | 5-CN | 3-S(O)CH₃ | CH₃ | H | H | |
| 5.092 | 5-CN | 3-S(O)₂CH₃ | CH₃ | H | H | |
| 5.093 | 5-CN | 3-CH₂CN | CH₃ | H | H | |
| 5.094 | 5-CN | 3-CH₂CN | CH₃ | CH₃ | H | |
| 5.095 | 5-CN | 3-SCH₂CH₃ | CH₃ | H | H | |
| 5.096 | H | H | CH₃ | H | H | |
| 5.097 | H | 3-SCH₃ | CH₃ | H | H | |
| 5.098 | H | 3-SCH₃ | CH₂CH₃ | H | H | |
| 5.099 | H | 3-S(O)CH₃ | CH₃ | H | H | |
| 5.100 | H | 3-S(O)₂CH₃ | CH₃ | H | H | |
| 5.101 | H | 3-CH₂CN | CH₃ | H | H | |
| 5.102 | H | 3-CH₂CN | CH₃ | CH₃ | H | |
| 5.103 | H | 3-SCH₂CH₃ | CH₃ | H | H | |
| 5.104 | H | H | CH₃ | H | CH₃ | |
| 5.105 | H | 3-SCH₃ | CH₃ | H | CH₃ | |
| 5.106 | H | 3-SCH₃ | CH₂CH₃ | H | CH₃ | |
| 5.107 | H | 3-S(O)CH₃ | CH₃ | H | CH₃ | |
| 5.108 | H | 3-S(O)₂CH₃ | CH₃ | H | CH₃ | |
| 5.109 | H | 3-CH₂CN | CH₃ | H | CH₃ | |
| 5.110 | H | 3-CH₂CN | CH₃ | CH₃ | CH₃ | |
| 5.111 | H | 3-SCH₂CH₃ | CH₃ | H | CH₃ | |
| 5.112 | H | H | CH₂OCH₃ | H | H | |
| 5.113 | H | 3-SCH₃ | CH₂OCH₃ | H | H | |
| 5.114 | H | 3-S(O)CH₃ | CH₂OCH₃ | H | H | |
| 5.115 | H | 3-S(O)₂CH₃ | CH₂OCH₃ | H | H | |
| 5.116 | H | CH₂CN | CH₂OCH₃ | H | H | |
| 5.117 | 6-OCH₃ | H | H | H | H | |
| 5.118 | 6-OCH₃ | 3-CF₃ | H | H | H | |
| 5.119 | 6-OCH₃ | 3-CHF₂ | H | H | H | |
| 5.120 | 6-OCH₃ | 3-SCH₃ | H | H | H | LogP |
| 5.121 | 6-OCH₃ | 3-S(O)CH₃ | H | H | H | NMR |
| 5.122 | 6-OCH₃ | 3-S(O)₂CH₃ | H | H | H | LogP |
| 5.123 | 6-OCH₃ | 3-SCF₃ | H | H | H | |
| 5.124 | 6-OCH₃ | 3-CH₂CN | H | H | H | NMR |
| 5.125 | 6-OCH₃ | 4-F-3-SCH₃ | H | H | H | LogP |
| 5.126 | 6-OCH₃ | 4-CN-3-SCH₃ | H | H | H | LogP |
| 5.127 | 6-OCH₃ | 3-SCH₂CH₃ | H | H | H | LogP |
| 5.128 | 6-OCH₃ | 3-S(O)CH₂CH₃ | H | H | H | |
| 5.129 | 6-OCH₃ | 3-S(O)₂CH₂CH₃ | H | H | H | |
| 5.130 | 6-OCH₃ | 3-OCH₃ | H | H | H | |
| 5.131 | 6-OCH₃ | 4-OCH₃-3-SCH₂CH₃ | H | H | H | LogP |
| 5.132 | 6-OCH₃ | 4-OCH3-3-S(O)CH₂CH₃ | H | H | H | LogP |
| 5.133 | 6-OCH₃ | 4-CH₃-3-SCH₂CH₃ | H | H | H | LogP |
| 5.134 | 6-OCH₃ | 4-CH3-3-S(O)CH₂CH₃ | H | H | H | LogP |
| 5.135 | 6-OCH₃ | 3-OCF₃ | H | H | H | LogP |
| 5.136 | 6-OCH₃ | 3-SCH(CH₃)₂ | H | H | H | LogP |
| 5.137 | 6-OCH₃ | 3-SCH₂CH=CH₂ | H | H | H | LogP |
| 5.138 | 6-OCH₃ | 4-OCH₃-3-SCH₃ | H | H | H | LogP |
| 5.139 | 6-OCH₃ | 2,4-F₂-3-SCH₃ | H | H | H | LogP |
| 5.140 | 6-OCH₃ | 2,4-F₂-3-S(O)CH₃ | H | H | H | LogP |
| 5.141 | 6-OCH₃ | 3,4-F₂ | H | H | H | LogP |
| 5.142 | 6-OCH₃ | 4-F-3-CH₃-5-SCH₃ | H | H | H | LogP |
| 5.143 | 6-OCH₃ | 2-SCH₃ | H | H | H | LogP |
| 5.144 | 6-OCH₃ | 4-F-3-CH₃-5-S(O)CH₃ | H | H | H | LogP |
| 5.145 | 6-OCH₃ | 2-S(O)CH₃ | H | H | H | LogP |
| 5.146 | 6-OCH₃ | 3,4-F₂-2-SCH₃ | H | H | H | LogP |
| 5.147 | 6-OCH₃ | 3,4-F₂-2-S(O)CH₃ | H | H | H | LogP |
| 5.148 | 6-OCH₃ | 4-F-3,5-(SCH₃)₂ | H | H | H | LogP |
| 5.149 | 6-CF₃ | H | H | H | H | |
| 5.150 | 6-CF₃ | 3-CF₃ | H | H | H | |
| 5.151 | 6-CF₃ | 3-CHF₂ | H | H | H | |
| 5.152 | 6-CF₃ | 3-SCH₃ | H | H | H | NMR |
| 5.153 | 6-CF₃ | 3-S(O)CH₃ | H | H | H | NMR |
| 5.154 | 6-CF₃ | 3-S(O)₂CH₃ | H | H | H | NMR |
| 5.155 | 6-CF₃ | 3-SCF₃ | H | H | H | |
| 5.156 | 6-CF₃ | 3-CH₂CN | H | H | H | NMR |
| 5.157 | 6-CF₃ | 4-F-3-SCH₃ | H | H | H | |
| 5.158 | 6-CF₃ | 3-OCH₃ | H | H | H | NMR |
| 5.159 | 2-OCH₂CH₃ | 3-OCH₃ | H | H | H | NMR |
| 5.160 | H | 4-CH₃-3-SCH₃ | H | H | H | NMR |
| 5.161 | 2-OCH₃ | 4-CH₃-3-SCH₃ | H | H | H | NMR |
| 5.162 | 2-OCH₂CH₃ | 4-CH₃-3-SCH₃ | H | H | H | NMR |
| 5.163 | 6-CF₃ | 4-CH₃-3-SCH₃ | H | H | H | NMR |
| 5.164 | 2-OCH(CH₃)₂ | 3-SCH₃ | H | H | H | NMR |
| 5.165 | 2-OCH(CH₃)₂ | 3-CH₂CN | H | H | H | NMR |
| 5.166 | 2-OCH(CH₃)₂ | 4-CH₃-SCH₃ | H | H | H | NMR |
| 5.167 | 2-OCH(CH₃)₂ | 3-OCH₃ | H | H | H | NMR |
| 5.168 | 2-OCH₃ | 4-CH₃-3-S(O)CH₃ | H | H | H | LogP |
| 5.169 | 2-OCH₃ | 4-CH₃-3-S(O)₂CH₃ | H | H | H | NMR |
| 5.170 | H | 4-CH₃-3-S(O)CH₃ | H | H | H | LogP |
| 5.171 | 2-OCH₂CH₃ | 4-CH₃-3-S(O)CH₃ | H | H | H | LogP |
| 5.172 | 2-OCH₂CH₃ | 4-CH₃-3-S(O)₂CH₃ | H | H | H | NMR |
| 5.173 | 6-CF₃ | 4-CH₃-3-S(O)CH₃ | H | H | H | NMR |
| 5.174 | 2-OCH(CH₃)₂ | 4-CH₃-3-S(O)CH₃ | H | H | H | NMR |
| 5.175 | 2-OCH(CH₃)₂ | 3-S(O)CH₃ | H | H | H | NMR |
| 5.176 | 2-OCH(CH₃)₂ | 3-S(O)₂CH₃ | H | H | H | NMR |
| 5.177 | 6-OCH(CH₃)₂ | 3-SCH₃ | H | H | H | LogP |
| 5.178 | 6-OCH(CH₃)₂ | 3-CH₂CN | H | H | H | LogP |
| 5.179 | 6-OCH(CH₃)₂ | 4-CH₃-3-SCH₃ | H | H | H | |
| 5.180 | 6-OCH(CH₃)₂ | 3-OCH₃ | H | H | H | |
| 5.181 | 6-OCH(CH₃)₂ | 3-S(O)CH₃ | H | H | H | LogP |
| 5.182 | 6-OCH(CH₃)₂ | 3-S(O)₂CH₃ | H | H | H | |
| 5.183 | 2,6-(OCH₃)₂ | 3-SCH₃ | H | H | H | LogP |
| 5.184 | 2,6-(OCH₃)₂ | 3-CH₂CN | H | H | H | LogP |
| 5.185 | 2,6-(OCH₃)₂ | 3-S(O)CH₃ | H | H | H | LogP |
| 5.186 | 6-SCH₃ | 3-SCH₃ | H | H | H | LogP |
| 5.187 | 6-SCH₃ | 3-CH₂CN | H | H | H | LogP |
| 5.188 | 6-SCH₃ | 3-S(O)CH₃ | H | H | H | LogP |
| 5.189 | 6-OCH₂CH₃ | 3-S(O)CH₃ | H | H | H | LogP |
| 5.190 | 6-OCH₂CH₃ | 3-SCH₃ | H | H | H | LogP |
| 5.191 | 2,6-(OCH₂CF₃)₂ | 3-SCH₃ | H | H | H | LogP |
| 5.192 | 2,6-(OCH₂CF₃)₂ | 3-CH₂CN | H | H | H | LogP |
| 5.193 | 2,6-(OCH₂CF₃)₂ | 3-S(O)CH₃ | H | H | H | LogP |
| 5.194 | 6-c-Pent | 3-SCH₃ | H | H | H | LogP |
| 5.195 | 6-c-Pent | 3-CH₂CN | H | H | H | LogP |
| 5.196 | 6-c-Pent | 3-S(O)CH₃ | H | H | H | LogP |
| 5.197 | 5-Br-6-OCH₃ | 3-SCH₃ | H | H | H | LogP |
| 5.198 | 5-F-6-OCH₃ | 4-F-3,5-(SCH₃)₂ | H | H | H | LogP |
| 5.199 | 5-F-6-OCH₂CH₃ | 3-SCH₃ | H | H | H | LogP |
| 5.200 | 6-S(O)₂CH₃ | 3-CH₂CN | H | H | H | LogP |

**Tabelle 6: Tabelle der Verbindungen (Ic-Q1)**

| | | | | | | | |
|---|---|---|---|---|---|---|---|
| 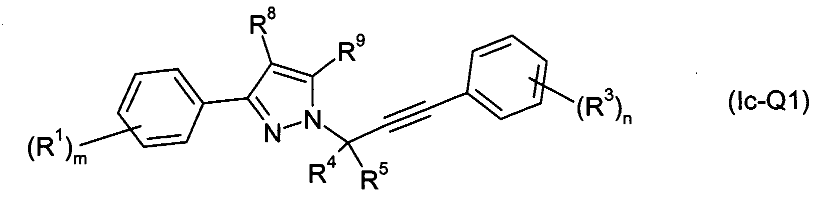 | | | | | | | |

| Nr. | (R¹)ₘ | (R³)ₙ | R⁴ | R⁵ | R⁸ | R⁹ | Daten |
|---|---|---|---|---|---|---|---|
| 6.001 | H | H | H | H | H | H | |
| 6.002 | H | 3-CF₃ | H | H | H | H | |
| 6.003 | H | 3-CHF₂ | H | H | H | H | |
| 6.004 | H | 3-SCH₃ | H | H | H | H | |
| 6.005 | H | 3-S(O)CH₃ | H | H | H | H | |
| 6.006 | H | 3-S(O)₂CH₃ | H | H | H | H | |
| 6.007 | H | 3-SCF₃ | H | H | H | H | |
| 6.008 | H | 3-S(O)CF₃ | H | H | H | H | |
| 6.009 | H | 3-S(O)₂CF₃ | H | H | H | H | |
| 6.010 | H | 3-CH₂CN | H | H | H | H | |
| 6.011 | H | 4-F-3-SCH₃ | H | H | H | H | |
| 6.012 | H | 4-F-3-S(O)CH₃ | H | H | H | H | |
| 6.013 | H | 4-F-3-S(O)₂CH₃ | H | H | H | H | |
| 6.014 | H | 3-SCH₂CH₃ | H | H | H | H | |
| 6.015 | H | 3-S(O)CH₂CH₃ | H | H | H | H | |
| 6.016 | H | 3-S(O)₂CH₂CH₃ | H | H | H | H | |
| 6.017 | H | 3-OCH₃ | H | H | H | H | |
| 6.018 | H | 3,5-(CF₃)₂ | H | H | H | H | |
| 6.019 | 2-I | H | H | H | H | H | |
| 6.020 | 2-I | 3-CF₃ | H | H | H | H | |
| 6.021 | 2-I | 3-CHF₂ | H | H | H | H | |
| 6.022 | 2-I | 3-SCH₃ | H | H | H | H | |
| 6.023 | 2-I | 3-S(O)CH₃ | H | H | H | H | |
| 6.024 | 2-I | 3-S(O)₂CH₃ | H | H | H | H | |
| 6.025 | 2-I | 3-CH₂CN | H | H | H | H | |
| 6.026 | 2-I | 4-F-3-SCH₃ | H | H | H | H | |
| 6.027 | 2-I | 4-F-3-S(O)CH₃ | H | H | H | H | |
| 6.028 | 2-I | 4-F-3-S(O)₂CH₃ | H | H | H | H | |
| 6.029 | 2-I | 3-SCH₂CH₃ | H | H | H | H | |
| 6.030 | 2-I | 3-S(O)CH₂CH₃ | H | H | H | H | |
| 6.031 | 2-I | 3-S(O)₂CH₂CH₃ | H | H | H | H | |
| 6.032 | 2-CN | H | H | H | H | H | |
| 6.033 | 2-CN | 3-CF₃ | H | H | H | H | |
| 6.034 | 2-CN | 3-CHF₂ | H | H | H | H | |
| 6.035 | 2-CN | 3-SCH₃ | H | H | H | H | |
| 6.036 | 2-CN | 3-S(O)CH₃ | H | H | H | H | |
| 6.037 | 2-CN | 3-S(O)₂CH₃ | H | H | H | H | |
| 6.038 | 2-CN | 3-CH₂CN | H | H | H | H | |
| 6.039 | 2-CN | 4-F-3-SCH₃ | H | H | H | H | |
| 6.040 | 2-CN | 4-F-3-S(O)CH₃ | H | H | H | H | |
| 6.041 | 2-CN | 4-F-3-S(O)₂CH₃ | H | H | H | H | |
| 6.042 | 2-CN | 3-SCH₂CH₃ | H | H | H | H | |
| 6.043 | 2-CN | 3-S(O)CH₂CH₃ | H | H | H | H | |
| 6.044 | 2-CN | 3-S(O)₂CH₂CH₃ | H | H | H | H | |
| 6.045 | 2-CN | 3-OCH₃ | H | H | H | H | |
| 6.046 | 2-OCH₃ | H | H | H | H | H | |
| 6.047 | 2-OCH₃ | 3-CF₃ | H | H | H | H | |
| 6.048 | 2-OCH₃ | 3-CHF₂ | H | H | H | H | |
| 6.049 | 2-OCH₃ | 3-OCH₃ | H | H | H | H | NMR |
| 6.050 | 2-OCH₃ | 3-SCH₃ | H | H | H | H | LogP |
| 6.051 | 2-OCH₃ | 3-S(O)CH₃ | H | H | H | H | |
| 6.052 | 2-OCH₃ | 3-S(O)₂CH₃ | H | H | H | H | |
| 6.053 | 2-OCH₃ | 3-CH₂CN | H | H | H | H | |
| 6.054 | 2-OCH₃ | 4-F-3-SCH₃ | H | H | H | H | |
| 6.055 | 2-OCH₃ | 4-F-3-S(O)CH₃ | H | H | H | H | |
| 6.056 | 2-OCH₃ | 4-F-3-S(O)₂CH₃ | H | H | H | H | |
| 6.057 | 2-OCH₃ | 3-SCH₂CH₃ | H | H | H | H | |
| 6.058 | 2-OCH₃ | 3-S(O)CH₂CH₃ | H | H | H | H | |
| 6.059 | 2-OCH₃ | 3-S(O)₂CH₂CH₃ | H | H | H | H | |
| 6.060 | 4-F-2-OCH₃ | H | H | H | H | H | |
| 6.061 | 4-F-2-OCH₃ | 3-CF₃ | H | H | H | H | |
| 6.062 | 4-F-2-OCH₃ | 3-CHF₂ | H | H | H | H | |
| 6.063 | 4-F-2-OCH₃ | 3-SCH₃ | H | H | H | H | |
| 6.064 | 4-F-2-OCH₃ | 3-S(O)CH₃ | H | H | H | H | |
| 6.065 | 4-F-2-OCH₃ | 3-S(O)₂CH₃ | H | H | H | H | |
| 6.066 | 4-F-2-OCH₃ | 3-CH₂CN | H | H | H | H | |
| 6.067 | 4-F-2-OCH₃ | 4-F-3-SCH₃ | H | H | H | H | |
| 6.068 | 4-F-2-OCH₃ | 4-F-3-S(O)CH₃ | H | H | H | H | |
| 6.069 | 4-F-2-OCH₃ | 4-F-3-S(O)₂CH₃ | H | H | H | H | |
| 6.070 | 4-F-2-OCH₃ | 3-SCH₂CH₃ | H | H | H | H | |
| 6.071 | 4-F-2-OCH₃ | 3-S(O)CH₂CH₃ | H | H | H | H | |
| 6.072 | 4-F-2-OCH₃ | 3-S(O)₂CH₂CH₃ | H | H | H | H | |
| 6.073 | 2-Cl | H | H | H | H | H | NMR |
| 6.074 | 2-Cl | 3-CF₃ | H | H | H | H | |
| 6.075 | 2-Cl | 3-CHF₂ | H | H | H | H | |
| 6.076 | 2-Cl | 3-OCH₃ | H | H | H | H | NMR |
| 6.077 | 2-Cl | 3-SCH₃ | H | H | H | H | NMR |
| 6.078 | 2-Cl | 3-S(O)CH₃ | H | H | H | H | |
| 6.079 | 2-Cl | 3-S(O)₂CH₃ | H | H | H | H | |
| 6.080 | 2-Cl | 3-CH₂CN | H | H | H | H | |
| 6.081 | 2-Cl | 4-F-3-SCH₃ | H | H | H | H | |
| 6.082 | 2-Cl | 4-F-3-S(O)CH₃ | H | H | H | H | |
| 6.083 | 2-Cl | 4-F-3-S(O)₂CH₃ | H | H | H | H | |
| 6.084 | 2-Cl | 3-SCH₂CH₃ | H | H | H | H | |
| 6.085 | 2-Cl | 3-S(O)CH₂CH₃ | H | H | H | H | |
| 6.086 | 2-Cl | 3-S(O)₂CH₂CH₃ | H | H | H | H | |
| 6.087 | 2,4-Cl₂ | H | H | H | H | H | |
| 6.088 | 2,4-Cl₂ | 3-CF₃ | H | H | H | H | |
| 6.089 | 2,4-Cl₂ | 3-CHF₂ | H | H | H | H | |
| 6.090 | 2,4-Cl₂ | 3-SCH₃ | H | H | H | H | |
| 6.091 | 2,4-Cl₂ | 3-S(O)CH₃ | H | H | H | H | |
| 6.092 | 2,4-Cl₂ | 3-S(O)₂CH₃ | H | H | H | H | |
| 6.093 | 2,4-Cl₂ | 3-CH₂CN | H | H | H | H | |
| 6.094 | 2,4-Cl₂ | 4-F-3-SCH₃ | H | H | H | H | |
| 6.095 | 2,4-Cl₂ | 4-F-3-S(O)CH₃ | H | H | H | H | |
| 6.096 | 2,4-Cl₂ | 4-F-3-S(O)₂CH₃ | H | H | H | H | |
| 6.097 | 2,4-Cl₂ | 3-SCH₂CH₃ | H | H | H | H | |
| 6.098 | 2,4-Cl₂ | 3-S(O)CH₂CH₃ | H | H | H | H | |
| 6.099 | 2,4-Cl₂ | 3-S(O)₂CH₂CH₃ | H | H | H | H | |
| 6.100 | 2,4-F₂ | H | H | H | H | H | NMR |
| 6.101 | 2,4-F₂ | 3-CF₃ | H | H | H | H | |
| 6.102 | 2,4-F₂ | 3-CHF₂ | H | H | H | H | |
| 6.103 | 2,4-F₂ | 3-OCH₃ | H | H | H | H | NMR |
| 6.104 | 2,4-F₂ | 3-SCH₃ | H | H | H | H | NMR |
| 6.105 | 2,4-F₂ | 3-S(O)CH₃ | H | H | H | H | |
| 6.106 | 2,4-F₂ | 3-S(O)₂CH₃ | H | H | H | H | |
| 6.107 | 2,4-F₂ | 3-CH₂CN | H | H | H | H | |
| 6.108 | 2,4-F₂ | 4-F-3-SCH₃ | H | H | H | H | |
| 6.109 | 2,4-F₂ | 4-F-3-S(O)CH₃ | H | H | H | H | |
| 6.110 | 2,4-F₂ | 4-F-3-S(O)₂CH₃ | H | H | H | H | |
| 6.111 | 2,4-F₂ | 3-SCH₂CH₃ | H | H | H | H | |
| 6.112 | 2,4-F₂ | 3-S(O)CH₂CH₃ | H | H | H | H | |
| 6.113 | 2,4-F₂ | 3-S(O)₂CH₂CH₃ | H | H | H | H | |
| 6.114 | 4-F-2-OCH₃ | H | H | H | H | H | |
| 6.115 | 4-F-2-OCH₃ | 3-CF₃ | H | H | H | H | |
| 6.116 | 4-F-2-OCH₃ | 3-CHF₂ | H | H | H | H | |
| 6.117 | 4-F-2-OCH₃ | 3-SCH₃ | H | H | H | H | |
| 6.118 | 4-F-2-OCH₃ | 3-S(O)CH₃ | H | H | H | H | |
| 6.119 | 4-F-2-OCH₃ | 3-S(O)₂CH₃ | H | H | H | H | |
| 6.120 | 4-F-2-OCH₃ | 3-CH₂CN | H | H | H | H | |
| 6.121 | 4-F-2-OCH₃ | 4-F-3-SCH₃ | H | H | H | H | |
| 6.122 | 4-F-2-OCH₃ | 4-F-3-S(O)CH₃ | H | H | H | H | |
| 6.123 | 4-F-2-OCH₃ | 4-F-3-S(O)₂CH₃ | H | H | H | H | |
| 6.124 | 4-F-2-OCH₃ | 3-SCH₂CH₃ | H | H | H | H | |
| 6.125 | 4-F-2-OCH₃ | 3-S(O)CH₂CH₃ | H | H | H | H | |
| 6.126 | 4-F-2-OCH₃ | 3-S(O)₂CH₂CH₃ | H | H | H | H | |
| 6.127 | 4-F | H | H | H | H | H | |
| 6.128 | 4-F | 3-CF₃ | H | H | H | H | |
| 6.129 | 4-F | 3-CHF₂ | H | H | H | H | |
| 6.130 | 4-F | 3-OCH₃ | H | H | H | H | |
| 6.131 | 4-F | 3-SCH₃ | H | H | H | H | |
| 6.132 | 4-F | 3-S(O)CH₃ | H | H | H | H | |
| 6.133 | 4-F | 3-S(O)₂CH₃ | H | H | H | H | |
| 6.134 | 4-F | 3-CH₂CN | H | H | H | H | |
| 6.135 | 4-F | 4-F-3-SCH₃ | H | H | H | H | |
| 6.136 | 4-F | 4-F-3-S(O)CH₃ | H | H | H | H | |
| 6.137 | 4-F | 4-F-3-S(O)₂CH₃ | H | H | H | H | |
| 6.138 | 4-F | 3-SCH₂CH₃ | H | H | H | H | |
| 6.139 | 4-F | 3-S(O)CH₂CH₃ | H | H | H | H | |
| 6.140 | 4-F | 3-S(O)₂CH₂CH₃ | H | H | H | H | |
| 6.141 | 4-CH₃ | H | H | H | H | H | NMR |
| 6.142 | 4-CH₃ | 3-SCF₃ | H | H | H | H | NMR |
| 6.143 | 4-CH₃ | 3-CHF₂ | H | H | H | H | |
| 6.144 | 4-CH₃ | 3-OCH₃ | H | H | H | H | NMR |
| 6.145 | 4-CH₃ | 3-SCH₃ | H | H | H | H | NMR |
| 6.146 | 4-CH₃ | 3-S(O)CH₃ | H | H | H | H | |
| 6.147 | 4-CH₃ | 3-S(O)₂CH₃ | H | H | H | H | NMR |
| 6.148 | 4-CH₃ | 3-CH₂CN | H | H | H | H | |
| 6.149 | 4-CH₃ | 4-F-3-SCH₃ | H | H | H | H | |
| 6.150 | 4-CH₃ | 4-F-3-S(O)CH₃ | H | H | H | H | |
| 6.151 | 4-CH₃ | 4-F-3-S(O)₂CH₃ | H | H | H | H | |
| 6.152 | 4-CH₃ | 3-SCH₂CH₃ | H | H | H | H | |
| 6.153 | 4-CH₃ | 3-S(O)CH₂CH₃ | H | H | H | H | |
| 6.154 | 4-CH₃ | 3-S(O)₂CH₂CH₃ | H | H | H | H | |
| 6.155 | 4-Cl | H | H | H | H | H | |
| 6.156 | 4-Cl | 3-CF₃ | H | H | H | H | |
| 6.157 | 4-Cl | 3-CHF₂ | H | H | H | H | |
| 6.158 | 4-Cl | 3-OCH₃ | H | H | H | H | |
| 6.159 | 4-Cl | 3-SCH₃ | H | H | H | H | |
| 6.160 | 4-Cl | 3-S(O)CH₃ | H | H | H | H | |
| 6.161 | 4-Cl | 3-S(O)₂CH₃ | H | H | H | H | |
| 6.162 | 4-Cl | 3-CH₂CN | H | H | H | H | |
| 6.163 | 4-Cl | 4-F-3-SCH₃ | H | H | H | H | |
| 6.164 | 4-Cl | 4-F-3-S(O)CH₃ | H | H | H | H | |
| 6.165 | 4-Cl | 4-F-3-S(O)₂CH₃ | H | H | H | H | |
| 6.166 | 4-Cl | 3-SCH₂CH₃ | H | H | H | H | |
| 6.167 | 4-Cl | 3-S(O)CH₂CH₃ | H | H | H | H | |
| 6.168 | 4-Cl | 3-S(O)₂CH₂CH₃ | H | H | H | H | |
| 6.169 | 4-OCF₃ | H | H | H | H | H | |
| 6.170 | 4-OCF₃ | 3-CF₃ | H | H | H | H | |
| 6.171 | 4-OCF₃ | 3-CHF₂ | H | H | H | H | |
| 6.172 | 4-OCF₃ | 3-OCH₃ | H | H | H | H | |
| 6.173 | 4-OCF₃ | 3-SCH₃ | H | H | H | H | |
| 6.174 | 4-OCF₃ | 3-S(O)CH₃ | H | H | H | H | |
| 6.175 | 4-OCF₃ | 3-S(O)₂CH₃ | H | H | H | H | |
| 6:176 | 4-OCF₃ | 3-CH₂CN | H | H | H | H | |
| 6.177 | 4-OCF₃ | 4-F-3-SCH₃ | H | H | H | H | |
| 6.178 | 4-OCF₃ | 4-F-3-S(O)CH₃ | H | H | H | H | |
| 6.179 | 4-OCF₃ | 4-F-3-S(O)₂CH₃ | H | H | H | H | |
| 6.180 | 4-OCF₃ | 3-SCH₂CH₃ | H | H | H | H | |
| 6.181 | 4-OCF₃ | 3-S(O)CH₂CH₃ | H | H | H | H | |
| 6.182 | 4-OCF₃ | 3-S(O)₂CH₂CH₃ | H | H | H | H | |
| 6.183 | 2-Cl | H | CH₃ | H | H | H | |
| 6.184 | 2-Cl | 3-CF₃ | CH₃ | H | H | H | |
| 6.185 | 2-Cl | 3-CHF₂ | CH₃ | H | H | H | |
| 6.186 | 2-Cl | 3-SCH₃ | CH₃ | H | H | H | |
| 6.187 | 2-Cl | 3-S(O)CH₃ | CH₃ | H | H | H | |
| 6.188 | 2-Cl | 3-S(O)₂CH₃ | CH₃ | H | H | H | |
| 6.189 | 2-Cl | 3-CH₂CN | CH₃ | H | H | H | |
| 6.190 | 2-Cl | 3-OCH₃ | CH₃ | H | H | H | |
| 6.191 | 2-Cl | H | H | H | CH₃ | H | |
| 6.192 | 2-Cl | 3-CF₃ | H | H | CH₃ | H | |
| 6.193 | 2-Cl | 3-CHF₂ | H | H | CH₃ | H | |
| 6.194 | 2-Cl | 3-SCH₃ | H | H | CH₃ | H | |
| 6.195 | 2-Cl | 3-S(O)CH₃ | H | H | CH₃ | H | |
| 6.196 | 2-Cl | 3-S(O)₂CH₃ | H | H | CH₃ | H | |
| 6.197 | 2-Cl | 3-CH₂CN | H | H | CH₃ | H | |
| 6.198 | 2-Cl | 3-OCH₃ | H | H | CH₃ | H | |
| 6.199 | 4-F-2-OCH₃ | H | H | H | CH₃ | H | |
| 6.200 | 4-F-2-OCH₃ | 3-CF₃ | H | H | CH₃ | H | |
| 6.201 | 4-F-2-OCH₃ | 3-CHF₂ | H | H | CH₃ | H | |
| 6.202 | 4-F-2-OCH₃ | 3-SCH₃ | H | H | CH₃ | H | |
| 6.203 | 4-F-2-OCH₃ | 3-S(O)CH₃ | H | H | CH₃ | H | |
| 6.204 | 4-F-2-OCH₃ | 3-S(O)₂CH₃ | H | H | CH₃ | H | |
| 6.205 | 4-F-2-OCH₃ | 3-CH₂CN | H | H | CH₃ | H | |
| 6.206 | 4-F-2-OCH₃ | H | H | H | H | CH₃ | |
| 6.207 | 4-F-2-OCH₃ | 3-CF₃ | H | H | H | CH₃ | |
| 6.208 | 4-F-2-OCH₃ | 3-CHF₂ | H | H | H | CH₃ | |
| 6.209 | 4-F-2-OCH₃ | 3-SCH₃ | H | H | H | CH₃ | |
| 6.210 | 4-F-2-OCH₃ | 3-S(O)CH₃ | H | H | H | CH₃ | |
| 6.211 | 4-F-2-OCH₃ | 3-S(O)₂CH₃ | H | H | H | CH₃ | |
| 6.212 | 4-F-2-OCH₃ | 3-CH₂CN | H | H | H | CH₃ | |
| 6.213 | 4-F-2-OCH₃ | H | H | H | -(CH₂)₄- | | |
| 6.214 | 4-F-2-OCH₃ | 3-CF₃ | H | H | -(CH₂)₄- | | |
| 6.215 | 4-F-2-OCH₃ | 3-CHF₂ | H | H | -(CH₂)₄- | | |
| 6.216 | 4-F-2-OCH₃ | 3-SCH₃ | H | H | -(CH₂)₄- | | |
| 6.217 | 4-F-2-OCH₃ | 3-S(O)CH₃ | H | H | -(CH₂)₄- | | |
| 6.218 | 4-F-2-OCH₃ | 3-S(O)₂CH₃ | H | H | -(CH₂)₄- | | |
| 6.219 | 4-F-2-OCH₃ | 3-CH₂CN | H | H | -(CH₂)₄- | | |
| 6.220 | 4-F-2-OCH₃ | H | CH₃ | H | -(CH₂)₄- | | |
| 6.221 | 4-F-2-OCH₃ | 3-CF₃ | CH₃ | H | -(CH₂)₄- | | |
| 6.222 | 4-F-2-OCH₃ | 3-CHF₂ | CH₃ | H | -(CH₂)₄- | | |
| 6.223 | 4-F-2-OCH₃ | 3-SCH₃ | CH₃ | H | -(CH₂)₄- | | |
| 6.224 | 4-F-2-OCH₃ | 3-S(O)CH₃ | CH₃ | H | -(CH₂)₄- | | |
| 6.225 | 4-F-2-OCH₃ | 3-S(O)₂CH₃ | CH₃ | H | -(CH₂)₄- | | |
| 6.226 | 4-F-2-OCH₃ | 3-CH₂CN | CH₃ | H | -(CH₂)₄- | | |
| 6.227 | 4-F-2-OCH₃ | H | H | H | Cl | H | |
| 6.228 | 4-F-2-OCH₃ | 3-CF₃ | H | H | Cl | H | |
| 6.229 | 4-F-2-OCH₃ | 3-CHF₂ | H | H | Cl | H | |
| 6.230 | 4-F-2-OCH₃ | 3-SCH₃ | H | H | Cl | H | |
| 6.231 | 4-F-2-OCH₃ | 3-S(O)CH₃ | H | H | Cl | H | |
| 6.232 | 4-F-2-OCH₃ | 3-S(O)₂CH₃ | H | H | Cl | H | |
| 6.233 | 4-F-2-OCH₃ | 3-CH₂CN | H | H | Cl | H | |

**Tabelle 7: Tabelle der Verbindungen der Formel (Id-Q1)**

| | | | | | | | |
|---|---|---|---|---|---|---|---|
| 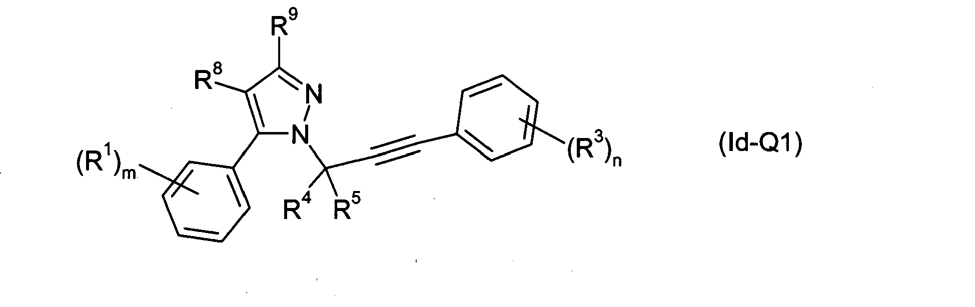 | | | | | | | |

| Nr. | (R¹)ₘ | (R³)ₙ | R⁴ | R⁵ | R⁸ | R⁹ | Daten |
|---|---|---|---|---|---|---|---|
| 7.001 | H | H | H | H | H | H | |
| 7.002 | H | 3-CF₃ | H | H | H | H | |
| 7.003 | H | 3-CHF₂ | H | H | H | H | |
| 7.004 | H | 3-SCH₃ | H | H | H | H | |
| 7.005 | H | 3-S(O)CH₃ | H | H | H | H | |
| 7.006 | H | 3-S(O)₂CH₃ | H | H | H | H | |
| 7.007 | H | 3-SCF₃ | H | H | H | H | |
| 7.008 | H | 3-S(O)CF₃ | H | H | H | H | |
| 7.009 | H | 3-S(O)₂CF₃ | H | H | H | H | |
| 7.010 | H | 3-CH₂CN | H | H | H | H | |
| 7.011 | H | 4-F-3-SCH₃ | H | H | H | H | |
| 7.012 | H | 4-F-3-S(O)CH₃ | H | H | H | H | |
| 7.013 | H | 4-F-3-S(O)₂CH₃ | H | H | H | H | |
| 7.014 | H | 3-SCH₂CH₃ | H | H | H | H | |
| 7.015 | H | 3-S(O)CH₂CH₃ | H | H | H | H | |
| 7.016 | H | 3-S(O)₂CH₂CH₃ | H | H | H | H | |
| 7.017 | H | 3-OCH₃ | H | H | H | H | |
| 7.018 | H | 3,5-(CF₃)₂ | H | H | H | H | |
| 7.019 | 2-I | H | H | H | H | H | |
| 7.020 | 2-I | 3-CF₃ | H | H | H | H | |
| 7.021 | 2-I | 3-CHF₂ | H | H | H | H | |
| 7.022 | 2-I | 3-SCH₃ | H | H | H | H | |
| 7.023 | 2-I | 3-S(O)CH₃ | H | H | H | H | |
| 7.024 | 2-I | 3-S(O)₂CH₃ | H | H | H | H | |
| 7.025 | 2-I | 3-CH₂CN | H | H | H | H | |
| 7.026 | 2-I | 4-F-3-SCH₃ | H | H | H | H | |
| 7.027 | 2-I | 4-F-3-S(O)CH₃ | H | H | H | H | |
| 7.028 | 2-I | 4-F-3-S(O)₂CH₃ | H | H | H | H | |
| 7.029 | 2-I | 3-SCH₂CH₃ | H | H | H | H | |
| 7.030 | 2-I | 3-S(O)CH₂CH₃ | H | H | H | H | |
| 7.031 | 2-I | 3-S(O)₂CH₂CH₃ | H | H | H | H | |
| 7.032 | 2-CN | H | H | H | H | H | |
| 7.033 | 2-CN | 3-CF₃ | H | H | H | H | |
| 7.034 | 2-CN | 3-CHF₂ | H | H | H | H | |
| 7.035 | 2-CN | 3-SCH₃ | H | H | H | H | |
| 7.036 | 2-CN | 3-S(O)CH₃ | H | H | H | H | |
| 7.037 | 2-CN | 3-S(O)₂CH₃ | H | H | H | H | |
| 7.038 | 2-CN | 3-CH₂CN | H | H | H | H | |
| 7.039 | 2-CN | 4-F-3-SCH₃ | H | H | H | H | |
| 7.040 | 2-CN | 4-F-3-S(O)CH₃ | H | H | H | H | |
| 7.041 | 2-CN | 4-F-3-S(O)₂CH₃ | H | H | H | H | |
| 7.042 | 2-CN | 3-SCH₂CH₃ | H | H | H | H | |
| 7.043 | 2-CN | 3-S(O)CH₂CH₃ | H | H | H | H | |
| 7.044 | 2-CN | 3-S(O)₂CH₂CH₃ | H | H | H | H | |
| 7.045 | 2-CN | 3-OCH₃ | H | H | H | H | |
| 7.046 | 2-OCH₃ | H | H | H | H | H | |
| 7.047 | 2-OCH₃ | 3-CF₃ | H | H | H | H | |
| 7.048 | 2-OCH₃ | 3-CHF₂ | H | H | H | H | |
| 7.049 | 2-OCH₃ | 3-OCH₃ | H | H | H | H | NMR |
| 7.050 | 2-OCH₃ | 3-SCH₃ | H | H | H | H | |
| 7.051 | 2-OCH₃ | 3-S(O)CH₃ | H | H | H | H | |
| 7.052 | 2-OCH₃ | 3-S(O)₂CH₃ | H | H | H | H | |
| 7.053 | 2-OCH₃ | 3-CH₂CN | H | H | H | H | |
| 7.054 | 2-OCH₃ | 4-F-3-SCH₃ | H | H | H | H | |
| 7.055 | 2-OCH₃ | 4-F-3-S(O)CH₃ | H | H | H | H | |
| 7.056 | 2-OCH₃ | 4-F-3-S(O)₂CH₃ | H | H | H | H | |
| 7.057 | 2-OCH₃ | 3-SCH₂CH₃ | H | H | H | H | |
| 7.058 | 2-OCH₃ | 3-S(O)CH₂CH₃ | H | H | H | H | |
| 7.059 | 2-OCH₃ | 3-S(O)₂CH₂CH₃ | H | H | H | H | |
| 7.060 | 4-F-2-OCH₃ | H | H | H | H | H | |
| 7.061 | 4-F-2-OCH₃ | 3-CF₃ | H | H | H | H | |
| 7.062 | 4-F-2-OCH₃ | 3-CHF₂ | H | H | H | H | |
| 7.063 | 4-F-2-OCH₃ | 3-SCH₃ | H | H | H | H | |
| 7.064 | 4-F-2-OCH₃ | 3-S(O)CH₃ | H | H | H | H | |
| 7.065 | 4-F-2-OCH₃ | 3-S(O)₂CH₃ | H | H | H | H | |
| 7.066 | 4-F-2-OCH₃ | 3-CH₂CN | H | H | H | H | |
| 7.067 | 4-F-2-OCH₃ | 4-F-3-SCH₃ | H | H | H | H | |
| 7.068 | 4-F-2-OCH₃ | 4-F-3-S(O)CH₃ | H | H | H | H | |
| 7.069 | 4-F-2-OCH₃ | 4-F-3-S(O)₂CH₃ | H | H | H | H | |
| 7.070 | 4-F-2-OCH₃ | 3-SCH₂CH₃ | H | H | H | H | |
| 7.071 | 4-F-2-OCH₃ | 3-S(O)CH₂CH₃ | H | H | H | H | |
| 7.072 | 4-F-2-OCH₃ | 3-S(O)₂CH₂CH₃ | H | H | H | H | |
| 7.073 | 2-Cl | H | H | H | H | H | NMR |
| 7.074 | 2-Cl | 3-CF₃ | H | H | H | H | |
| 7.075 | 2-Cl | 3-CHF₂ | H | H | H | H | |
| 7.076 | 2-Cl | 3-OCH₃ | H | H | H | H | NMR |
| 7.077 | 2-Cl | 3-SCH₃ | H | H | H | H | NMR |
| 7.078 | 2-Cl | 3-S(O)CH₃ | H | H | H | H | |
| 7.079 | 2-Cl | 3-S(O)₂CH₃ | H | H | H | H | |
| 7.080 | 2-Cl | 3-CH₂CN | H | H | H | H | |
| 7.081 | 2-Cl | 4-F-3-SCH₃ | H | H | H | H | |
| 7.082 | 2-Cl | 4-F-3-S(O)CH₃ | H | H | H | H | |
| 7.083 | 2-Cl | 4-F-3-S(O)₂CH₃ | H | H | H | H | |
| 7.084 | 2-Cl | 3-SCH₂CH₃ | H | H | H | H | |
| 7.085 | 2-Cl | 3-S(O)CH₂CH₃ | H | H | H | H | |
| 7.086 | 2-Cl | 3-S(O)₂CH₂CH₃ | H | H | H | H | |
| 7.087 | 2,4-Cl₂ | H | H | H | H | H | |
| 7.088 | 2,4-Cl₂ | 3-CF₃ | H | H | H | H | |
| 7.089 | 2,4-Cl₂ | 3-CHF₂ | H | H | H | H | |
| 7.090 | 2,4-Cl₂ | 3-SCH₃ | H | H | H | H | |
| 7.091 | 2,4-Cl₂ | 3-S(O)CH₃ | H | H | H | H | |
| 7.092 | 2,4-Cl₂ | 3-S(O)₂CH₃ | H | H | H | H | |
| 7.093 | 2,4-Cl₂ | 3-CH₂CN | H | H | H | H | |
| 7.094 | 2,4-Cl₂ | 4-F-3-SCH₃ | H | H | H | H | |
| 7.095 | 2,4-Cl₂ | 4-F-3-S(O)CH₃ | H | H | H | H | |
| 7.096 | 2,4-Cl₂ | 4-F-3-S(O)₂CH₃ | H | H | H | H | |
| 7.097 | 2,4-Cl₂ | 3-SCH₂CH₃ | H | H | H | H | |
| 7.098 | 2,4-Cl₂ | 3-S(O)CH₂CH₃ | H | H | H | H | |
| 7.099 | 2,4-Cl₂ | 3-S(O)₂CH₂CH₃ | H | H | H | H | |
| 7.100 | 2,4-F₂ | H | H | H | H | H | NMR |
| 7.101 | 2,4-F₂ | 3-CF₃ | H | H | H | H | |
| 7.102 | 2,4-F₂ | 3-CHF₂ | H | H | H | H | |
| 7.103 | 2,4-F₂ | 3-OCH₃ | H | H | H | H | NMR |
| 7.104 | 2,4-F₂ | 3-SCH₃ | H | H | H | H | NMR |
| 7.105 | 2,4-F₂ | 3-S(O)CH₃ | H | H | H | H | |
| 7.106 | 2,4-F₂ | 3-S(O)₂CH₃ | H | H | H | H | |
| 7.107 | 2,4-F₂ | 3-CH₂CN | H | H | H | H | |
| 7.108 | 2,4-F₂ | 4-F-3-SCH₃ | H | H | H | H | |
| 7.109 | 2,4-F₂ | 4-F-3-S(O)CH₃ | H | H | H | H | |
| 7.110 | 2,4-F₂ | 4-F-3-S(O)₂CH₃ | H | H | H | H | |
| 7.111 | 2,4-F₂ | 3-SCH₂CH₃ | H | H | H | H | |
| 7.112 | 2,4-F₂ | 3-S(O)CH₂CH₃ | H | H | H | H | |
| 7.113 | 2,4-F₂ | 3-S(O)₂CH₂CH₃ | H | H | H | H | |
| 7.114 | 4-F-2-OCH₃ | H | H | H | H | H | |
| 7.115 | 4-F-2-OCH₃ | 3-CF₃ | H | H | H | H | |
| 7.116 | 4-F-2-OCH₃ | 3-CHF₂ | H | H | H | H | |
| 7.117 | 4-F-2-OCH₃ | 3-SCH₃ | H | H | H | H | |
| 7.118 | 4-F-2-OCH₃ | 3-S(O)CH₃ | H | H | H | H | |
| 7.119 | 4-F-2-OCH₃ | 3-S(O)₂CH₃ | H | H | H | H | |
| 7.120 | 4-F-2-OCH₃ | 3-CH₂CN | H | H | H | H | |
| 7.121 | 4-F-2-OCH₃ | 4-F-3-SCH₃ | H | H | H | H | |
| 7.122 | 4-F-2-OCH₃ | 4-F-3-S(O)CH₃ | H | H | H | H | |
| 7.123 | 4-F-2-OCH₃ | 4-F-3-S(O)₂CH₃ | H | H | H | H | |
| 7.124 | 4-F-2-OCH₃ | 3-SCH₂CH₃ | H | H | H | H | |
| 7.125 | 4-F-2-OCH₃ | 3-S(O)CH₂CH₃ | H | H | H | H | |
| 7.126 | 4-F-2-OCH₃ | 3-S(O)₂CH₂CH₃ | H | H | H | H | |
| 7.127 | 4-F | H | H | H | H | H | |
| 7.128 | 4-F | 3-CF₃ | H | H | H | H | |
| 7.129 | 4-F | 3-CHF₂ | H | H | H | H | |
| 7.130 | 4-F | 3-OCH₃ | H | H | H | H | |
| 7.131 | 4-F | 3-SCH₃ | H | H | H | H | |
| 7.132 | 4-F | 3-S(O)CH₃ | H | H | H | H | |
| 7.133 | 4-F | 3-S(O)₂CH₃ | H | H | H | H | |
| 7.134 | 4-F | 3-CH₂CN | H | H | H | H | |
| 7.135 | 4-F | 4-F-3-SCH₃ | H | H | H | H | |
| 7.136 | 4-F | 4-F-3-S(O)CH₃ | H | H | H | H | |
| 7.137 | 4-F | 4-F-3-S(O)₂CH₃ | H | H | H | H | |
| 7.138 | 4-F | 3-SCH₂CH₃ | H | H | H | H | |
| 7.139 | 4-F | 3-S(O)CH₂CH₃ | H | H | H | H | |
| 7.140 | 4-F | 3-S(O)₂CH₂CH₃ | H | H | H | H | |
| 7.141 | 4-CH₃ | H | H | H | H | H | NMR |
| 7.142 | 4-CH₃ | 3-CF₃ | H | H | H | H | |
| 7.143 | 4-CH₃ | 3-CHF₂ | H | H | H | H | |
| 7.144 | 4-CH₃ | 3-OCH₃ | H | H | H | H | NMR |
| 7.145 | 4-CH₃ | 3-SCH₃ | H | H | H | H | NMR |
| 7.146 | 4-CH₃ | 3-S(O)CH₃ | H | H | H | H | |
| 7.147 | 4-CH₃ | 3-S(O)₂CH₃ | H | H | H | H | |
| 7.148 | 4-CH₃ | 3-CH₂CN | H | H | H | H | |
| 7.149 | 4-CH₃ | 4-F-3-SCH₃ | H | H | H | H | |
| 7.150 | 4-CH₃ | 4-F-3-S(O)CH₃ | H | H | H | H | |
| 7.151 | 4-CH₃ | 4-F-3-S(O)₂CH₃ | H | H | H | H | |
| 7.152 | 4-CH₃ | 3-SCH₂CH₃ | H | H | H | H | |
| 7.153 | 4-CH₃ | 3-S(O)CH₂CH₃ | H | H | H | H | |
| 7.154 | 4-CH₃ | 3-S(O)₂CH₂CH₃ | H | H | H | H | |
| 7.155 | 4-Cl | H | H | H | H | H | |
| 7.156 | 4-Cl | 3-CF₃ | H | H | H | H | |
| 7.157 | 4-Cl | 3-CHF₂ | H | H | H | H | |
| 7.158 | 4-Cl | 3-OCH₃ | H | H | H | H | |
| 7.159 | 4-Cl | 3-SCH₃ | H | H | H | H | |
| 7.160 | 4-Cl | 3-S(O)CH₃ | H | H | H | H | |
| 7.161 | 4-Cl | 3-S(O)₂CH₃ | H | H | H | H | |
| 7.162 | 4-Cl | 3-CH₂CN | H | H | H | H | |
| 7.163 | 4-Cl | 4-F-3-SCH₃ | H | H | H | H | |
| 7.164 | 4-Cl | 4-F-3-S(O)CH₃ | H | H | H | H | |
| 7.165 | 4-Cl | 4-F-3-S(O)₂CH₃ | H | H | H | H | |
| 7.166 | 4-Cl | 3-SCH₂CH₃ | H | H | H | H | |
| 7.167 | 4-Cl | 3-S(O)CH₂CH₃ | H | H | H | H | |
| 7.168 | 4-Cl | 3-S(O)₂CH₂CH₃ | H | H | H | H | |
| 7.169 | 4-OCF₃ | H | H | H | H | H | |
| 7.170 | 4-OCF₃ | 3-CF₃ | H | H | H | H | |
| 7.171 | 4-OCF₃ | 3-CHF₂ | H | H | H | H | |
| 7.172 | 4-OCF₃ | 3-OCH₃ | H | H | H | H | |
| 7.173 | 4-OCF₃ | 3-SCH₃ | H | H | H | H | |
| 7.174 | 4-OCF₃ | 3-S(O)CH₃ | H | H | H | H | |
| 7.175 | 4-OCF₃ | 3-S(O)₂CH₃ | H | H | H | H | |
| 7.176 | 4-OCF₃ | 3-CH₂CN | H | H | H | H | |
| 7.177 | 4-OCF₃ | 4-F-3-SCH₃ | H | H | H | H | |
| 7.178 | 4-OCF₃ | 4-F-3-S(O)CH₃ | H | H | H | H | |
| 7.179 | 4-OCF₃ | 4-F-3-S(O)₂CH₃ | H | H | H | H | |
| 7.180 | 4-OCF₃ | 3-SCH₂CH₃ | H | H | H | H | |
| 7.181 | 4-OCF₃ | 3-S(O)CH₂CH₃ | H | H | H | H | |
| 7.182 | 4-OCF₃ | 3-S(O)₂CH₂CH₃ | H | H | H | H | |
| 7.183 | 2-Cl | H | CH₃ | H | H | H | |
| 7.184 | 2-Cl | 3-CF₃ | CH₃ | H | H | H | |
| 7.185 | 2-Cl | 3-CHF₂ | CH₃ | H | H | H | |
| 7.186 | 2-Cl | 3-SCH₃ | CH₃ | H | H | H | |
| 7.187 | 2-Cl | 3-S(O)CH₃ | CH₃ | H | H | H | |
| 7.188 | 2-Cl | 3-S(O)₂CH₃ | CH₃ | H | H | H | |
| 7.189 | 2-Cl | 3-CH₂CN | CH₃ | H | H | H | |
| 7.190 | 2-Cl | 3-OCH₃ | CH₃ | H | H | H | |
| 7.191 | 2-Cl | H | H | H | CH₃ | H | |
| 7.192 | 2-Cl | 3-CF₃ | H | H | CH₃ | H | |
| 7.193 | 2-Cl | 3-CHF₂ | H | H | CH₃ | H | |
| 7.194 | 2-Cl | 3-SCH₃ | H | H | CH₃ | H | |
| 7.195 | 2-Cl | 3-S(O)CH₃ | H | H | CH₃ | H | |
| 7.196 | 2-Cl | 3-S(O)₂CH₃ | H | H | CH₃ | H | |
| 7.197 | 2-Cl | 3-CH₂CN | H | H | CH₃ | H | |
| 7.198 | 2-Cl | 3-OCH₃ | H | H | CH₃ | H | |
| 7.199 | 4-F-2-OCH₃ | H | H | H | CH₃ | H | |
| 7.200 | 4-F-2-OCH₃ | 3-CF₃ | H | H | CH₃ | H | |
| 7.201 | 4-F-2-OCH₃ | 3-CHF₂ | H | H | CH₃ | H | |
| 7.202 | 4-F-2-OCH₃ | 3-SCH₃ | H | H | CH₃ | H | |
| 7.203 | 4-F-2-OCH₃ | 3-S(O)CH₃ | H | H | CH₃ | H | |
| 7.204 | 4-F-2-OCH₃ | 3-S(O)₂CH₃ | H | H | CH₃ | H | |
| 7.205 | 4-F-2-OCH₃ | 3-CH₂CN | H | H | CH₃ | H | |
| 7.206 | 4-F-2-OCH₃ | H | H | H | H | CH₃ | |
| 7.207 | 4-F-2-OCH₃ | 3-CF₃ | H | H | H | CH₃ | |
| 7.208 | 4-F-2-OCH₃ | 3-CHF₂ | H | H | H | CH₃ | |
| 7.209 | 4-F-2-OCH₃ | 3-SCH₃ | H | H | H | CH₃ | |
| 7.210 | 4-F-2-OCH₃ | 3-S(O)CH₃ | H | H | H | CH₃ | |
| 7.211 | 4-F-2-OCH₃ | 3-S(O)₂CH₃ | H | H | H | CH₃ | |
| 7.212 | 4-F-2-OCH₃ | 3-CH₂CN | H | H | H | CH₃ | |
| 7.213 | 4-F-2-OCH₃ | H | H | H | -(CH₂)₄- | | |
| 7.214 | 4-F-2-OCH₃ | 3-CF₃ | H | H | -(CH₂)₄- | | |
| 7.215 | 4-F-2-OCH₃ | 3-CHF₂ | H | H | -(CH₂)₄- | | |
| 7.216 | 4-F-2-OCH₃ | 3-SCH₃ | H | H | -(CH₂)₄- | | |
| 7.217 | 4-F-2-OCH₃ | 3-S(O)CH₃ | H | H | -(CH₂)₄- | | |
| 7.218 | 4-F-2-OCH₃ | 3-S(O)₂CH₃ | H | H | -(CH₂)₄- | | |
| 7.219 | 4-F-2-OCH₃ | 3-CH₂CN | H | H | -(CH₂)₄- | | |
| 7.220 | 4-F-2-OCH₃ | H | CH₃ | H | -(CH₂)₄- | | |
| 7.221 | 4-F-2-OCH₃ | 3-CF₃ | CH₃ | H | -(CH₂)₄- | | |
| 7.222 | 4-F-2-OCH₃ | 3-CHF₂ | CH₃ | H | -(CH₂)₄- | | |
| 7.223 | 4-F-2-OCH₃ | 3-SCH₃ | CH₃ | H | -(CH₂)₄- | | |
| 7.224 | 4-F-2-OCH₃ | 3-S(O)CH₃ | CH₃ | H | -(CH₂)₄- | | |
| 7.225 | 4-F-2-OCH₃ | 3-S(O)₂CH₃ | CH₃ | H | -(CH₂)₄- | | |
| 7.226 | 4-F-2-OCH₃ | 3-CH₂CN | CH₃ | H | -(CH₂)₄- | | |
| 7.227 | 4-F-2-OCH₃ | H | H | H | Cl | H | |
| 7.228 | 4-F-2-OCH₃ | 3-CF₃ | H | H | Cl | H | |
| 7.229 | 4-F-2-OCH₃ | 3-CHF₂ | H | H | Cl | H | |
| 7.230 | 4-F-2-OCH₃ | 3-SCH₃ | H | H | Cl | H | |
| 7.231 | 4-F-2-OCH₃ | 3-S(O)CH₃ | H | H | Cl | H | |
| 7.232 | 4-F-2-OCH₃ | 3-S(O)₂CH₃ | H | H | Cl | H | |
| 7.233 | 4-F-2-OCH₃ | 3-CH₂CN | H | H | Cl | H | |

"NMR" der Beispielverbindungen wurde jeweils als ¹H-NMR-Spektum bei 300 MHz (CDCl₃) (¹H-Kernresonanz-Daten) gemessen. In der nachfolgenden Tabelle 8 sind charakterische chemische Verschiebungen δ (ppm) für einige Beispielverbindungen aufgeführt.

**Tabelle 8: NMR-Daten zu Verbindungen aus Tabellen 1 bis 7**

| Beisp. Nr. | ¹H-NMR-Daten (CDCl₃; δ in ppm) |
|---|---|
| 1.001 | 5,00 (s, 2H); 7,30 (m, 3H); 7,39 (m, 3H); 7,47 (m, 2H); 7,61 (m, 2H); 8,18 (s, 1H) |
| 1.002 | 5,0 (s, 2H); 7,4 (m, 4H); 7,6 (m, 4H); 7,74 (s, 1H); 8,18 (s, 1H) |
| 1.004 | 2,47 (s, 3H); 5,01 (s, 2H); 7,2 (m, 3H); 7,32 (m, 1H); 7,4 (m, 3H); 7,62 (m, 2H); 8,16 (s, 1H) |
| 1.006 | 3,04 (s, 3H); 5,01 (s, 2H); 7,40 (m, 3H), 7,53 (t, 1H); 7,61 (m, 2H); 7,72 (m, 1H); 7,88 (m, 1H); 8,05 (m, 1H); 8,16 (s, 1H) |
| 1.007 | 5,0 (s, 2H); 7,39 (m, 4H); 7,6 (m, 5H); 8,16 (s, 1H) |
| 1.008 | 5,0 (s, 2H); 7,4 (m, 3H); 7,6 (m, 3H); 7,75 (m, 2H), 7,85 (br s, 1H); 8,18 (s, 1H) |
| 1.009 | 5,0 (s, 2H); 7,4 (m, 3H); 7,62 (m, 3H); 7,88 (m, 1H); 7,97 (m, 1H); 8,12 (br s, 1H); 8,16 (s, 1H) |
| 1.010 | 3,72 (s, 2H); 5,0 (s, 2H); 7,3 - 7,45 (m, 7H); 7,62 (m, 2H); 8,16 (s, 1H) |
| 1.017 | 3,79 (s, 3H); 5,0 (s, 2H); 6,88 (m, 1H); 7,01 (m, 1H); 7,07 (m, 1H); 7,12 (m, 1H); 7,39 (m, 3H); 7,61 (m, 2H); 8,17 (s, 1H) |
| 1.018 | 4,99 (s, 2H); 7,39 (m, 3H); 7,61 (m, 2H); 7,81 (s, 1H); 7,9 (s, 2H); 8,16 (s,1H) |
| 1.022 | 2,49 (s, 3H); 5,02 (s; 2H); 7,06 (m, 1H); 7,2 (m, 3H); 7,34 (m, 2H); 7,85 (m, 2H); 8,42 (s, 1H) |
| 1.023 | 2,72 (s, 3H); 5,02 (s; 2H); 7,08 (m, 1H); 7,34 (m, 1H); 7,48 (m, 1H); 7,6 (m, 2H); 7,73 (m, 1H); 7,86 (m, 2H); 8,42 (s, 1H) |
| 1.024 | 3,05 (s, 3H); 5,02 (s; 2H); 7,08 (m, 1H); 7,36 (m, 1H); 7,53 (m, 1H); 7,74 (m, 1H); 7,87 (m, 3H); 8,05 (m, 1H); 8,43 (s, 1H) |
| 1.035 | 2,47 (s, 3H); 5,03 (s; 2H); 7,2 (m, 3H); 7,34 (s, 1H); 7,47 (m, 1H); 7,6 (t, 1H); 7,68 (m, 1H); 8,02 (d, 1H); 8,52 (s, 1H) |
| 1.036 | 2,73 (s, 3H); 5,06 (s; 2H); 7,5 (m, 2H); 7,6 (m, 3H); 7,7 (m, 1H); 7,75 (m, 1H); 8,02 (d, 1H); 8,53 (s, 1H) |
| 1.037 | 3,06 (s, 3H); 5,06 (s; 2H); 7,5 - 7,75 (m, 5H); 7,9 (m, 1H); 8,03 (m, 2H); 8,53 (s, 1H) |
| 1.038 | 3,71 (s, 2H); 5,05 (s, 2H); 7,32 (m, 2H); 7,47 (m, 3H); 7,61 (m, 1H); 7,68 (m, 1H); 8,03 (m, 1H); 8,53 (s, 1H) |
| 1.049 | 2,43 (s, 3H); 3,83 (s, 3H); 4,98 (s, 2H); 6,88 (d, 1H); 6,96 (t, 1H); 7,2 (m, 3H); 7,37 (m, 2H); 7,81 (m, 1H); 8,58 (s, 1H) |
| 1.050 | 2,75 (s, 3H); 3,83 (s, 3H); 5,0 (s, 2H); 6,9 (d, 1H); 6,96 (t, 1H); 7,38 (m, 1H); 7,5 (m, 1H); 7,6 (m, 2H); 7,72 (s, 1H); 7,81 (d, 1H); 8,58 (s, 1H) |
| 1.051 | 3,05 (s, 3H); 3,83 (s, 3H); 5,0 (s, 2H); 6,9 (d, 1H); 6,98 (t, 1H); 7,38 (m, 1H); 7,53 (m, 1H); 7,72 (m, 1H); 7,81 (m, 1H); 7,88 (m, 1H); 8,04 (s, 1H); 8,58 (s, 1H) |
| 1.062 | 2,47 (s, 3H); 3,82 (s, 3H); 4,97 (s, 2H); 6,6 - 6,7 (m, 2H); 7,2 (m, 3H); 7,34 (s, 1H); 7,8 (m, 1H); 8,47 (s, 1H) |
| 1.063 | 2,73 (s, 3H); 3,82 (s, 3H); 4,96 (s, 2H); 6,6 - 6,7 (m, 2H); 7,48 (m, 1H); 7,6 (m, 2H); 7,71 (s, 1H); 7,8 (m, 1 H); 8,46 (s, 1 H) |
| 1.064 | 3,04 (s, 3H); 3,82 (s, 3H); 4,96 (s, 2H); 6,6 - 6,7 (m, 2H); 7,53 (m, 1H); 7,72 (m, 1H); 7,8 (m, 1H); 7,88 (m, 1H); 8,05 (m, 1H); 8,45 (s, 1H) |
| 1.065 | 3,71 (s, 2H); 3,83 (s, 3H); 4,97 (s, 2H); 6,64 (m, 2H); 7,3 (m, 2H); 7,43 (m, 2H); 7,8 (m, 1H); 8,47 (s, 1H) |
| 1.114 | 2,47 (s, 3H); 3,87 (s, 3H); 5,0 (s, 2H); 7,1 (s, 1H); 7,2 (m, 4H); 7,34 (s, 1H); 7,97 (m, 1H); 8,47 (s, 1H) |
| 1.115 | 2,73 (s, 3H); 3,9 (s, 3H); 5,01 (s, 2H); 7,1 (s, 1H); 7,2 (d, 1H); 7,47 (m, 1H); 7,6 (m, 2H); 7,72 (s, 1H); 7,95 (d, 1H); 8,53 (s, 1H) |
| 1.116 | 3,05 (s, 3H); 3,9 (s, 3H); 5,01 (s, 2H); 7,1 (s, 1H); 7,22 (d, 1H); 7,54 (m, 1H); 7,72 (m, 1H); 7,87 (m, 1H); 7,93 (d, 1H); 8,05 (m, 1H); 8,53 (s, 1H) |
| 1.117 | 3,71 (s, 2H); 3,89 (s, 3H); 5,0 (s, 2H); 7,1 (s, 1H); 7,25 (m, 3H); 7,43 (m, 2H); 7,93 (d, 1H); 8,53 (s, 1H) |
| 1.127 | 3,78 (s, 3H); 4,98 (s, 2H); 6,87 (m, 1H); 7,0 (m, 1H); 7,08 (m, 3H); 7,21 (m, 1H); 7,6 (m, 2H); 8,12 (s, 1H) |
| 1.128 | 2,42 (s, 3H); 4,98 (s, 2H); 7,05 (m, 2H); 7,2 (m, 3H); 7,35 (m, 1 H); 7,6 (m, 2H); 8,13 (s, 1H) |
| 1.131 | 3,72 (s, 2H); 4,98 (s, 2H); 7,08 (m, 2H); 7,27 - 7,37 (m, 2H); 7,43 (m, 2H); 7,6 (m, 2H); 8,12 (s, 1H) |
| 1.138 | 4,99 (s, 2H); 7,09 (m, 2H); 7,62 (m, 2H); 7,81 (s, 1H); 7,9 (s, 2H); 8,16 (s,1H) |
| 1.156 | 3,79 (s, 3H); 4,98 (s, 2H); 6,88 (m, 1H); 7,0 (m, 1H); 7,06 (m, 1H); 7,21 (m, 1H); 7,36 (m, 2H); 7,55 (m, 2H); 8,12 (s, 1H) |
| 1.157 | 2,45 (s, 3H); 4,99 (s, 2H); 7,21 (m, 3H); 7,36 (m, 3H); 7,57 (m, 2H); 8,12 (s, 1H) |
| 1.160 | 3,76 (s, 2H); 4,99 (s, 2H); 7,28 - 7,45 (m, 6H); 7,58 (m, 2H); 8,12 (s, 1H) |
| 1.167 | 5,0 (s, 2H); 7,21 (d, 2H); 7,31 (m; 3H); 7,45 (m, 2H); 7,65 (m, 2H); 8,13 (s, 1H) |
| 1.170 | 3,79 (s, 3H); 5,0 (s, 2H); 6,88 (m, 1H); 6,99 (m, 1 H); 7,06 (m, 1H); 7,22 (m, 3H); 7,64 (m, 2H); 8,14 (s, 1H) |
| 1.182 | 2,28 (s, 3H); 2,32 (s, 3H); 5,01 (s, 2H); 7,1 - 7,4 (m, 7H); 7,67 (m, 2H) |
| 1.183 | 2,3 (s, 3H); 5,02 (s, 2H); 7,4 (m, 4H); 7,57 (m, 1 H); 7,65 (m, 3H); 7,74 (s, 1H) |
| 1.195 | 2,28 (s, 3H); 2,47 (s, 3H); 5,0 (s, 2H); 7,2 - 7,42 (m, 8H) |
| 1.196 | E-Isomer: 2,29 (s, 3H); 2,73 (s, 3H); 5,0 (s, 2H); 7,25 - 7,63 (m, 7H); 7,74 (m, 1H) Z-Isomer: 2,21 (s, 3H); 2,73 (s, 3H); 4,81 (s, 2H); 7,25 - 7,63 (m, 7H); 7,7 (m, 1H) |
| 1.197 | E-Isomer: 2,29 (s, 3H); 3,05 (s, 3H); 5,0 (s, 2H); 7,3 - 7,42 (m, 4H); 7,55 (m, 1H); 7,74 (m, 1H); 7,9 (m, 1H); 8,05 (m, 1H) Z-Isomer: 2,26 (s, 3H); 3,05 (s, 3H); 4,82 (s, 2H); 7,18 (m, 1H); 7,3 - 7,42 (m, 7H); 7,54 (m, 1H); 7,7 (m, 1H); 7,89 (m, 1H); 8,01 (m, 1H) |
| 1.215 | 2,25 (s, 3H); 2,47 (s, 3H); 3,83 (s, 3H); 4,99 (s, 2H); 6,88 - 6,98 (m, 2H); 7,2 (m, 3H); 7,34 (m, 3H) |
| 1.216 | E- Isomer: 2,25 (s, 3H); 2,72 (s, 3H); 3,82 (s, 3H); 4,99 (s, 2H); 6,85 (m, 2H); 7,33 (m, 2H); 7,48 (m, 1H); 7,6 (m, 2H); 7,71 (m, 1H) Z-Isomer: 2,14 (s, 3H); 2,72 (s, 3H); 3,82 (s, 3H); 4,9 (s, 2H) |
| 1.217 | 2,25 (s, 3H); 3,05 (s, 3H); 3,82 (s, 3H); 5,0 (s, 2H); 6,85 (m, 2H); 7,35 (m, 2H); 7,53 (m, 1H); 7,73 (m, 1H); 7,88 (m, 1H); 8,06 (m, 1H) |
| 1.251 | 1,1 (m, 2H); 1,33 (m, 2H); 2,5 (m, 1H); 3,78 (s, 3H); 4,17 (s, 2H); 6,85 - 7,55 (m, 8H) |
| 1.365 | 4,96 (s, 2H); 5,99 (s, 2H); 6,8 (d, 1H); 6,98 (dd, 1H); 7,24 (d, 1H); 7,42 (t, 1H); 7,56 (d, 1H); 7,62 (d, 1H); 7,73 (s, 1H); 8,06 (s, 1H) |
| 1.369 | 2,32 (s, 3H); 5,0 (s, 2H); 7,1 - 7,3 (m, 4H); 7,44 (m, 2H); 7,74 (m, 1 H); 8,06 (s, 1H) |
| 1.371 | 5,01 (s, 2H); 7,44 (m, 3H); 7,58 (m, 1H); 7,63 (m, 1H); 7,74 (m, 2H); 8,07 (s, 1H) |
| 1.372 | 2,45 (s, 3H); 4,98 (s, 2H); 6,87 (m, 3H); 7,15 - 7,3 (m, 8H); 7,96 (m, 1 H); 8,47 (s, 1H) |
| 1.373 | 3,72 (s, 2H); 4,98 (s, 2H); 6,87 (m, 3H); 7,17 (m, 1H); 7,25 - 7,32 (m, 7H); 7,98 (m, 1H); 8,45 (s, 1H) |
| 1.374 | 3,78 (s, 3H); 3,82 (s, 3H); 4,96 (s, 2H); 6,6 (m, 2H); 6,87 (m, 1H); 7,0 (m, 1H); 7,05 (m, 1H); 7,2 (t, 1H); 7,8 (m, 1H); 8,45 (s, 1H) |
| 1.375 | 3,78 (s, 3H); 3,88 (s, 3H); 5,01 (s, 2H); 6,87 (m, 1H); 7,0 (m, 1H); 7,05 (m, 2H); 7,2 (m, 2H); 7,93 (m, 1H); 8,54 (s, 1H) |
| 1.376 | 3,78 (s, 3H); 5,0 (s, 2H); 6,87 (m, 4H); 7,0 (m, 1H); 7,05 (m, 1H); 7,1 - 7,4 (m, 5H); 7,97 (m, 1H); 8,48 (s, 1H) |
| 1.377 | 2,3 (s, 3H); 2,33 (s, 3H); 2,45 (s, 3H); 7,07 (m, 1H); 7,1 - 7,4 (m, 6H) |
| 1.378 | 2,38 (s, 3H); 2,65 (s, 3H); 3,9 (s, 3H); 5,0 (s, 2H); 7,1 (s, 1H); 7,15 (d, 1H); 7,21 (d, 1H); 7,45 (dd, 1H); 7,95 (d, 1H); 8,06 (d, 1H); 8,53 (s; 1H) |
| 1.381 | 2,72 (s, 3H); 3,86 (s, 3H); 3,88 (s, 3H); 5,0 (s, 2H); 6,95 (m, 1H); 7,05 (t, 1H); 7,45 (m, 2H); 7,6 (m, 2H); 7,7 (m, 1H); 8,51 (s, 1H) |
| 1.382 | 2,22 (s, 3H); 2,32 (s, 3H); 3,81 (s, 3H); 6,9 (m, 2H); 7,07 (d, 1H); 7,18 (dd, 1H); 7,22 (m, 1 H); 7,35 (m, 2H) |
| 1.383 | 1,14 (t, 3H); 2,75 (q, 2H); 3,79 (s, 3H); 5,01 (s, 2H); 6,88 (m, 1H); 7,0 (m, 1H); 7,07 (m, 1H); 7,21 (t, 1H); 7,47 (m, 2H); 7,78 (m, 1 H) |
| 1.384 | 1,15 (t, 3H); 2,75 (q, 2H); 3,79 (s, 3H); 5,0 (s, 2H); 7,47 (m, 3H); 7,57 (d, 1H); 7,63 (d, 1H); 7,72 (m, 1H); 7,78 (m, 1H) |
| 1.385 | 1,15 (t, 3H); 2,47 (s, 3H); 2,75 (q, 2H); 5,0 (s, 2H); 7,2 (m, 3H); 7,32 (m, 1H); 7,47 (m, 2H); 7,78 (m, 1H) |
| 2.049 | 2,44 (s, 3H); 2,95 (s, 3H); 3,84 (s, 3H); 4,31 (s, 2H); 6,6 (m, 2H); 7,17 (m, 3H); 7,28 (s, 1H); 7,68 (s, 1H); 7,84 (m, 1H) |
| 3.022 | 2,45 (s, 3H); 5,02 (s, 2H); 7,2 (m, 3H); 7,35 (s, 1H); 7,75 (d, 1H); 7,82 (dd, 1H); 8,2 (s, 1H); 8,68 (d,1H) |
| 3.023 | 2,73 (s, 3H); 5,03 (s, 2H); 7,48 (m, 1H); 7,6 (m, 2H); 7,75 (m, 2H); 7,85 (m, 1H); 8,2 (s, 1H); 8,68 (d, 1H) |
| 3.024 | 3,05 (s, 3H); 5,02 (s, 2H); 7,53 (m, 1H); 7,75 (m, 2H); 7,85 (m, 2H); 8,04 (m, 1H); 8,2 (s, 1H); 8,68 (d, 1H) |
| 3.061 | 2,47 (s, 3H); 5,18 (s, 2H); 7,2 (m, 3H); 7,35 (m, 1H); 7,44 (m, 1H); 8,02 (m, 1H); 8,59 (m, 1H); 8,9 (m, 1H) |
| 3.062 | 2,73 (s, 3H); 5,18 (s, 2H); 7,48 (m, 2H); 7,59 (m, 1H); 7,63 (m, 1H); 7,73 (m, 1H); 8,02 (m, 1H); 8,59 (m, 1H); 8,9 (m, 1H) |
| 3.063 | 3,04 (s, 3H); 5,19 (s, 2H); 7,52 (m, 2H); 7,73 (m, 1H); 7,9 (m, 1H); 8,04 (m, 2H); 8,59 (m, 1H); 8,9 (m, 1H) |
| 3.074 | 2,45 (s, 3H); 2,58 (s, 3H); 5,02 (s, 2H); 7.15 (d, 1H); 7,2 (m, 3H); 7,33 (m, 1H); 7,59 (t, 1H); 7,67 (d, 1H); 8,22 (s, 1H) |
| 3.122 | 2,43 (s, 6H); 5,08 (s, 2H); 7,13 (d, 2H); 8,12 (s, 1H); 8,23 (s, 1H); 8,73 (s, 1H) |
| 4.004 | 2,47 (s, 3H); 5,09 (s, 2H); 7,2 (m, 3H); 7,35 (m, 1H); 8,24 (s, 1H); 8,58 (m, 2H); 9,1 (m, 1H) |
| 4.005 | 2,74 (s, 3H); 5,08 (s, 2H); 7,5 (m, 1H); 7,6 (m, 2H); 7,75 (s, 1H); 8,15 (s, 1H); 8,58 (m, 1H); 9,12 (s, 1H) |
| 4.006 | 3, 05 (s, 3H); 5,1 (s, 2H); 7,55 (m, 1H); 7,73 (m, 1H); 7,9 (m, 1H); 8,05 (m, 1H); 8,25 (s, 1H); 8,58 (m, 1H); 9,13 (m, 1H) |
| 4.010 | 3,72 (s, 2H); 5,09 (s, 2H); 7,27 - 7,45 (m, 4H); 8,25 (s, 1H); 8,6 (m, 2H); 9,12 (m, 1H) |
| 4.017 | 3,79 (s, 3H); 5,08 (s, 2H); 6,88 (m, 1H); 7,0 (m, 1H); 7,07 (m, 1H); 7,21 (m, 1H); 8,24 (s, 1H); 8,58 (m, 2H); 9,12 (m, 1H) |
| 4.077 | 2,3 (s, 3H); 2,46 (s, 3H); 5,1 (s, 1H); 7,05 - 7,22 (m, 3H); 8,24 (s, 1H); 8,58 (m, 2H); 9,12 (m, 1H) |
| 5.004 | 2,47 (s, 3H); 5,02 (s, 2H); 7,2 (m, 3H); 7,35 (m, 2H); 8,0 (m, 1H); 8,18 (s, 1H); 8,6 (m, 1H); 8,77 (m, 1H) |
| 5.006 | 3,03 (s, 3H); 5,02 (s, 2H); 7,34 (m, 1H); 7,53 (t, 1H); 7,72 (m, 1H); 7,88 (m, 1H); 8,0 (m, 1H); 8,04 (m, 1H); 8,18 (s, 1H); 8,61 (m, 1H); 8,78 (m, 1H) |
| 5.010 | 3,72 (s, 2H); 5,0 (s, 2H); 7,3 (m, 3H); 7,42 (m, 2H); 8,0 (m, 1H); 8,18 (s, 1H); 8,6 (m, 1H); 8,78 (s, 1H) |
| 5.017 | 3,79 (s, 3H); 5,02 (s, 2H); 6,88 (m, 1H); 7,0 (s, 1H); 7,05 (d, 1H); 7,2 (m, 1H); 7,33 (m, 1H); 8,0 (m, 1H); 8,18 (s, 1H); 8,6 (m, 1H); 8,78 (m, 1H) |
| 5.022 | 2,47 (s, 3H); 3,97 (s, 3H); 4,98 (s, 2H); 6,9 (m, 1H); 7,2 (m, 3H); 7,35 (m, 1H); 8,08 (m, 1H); 8,2 (m, 1H); 8,4 (s, 1H) |
| 5.024 | 3,03 (s, 3H); 3,98 (s, 3H); 5,0 (s, 2H); 6,9 (m, 1H); 7,53 (t, 1H); 7,72 (m, 1H); 7,88 (m, 1H); 8,05 (m, 1H); 8,09 (m, 1H); 8,2 (m, 1H); 8,42 (s, 1H) |
| 5.026 | 3,73 (s, 2H); 3,98 (s, 3H); 4,98 (s, 2H); 6,9 (m, 1H); 7,2 (m, 2H); 7,43 (m, 2H); 8,1 (m, 1H); 8,2 (m, 1H); 8,42 (s, 1H) |
| 5.031 | 3,79 (s, 3H); 3,98 (s, 3H); 4,99 (s, 2H); 6,88 (m, 2H); 7,0 (s, 1H); 7,05 (d, 1H); 7,2 (m, 1H); 8,1 (m, 1H); 8,2 (m, 1H); 8,41 (s, 1H) |
| 5.032 | 1,4 (t, 3H); 2,47 (s, 3H); 4,4 (q, 2H); 4,98 (s, 2H); 6,87 (m, 1H); 7,2 (m, 3H); 7,37 (m, 1H); 8,08 (m, 1H); 8,18 (m, 1H); 8,44 (s, 1H) |
| 5.033 | 1,4 (t, 3H); 2,72 (s, 3H); 4,4 (q, 2H); 5,0 (s, 2H); 6,88 (m, 1H); 7,48 (t, 1H); 7,6 (m, 2H); 7,73 (m, 1H); 8,08 (m, 1H); 8,18 (m, 1H); 8,43 (s, 1H) |
| 5.034 | 1,4 (t, 3H); 3,04 (s, 3H); 4,4 (q, 2H); 5,0 (s, 2H); 6,9 (m, 1H); 7,53 (t, 1H); 7,72 (m, 1H); 7,9 (m, 1H); 8,04 (m, 1H); 8,1 (m, 1H); 8,19 (m, 1H); 8,43 (s, 1H) |
| 5.036 | 1,4 (t, 3H); 3,73 (s, 2H); 4,4 (q, 2H); 4,98 (s, 2H); 6,88 (m, 1H); 7,3 (m, 2H); 7,43 (m, 2H); 8,1 (m, 1H); 8,18 (m, 1H); 8,43 (s, 1H) |
| 5.053 | 2,47 (s, 3H); 4,8 (q, 2H); 4,98 (s, 2H); 6,88 (d, 1H); 7,2 (m, 3H); 7,34 (s, 1H); 8,03 (m, 1H); 8,1 (s, 1H); 8,21 (m, 1H) |
| 5.066 | 2,47 (s, 3H); 5,08 (s, 2H); 7,2 (m, 3H); 7,37 (m, 1H); 7,58 (d, 1H); 8,47 (m, 1H); 8,9 (d, 1H); 9,35 (s, 1H) |
| 5.067 | 2,74 (s, 3H); 5,08 (s, 2H); 7,5 (m, 1 H); 7,6 (m, 3H); 7,75 (m, 1H); 8,47 (m, 1H); 8,8 (d, 1H); 9,35 (s, 1H) |
| 5.068 | 3,05 (s, 3H); 5,08 (s, 2H); 7,55 (m, 2H); 7,73 (m, 1H); 7,9 (m, 1H); 8,05 (m, 1H); 8,47 (m, 1H); 8,8 (d, 1H); 9,35 (s, 1H) |
| 5.070 | 3,74 (s, 2H); 5,03 (s, 2H); 7,3 (m, 2H); 7,43 (m, 2H); 7,57 (d, 1H); 8,47 (m, 1H); 8,8 (d, 1H); 9,35 (s, 1H) |
| 5.075 | 3,8 (s, 3H); 5,08 (s, 2H); 6,9 (m, 1H); 7,0 (m, 1H); 7,07 (m, 1H); 7,2 (m, 1H); 7,57 (d, 1H); 8,47 (m, 1H); 8,8 (d, 1H); 9,35 (s, 1H) |
| 5.121 | 2,73 (s, 3H); 3,97 (s, 3H); 4,98 (s, 2H); 6,78 (d, 1H); 7,48 (t, 1H); 7,6 (m, 2H); 7,72 (s, 1H); 7,98 (m, 1H); 8,1 (s, 1H); 8,22 (m, 1H) |
| 5.124 | 3,7 (s, 2H); 4,96 (s, 2H); 6,8 (d, 1H); 7,3 (m, 2H); 7,42 (m, 2H); 7,98 (m, 1H); 8,1 (s, 1H); 8,22 (m, 1H) |
| 5.152 | 2,47 (s,3H); 5,03 (s, 2H); 7,2 (m, 3H); 7,34 (s, 1H); 7,7 (d, 1H); 8,17 (m, 1H), 8,21 (s, 1H); 8,87 (s,1H) |
| 5.153 | 2,74 (s, 3H); 5,04 (s, 2H); 7,5 (t, 1H); 7,6 (m, 1H); 7,75 (m, 2H); 8,18 (m, 1H); 8,21 (s, 1H); 8,9 (s, 1H) |
| 5.154 | 3,05 (s, 3H); 5, 07 (s, 2H); 7,55 (t, 1H); 7,73 (m, 1H); 7,9 (m, 1H); 8,04 (m, 1H) ;8,18 (m, 1H); 8,21 (s, 1H); 8,88 (s, 1H) |
| 5.156 | 3,72 (s, 2H); 5,02 (s, 2H); 7,3 (m, 2H); 7,43 (m, 2H); 8,18 (m, 1H); 8,22 (s, 1H); 8,9 (s, 1H) |
| 5.158 | 3,78 (s, 3H); 5,03 (s, 2H); 6,88 (m, 1H); 6,98 (m, 1H); 7,05 (m, 1H); 7,2 (m, 1H); 7,7 (d, 1H); 8,15 (m, 1H); 8,2 (s, 1H); 8,86 (s, 1H) |
| 5.159 | 1,4 (t, 3H); 3,78 (s, 3H); 4,4 (q, 2H); 5,0 (s, 2H); 6,88 (m, 2H); 7,0 (s, 1H); 7,05 (d, 1H); 7,2 (m, 1H); 8,1 (m, 1H); 8,18 (m, 1H); 8,43 (s, 1H) |
| 5.160 | 2,3 (s, 3H); 2,43 (s, 3H); 5,02 (s, 2H); 7,05 (d, 1H); 7,15 (m, 1H); 7,22 (m, 1H); 7,32 (m, 1H); 8,0 (m, 1H); 8,18 (s, 1H); 8,6 (m, 1H); 8,77 (m, 1H) |
| 5.161 | 2,3 (s, 3H); 2,45 (s, 3H); 3,96 (s, 3H); 4,98 (s, 2H); 6,9 (m, 1H); 7,06 (d, 1H); 7,15 (m, 1H); 7,22 (m, 1H); 8,06 (m, 1H); 8,19 (m, 1H); 8,42 (s, 1H) |
| 5.162 | 1,4 (t, 3H); 2,31 (s, 3H); 2,45 (s, 3H); 4,4 (q, 2H); 5,0 (s, 2H); 6,87 (m, 1H); 7,06 (d, 1H); 7,15 (m, 1H); 7,22 (m, 1H); 8,08 (m, 1H); 8,18 (m, 1H); 8,43 (s, 1H) |
| 5.163 | 2,3 (s, 3H); 2,45 (s, 3H); 5,05 (s, 2H); 7,0 - 7,2 (m, 3H); 7,7 (d, 1 H); 8,15 (m, 1H); 8,2 (s, 1H); 8,87 (s, 1H) |
| 5.164 | 1,36 (d, 6H); 2,43 (s, 3H); 4,99 (s, 2H); 5,38 (sep, 1H); 6,83 (m, 1H); 7,2 (m, 3H); 7,35 (m, 1H); 8,06 (m, 1H); 8,15 (m, 1H); 8,42 (s, 1H) |
| 5.165 | 1,36 (d, 6H); 3,73 (s, 2H); 5,0 (s, 2H); 5,38 (sep, 1H); 6,84 (m, 1H); 7,3 (m, 3H); 7,42 (m, 1H); 8,07 (m, 1H); 8,18 (m, 1H); 8,42 (s, 1H) |
| 5.166 | 1,34 (d, 6H); 2,3 (s, 3H); 2,45 (s, 3H); 5,0 (s, 2H); 5,38 (sep, 1H); 6,82 (m, 1H); 7,05 (d, 1H); 7,17 (m, 1H); 7,22 (m, 1H); 8,08 (m, 1H); 8,17 (m, 1H); 8,43 (s, 1H) |
| 5.167 | 1,35 (d, 6H); 3,79 (s, 3H); 5,0 (s, 2H); 5,36 (sep, 1H); 6,85 (m, 2H); 7,0 (m, 1H); 7,07 (m, 1H); 7,2 (m, 1H); 8,1 (m, 1H); 8,17 (m, 1H); 8,43 (s, 1H) |
| 5.169 | 2,7 (t, 3H); 3,04 (s, 3H); 3,98 (s, 3H); 4,98 (s, 2H); 6,92 (m, 1H); 7,3 (d, 1H); 7,59 (m, 1H); 8,1 (m, 1H); 8,15 (m, 1H); 8,2 (m, 1H); 8,41 (s, 1H) |
| 5.172 | 1,4 (t, 3H); 2,7 (t, 3H); 3,04 (s, 3H); 4,4 (q, 2H); 4,98 (s, 2H); 6,88 (m, 1H); 7,3 (d, 1H); 7,58 (m, 1H); 8,08 (m, 1H); 8,17 (m, 2H); 8,43 (s, 1H) |
| 5.173 | 2,38 (s, 3H); 2,67 (s, 3H); 5,02 (s, 2H); 7,17 (d, 1H); 7,43 (m, 1H); 7,71 (d, 1H); 8,07 (m, 1H); 8,2 (m, 1H); 8,22 (s, 1H); 8,88 (s, 1H) |
| 5.174 | 1,36 (d, 6H); 2,38 (s, 3H); 2,66 (s, 3H); 4,99 (s, 2H); 5,38 (sep, 1H); 6,85 (m, 1H); 7,15 (d, 1H); 7,47 (m, 1H); 8,08 (m, 2H); 8,18 (m, 1H); 8,42 (s, 1H) |
| 5.175 | 1,36 (d, 6H); 2,73 (s, 3H); 5,0 (s, 2H); 5,38 (sep, 1H); 6,87 (m, 1H); 7,48 (t, 1H); 7,6 (m, 2H); 7,74 (m, 1H); 8,08 (m, 1H); 8,19 (m, 1H); 8,42 (s, 1H) |
| 5.176 | 1,35 (d, 6H); 3,04 (s, 3H); 5,0 (s, 2H); 5,38 (sep, 1H); 6,87 (m, 1H); 7,53 (t, 1H); 7,73 (m, 1H); 7,9 (m, 1H); 8,08 (m, 2H); 8,19 (m, 1H); 8,43 (s, 1H) |
| 6.049 | 3,8 (s, 3H); 3,9 (s, 3H); 5,22 (s, 2H); 6,82 (d, 1H); 6,9 (m, 1H); 6,95 - 7,1 (m, 4H); 7,2 - 7,35 (m, 2H); 7,74 (d, 1H); 7,93 (m, 1H) |
| 6.073 | 5,24 (s, 2H); 6,94 (d, 1H); 7,2 - 7,4 (m, 5H); 7,45 (m, 3H); 7,77 (d, 1H); 7,81 (m, 1H) |
| 6.076 | 3,8 (s, 3H); 5,23 (s, 2H); 6,84 (d, 1H); 6,94 (m, 1H); 7,0 (m, 1H); 7,07 (m, 1H); 7,2 - 7,35 (m, 3H); 7,45 (m, 1H); 7,75 (d, 1H); 7,81 (m, 1H) |
| 6.077 | 2,49 (s, 3H); 5,22 (s, 2H); 6,82 (d, 1H); 7,2 - 7,35 (m, 6H); 7,45 (m, 1H); 7,76 (d, 1H); 7,8 (m, 1H) |
| 6.100 | 5,21 (s, 2H); 6,7 (m, 1H); 6,9 (m, 2H); 7,35 (m, 3H); 7,48 (m, 2H); 7,75 (d, 1H); 8,0 (m, 1H) |
| 6.103 | 3,8 (s, 3H); 5,21 (s, 2H); 6,71 (d, 1H); 6,85 - 6,95 (m, 3H); 7,0 (m, 1H); 7,07 (m, 1H); 7,21 (m, 1H); 7,75 (d, 1H); 7,99 (m, 1H) |
| 6.104 | 2,45 (s, 3H); 5,22 (s, 2H); 6,7 (m, 1H); 6,85 - 6,95 (m, 2H); 7,23 (m, 3H); 7,34 (s, 1H); 7,74 (m, 1H); 7,98 (m, 1H) |
| 6.141 | 2,37 (s, 3H); 5,2 (s, 2H); 6,58 (d, 1H); 7,2 (d, 2H); 7,34 (m, 3H); 7,48 (m, 2H); 7,7 (m, 3H) |
| 6.142 | 2,37 (s, 3H); 5,21 (s, 2H); 6,59 (d, 1H); 7,2 (d, 2H); 7,39 (t, 1H); 7,57 (m, 1H); 7,62 (m, 1H); 7,7 (m, 3H); 7,77 (s, 1H) |
| 6.144 | 2,38 (s, 3H); 3,8 (s, 3H); 5,2 (s, 2H); 6,58 (d, 1H); 6,9 (m, 1H); 6,99 (m, 1H); 7,07 (m, 1H); 7,2 (m, 3H); 7,7 (m, 3H) |
| 6.145 | 2,37 (s, 3H); 2,47 (s, 3H); 5,21 (s, 2H); 6,58 (d, 1H); 7,2 (m, 5H); 7,33 (s, 1H); 7,7 (m, 3H) |
| 6.147 | 2,38 (s, 3H); 3,05 (s, 3H); 5,22 (s, 2H); 6,6 (d, 1H); 7,2 (d, 2H); 7,58 (t, 1H); 7,7 (m, 4H); 7,9 (m, 1H); 8,04 (m, 1H) |
| 7.049 | 3,76 (s, 3H); 3,84 (s, 3H); 5,01 (s, 2H); 6,3 (m, 1H); 6,84 (m, 2H); 6,93 (m, 1H); 7,04 (m, 2H); 7,17 (m, 1H); 7,35 (dd, 1H); 7,42 (m, 1H); 7,61 (m, 1H) |
| 7.073 | 5,03 (s, 2H); 6,36 (d, 1H); 7,2 - 7,55 (m, 9H); 7,64 (d, 1H) |
| 7.076 | 3,78 (s, 3H); 5,02 (s, 2H); 6,36 (d, 1H); 6,84 (m, 2H); 6,92 (m, 1H); 7,18 (m, 1H); 7,35 - 7,45 (m, 3H); 7,53 (m, 1H); 7,63 (d, 1H) |
| 7.077 | 2,45 (s, 3H); 5,01 (s, 2H); 6,38 (d, 1H); 7,08 (m, 1H); 7,19 (m, 3H); 7,35 -7,45 (m, 3H); 7,53 (m, 1H); 7,62 (d, 1H) |
| 7.100 | 5,09 (s, 2H); 6,38 (d, 1H); 7,0 (m, 2H); 7,29 (m, 5H); 7,45 (m, 1H); 7,62 (d, 1H) |
| 7.103 | 3,77 (s, 3H); 5,08 (s, 2H); 6,39 (d, 1H); 6,82 - 7,02 (m, 5H); 7,08 (m, 1 H); 7,47 (m, 1 H); 7,61 (d, 1H) |
| 7.104 | 2,46 (s, 3H); 5,08 (s, 2H); 6,39 (m, 1H); 7,0 (m, 2H); 7,08 (m, 1H); 7,18 (m, 3H); 7,48 (m, 1H); 7,63 (m, 1H) |
| 7.141 | 2,42 (s, 3H); 5,21 (s, 2H); 6,32 (d, 1H); 7,29 (m, 5H); 7,46 (d, 2H); 7,58 (d, 1 H) |
| 7.144 | 2,42 (s, 3H); 3,78 (s, 3H); 5,1 (s, 2H); 6,33 (m, 1H); 6,86 (m, 1H); 6,93 (m, 1H); 7,0 (m, 1H); 7,18 (m, 1H); 7,3 (d, 2H); 7,45 (d, 2H); 7,59 (m, 1H) |
| 7.145 | 2,43 (s, 3H); 2,47 (s, 3H); 5,11 (s, 2H); 6,33 (d, 1H); 7,15 - 7,3 (m, 6H); 7,43 (d, 2H); 7,6 (m, 1H) |

In der nachfolgenden Tabelle 9 sind log P-Daten zu den Beispielverbindungen aufgeführt.

Die log P-Daten wurden in Übereinstimmung mit der EEC Direktive 79/831, Anlage V.A8 (Annex V.A8) mittels HPLC (High Performance Liquid Chromatography) auf einer Umkehrphase-Säule (reversed-phase column) (C18) mit folgenden Methoden bestimmt:
Temperatur: 40°C; Mobile Phase: 0,1 % bzw. 0,06 %ige wässrige Ameisensäure oder 0,1 % wässriger Phosphorsäure und Acetonitril; linearer Gradient von 10% Acetonitril bis 90% bzw. 95 % Acetonitril. Die Kalibrierung wurde ausgeführt mit Hilfe unverzweigter Alkan-2-one (bestehend aus 3 bis 13 bzw.16 Kohlenstoff-Atomen) mit bekannten IogP-Werten (Bestimmung der IogP-Werte über die Retentionszeiten mittels linearer Interpolation zwischen zwei nachfolgenden Alkanonen).
Die lambda- max-Werte wurden bestimmt über die Maxima der chromatographischen Signale der UV-Spectren von 190 nm bis 400 bzw. 450 nm.

**Tabelle 9: log P-Daten zu Verbindungen aus Tabellen 1 bis 7**

| Nr. | LogP-Werte |
|---|---|
| 1.053 | 4,51 |
| 1.056 | 4,9 |
| 1.066 | 4,58 |
| 1.069 | 4,9 |
| 1.301 | 4,85 |
| 1.379 | 4,11 |
| 1.380 | 3,88 |
| 1.386 | 4,23 |
| 1.387 | 3,22 |
| 1.388 | 4,4 |
| 1.389 | 2,42 |
| 1.393 | 3,77 |
| 1.394 | 3,94 |
| 1.395 | 3,97 |
| 1.396 | 2,04 |
| 1.397 | 2,86 |
| 1.398 | 4,45 |
| 1.399 | 4,53 |
| 1.401 | 3,07 |
| 1.402 | 3,19 |
| 1.403 | 4,45 |
| 1.404 | 5,13 |
| 1.405 | 4,84 |
| 1.406 | 3,87 |
| 1.407 | 4,92 |
| 1.408 | 5,53 |
| 1.409 | 5,36 |
| 1.410 | 4,18 |
| 1.411 | 4,54 |
| 1.412 | 5,13 |
| 1.413 | 4,97 |
| 1.414 | 3,88 |
| 1.415 | 4,42 |
| 1.416 | 5,03 |
| 1.417 | 4,84 |
| 1.418 | 3,72 |
| 1.419 | 4,84 |
| 1.420 | 2,93 |
| 1.421 | 3,46 |
| 1.422 | 4,12 |
| 1.423 | 3,14 |
| 1.424 | 5,33 |
| 1.425 | 5,38 |
| 1.426 | 3,05 |
| 1.427 | 3,15 |
| 1.428 | 4,72 |
| 1.429 | 4,77 |
| 1.430 | 5,24 |
| 1.431 | 5,26 |
| 1.432 | 5,24 |
| 1.433 | 4,32 |
| 1.434 | 4,24 |
| 1.435 | 4,54 |
| 1.436 | 4,48 |
| 1.437 | 2,66 |
| 1.438 | 2,75 |
| 1.439 | 4,29 |
| 1.440 | 5,01 |
| 1.441 | 5,05 |
| 1.442 | 4,29 |
| 1.443 | 3,43 |
| 1.444 | 3,32 |
| 1.445 | 2,73 |
| 1.446 | 2,86 |
| 1.447 | 4,58 |
| 1.448 | 2,78 |
| 1.449 | 5,17 |
| 1.450 | 5,05 |
| 3.085 | 3,79 |
| 3.086 | 1,57 |
| 3.091 | 4,18 |
| 3.092 | 1,75 |
| 3.093 | 2,4 |
| 3.094 | 2,96 |
| 3.095 | 4,77 |
| 3.096 | 2,21 |
| 3.097 | 2,93 |
| 3.098 | 3,5 |
| 3.120 | 4,92 |
| 3.121 | 2,96 |
| 3.125 | 4,76 |
| 3.126 | 2,78 |
| 4.078 | 4,18 |
| 4.080 | 2,99 |
| 4.084 | 3,34 |
| 4.086 | 2,4 |
| 4.090 | 1,57 |
| 4.091 | 1,99 |
| 4.092 | 4,03 |
| 4.094 | 3,09 |
| 4.098 | 2,18 |
| 4.100 | 4,03 |
| 4.106 | 2,21 |
| 5.040 | 4,23 |
| 5.041 | 2,28 |
| 5.044 | 3,24 |
| 5.120 | 4 |
| 5.122 | 2,5 |
| 5.125 | 2,94 |
| 5.126 | 3,49 |
| 5.127 | 4,34 |
| 5.131 | 4,03 |
| 5.132 | 2,42 |
| 5.133 | 4,9 |
| 5.134 | 2,51 |
| 5.135 | 4,26 |
| 5.136 | 4,8 |
| 5.137 | 4,72 |
| 5.138 | 3,83 |
| 5.139 | 4,1 |
| 5.140 | 2,17 |
| 5.141 | 3,83 |
| 5.142 | 4,54 |
| 5.143 | 3,79 |
| 5.144 | 2,76 |
| 5.145 | 2,19 |
| 5.146 | 4,11 |
| 5.147 | 2,17 |
| 5.148 | 4,57 |
| 5.168 | 2,42 |
| 5.170 | 1,37 |
| 5.171 | 2,83 |
| 5.177 | 4,87 |
| 5.178 | 3,69 |
| 5.181 | 2,77 |
| 5.183 | 4,93 |
| 5.184 | 3,79 |
| 5.185 | 2,89 |
| 5.186 | 4,22 |
| 5.187 | 3,18 |
| 5.188 | 2,23 |
| 5.189 | 2,42 |
| 5.190 | 4,42 |
| 5.191 | 5,08 |
| 5.192 | 4,03 |
| 5.193 | 3,4 |
| 5.194 | 5,44 |
| 5.195 | 4,27 |
| 5.196 | 3,43 |
| 5.197 | 4,81 |
| 5.198 | 4,65 |
| 5.199 | 4,68 |
| 5.200 | 2,18 |
| 6.050 | 3,43 |

### (B) Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man

| | |
|---|---|
| 75 Gewichtsteile | einer Verbindung der Formel (I), |
| 10 | " ligninsulfonsaures Calcium, |
| 5 | " Natriumlaurylsulfat, |
| 3 | " Polyvinylalkohol und |
| 7 | " Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gewichtsteile einer Verbindung der Formel (I), 5 " 2,2'-dinaphthylmethan-6,6'-disulfonsaures Natrium 2 " oleoylmethyltaurinsaures Natrium, 1 Gewichtsteil Polyvinylalkohol, 17 Gewichtsteile Calciumcarbonat und 50 " Wasser auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### (C) Biologische Beispiele

### 1. Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. Auflaufschäden erfolgte nach dem Auflaufen der Versuchspflanzen nach einer Versuchszeit von 3 bis 4 Wochen im Vergleich zu unbehandelten Kontrollen. Wie die Testergebnisse zeigten, wiesen die erfindungsgemäßen Verbindungen eine gute herbizide Vorauflaufwirksamkeit gegen ein breites Spektrum von Ungräsern und Unkräutern auf. Beispielsweise zeigten die Beispiele 1.049, 1.050, 1.062, 1.063, 1.115, 1.128, 1.301, 3.086, 5.022, 5.040, 5.041, 5.053, 5.120, 5.121, 5.152, 5.177, 5.181, 5.189, 5.190 aus den Tabellen 1 bis 5 im Test sehr gute herbizide Wirkung gegen Schadpflanzen wie Echinochloa crus-galli, Setaria viridis, Stellaria media und Veronica persica im Vorauflaufverfahren bei einer Aufwandmenge von 1,28 kg oder weniger Aktivsubstanz pro Hektar.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt. Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 I/ha auf die grünen Pflanzenteile gesprüht. Nach ca. 3 bis 4 Wochen Standzeit der Versuchspflanzen im Gewächshaus unter optimalen Wachstumsbedingungen wurde die Wirkung der Präparate optisch im Vergleich zu unbehandelten Kontrollen bonitiert. Die erfindungsgemäßen Mittel wiesen auch im Nachauflauf eine gute herbizide Wirksamkeit gegen ein breites Spektrum wirtschaftlich wichtiger Ungräser und Unkräuter auf. Beispielsweise zeigten die Beispiele Nr. 1.006, 1.049, 1.050, 1.063, 1.115, 1.128, 1.157, 1.301, 3.022, 3.023, 3.086, 5.022, 5.040, 5.041, 5.053, 5.120, 5.121, 5.152, 5.177, 5.181, 5.185, 5.188, 5.189, 5.190, 6.077 aus den Tabellen 1 bis 6 sehr gute herbizide Wirkung gegen Schadpflanzen wie Echinochloa crus-galli, Setaria viridis, Abuthilon theophrasti, Amaranthus retroflexus und Veronica persica im Nachauflaufverfahren bei einer Aufwandmenge von 1,28 kg und weniger Aktivsubstanz pro Hektar.

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt. Ein Teil der Töpfe wurde sofort wie unter Abschnitt 1 beschrieben behandelt, die übrigen im Gewächshaus aufgestellt, bis die Pflanzen zwei bis drei echte Blätter entwickelt hatten und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. In der Regel schonten die Verbindungen bei geeigneten Aufwandmengen auch Gramineen-Kulturen wie z.B. Gerste, Weizen, Roggen, Sorghum, Mais oder Reis. Die Verbindungen der Formel (I) zeigten teilweise eine hohe Selektivität und eignen sich deshalb zur Bekämpfung von unerwünschten Pflanzenwuchs in landwirtschaftlichen Kulturen.

## Patentansprüche

1. Verbindungen der Formel (I) oder deren Salze, 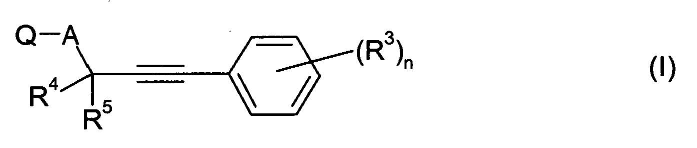 in welcher
Q einen carbocyclischen aromatischen Rest mit 6 Ringatomen oder einen heteroaromatischen Rest mit 5 bis 6 Ringatomen, wobei im Falle des heteroaromatischen Restes der Ring 1, 2, 3 oder 4 Stickstoffatome oder 1 Schwefelatom oder 1 Schwefelatom und 1 Stickstoffatom als Heteroringatome aufweist und wobei der carbocyclische aromatische Rest oder der heteroaromatische Rest an einer oder mehreren CH-Gruppen des Rings unsubstituiert oder mit einem Rest R¹ substituiert ist (CH wird zu CR¹), wobei die gegebenenfalls vorhandene Substitution der Gruppe Q durch Reste R¹ abgekürzt durch die Formel (R¹)ₘ definiert ist und der Rest oder die m Reste R¹ am Ring unabhängig voneinander wie nachstehend definiert sind, und wobei der heteroaromatische Rest, wenn er eine NH-Gruppe im Ring enthält, an der NH-Gruppe unsubstituiert oder mit einem Rest R² substituiert ist (NH wird zu NR²), wobei der Rest R² wie nachstehend definiert ist,
A eine ungesättigte divalente Gruppe der Formel (A1), (A2), (A3), (A4) oder (A5), 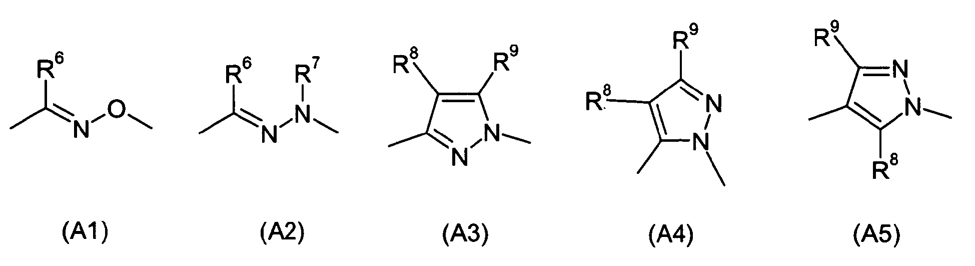 wobei der Rest Q mit der Gruppe A an dem in der jeweiligen Formel (A1) bis (A5) gezeigten ungesättigten C-Atom der Gruppe A gebunden ist und die Gruppe CR⁴R⁵ mit der Gruppe A an dem in der jeweiligen Formel (A1) bis (A5) gezeigten gesättigten Heteroatom der Gruppe A gebunden ist und wobei die Doppelbindung in der Gruppe der Formel (A1) oder (A2) Z- oder E-konfiguriert ist und wobei R⁶, R⁷, R⁸ und R⁹ wie nachstehend definiert sind,
(R¹)ₘ m Substituenten R¹ bedeutet, wobei R¹, im Fall dass m = 1 ist, oder jeder der Reste R¹ jeweils unabhängig voneinander, im Fall dass m größer als 1 ist, einen Rest aus den Gruppen (R¹a) bis (R¹j) bedeutet, enthaltend die Reste (R¹a) Halogen, Cyano, Azido, SF₅, Isocyanato und Nitro, (R¹b) Reste der Formel -NR^{a}R^{b},
worin jeder der Reste R^{a} und R^{b} unabhängig voneinander für einen Rest aus der Gruppe, welche die Reste
(1) Wasserstoff, (C₁-C₈)Alkyl, (C₂-C₈)Alkenyl und (C₂-C₈)Alkinyl,
(2) Phenyl und Benzyl,
wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
(3) [(C₁-C₈)Alkanoyl, das unsubstituiert oder durch einen oder mehrere Reste aus der Gruppe Halogen, CN, (C₁-C₆)Alkylamino, Di[(C₁-C₆)alkyl]-amino und (C₁-C₆)Alkoxy substituiert ist,
(4) [(C₁-C₈)Alkoxy]-carbonyl, das unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist,
(5) (C₁-C₄)Alkylsulfinyl und (C₁-C₄)Alkylsulfonyl, wobei jeder der beiden letztgenannten Reste unsubstituiert oder durch einen oder mehrere Reste aus der Gruppe Halogen, CN, (C₁-C₄)Alkoxy und (C₁-C₄)Haloalkoxy substituiert ist,
enthält, steht
oder
worin einer der Reste R^{a} und R^{b} wie oben definiert ist und der andere für Hydroxy oder (C₁-C₆)Alkoxy steht
oder
worin R^{a} und R^{b} gemeinsam für eine (C₂-C₅)Alkylen-Kette, welche durch ein oder mehrere Heteroatome aus der Gruppe O und S unterbrochen sein kann und welche unsubstituiert oder durch einen oder mehrere (C₁-C₄)Alkylreste substituiert ist, stehen
oder
worin R^{a} und R^{b} gemeinsam mit dem N-Atom der Gruppe NR^{a}R^{b} für N-Phthalimido stehen,
(R¹c) Reste der Formel -CO₂R,
worin R für Wasserstoff, (C₁-C₈)Alkyl, (C₂-C₈)Alkenyl oder (C₂-C₈)Alkinyl, wobei jeder der letztgenannten 3 Reste unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist, steht,
(R¹d) Reste der Formeln -CO-NR^{a}R^{b} und -CS-NR^{a}R^{b},
worin jeder der Reste R^{a} und R^{b} in den letztgenannten beiden Formeln unabhängig voneinander für einen Rest aus der Gruppe, welche
(1) Wasserstoff und (C₁-C₈)Alkyl, das unsubstituiert oder durch einen oder mehrere Reste aus der Gruppe Carboxy, [(C₁-C₈)Alkoxy]-carbonyl und CN substituiert ist,
(2) (C₂-C₈)Alkenyl und (C₂-C₈)Alkinyl,
(3) Phenyl und Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
enthält, steht oder
worin R^{a} und R^{b} gemeinsam für eine (C₂-C₅)Alkylen-Kette, welche durch ein oder mehrere Heteroatome aus der Gruppe O und S unterbrochen sein kann und welche unsubstituiert oder durch einen oder mehrere (C₁-C₄)Alkylreste substituiert ist, stehen,
(R¹e) Reste der Formel -CO-R,
worin R für Wasserstoff, (C₁-C₆)Alkyl, (C₁-C₆)Haloalkyl oder (C₃-C₆)Cycloalkyl, wobei der Cycloalkylrest unsubstituiert oder durch einen oder mehrere Reste aus der Gruppe (C₁-C₄)Alkyl substituiert ist, steht,
(R¹f) Reste der Formel -C(R^{a})=N-OR^{b},
worin
R^{a} für Wasserstoff, (C₁-C₆)Alkyl, (C₁-C₆)Haloalkyl oder (C₃-C₆)Cycloalkyl, wobei der Cycloalkylrest unsubstituiert oder durch einen oder mehrere (C₁-C₄)Alkylreste substituiert ist, und
R^{b} für Wasserstoff, (C₁-C₈)Alkyl, (C₁-C₈)Haloalkyl, (C₂-C₈)Alkenyl, (C₂-C₈)Haloalkenyl oder (C₂-C₈)Alkinyl stehen,
(R¹g) Reste der Formel -C(SR^{a})=NR^{b}, worin
R^{a} für (C₁-C₆)Alkyl und
R^{b} für Wasserstoff oder (C₁-C₆)Alkyl stehen,
(R¹h) Reste der Formel -OR, worin R
(1) für Wasserstoff, (C₁-C₈)Alkyl, (C₂-C₈)Alkenyl oder (C₂-C₈)Alkinyl
(2) oder für (C₁-C₈)Alkyl, das unsubstituiert oder durch einen oder mehrere Reste aus der Gruppe Halogen, CN, Hydroxy, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist,
(3) oder für [(C₁-C₈)Alkoxy]-carbonyl, das unsubstituiert oder durch einen oder mehrere Reste aus der Gruppe Halogen, CN, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist,
(4) oder für Phenyl oder Phenyl-(C₁-C₆)alkyl, wobei jeder der letztgenannten beiden Reste im Phenylring unsubstituiert oder durch einen oder mehrere Reste aus der Gruppe Halogen, CN, Hydroxy, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, [(C₁-C₄)Alkoxy]-carbonyl, [(C₁-C₄)Haloalkoxy]-carbonyl, R'R"N-CO-, worin jeder Rest R' und R" unabhängig voneinander H, (C₁-C₄)Alkyl oder (C₁-C₄)Haloalkyl bedeutet, (C₁-C₄)Alkylsulfinyl und (C₁-C₄)Alkylsulfonyl substituiert ist, steht,
(R¹i) Reste der Formel -SR, -S(=O)-R, -O-S(=O)-R, -S(=O)₂-R, und -O-S(=O)₂-R,
wobei in jedem der letztgenannten 5 Formeln R jeweils unabhängig voneinander von den anderen Resten R für Wasserstoff, (C₁-C₈)Alkyl, (C₂-C₈)Alkenyl oder (C₂-C₈)Alkinyl
oder für (C₁-C₈)Alkyl, das durch einen oder mehrere Reste aus der Gruppe Halogen, CN und (C₁-C₄)Alkoxy substituiert ist, steht,
(R¹j) (C₁-C₈)Alkyl, (C₂-C₈)Alkenyl, (C₂-C₈)Alkinyl, (C₃-C₆)Cycloalkyl, (C₅-C₆)Cycloalkenyl, Heterocyclyl mit 3 bis 6 Ringatomen und einem oder mehreren Heteroringatomen aus der Gruppe N, O und S oder Phenyl,
wobei jeder der 7 letztgenannten Reste unsubstituiert oder durch einen oder mehrere Reste aus den Gruppen (G¹a) bis (G¹L) substituiert ist, enthaltend die Reste
(G¹a) Halogen, Cyano, Azido, SF₅, Isocyanato und Nitro,
(G¹b) (C₃-C₆)Cycloalkyl und (C₅-C₆)Cycloalkenyl,
wobei jeder der letztgenannten 2 Reste unsubstituiert oder durch einen oder mehrere Reste aus der Gruppe Halogen und (C₁-C₃)Alkyl substituiert ist,
(G¹c) Phenyl und Pyridyl,
wobei jeder der letztgenannten 2 Reste unsubstituiert oder durch einen oder mehrere Reste aus der Gruppe enthaltend Halogen, Hydroxy, (C₁-C₄)Alkyl, (C₁-C₄)Haloalkyl, (C₁-C₄)Alkoxy, (C₁-C₄)Haloalkoxy, (C₁-C₄)Alkylthio, [(C₁-C₄)Alkoxy]-carbonyl, [(C₁-C₄)Haloalkoxy]-carbonyl, R'R"N-CO-, worin jeder Rest R' und R" unabhängig voneinander H, (C₁-C₄)Alkyl oder (C₁-C₄)Haloalkyl bedeutet oder R' und R" gemeinsam mit dem N-Atom einen gesättigten oder ungesättigten heterocyclischen Ring mit 5 oder 6 Ringatomen, der neben dem N-Atom noch ein N-Atom oder Sauerstoffatom als Heteroatom enthalten kann, bedeuten, (C₁-C₄)Alkylsulfinyl und (C₁-C₄)Alkylsulfonyl substituiert ist,
(G¹d) Reste der Formel -NR^{a}R^{b}, worin jeder der Reste R^{a} und R^{b} unabhängig voneinander für einen Rest aus der Gruppe, welche die Reste
(1) Wasserstoff, (C₁-C₈)Alkyl, (C₂-C₈)Alkenyl und (C₂-C₈)Alkinyl,
(2) Phenyl und Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
(3) [(C₁-C₈)Alkyl]-carbonyl, das unsubstituiert oder durch einen oder mehrere Reste aus der Gruppe Halogen, CN, (C₁-C₆)Alkylamino, Di[(C₁-C₆)alkyl]-amino und (C₁-C₆)Alkoxy substituiert ist,
(4) [(C₁-C₈)Alkoxy]-carbonyl, das unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist,
(5) (C₁-C₄)Alkylsulfinyl und (C₁-C₄)Alkylsulfonyl, wobei jeder der beiden letztgenannten Reste unsubstituiert oder durch einen oder mehrere Reste aus der Gruppe Halogen, CN, (C₁-C₄)Alkoxy und (C₁-C₄)Haloalkoxy substituiert ist,
enthält, steht oder
R^{a} und R^{b} gemeinsam für eine (C₂-C₅)Alkylen-kette, welche durch ein oder mehrere Heteroatome aus der Gruppe O und S unterbrochen sein kann und welche unsubstituiert oder durch einen oder mehrere (C₁-C₄)Alkylreste substituiert ist, stehen,
(G¹e) Reste der Formel -CO₂R,
worin R für Wasserstoff, (C₁-C₈)Alkyl, (C₂-C₈)Alkenyl oder (C₂-C₈)Alkinyl, wobei jeder der letztgenannten 3 Reste unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist, steht,
(G¹f) Reste der Formeln -CO-NR^{a}R^{b} und -CS-NR^{a}R^{b}, worin R^{a} und R^{b} in den letztgenannten beiden Formeln jeweils unabhängig voneinander für einen Rest aus der Gruppe, welche die Reste
(1) Wasserstoff und (C₁-C₈)Alkyl, das unsubstituiert oder durch einen oder mehrere Reste aus der Gruppe Carboxy, [(C₁-C₈)Alkoxy]-carbonyl und CN substituiert ist,
(2) (C₂-C₈)Alkenyl und (C₂-C₈)Alkinyl,
(3) Phenyl und Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
enthält, stehen oder
R^{a} und R^{b} gemeinsam für eine (C₂-C₅)Alkylen-kette, welche durch ein oder mehrere Heteroatome aus der Gruppe 0 und S unterbrochen sein kann und welche unsubstituiert oder durch einen oder mehrere (C₁-C₄)Alkylreste substituiert ist, stehen,
(G¹g) Reste der Formel -CO-R,
worin R für Wasserstoff, (C₁-C₆)Alkyl, (C₁-C₆)Haloalkyl oder (C₃-C₆)Cycloalkyl, wobei der Cycloalkylrest unsubstituiert oder durch einen oder mehrere (C₁-C₄)Alkylreste substituiert ist, steht,
(G¹h) Reste der Formel -C(R^{a})=N-OR^{b}, worin
R^{a} für Wasserstoff, (C₁-C₆)Alkyl, (C₁-C₆)Haloalkyl oder (C₃-C₆)Cycloalkyl, wobei der Cycloalkylrest unsubstituiert oder durch einen oder mehrere (C₁-C₄)Alkylreste substituiert ist, und
R^{b} für Wasserstoff, (C₁-C₈)Alkyl, (C₁-C₈)Haloalkyl, (C₂-C₈)Alkenyl, (C₂-C₈)Haloalkenyl oder (C₂-C₈)Alkinyl stehen,
(G¹i) Reste der Formel -C(SR^{a})=NR^{b}, worin
R^{a} für (C₁-C₆)Alkyl und
R^{b} für Wasserstoff oder (C₁-C₆)Alkyl stehen,
(G¹j) Reste der Formel -OR,
worin R
(1) für Wasserstoff, (C₁-C₈)Alkyl, (C₂-C₈)Alkenyl oder (C₂-C₈)Alkinyl,
(2) oder für (C₁-C₈)Alkyl, das unsubstituiert oder durch einen oder mehrere Reste aus der Gruppe Halogen, CN, Hydroxy, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist,
(3) oder für [(C₁-C₈)Alkoxy]-carbonyl, das unsubstituiert oder durch einen oder mehrere Reste aus der Gruppe Halogen, CN, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist,
(4) oder für Phenyl oder Phenyl-(C₁-C₆)alkyl, wobei jeder der letztgenannten beiden Reste im Phenylring unsubstituiert oder durch einen oder mehrere Reste aus der Gruppe Halogen, CN, Hydroxy, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, [(C₁-C₄)Alkoxy]-carbonyl, [(C₁-C₄)Haloalkoxy]-carbonyl, R'R"N-CO-, worin jeder Rest R' und R" unabhängig voneinander H, (C₁-C₄)Alkyl oder (C₁-C₄)Haloalkyl bedeutet oder R' und R" gemeinsam mit dem N-Atom einen gesättigten oder ungesättigten heterocyclischen Ring mit 5 oder 6 Ringatomen, der neben dem N-Atom noch ein N-Atom oder Sauerstoffatom als Heteroatom enthalten kann, bedeuten, (C₁-C₄)Alkylsulfinyl und (C₁-C₄)Alkylsulfonyl substituiert ist,
steht,
(G¹k) Reste der Formel -SR, -S(=O)-R, -O-S(=O)-R, -S(=O)₂-R und -O-S(=O)₂-R,
wobei in jedem der letztgenannten 5 Formeln R jeweils unabhängig von den anderen Resten R für Wasserstoff, (C₁-C₈)Alkyl, (C₂-C₈)Alkenyl oder (C₂-C₈)Alkinyl oder für (C₁-C₈)Alkyl, das durch einen oder mehrere Reste aus der Gruppe Halogen, CN und (C₁-C₄)Alkoxy substituiert ist, steht,
(G¹L) und, nur im Falle R¹ = (C₃-C₆)Cycloalkyl, (C₅-C₆)Cycloalkenyl, Heterocyclyl oder Phenyl als Basisgruppe, auch die Reste (C₁-C₄)Alkyl, (C₁-C₄)Haloalkyl und (C₁-C₄)Alkoxy-(C₁-C₄)alkyl, oder
zwei benachbarte Reste R¹ gemeinsam eine (C₂-C₇)Alkylen- oder (C₃-C₇)Alkenylengruppe, welche durch 1, 2 oder 3 Heteroatome aus der Gruppe O, S und N unterbrochen sein kann und welche unsubstituiert oder durch einen oder mehrere Reste aus der Gruppe Halogen, CN, (C₁-C₄)Alkyl, (C₁-C₄)Haloalkyl und (C₁-C₄)Alkoxy substituiert ist, bedeuten,
m eine ganze Zahl von 0 bis zur Anzahl der CH-Gruppen im Ring des Grundkörpers des Restes der Formel Q bedeutet,
R² Wasserstoff, (C₁-C₆)Alkyl, (C₂-C₆)Alkenyl, (C₂-C₆)Alkinyl, (C₃-C₆)Cycloalkyl, (C₅-C₆)Cycloalkenyl, Heterocyclyl mit 3 bis 6 Ringatomen und 1, 2 oder 3 Heteroringatomen aus der Gruppe N, O und S oder Phenyl bedeutet, wobei jeder der 7 letztgenannten Reste unsubstituiert oder durch einen oder mehrere Reste aus den Gruppen (G²a) bis (G²h) substituiert ist, enthaltend die Reste
(G²a) Halogen, Cyano, Azido, SF₅, Isocyanato und Nitro,
(G²b) (C₃-C₆)Cycloalkyl und (C₅-C₆)Cycloalkenyl,
wobei jeder der letztgenannten 2 Reste unsubstituiert oder durch einen oder mehrere Reste aus der Gruppe Halogen und (C₁-C₃)Alkyl substituiert ist,
(G²c) Phenyl und Pyridyl,
wobei jeder der letztgenannten 2 Reste unsubstituiert oder durch einen oder mehrere Reste aus der Gruppe, welche die Reste
(1) Halogen, Hydroxy, (C₁-C₄)Alkyl, (C₁-C₄)Haloalkyl, (C₁-C₄)Alkoxy, (C₁-C₄)Haloalkoxy, (C₁-C₄)Alkylthio, [(C₁-C₄)Alkoxy]-carbony und [(C₁-C₄)Haloalkoxy]-carbonyl,
(2) (C₁-C₄)Alkylsulfinyl und (C₁-C₄)Alkylsulfonyl enthält, substituiert ist,
(G²d) Reste der Formel -CO₂R,
worin R für Wasserstoff, (C₁-C₈)Alkyl, (C₂-C₈)Alkenyl oder (C₂-C₈)Alkinyl, wobei jeder der letztgenannten 3 Reste unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist, steht,
(G²e) Reste der Formeln -CO-NR^{a}R^{b} und -CS-NR^{a}R^{b},
worin jeder der Reste R^{a} und R^{b} in den letztgenannten beiden Formeln jeweils unabhängig voneinander für einen Rest aus der Gruppe, welche die Reste
(1) Wasserstoff und (C₁-C₈)Alkyl, das unsubstituiert oder durch einen oder mehrere Reste aus der Gruppe Carboxy, [(C₁-C₈)Alkoxy]-carbonyl und CN substituiert ist,
(2) (C₂-C₈)Alkenyl und (C₂-C₈)Alkinyl,
(3) Phenyl und Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkyisulfinyl und (C₁-C₄)Alkylsulfonyl substituiert ist,
enthält, steht oder
R^{a} und R^{b} gemeinsam für eine (C₂-C₅)Alkylen-kette stehen, welche durch ein oder mehrere Heteroatome aus der Gruppe O und S unterbrochen sein kann und welche unsubstituiert oder durch einen oder mehrere (C₁-C₄)Alkylreste substituiert ist,
(G²f) Reste der Formel -OR,
worin R für Wasserstoff, (C₁-C₈)Alkyl, (C₂-C₈)Alkenyl oder (C₂-C₈)Alkinyl,
oder für (C₁-C₈)Alkyl, das unsubstituiert oder durch einen oder mehrere Reste aus der Gruppe Halogen, CN, Hydroxy, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist,
oder für [(C₁-C₈)Alkoxy]-carbonyl, das unsubstituiert oder durch einen oder mehrere Reste aus der Gruppe Halogen, CN, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist,
oder für Phenyl oder Phenyl-(C₁-C₆)alkyl, wobei jeder der letztgenannten beiden Reste im Phenylring unsubstituiert oder durch einen oder mehrere Reste aus der Gruppe, welche die Reste
(1) Halogen, CN, Hydroxy, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, [(C₁-C₄)Alkoxy]-carbonyl, [(C₁-C₄)Haloalkoxy]-carbonyl,
(2) (C₁-C₄)Alkylsulfinyl und (C₁-C₄)Alkylsulfonyl, enthält, substituiert ist,
steht,
(G²g) Reste der Formel -SR, -S(=O)-R, -O-S(=O)-R, -S(=O)₂-R, und -O-S(=O)₂-R, wobei in jedem der letztgenannten 5 Formeln R jeweils unabhängig voneinander von den anderen Resten R für Wasserstoff, (C₁-C₈)Alkyl, (C₂-C₈)Alkenyl oder (C₂-C₈)Alkinyl oder für (C₁-C₈)Alkyl, das unsubstituiert oder durch einen oder mehrere Reste aus der Gruppe Halogen, CN und (C₁-C₄)Alkoxy substituiert ist, steht,
(G²h) und, nur für den Fall R² = (C₃-C₆)Cycloalkyl, (C₅-C₆)Cycloalkenyl, Heterocyclyl oder Phenyl als Basisgruppen, auch die Reste (C₁-C₄)Alkyl, (C₁-C₄)Haloalkyl und (C₁-C₄)Alkoxy-(C₁-C₄)alkyl,
(R³)ₙ n Substituenten R¹ bedeutet, wobei R¹, im Fall dass n = 1 ist, oder jeder der Reste R¹ jeweils unabhängig voneinander, im Fall dass n größer als 1 ist, einen Rest aus den Gruppen (R³a) bis (R³n) bedeutet, enthaltend die Reste (R³a) Halogen, Cyano, Azido, SF₅, Isocyanato und Nitro,
(R³b) Reste der Formel -NR^{a}R^{b}, worin jeder der Reste R^{a} und R^{b} unabhängig voneinander für einen Rest aus der Gruppe, welche die Reste
(1) Wasserstoff, (C₁-C₈)Alkyl, (C₂-C₈)Alkenyl und (C₂-C₈)Alkinyl,
(2) Phenyl und Benzyl,
wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
(3) (C₁-C₈)Alkanoyl, [(C₂-C₆)Alkenyl]-carbonyl und [(C₂-C₆)Alkinyl]-carbonyl, wobei jeder der letztgenannten drei Reste unsubstituiert oder durch einen oder mehrere Reste aus der Gruppe Halogen, CN, Amino, (C₁-C₆)Alkoxy, (C₁-C₆)Alkylamino, Di[(C₁-C₆)alkyl]-amino, [(C₁-C₆)Alkoxy]-carbonyl, [(C₁-C₆)Alkoxy]-carbonylamino, Mono- und Di-[(C₁-C₆)Alkanoyl]amino und Mono- und Di-[(C₁-C₆)alkylsulfonyl]amino substituiert ist,
(4) Phenyl-carbonyl und Benzyl-carbonyl, wobei jeder der letztgenannten beiden Reste im Phenylteil unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
(5) [(C₁-C₈)Alkoxy]-carbonyl, [(C₂-C₆)Alkenyloxy]-carbonyl und [(C₂-C₆)Alkinyloxy]-carbonyl, wobei jeder der letztgenannten drei Reste unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist,
(6) (C₁-C₄)Alkylsulfinyl und (C₁-C₄)Alkylsulfonyl, wobei jeder der beiden letztgenannten Reste unsubstituiert oder durch einen oder mehrere Reste aus der Gruppe Halogen, CN, (C₁-C₄)Alkoxy und (C₁-C₄)Haloalkoxy substituiert ist,
enthält, steht
oder
worin einer der Reste R^{a} und R^{b} wie oben definiert ist und der andere für Hydroxy oder (C₁-C₆)Alkoxy steht
oder
worin R^{a} und R^{b} gemeinsam für eine (C₂-C₅)Alkylen-kette, welche durch ein oder mehrere Heteroatome aus der Gruppe O und S unterbrochen sein kann und welche unsubstituiert oder durch einen oder mehrere (C₁-C₄)Alkylreste substituiert ist, stehen
oder
worin R^{a} und R^{b} gemeinsam mit dem N-Atom der Gruppe NR^{a}R^{b} für N-Phthalimido stehen,
(R³c) Reste der Formel -CO₂R,
worin R für Wasserstoff, (C₁-C₈)Alkyl, (C₂-C₈)Alkenyl oder (C₂-C₈)Alkinyl, wobei jeder der letztgenannten 3 Reste unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkyisulfinyl und (C₁-C₄)Alkylsulfonyl substituiert ist, substituiert ist, steht,
(R³d) Reste der Formeln -CO-NR^{a}R^{b}, -CS-NR^{a}R^{b} und -SO₂-NR^{a}R^{b},
worin jeder der Reste R^{a} und R^{b} in den letztgenannten 3 Formeln unabhängig voneinander für einen Rest aus der Gruppe, welche die Reste
(1) Wasserstoff und (C₁-C₈)Alkyl, das unsubstituiert oder durch einen oder mehrere Reste aus der Gruppe Carboxy, [(C₁-C₈)Alkoxy]-carbonyl und CN substituiert ist,
(2) (C₂-C₈)Alkenyl und (C₂-C₈)Alkinyl,
(3) Phenyl und Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
enthält, steht oder
R^{a} und R^{b} gemeinsam für eine (C₂-C₅)Alkylen-Kette, welche durch ein oder mehrere Heteroatome aus der Gruppe O und S unterbrochen sein kann und welche unsubstituiert oder durch einen oder mehrere (C₁-C₄)Alkylreste substituiert ist, stehen,
(R³e) Reste der Formel -CO-R,
worin R für Wasserstoff, (C₁-C₆)Alkyl, (C₁-C₆)Haloalkyl, [(C₁-C₈)Alkoxy]-carbonyl, [(C₁-C₈)Alkoxy]-oxalyl oder (C₃-C₆)Cycloalkyl, wobei der Cycloalkylrest unsubstituiert oder durch einen oder mehrere (C₁-C₄)Alkylreste substituiert ist, steht,
(R³f) Reste der Formel -C(R^{a})=N-OR^{b}, worin
R^{a} für Wasserstoff, (C₁-C₆)Alkyl, (C₁-C₆)Haloalkyl oder (C₃-C₆)Cycloalkyl, wobei der Cycloalkylrest unsubstituiert oder durch einen oder mehrere (C₁-C₄)Alkylreste substituiert ist, und
R^{b} für Wasserstoff, (C₁-C₈)Alkyl, (C₁-C₈)Haloalkyl, (C₂-C₈)Alkenyl, (C₂-C₈)Haloalkenyl oder (C₂-C₈)Alkinyl stehen,
(R³g) Reste der Formel -C(SR^{a})=NR^{b}, worin
R^{a} für (C₁-C₆)Alkyl und
R^{b} für Wasserstoff oder (C₁-C₆)Alkyl stehen,
(R³h) Reste der Formel -OR, worin R für Wasserstoff, (C₁-C₈)Alkyl, (C₂-C₈)Alkenyl oder (C₂-C₈)Alkinyl steht oder
R für (C₁-C₈)Alkyl, das durch einen oder mehrere Reste aus der Gruppe Halogen, CN, Hydroxy, Carboxy, Amino, (C₁-C₆)Alkoxy, (C₁-C₆)Alkylthio, (C₁-C₆)Alkylsulfinyl, (C₁-C₆)Alkylsulfonyl, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₆)Haloalkoxy, (C₁-C₄)Alkoxy-(C₁-C₄)alkoxy, (C₁-C₄)Alkylamino und Di[(C₁-C₄)alkyl]-amino substituiert ist, steht
oder
R für [(C₁-C₆)Alkyl]-carbonyl, [(C₁-C₆)Haloalkyl]-carbonyl steht oder
R für [(C₁-C₈)Alkoxy]-carbonyl, das unsubstituiert oder durch einen oder mehrere Reste aus der Gruppe Halogen, CN, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist, steht oder
R für Mono- oder Di-[(C₁-C₈)alkyl]-aminocarbonyl, Mono- oder Di-[(C₁-C₈)alkanoyl]-aminocarbonyl, Mono- oder Di-[(C₁-C₈)alkylsulfonyl]-aminocarbonyl, N-[(C₁-C₈)Alkyl]-N-[(C₁-C₈)alkanoyl]-aminocarbonyl, N-[(C₁-C₈)Alkyl]-N-[(C₁-C₈)alkylsulfonyl]-aminocarbonyl, N-[(C₁-C₈)Alkanoyl]-N-[(C₁-C₈)alkylsulfonyl]-aminocarbonyl oder für einen Rest der Formel -C(=O)NR'R', worin die R' gemeinsam eine Alkylen-kette mit 2 bis 5 C-Atomen bilden, die noch durch ein Sauerstoff oder Stickstoffatom unterbrochen sein kann, steht oder
R für Mono- oder Di-[(C₁-C₈)alkyl]-aminothiocarbonyl, Mono- oder Di-[(C₁-C₈)alkanoyl]-aminothiocarbonyl, Mono- oder Di-[(C₁-C₈)alkylsulfonyl]-aminothiocarbonyl, N-[(C₁-C₈)Alkyl]-N-[(C₁-C₈)alkanoyl]-aminothiocarbonyl, N-[(C₁-C₈)Alkyl]-N-[(C₁-C₈)alkylsulfonyl]-aminothiocarbonyl, N-[(C₁-C₈)Alkanoyl]-N-[(C₁-C₈)alkylsulfonyl]-aminothiocarbonyl oder für einen Rest der Formel -C(S=)-NR'R', worin die R' gemeinsam eine Alkylen-Kette mit 2 bis 5 C-Atomen, die noch durch ein Sauerstoff oder Stickstoffatom unterbrochen sein kann, bilden, steht oder
R für Phenyl oder Phenyl-(C₁-C₆)alkyl, wobei jeder der letztgenannten beiden Reste im Phenylring unsubstituiert oder durch einen oder mehrere Reste aus der Gruppe Halogen, CN, Hydroxy, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, [(C₁-C₄)Alkoxy]-carbonyl, [(C₁-C₄)Haloalkoxy]-carbonyl, R'R"N-CO-, worin jeder Rest R' und R" unabhängig voneinander H, (C₁-C₄)Alkyl oder (C₁-C₄)Haloalkyl bedeutet, (C₁-C₄)Alkylsulfinyl und (C₁-C₄)Alkylsulfonyl substituiert ist, steht,
(R³i) Reste der Formel -SR,
worin R
für Wasserstoff, (C₁-C₈)Alkyl, (C₂-C₈)Alkenyl oder (C₂-C₈)Alkinyl oder für (C₁-C₈)Alkyl, das durch einen oder mehrere Reste aus der Gruppe Halogen, CN und (C₁-C₄)Alkoxy substituiert ist, steht oder
R für [(C₁-C₆)Alkyl]-carbonyl oder [(C₁-C₆)Haloalkyl]-carbonyl steht oder
R für [(C₁-C₈)Alkoxy]-carbonyl, das unsubstituiert oder durch einen oder mehrere Reste aus der Gruppe Halogen, CN, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist, steht oder
R für Mono- oder Di-[(C₁-C₈)alkyl]-aminocarbonyl, Mono- oder Di-[(C₁-C₈)alkanoyl]-aminocarbonyl, Mono- oder Di-[(C₁-C₈)alkylsulfonyl]-aminocarbonyl, N-[(C₁-C₈)Alkyl]-N-[(C₁-C₈)alkanoyl]-aminocarbonyl, N-[(C₁-C₈)Alkyl]-N-[(C₁-C₈)alkylsulfonyl]-aminocarbonyl, N-[(C₁-C₈)Alkanoyl]-N-[(C₁-C₈)alkylsulfonyl]-aminocarbonyl oder für einen Rest der Formel -C(=O)NR'R', worin die R' gemeinsam eine Alkylen-kette mit 2 bis 5 C-Atomen bilden, die noch durch ein Sauerstoff oder Stickstoffatom unterbrochen sein kann, steht,
(R³j) Reste der Formel -S(=O)-R und -S(=O)₂-R,
wobei in jedem der letztgenannten beiden Formeln R jeweils unabhängig voneinander für (C₁-C₈)Alkyl, (C₂-C₈)Alkenyl oder (C₂-C₈)Alkinyl oder für (C₁-C₈)Alkyl, das durch einen oder mehrere Reste aus der Gruppe Halogen, CN und (C₁-C₄)Alkoxy substituiert ist, steht,
(R³k) Reste der Formel -O-S(=O)₂-R,
wobei R für Wasserstoff, (C₁-C₈)Alkyl, (C₂-C₈)Alkenyl oder (C₂-C₈)Alkinyl oder für (C₁-C₈)Alkyl, das durch einen oder mehrere Reste aus der Gruppe Halogen, CN und (C₁-C₄)Alkoxy substituiert ist, oder für Phenyl, das durch einen oder mehrere Reste aus der Gruppe Halogen, CN, Nitro, Hydroxy, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Haloalkylsulfinyl, (C₁-C₄)Alkylsulfonyl und (C₁-C₄)Haloalkylsulfonyl substituiert ist, steht,
(R³L) Reste der Formel -N=C(OR^{a})R^{b},
wobei jeder der Reste R^{a} und R^{b} unabhängig voneinander für Wasserstoff oder (C₁-C₆)Alkyl stehen,
(R³m) Reste der Formel -N(OR^{a})COR^{b}
wobei jeder der Reste R^{a} und R^{b} unabhängig voneinander für Wasserstoff, (C₁-C₆)Alkyl oder Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, stehen,
(R³n) (C₁-C₈)Alkyl, (C₂-C₈)Alkenyl, (C₂-C₈)Alkinyl, (C₃-C₆)Cycloalkyl, (C₅-C₆)Cycloalkenyl, Heterocyclyl mit 3 bis 6 Ringatomen und einem oder mehreren Heteroringatomen aus der Gruppe N, O und S oder Phenyl,
wobei jeder der 7 letztgenannten Reste unsubstituiert oder durch einen oder mehrere Reste aus den Gruppen (G³a) bis (G³m) substituiert ist, enthaltend die Reste
(G³a) Halogen, Cyano, Azido, SF₅, Isocyanato und Nitro,
(G³b) (C₃-C₆)Cycloalkyl, (C₅-C₆)Cycloalkenyl und einen gesättigten oder ungesättigten nicht-aromatischen heterocyclischen Rest mit 3 bis 6 Ringatomen und 1, 2 oder 3 Heteroringatomen aus der Gruppe N, O und S,
wobei jeder der letztgenannten 3 Reste unsubstituiert oder durch einen oder mehrere Reste aus der Gruppe Halogen und (C₁-C₃)Alkyl substituiert ist,
(G³c) Phenyl und einen aromatischen heterocyclischen Rest mit 5 oder 6 Ringatomen und 1, 2 oder 3 Heteroringatomen aus der Gruppe N, O und S,
wobei jeder der letztgenannten 2 Reste unsubstituiert oder durch einen oder mehrere Reste aus der Gruppe Halogen, Hydroxy, (C₁-C₄)Alkyl, (C₁-C₄)Haloalkyl, (C₁-C₄)Alkoxy, (C₁-C₄)Haloalkoxy, (C₁-C₄)Alkylthio, [(C₁-C₄)Alkoxy]-carbonyl, [(C₁-C₄)Haloalkoxy]-carbonyl, R'R"N-CO-, worin jeder Rest R' und R" unabhängig voneinander H, (C₁-C₄)Alkyl oder (C₁-C₄)Haloalkyl bedeutet oder R' und R" gemeinsam mit dem N-Atom einen gesättigten oder ungesättigten heterocyclischen Ring mit 5 oder 6 Ringatomen, der neben dem N-Atom noch ein N-Atom oder Sauerstoffatom als Heteroatom enthalten kann, bedeuten, (C₁-C₄)Alkylsulfinyl und (C₁-C₄)Alkylsulfonyl substituiert ist,
(G³d) Reste der Formel -NR^{a}R^{b},
worin jeder der Reste R^{a} und R^{b} unabhängig voneinander für einen Rest aus der Gruppe, welche die Reste
(1) Wasserstoff, (C₁-C₈)Alkyl, (C₂-C₈)Alkenyl und (C₂-C₈)Alkinyl,
(2) Phenyl oder Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
(3) [(C₁-C₈)Alkanoyl, das unsubstituiert oder durch einen oder mehrere Reste aus der Gruppe Halogen, CN, (C₁-C₆)Alkylamino, Di[(C₁-C₆)alkyl]-amino und (C₁-C₆)Alkoxy substituiert ist,
(4) Phenyl-carbonyl und Benzyl-carbonyl, wobei jeder der letztgenannten beiden Reste im Phenylteil unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
(5) [(C₁-C₈)Alkoxy]-carbonyl, das unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist,
(6) (C₁-C₄)Alkylsulfinyl und (C₁-C₄)Alkylsulfonyl, wobei jeder der beiden letztgenannten Reste unsubstituiert oder durch einen oder mehrere Reste aus der Gruppe Halogen, CN, (C₁-C₄)Alkoxy und (C₁-C₄)Haloalkoxy substituiert ist, enthält, steht
oder
worin einer der Reste R^{a} und R^{b} wie oben definiert ist und der andere für Hydroxy oder (C₁-C₆)Alkoxy steht
oder
worin R^{a} und R^{b} gemeinsam für eine (C₂-C₅)Alkylen-kette, welche durch ein oder mehrere Heteroatome aus der Gruppe O und S unterbrochen sein kann und welche unsubstituiert oder durch einen oder mehrere (C₁-C₄)Alkylreste substituiert ist, stehen
oder
worin R^{a} und R^{b} gemeinsam mit dem N-Atom der Gruppe NR^{a}R^{b} für N-Phthalimido stehen,
(G³e) Reste der Formel -CO₂R,
worin R für Wasserstoff, (C₁-C₈)Alkyl, (C₂-C₈)Alkenyl oder (C₂-C₈)Alkinyl, wobei jeder der letztgenannten 3 Reste unsubstituiert oder durch einen oder mehrere Reste aus der Gruppe Halogen, (C₁-C₄)Alkoxy, (C₁-C₄)Haloalkoxy, (C₁-C₄)Alkoxy-(C₁-C₄)alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, substituiert ist, steht,
(G³f) Reste der Formeln -CO-NR^{a}R^{b} und -CS-NR^{a}R^{b},
worin jeder der Reste R^{a} und R^{b} in den letztgenannten beiden Formeln jeweils unabhängig voneinander für einen Rest aus der Gruppe, welche die Reste
(1) Wasserstoff und (C₁-C₈)Alkyl, das unsubstituiert oder durch einen oder mehrere Reste aus der Gruppe Carboxy, [(C₁-C₈)Alkoxy]-carbonyl und CN substituiert ist,
(2) (C₂-C₈)Alkenyl und (C₂-C₈)Alkinyl,
(3) Phenyl und Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
enthält, steht oder
R^{a} und R^{b} gemeinsam für eine (C₂-C₅)Alkylen-kette, welche durch ein oder mehrere Heteroatome aus der Gruppe O und S unterbrochen sein kann und welche unsubstituiert oder durch einen oder mehrere (C₁-C₄)Alkylreste substituiert ist, stehen,
(G³g) Reste der Formel -CO-R,
worin R für Wasserstoff, (C₁-C₆)Alkyl, (C₁-C₆)Haloalkyl oder (C₃-C₆)Cycloalkyl, wobei der Cycloalkylrest unsubstituiert oder durch einen oder mehrere (C₁-C₄)Alkylreste substituiert ist, steht,
(G³h) Reste der Formel -C(R^{a})=N-OR^{b}, worin
R^{a} für Wasserstoff, (C₁-C₆)Alkyl, (C₁-C₆)Haloalkyl oder (C₃-C₆)Cycloalkyl, wobei der Cycloalkylrest unsubstituiert oder durch einen oder mehrere (C₁-C₄)Alkylreste substituiert ist, und
R^{b} für Wasserstoff, (C₁-C₈)Alkyl, (C₁-C₈)Haloalkyl, (C₂-C₈)Alkenyl, (C₂-C₈)Haloalkenyl oder (C₂-C₈)Alkinyl stehen,
(G³i) Reste der Formel -C(SR^{a})=NR^{b}, worin
R^{a} für (C₁-C₆)Alkyl und
R^{b} für Wasserstoff oder (C₁-C₆)Alkyl stehen,
(G³j) Reste der Formel -OR,
worin R für Wasserstoff, (C₁-C₈)Alkyl, (C₂-C₈)Alkenyl oder (C₂-C₈)Alkinyl steht
oder
R für (C₁-C₈)Alkyl, das unsubstituiert oder durch einen oder mehrere Reste aus der Gruppe Halogen, CN, Hydroxy, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist, steht oder
R für [(C₁-C₈)Alkoxy]-carbonyl, das unsubstituiert oder durch einen oder mehrere Reste aus der Gruppe Halogen, CN, (C₁-C₄)Alkoxy, (C₁-C₄)Haloalkoxy, (C₁-C₄)Alkoxy-(C₁-C₄)alkoxy, (C₁-C₄)Alkylamino und Di[(C₁-C₄)alkyl]-amino substituiert ist, steht oder
R für Phenyl oder Phenyl-(C₁-C₆)alkyl, wobei jeder der letztgenannten beiden Reste im Phenylring unsubstituiert oder durch einen oder mehrere Reste aus der Gruppe Halogen, CN, Hydroxy, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, [(C₁-C₄)Alkoxy]-carbonyl, [(C₁-C₄)Haloalkoxy]-carbonyl, R'R"N-CO-, worin jeder Rest R' und R" unabhängig voneinander H, (C₁-C₄)Alkyl oder (C₁-C₄)Haloalkyl bedeutet oder R' und R" gemeinsam mit dem N-Atom einen gesättigten oder ungesättigten heterocyclischen Ring mit 5 oder 6 Ringatomen, der neben dem N-Atom noch ein N-Atom oder Sauerstoffatom als Heteroatom enthalten kann, bedeuten, (C₁-C₄)Alkylsulfinyl und (C₁-C₄)Alkylsulfonyl substituiert ist, steht
oder
R für Si[(C₁-C₄)alkyl]₃ steht,
(G³k) Reste der Formel -SR, -S(=O)-R, -O-S(=O)-R, -S(=O)₂-R und -O-S(=O)₂-R,
wobei in jedem der letztgenannten 5 Formeln R jeweils unabhängig voneinander von den anderen Resten R für Wasserstoff, (C₁-C₈)Alkyl, (C₂-C₈)Alkenyl oder (C₂-C₈)Alkinyl oder für (C₁-C₈)Alkyl, das durch einen oder mehrere Reste aus der Gruppe Halogen, CN und (C₁-C₄)Alkoxy substituiert ist, steht,
(G³L) Reste der Formeln -Si[(C₁-C₄)alkyl]₃, -S⁺[(C₁-C₄)alkyl]₂, und -N⁺[(C₁-C₄)alkyl]₃, wobei in den Fällen der kationischen Reste ein Anionäquivalent als Gegenion vorliegt,
(G³m) und, nur für den Fall R³ = (C₃-C₆)Cycloalkyl, (C₅-C₆)Cycloalkenyl, Heterocyclyl oder Phenyl als Basisgruppe, auch die Reste (C₁-C₄)Alkyl, (C₁-C₄)Haloalkyl und (C₁-C₄)Alkoxy-(C₁-C₄)alkyl, oder
zwei benachbarte Reste R³ gemeinsam eine (C₂-C₇)Alkylen- oder (C₃-C₇)Alkenylengruppe, welche durch 1, 2 oder 3 Heteroatome aus der Gruppe O, S und N unterbrochen sein kann und welche unsubstituiert oder durch einen oder mehrere Reste aus der Gruppe Halogen, CN, (C₁-C₄)Alkyl, (C₁-C₄)Haloalkyl und (C₁-C₄)Alkoxy substituiert ist, bedeuten,
n eine ganze Zahl von 0 bis 5 bedeutet,
R⁴ H, (C₁-C₄)Alkyl, (C₁-C₄)Haloalkyl, (C₃-C₆)Cycloalkyl, das unsubstituiert oder durch einen oder mehrere Reste aus der Gruppe Halogen und (C₁-C₃)Alkyl substituiert ist, bedeutet und
R⁵ H, (C₁-C₄)Alkyl, (C₁-C₄)Haloalkyl, (C₃-C₆)Cycloalkyl, das unsubstituiert oder durch einen oder mehrere Reste aus der Gruppe Halogen und (C₁-C₃)Alkyl substituiert ist, bedeutet oder
R⁴ und R⁵ gemeinsam mit dem sie verbindenden C-Atom einen 3- bis 6-gliedrigen Ring, der carbocyclisch ist oder heterocyclisch mit 1 oder 2 Heteroringatomen aus der Gruppe N, O und S ist, wobei der Ring unsubstituiert oder substituiert ist, vorzugsweise unsubstituiert oder durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist, bedeuten,
R⁶ H, (C₁-C₆)Alkyl, (C₂-C₆)Alkenyl oder (C₂-C₆)Alkinyl,
wobei jeder der letztgenannten 3 Reste unsubstituiert oder durch einen oder mehrere Reste aus der Gruppe, welche die Reste
(1) Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
(2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
(3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
(4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkylaminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten, substituiert ist,
enthält, substituiert ist, bedeutet
oder
R⁶ Phenyl, Pyridyl, Phenoxy, (C₃-C₆)Cycloalkyl, (C₅-C₆)Cycloalkenyl oder einen gesättigten heterocyclischen Rest mit 3 bis 6 Ringatomen und 1 oder 2 Heteroringatomen aus der Gruppe N, O und S,
wobei jeder der letztgenannten 6 Reste unsubstituiert oder durch einen oder mehrere Reste aus der Gruppe, welche die Reste
(1) (C₁-C₆)Alkyl, (C₂-C₆)Alkenyl, (C₂-C₆)Haloalkenyl, (C₂-C₆)Alkinyl, (C₂-C₆)Haloalkinyl, Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
(2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder
R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist.
(3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
(4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkylaminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
substituiert ist,
enthält, substituiert ist, bedeutet,
R⁷ H, (C₁-C₆)Alkyl, (C₂-C₆)Alkenyl, (C₂-C₆)Alkinyl,
wobei jeder der letztgenannten 3 Reste unsubstituiert oder durch einen oder mehrere Reste aus der Gruppe, welche die Reste
(1) Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
(2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder
R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
(3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
(4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthlo, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkylaminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
substituiert ist,
enthält, substituiert ist, bedeutet
oder
R⁷ Phenyl, Pyridyl, Phenoxy, (C₃-C₆)Cycloalkyl, (C₅-C₆)Cycloalkenyl oder einen gesättigten heterocyclischen Rest mit 3 bis 6 Ringatomen und 1 oder 2 Heteroringatomen aus der Gruppe N, O und S,
wobei jeder der letztgenannten 6 Reste unsubstituiert oder durch einen oder mehrere Reste aus der Gruppe, welche die Reste
(1) (C₁-C₆)Alkyl, (C₂-C₆)Alkenyl, (C₂-C₆)Haloalkenyl, (C₂-C₆)Alkinyl, (C₂-C₆)Haloalkinyl, Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
(2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder
R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
(3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
(4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkylaminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
substituiert ist,
enthält, substituiert ist, bedeutet
oder
R⁷ Formyl oder einen Rest der Formel CO-R, CO-OR, SO-R oder S(O)₂-R bedeutet,
worin R jeweils für (C₁-C₆)Alkyl, (C₂-C₆)Alkenyl, (C₂-C₆)Alkinyl,
wobei jeder der letztgenannten 3 Reste unsubstituiert oder durch einen oder mehrere Reste aus der Gruppe, welche die Reste
(1) Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
(2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder
R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
(3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
(4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthlo, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkylaminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten, substituiert ist,
enthält, substituiert ist, steht
oder
worin R für Phenyl, Pyridyl, Phenoxy, (C₃-C₆)Cycloalkyl, (C₅-C₆)Cycloalkenyl oder einen gesättigten heterocyclischen Rest mit 3 bis 6 Ringatomen und 1 oder 2 Heteroringatomen aus der Gruppe N, O und S,
wobei jeder der letztgenannten 6 Reste unsubstituiert oder durch einen oder mehrere Reste aus der Gruppe, welche die Reste
(1) (C₁-C₆)Alkyl, (C₂-C₆)Alkenyl, (C₂-C₆)Haloalkenyl, (C₂-C₆)Alkinyl, (C₂-C₆)Haloalkinyl, Halogen, Cyano, Nitro, OH, SH, (C₁-C₃)Alkoxy, (C₁-C₃)Alkylthlo, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
(2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
(3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
(4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkylaminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
substituiert ist,
enthält, substituiert ist, steht,
oder
R⁷ einen Rest der Formel CO-NR*R** bedeutet,
worin jeder der Reste R* und R** unabhängig voneinander für Wasserstoff, (C₁-C₆)Alkyl, (C₂-C₆)Alkenyl oder (C₂-C₆)Alkinyl,
wobei jeder der letztgenannten 3 Reste unsubstituiert oder durch einen oder mehrere Reste aus der Gruppe, welche die Reste
(1) Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
(2) Reste der Formel CO-NR^{a}R^{b}, worin jeder der Reste R^{a} und R^{b} unabhängig voneinander für Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder
R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
(3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
(4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthlo, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkylaminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
substituiert ist,
enthält, substituiert ist, oder
für Phenyl, Pyridyl, Phenoxy, (C₃-C₆)Cycloalkyl, (C₅-C₆)Cycloalkenyl oder einen gesättigten heterocyclischen Rest mit 3 bis 6 Ringatomen und 1 oder 2 Heteroringatomen aus der Gruppe N, O und S,
wobei jeder der letztgenannten 6 Reste unsubstituiert oder durch einen oder mehrere Reste aus der Gruppe, welche die Reste
(1) (C₁-C₆)Alkyl, (C₂-C₆)Alkenyl, (C₂-C₆)Haloalkenyl, (C₂-C₆)Alkinyl, (C₂-C₆)Haloalkinyl, Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
(2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder
R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist,
(3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
(4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkylaminocarbonyl, Di-[(C₁-C₃)haloalkyl]-aminocarbonyl, N-(C₁-C₃)haloalkyl-N-(C₁-C₃)alkyl-amino-carbonyl und Reste der Formel CO-NR'₂,
wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
substituiert ist,
enthält, substituiert ist, steht,
oder worin R* und R** gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bilden, und
R⁸ und R⁹ jeweils unabhängig voneinander Reste aus den Gruppen (i), (ii), (iii), (iv), (v), (vi) und (vii) bedeuten, wobei die Gruppen (i) bis (vii) die Reste
(i) Wasserstoff, Halogen, Cyano, Carboxy, Formyloxy, Nitro, Hydroxy und Thio,
(ii) (C₁-C₆)Alkyl, (C₂-C₆)Alkenyl und (C₂-C₆)Alkinyl,
wobei jeder der letztgenannten 3 Reste unsubstituiert oder durch einen oder mehrere Reste aus der Gruppe, welche die Reste
(1) Halogen, Cyano, Nitro, OH, SH, (C₁-C₃)Alkoxy, (C₁-C₃)Alkylthio, (C₁-C₃)Haloalkylthlo, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
(2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl oder (C₁-C₃)Alkylsulfonyl, (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
(3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
(4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthlo, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkyl-aminocarbonyl, Di-[(C₁-C₃)haloalkyl]-aminocarbonyl, N-(C₁-C₃)haloalkyl-N-(C₁-C₃)alkyl-aminocarbonyl und Reste der Formel CO-NR'₂,
wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten, substituiert ist,
enthält, substituiert ist,
(iii) Phenyl, Pyridyl, Phenoxy, (C₃-C₆)Cycloalkyl, (C₅-C₆)Cycloalkenyl oder einen gesättigten heterocyclischen Rest mit 3 bis 6 Ringatomen und 1 oder 2 Heteroringatomen aus der Gruppe N, O und S,
wobei jeder der letztgenannten 6 Reste unsubstituiert oder durch einen oder mehrere Reste aus der Gruppe, welche die Reste
(1) (C₁-C₆)Alkyl, (C₂-C₆)Alkenyl, (C₂-C₆)Haloalkenyl, (C₂-C₆)Alkinyl, (C₂-C₆)Haloalkinyl, Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
(2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
(3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
(4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkyl-aminocarbonyl, Di-[(C₁-C₃)haloalkyl]-aminocarbonyl, N-(C₁-C₃)haloalkyl-N-(C₁-C₃)alkyl-aminocarbonyl und Reste der Formel CO-NR'₂,
wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten, substituiert ist,
enthält, substituiert ist,
(iv) (C₁-C₆)Alkoxy, (C₁-C₆)Alklthio und (C₂-C₆)Alkenyloxy, wobei jeder der letztgenannten beiden Reste unsubstituiert oder durch einen oder mehrere Reste aus der Gruppe Halogen, CN, OH, SH, (C₁-C₃)Alkoxy, (C₁-C₃)Haloalkoxy und (C₃-C₆)Cycloalkoxy, das unsubstituiert oder durch einen oder mehrere Reste aus der Gruppe Halogen und (C₁-C₃)Alkyl substituiert ist, substituiert ist,
(v) (C₃-C₆)Cycloalkoxy und (C₅-C₆)Cycloalkenyloxy, wobei jeder der letztgenannten beiden Reste unsubstituiert oder durch einen oder mehrere Reste aus der Gruppe Halogen, CN, OH, SH, (C₁-C₃)Alkyl, (C₁-C₃)Haloalkyl, (C₁-C₃)Alkoxy und (C₁-C₃)Haloalkoxy substituiert ist,
(vi) Reste der Formeln -CO-OR, -SO-R, -S(O)₂-R, -O-CO-OR, -O-SO-R und -O-S(O)₂-R,
wobei jedes R in den letztgenannten 6 Formeln unabhängig von den anderen Resten R jeweils für (C₁-C₆)Alkyl, (C₂-C₆)Alkenyl oder (C₂-C₆)Alkinyl,
wobei jeder der letztgenannten 3 Reste unsubstituiert oder durch einen oder mehrere Reste aus der Gruppe, welche die Reste
(1) Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
(2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
(3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
(4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkyl-aminocarbonyl, Di-[(C₁-C₃)haloalkyl]-aminocarbonyl, N-(C₁-C₃)haloalkyl-N-(C₁-C₃)alkyl-aminocarbonyl und Reste der Formel CO-NR'₂,
wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten, substituiert ist,
enthält, substituiert ist, steht
oder
worin jedes R unabhängig von den anderen Resten R jeweils für Phenyl, Pyridyl, Phenoxy, (C₃-C₆)Cycloalkyl, (C₅-C₆)Cycloalkenyl oder einen gesättigten heterocyclischen Rest mit 3 bis 6 Ringatomen und 1 oder 2 Heteroringatomen aus der Gruppe N, O und S,
wobei jeder der letztgenannten 6 Reste unsubstituiert oder durch einen oder mehrere Reste aus der Gruppe, welche die Reste
(1) (C₁-C₆)Alkyl, (C₂-C₆)Alkenyl, (C₂-C₆)Haloalkenyl, (C₂-C₆)Alkinyl, (C₂-C₆)Haloalkinyl, Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
(2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist bedeuten,
(3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
(4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkyl-aminocarbonyl, Di-[(C₁-C₃)haloalkyl]-aminocarbonyl, N-(C₁-C₃)haloalkyl-N-(C₁-C₃)alkyl-aminocarbonyl und Reste der Formel CO-NR'₂,
wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten, substituiert ist,
enthält, substituiert ist, steht,
(vii) Reste der Formeln -CO-NR*R**, -O-CO-NR*R**, -O-CO-NR*R** und O-CO-NR*R**,
wobei in den letztgenannten 4 Formeln jeder der Reste R* und R** unabhängig voneinander für
Wasserstoff, (C₁-C₆)Alkyl, (C₂-C₆)Alkenyl oder (C₂-C₆)Alkinyl,
wobei jeder der letztgenannten 3 Reste unsubstituiert oder durch einen oder mehrere Reste aus der Gruppe, welche die Reste
(1) Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
(2) Reste der Formel CO-NR^{a}R^{b}, worin jeder der Reste R^{a} und R^{b} unabhängig voneinander für Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
(3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
(4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkyl-aminocarbonyl, Di-[(C₁-C₃)haloalkyl]-aminocarbonyl, N-(C₁-C₃)haloalkyl-N-(C₁-C₃)alkyl-aminocarbonyl und Reste der Formel CO-NR'₂,
wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
substituiert ist,
enthält, substituiert ist, oder
für Phenyl, Pyridyl, Phenoxy, (C₃-C₆)Cycloalkyl, (C₅-C₆)Cycloalkenyl oder einen gesättigten heterocyclischen Rest mit 3 bis 6 Ringatomen und 1 oder 2 Heteroringatomen aus der Gruppe N, O und S,
wobei jeder der letztgenannten 6 Reste unsubstituiert oder durch einen oder mehrere Reste aus der Gruppe, welche die Reste
(1) (C₁-C₆)Alkyl, (C₂-C₆)Alkenyl, (C₂-C₆)Haloalkenyl, (C₂-C₆)Alkinyl, (C₂-C₆)Haloalkinyl, Halogen, Cyano, Nitro, OH, SH, (C₁-C₃)Alkoxy, (C₁-C₃)Alkylthio, (C₁-C₃)Haloalkylthio, (C₁-C₃)Haloalkoxy, (C₁-C₆)Alkanoyl, (C₁-C₆)Haloalkanoyl, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl und (C₁-C₃)Haloalkylsulfonyl,
(2) Reste der Formel CO-NR^{a}R^{b}, worin jeder der Reste R^{a} und R^{b} unabhängig voneinander Wasserstoff, (C₁-C₃)Alkyl und (C₁-C₃)Haloalkyl, (C₁-C₃)Alkanoyl, (C₁-C₃)Haloalkanoyl, [(C₁-C₃)Alkoxy]-carbonyl, [(C₁-C₃)Haloalkoxy]-carbonyl, (C₁-C₃)Alkylsulfinyl, (C₁-C₃)Haloalkylsulfinyl, (C₁-C₃)Alkylsulfonyl oder (C₁-C₃)Haloalkylsulfonyl bedeutet oder R^{a} und R^{b} gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten,
(3) (C₃-C₆)Cycloalkyl und (C₅-C₆)Cycloalkenyl, wobei jeder der letztgenannten beiden Reste unsubstituiert oder durch einen oder mehrere Reste aus der Gruppe Halogen und (C₁-C₃)Alkyl substituiert ist,
(4) Phenyl, Pyridyl und Phenoxy, wobei jeder der letztgenannten 3 Reste unsubstituiert oder durch einen oder mehrere Reste aus der Gruppe Halogen, (C₁-C₄)Alkyl, (C₁-C₄)Haloalkyl, OH, (C₁-C₄)Alkoxy, (C₁-C₄)Haloalkoxy, (C₁-C₄)Alkylthio, (C₁-C₄)Haloalkylthio, CN, [(C₁-C₆)Alkoxy]-carbonyl, [(C₁-C₆)Haloalkoxy]-carbonyl, (C₁-C₃)Alkylsulfinyl, (C₁-C₃)Haloalkylsulfinyl, (C₁-C₃)Alkylsulfonyl, (C₁-C₃)Haloalkylsulfonyl, Aminocarbonyl, Mono-(C₁-C₃)alkyl-aminocarbonyl, Di(C₁-C₃)alkyl-aminocarbonyl, Mono-(C₁-C₃)haloalkyl-aminocarbonyl, Di-[(C₁-C₃)haloalkyl]-aminocarbonyl, N-(C₁-C₃)haloalkyl-N-(C₁-C₃)alkyl-aminocarbonyl und Reste der Formel CO-NR'₂,
wobei die R' gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bedeuten, substituiert ist, enthält, substituiert ist, steht,
oder worin R* und R** gemeinsam mit dem sie verbindenden N-Atom einen gesättigten oder teilweise ungesättigten (nicht aromatischen) heterocyclischen Rest mit 3 bis 7 Ringatomen, der neben dem N-Atom als Heteroringatom noch 1 oder 2 weitere Heteroringatome aus der Gruppe N, O und S enthalten kann und der unsubstitutiert oder durch einen oder mehrere Reste aus der Gruppe (C₁-C₃)Alkyl und Oxo substituiert ist, bilden,
enthalten, oder
R⁸ und R⁹ bedeuten gemeinsam eine (C₂-C₅)Alkylen-Kette, welche durch ein oder mehrere Heteroatome aus der Gruppe N, O und S unterbrochen sein kann und welche unsubstituiert oder durch einen oder mehrere Reste aus der Gruppe Halogen und (C₁-C₄)Alkyl substituiert ist.

2. Verbindungen nach Anspruch 1, **dadurch gekennzeichnet, dass**
(R¹)ₘ m Substituenten R¹ bedeutet, wobei R¹, im Fall dass m = 1 ist, oder jeder der Reste R¹ jeweils unabhängig voneinander, im Fall dass m größer als 1 ist, einen Rest aus den Gruppen (R¹a) bis (R¹j) bedeutet, enthaltend die Reste (R¹a) Halogen, Cyano, Isocyanato und Nitro,
(R¹b) Reste der Formel -NR^{a}R^{b},
worin jeder der Reste R^{a} und R^{b} unabhängig voneinander für einen Rest aus der Gruppe, welche die Reste
(1) Wasserstoff, (C₁-C₆)Alkyl, (C₂-C₆)Alkenyl und (C₂-C₆)Alkinyl,
(2) Phenyl und Benzyl,
wobei jeder der letztgenannten beiden Reste im Ring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy und (C₁-C₄)Haloalkoxy substituiert ist,
(3) [(C₁-C₆)Alkanoyl und [(C₁-C₆)Haloalkanoyl,
(4) [(C₁-C₆)Alkoxy]-carbonyl und [(C₁-C₆)Haloalkyloxy]-carbonyl,
(5) (C₁-C₄)Alkylsulfinyl, (C₁-C₄)Haloalkylsulfinyl, (C₁-C₄)Alkylsulfonyl und (C₁-C₄)Haloalkylsulfonyl
enthält, steht
oder
worin einer der Reste R^{a} und R^{b} wie oben definiert ist und der andere für Hydroxy oder (C₁-C₆)Alkoxy steht
oder
worin R^{a} und R^{b} gemeinsam für eine (C₂-C₅)Alkylen-kette, welche durch ein Heteroatom aus der Gruppe O und S unterbrochen sein kann und welche unsubstituiert oder durch einen oder mehrere (C₁-C₄)Alkylreste substituiert ist, stehen,
(R¹c) Reste der Formel -CO₂R,
worin R für Wasserstoff, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, (C₁-C₄)Alkoxy und Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist, substituiert ist,
(R¹d) Reste der Formeln -CO-NR^{a}R^{b} und -CS-NR^{a}R^{b},
worin jeder der Reste R^{a} und R^{b} in den letztgenannten beiden Formeln unabhängig voneinander für einen Rest aus der Gruppe, welche
(1) Wasserstoff und (C₁-C₄)Alkyl, das unsubstituiert oder durch einen oder mehrere Reste aus der Gruppe (C₁-C₆)Alkoxy]-carbonyl und CN substituiert ist,
(2) (C₂-C₆)Alkenyl und (C₂-C₆)Alkinyl,
(3) Phenyl und Benzyl, wobei jeder der letztgenannten beiden Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthlo, (C₁-C₄)Alkylsulfinyl und (C₁-C₄)Alkylsulfonyl substituiert ist,
enthält, steht oder
worin R^{a} und R^{b} gemeinsam für eine (C₂-C₅)Alkylen-kette, welche durch ein oder mehrere Heteroatome aus der Gruppe O und S unterbrochen sein kann und welche unsubstituiert oder durch einen oder mehrere (C₁-C₄)Alkylreste substituiert ist, stehen,
(R¹e) Reste der Formel -CO-R,
worin R für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, steht,
(R¹f) Reste der Formel -C(R^{a})=N-OR^{b}, worin
R^{a} für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, und
R^{b} für Wasserstoff, (C₁-C₆)Alkyl, (C₁-C₆)Haloalkyl, (C₂-C₆)Alkenyl, (C₂-C₆)Haloalkenyl oder (C₂-C₆)Alkinyl, stehen,
(R¹g) Reste der Formel -C(SR^{a})=NR^{b}, worin
R^{a} für (C₁-C₄)Alkyl und
R^{b} für Wasserstoff oder (C₁-C₄)Alkyl stehen,
(R¹h) Reste der Formel -OR, worin R
(1) für Wasserstoff, (C₁-C₆)Alkyl, (C₂-C₆)Alkenyl oder (C₂-C₆)Alkinyl
(2) oder für (C₁-C₆)Alkyl, das unsubstituiert oder durch einen oder mehrere Reste aus der Gruppe Halogen, CN, (C₁-C₄)Alkoxy, (C₁-C₄)Haloalkoxy und (C₁-C₄)Alkoxy-(C₁-C₄)alkoxy substituiert ist,
(3) oder für [(C₁-C₆)Alkoxy]-carbonyl, das unsubstituiert oder durch einen oder mehrere Reste aus der Gruppe Halogen, (C₁-C₄)Alkoxy, (C₁-C₄)Haloalkoxy und (C₁-C₄)Alkoxy-(C₁-C₄)alkoxy substituiert ist,
(4) oder für Phenyl oder Phenyl-(C₁-C₄)alkyl, wobei jeder der letztgenannten beiden Reste im Phenylring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy und (C₁-C₄)Alkylthio substituiert ist,
steht,
vorzugsweise die Reste (C₁-C₄)Alkoxy und (C₁-C₄)Haloalkoxy,
(R¹i) Reste der Formel -SR, -S(=O)-R und -S(=O)₂-R,
wobei in jedem der letztgenannten 3 Formeln R jeweils unabhängig voneinander von den anderen Resten R für Wasserstoff, (C₁-C₆)Alkyl, (C₂-C₆)Alkenyl oder (C₂-C₆)Alkinyl
oder für (C₁-C₆)Alkyl, das durch einen oder mehrere Reste aus der Gruppe Halogen, CN und (C₁-C₄)Alkoxy substituiert ist,
steht,
(R¹j) (C₁-C₆)Alkyl, (C₂-C₆)Alkenyl, (C₂-C₆)Alkinyl, (C₃-C₆)Cycloalkyl, (C₅-C₆)Cycloalkenyl, Heterocyclyl mit 3 bis 6 Ringatomen und einem bis 3 Heteroringatomen aus der Gruppe N, O und S oder Phenyl, wobei jeder der 7 letztgenannten Reste unsubstituiert oder durch einen oder mehrere Reste aus den Gruppen (G¹a) bis (G¹L) substituiert ist, enthaltend die Reste
(G¹a) Halogen und Cyano,
(G¹b) (C₃-C₆)Cycloalkyl, das unsubstituiert oder durch einen oder mehrere (C₁-C₃)Alkylreste substituiert ist,
(G¹c) Phenyl und Pyridyl,
wobei jeder der letztgenannten 2 Reste unsubstituiert oder durch einen oder mehrere Reste aus der Gruppe enthaltend Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
(G¹d) Reste der Formel -NR^{a}R^{b}, worin jeder der Reste R^{a} und R^{b} unabhängig voneinander für einen Rest aus der Gruppe, welche die Reste
(1) Wasserstoff, (C₁-C₆)Alkyl, (C₂-C₆)Alkenyl und (C₂-C₆)Alkinyl,
(2) Phenyl und Benzyl,
wobei jeder der letztgenannten beiden Reste im Ring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy und (C₁-C₄)Haloalkoxy substituiert ist,
(3) [(C₁-C₆)Alkanoyl und [(C₁-C₆)Haloalkanoyl,
(4) [(C₁-C₆)Alkoxy]-carbonyl und [(C₁-C₆)Haloalkyloxy]-carbonyl,
(5) (C₁-C₄)Alkylsulfinyl, (C₁-C₄)Haloalkylsulfinyl, (C₁-C₄)Alkylsulfonyl und (C₁-C₄)Haloalkylsulfonyl
enthält, steht oder
R^{a} und R^{b} gemeinsam für eine (C₂-C₅)Alkylen-kette, welche ein Heteroatom aus der Gruppe O und S unterbrochen sein kann und welche unsubstituiert oder durch einen oder mehrere (C₁-C₃)Alkylreste substituiert ist, stehen,
(G¹e) Reste der Formel -CO₂R,
worin R für Wasserstoff, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, (C₁-C₄)Alkoxy und Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist, substituiert ist, steht,
(G¹f) Reste der Formeln -CO-NR^{a}R^{b} und -CS-NR^{a}R^{b},
worin jeder der Reste R^{a} und R^{b} unabhängig voneinander für einen Rest aus der Gruppe, welche die Reste
(1) Wasserstoff und (C₁-C₄)Alkyl, das unsubstituiert oder durch einen oder mehrere Reste aus der Gruppe (C₁-C₆)Alkoxy]-carbonyl und CN substituiert ist,
(2) (C₂-C₆)Alkenyl und (C₂-C₆)Alkinyl, vorzugsweise (C₃-C₆)Alkenyl und (C₃-C₆)Alkinyl,
(3) Phenyl und Benzyl, wobei jeder der letztgenannten beiden letztgenannten Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
enthält, steht oder
R^{a} und R^{b} gemeinsam für eine (C₂-C₅)Alkylen-kette, welche durch ein Heteroatom aus der Gruppe O und S unterbrochen sein kann und welche unsubstituiert oder durch einen oder mehrere (C₁-C₃)Alkylreste substituiert ist, stehen,
(G¹g) Reste der Formel -CO-R,
worin R für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, steht,
(G¹h) Reste der Formel -C(R^{a})=N-OR^{b}, worin
R^{a} für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, und
R^{b} für Wasserstoff, (C₁-C₆)Alkyl, (C₁-C₆)Haloalkyl, (C₂-C₆)Alkenyl, (C₂-C₆)Haloalkenyl oder (C₂-C₆)Alkinyl stehen,
(G¹i) Reste der Formel -C(SR^{a})=NR^{b}, worin
R^{a} für (C₁-C₄)Alkyl und
R^{b} für Wasserstoff oder (C₁-C₄)Alkyl stehen,
(G¹j) Reste der Formel -OR, worin R
(1) für Wasserstoff, (C₁-C₆)Alkyl, (C₂-C₆)Alkenyl oder (C₂-C₆)Alkinyl
(2) oder für (C₁-C₆)Alkyl, das unsubstituiert oder durch einen oder mehrere Reste aus der Gruppe Halogen, CN, (C₁-C₄)Alkoxy, (C₁-C₄)Haloalkoxy und (C₁-C₄)Alkoxy-(C₁-C₄)alkoxy substituiert ist,
(3) oder für [(C₁-C₆)Alkoxy]-carbonyl, das unsubstituiert oder durch einen oder mehrere Reste aus der Gruppe Halogen, (C₁-C₄)Alkoxy, (C₁-C₄)Haloalkoxy und (C₁-C₄)Alkoxy-(C₁-C₄)alkoxy substituiert ist,
(4) oder für Phenyl oder Phenyl-(C₁-C₄)alkyl, wobei jeder der letztgenannten beiden Reste im Phenylring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy und (C₁-C₄)Alkylthio substituiert ist,
steht,
(G¹k) Reste der Formel -SR, -S(=O)-R und -S(=O)₂-R,
wobei in jedem der letztgenannten 3 Formeln R jeweils unabhängig voneinander von den anderen Resten R für Wasserstoff, (C₁-C₆)Alkyl, (C₂-C₆)Alkenyl oder (C₂-C₆)Alkinyl oder für (C₁-C₆)Alkyl, das durch einen oder mehrere Reste aus der Gruppe Halogen, CN und (C₁-C₄)Alkoxy substituiert ist, steht,
(G¹L) und, nur für den Fall R¹ = (C₃-C₆)Cycloalkyl, (C₅-C₆)Cycloalkenyl, Heterocyclyl oder Phenyl als Basisgruppe, auch die Reste (C₁-C₄)Alkyl, (C₁-C₄)Haloalkyl und (C₁-C₄)Alkoxy-(C₁-C₄)alkyl, oder
zwei benachbarte Reste R¹ gemeinsam eine (C₂-C₇)Alkylen- oder (C₃-C₇)Alkenylengruppe, welche durch 1 oder 2 Heteroatome aus der Gruppe O, S und N unterbrochen sein kann und welche unsubstituiert oder durch einen oder mehrere Reste aus der Gruppe Halogen, CN, (C₁-C₃)Alkyl, (C₁-C₃)Haloalkyl und (C₁-C₃)Alkoxy substituiert ist, bedeuten, und
m eine ganze Zahl von 0 bis zur Anzahl der CH-Gruppen im Ring des Restes der Formel Q bedeutet,
R² Wasserstoff, (C₁-C₄)Alkyl, (C₂-C₄)Alkenyl, (C₂-C₄)Alkinyl, (C₃-C₆)Cycloalkyl, (C₅-C₆)Cycloalkenyl, Heterocyclyl mit 3 bis 6 Ringatomen und 1, 2 oder 3 Heteroringatome aus der Gruppe N, O und S oder Phenyl bedeutet, wobei jeder der 7 letztgenannten Reste unsubstituiert oder durch einen oder mehrere Reste aus den Gruppen (G²a) bis (G²h) substituiert ist, enthaltend die Reste
(G²a) Halogen und Cyano,
(G²b) (C₃-C₆)Cycloalkyl, das unsubstituiert oder durch einen oder mehrere (C₁-C₃)Alkylreste substituiert ist,
(G²c) Phenyl und Pyridyl,
wobei jeder der letztgenannten 2 Reste unsubstituiert oder durch einen oder mehrere Reste aus der Gruppe enthaltend Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, vorzugsweise Phenyl,
(G²d) Reste der Formel -CO₂R,
worin R für Wasserstoff, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, (C₁-C₄)Alkoxy und Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist, substituiert ist, steht,
(G²e) Reste der Formeln -CO-NR^{a}R^{b} und -CS-NR^{a}R^{b},
worin jeder der Reste R^{a} und R^{b} unabhängig voneinander für einen Rest aus der Gruppe, welche die Reste
(1) Wasserstoff und (C₁-C₄)Alkyl, das unsubstituiert oder durch einen oder mehrere Reste aus der Gruppe (C₁-C₆)Alkoxy]-carbonyl und CN substituiert ist,
(2) (C₂-C₆)Alkenyl und (C₂-C₆)Alkinyl,
(3) Phenyl und Benzyl, wobei jeder der beiden letztgenannten Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl, vorzugsweise aus der Gruppe Halogen, (C₁-C₄)Alkyl, (C₁-C₄)Haloalkyl und (C₁-C₄)Alkoxy substituiert ist,
enthält, steht oder
R^{a} und R^{b} gemeinsam für eine (C₂-C₅)Alkylen-kette, welche durch ein Heteroatom aus der Gruppe O und S unterbrochen sein kann und welche unsubstituiert oder durch einen oder mehrere (C₁-C₃)Alkylreste substituiert ist, stehen,
(G²f) Reste der Formel -OR, worin R
(1) für Wasserstoff, (C₁-C₆)Alkyl, (C₂-C₆)Alkenyl oder (C₂-C₆)Alkinyl
(2) oder für (C₁-C₆)Alkyl, das unsubstituiert oder durch einen oder mehrere Reste aus der Gruppe Halogen, CN, (C₁-C₄)Alkoxy, (C₁-C₄)Haloalkoxy und (C₁-C₄)Alkoxy-(C₁-C₄)alkoxy substituiert ist,
(3) oder für [(C₁-C₆)Alkoxy]-carbonyl, das unsubstituiert oder durch einen oder mehrere Reste aus der Gruppe Halogen, (C₁-C₄)Alkoxy, (C₁-C₄)Haloalkoxy und (C₁-C₄)Alkoxy-(C₁-C₄)alkoxy substituiert ist,
(4) oder für Phenyl oder Phenyl-(C₁-C₄)alkyl, wobei jeder der letztgenannten beiden Reste im Phenylring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy und (C₁-C₄)Alkylthio substituiert ist, steht,
(G²g) Reste der Formel -SR, -S(=O)-R und -S(=O)₂-R,
wobei in jedem der letztgenannten 3 Formeln R jeweils unabhängig voneinander von den anderen Resten R für Wasserstoff, (C₁-C₆)Alkyl, (C₂-C₆)Alkenyl oder (C₂-C₆)Alkinyl
oder für (C₁-C₆)Alkyl, das durch einen oder mehrere Reste aus der Gruppe Halogen, CN und (C₁-C₄)Alkoxy substituiert ist, steht,
(G²h) und, nur für den Fall R² = (C₃-C₆)Cycloalkyl, (C₅-C₆)Cycloalkenyl, Heterocyclyl oder Phenyl als Basisgruppen, auch die Reste (C₁-C₄)Alkyl, (C₁-C₄)Haloalkyl und (C₁-C₄)Alkoxy-(C₁-C₄)alkyl,
(R³)ₙ n Substituenten R¹ bedeutet, wobei R¹, im Fall dass n = 1 ist, oder jeder der Reste R¹ jeweils unabhängig voneinander, im Fall dass n größer als 1 ist, einen Rest aus den Gruppen (R³a) bis (R³n) bedeutet, enthaltend die Reste
(R³a) Halogen, Cyano, Isocyanato und Nitro,
(R³b) Reste der Formel -NR^{a}R^{b}, worin jeder der Reste R^{a} und R^{b} unabhängig voneinander für einen Rest aus der Gruppe, welche die Reste
(1) Wasserstoff, (C₁-C₆)Alkyl, (C₂-C₆)Alkenyl und (C₂-C₆)Alkinyl,
(2) Phenyl und Benzyl,
wobei jeder der letztgenannten beiden Reste im Ring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy und (C₁-C₄)Haloalkoxy substituiert ist,
(3) [(C₁-C₆)Alkanoyl und [(C₁-C₆)Haloalkanoyl,
(4) Phenyl-carbonyl und Benzyl-carbonyl, wobei jeder der letztgenannten beiden Reste im Phenylteil unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist,
(5) [(C₁-C₆)Alkoxy]-carbonyl, das unsubstituiert oder durch einen oder mehrere Reste aus der Gruppe Halogen, (C₁-C₄)Alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist, substituiert ist,
(6) (C₁-C₄)Alkylsulfinyl, (C₁-C₄)Haloalkylsulfinyl, (C₁-C₄)Alkylsulfonyl und (C₁-C₄)Haloalkylsulfonyl
enthält, steht
oder
worin einer der Reste R^{a} und R^{b} wie oben definiert ist und der andere für Hydroxy oder (C₁-C₄)Alkoxy steht
oder
worin R^{a} und R^{b} gemeinsam für eine (C₂-C₅)Alkylen-kette, welche durch ein oder zwei Heteroatome aus der Gruppe O und S unterbrochen sein kann und welche unsubstituiert oder durch einen oder mehrere (C₁-C₃)Alkylreste substituiert ist, stehen
oder
worin R^{a} und R^{b} gemeinsam mit dem N-Atom der Gruppe NR^{a}R^{b} für N-Phthalimido stehen,
(R³c) Reste der Formel -CO₂R,
worin R für Wasserstoff, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, (C₁-C₄)Alkoxy und Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist, substituiert ist, steht
(R³d) Reste der Formeln -CO-NR^{a}R^{b}, -CS-NR^{a}R^{b} und -SO₂NR^{a}R^{b},
worin jeder der Reste R^{a} und R^{b} in den letztgenannten 3 Formeln unabhängig voneinander für einen Rest aus der Gruppe, welche die Reste
(1) Wasserstoff und (C₁-C₄)Alkyl, das unsubstituiert oder durch einen oder mehrere Reste aus der Gruppe (C₁-C₆)Alkoxy]-carbonyl und CN substituiert ist,
(2) (C₂-C₆)Alkenyl und (C₂-C₆)Alkinyl,
(3) Phenyl und Benzyl, wobei jeder der letztgenannten beiden letztgenannten Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, enthält, steht oder
worin R^{a} und R^{b} gemeinsam für eine (C₂-C₅)Alkylen-kette, welche durch ein oder zwei Heteroatome aus der Gruppe O und S unterbrochen sein kann und welche unsubstituiert oder durch einen oder mehrere (C₁-C₄)Alkylreste substituiert ist, stehen,
(R³e) Reste der Formel -CO-R,
worin R für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, steht,
(R³f) Reste der Formel -C(R^{a})=N-OR^{b}, worin
R^{a} für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, und
R^{b} für Wasserstoff, (C₁-C₆)Alkyl, (C₁-C₆)Haloalkyl, (C₂-C₆)Alkenyl, (C₂-C₆)Haloalkenyl oder (C₂-C₆)Alkinyl stehen,
(R³g) Reste der Formel -C(SR^{a})=NR^{b}, worin
R^{a} für (C₁-C₄)Alkyl und
R^{b} für Wasserstoff oder (C₁-C₄)Alkyl stehen,
(R³h) Reste der Formel -OR, worin
(1) für Wasserstoff, (C₁-C₆)Alkyl, (C₂-C₆)Alkenyl oder (C₂-C₆)Alkinyl
(2) oder für (C₁-C₆)Alkyl, das unsubstituiert oder durch einen oder mehrere Reste aus der Gruppe Halogen, CN, (C₁-C₄)Alkoxy, (C₁-C₄)Haloalkoxy und (C₁-C₄)Alkoxy-(C₁-C₄)alkoxy substituiert ist,
(3) oder für [(C₁-C₆)Alkoxy]-carbonyl, das unsubstituiert oder durch einen oder mehrere Reste aus der Gruppe Halogen, (C₁-C₄)Alkoxy, (C₁-C₄)Haloalkoxy und (C₁-C₄)Alkoxy-(C₁-C₄)alkoxy substituiert ist,
(4) oder für Phenyl oder Phenyl-(C₁-C₄)alkyl, wobei jeder der letztgenannten beiden Reste im Phenylring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy und (C₁-C₄)Alkylthio substituiert ist,
steht,
(R³i) Reste der Formel -SR, worin R
für Wasserstoff, (C₁-C₆)Alkyl, (C₂-C₆)Alkenyl oder für (C₁-C₆)Alkyl, das durch einen oder mehrere Reste aus der Gruppe Halogen, CN und (C₁-C₄)Alkoxy substituiert ist, steht oder
R für [(C₁-C₄)Alkyl]-carbonyl oder [(C₁-C₄)Haloalkyl]-carbonyl steht oder
R für [(C₁-C₆)Alkoxy]-carbonyl, das unsubstituiert oder durch einen oder mehrere Reste aus der Gruppe Halogen, CN, (C₁-C₄)Alkoxy und (C₁-C₄)Haloalkoxy, substituiert ist, steht oder
R für Mono- oder Di-[(C₁-C₆)alkyl]-aminocarbonyl, Mono- oder Di-[(C₁-C₆)alkanoyl]-aminocarbonyl, Mono- oder Di-[(C₁-C₆)alkylsulfonyl]-aminocarbonyl, N-[(C₁-C₆)Alkyl]-N-[(C₁-C₆)alkanoyl]-aminocarbonyl, N-[(C₁-C₆)Alkyl]-N-[(C₁-C₆)alkylsulfonyl]-aminocarbonyl, N-[(C₁-C₆)Alkanoyl]-N-[(C₁-C₆)alkylsulfonyl]-aminocarbonyl oder für einen Rest der Formel -C(=O)NR'R', worin die R' gemeinsam eine Alkylen-kette mit 2 bis 5 C-Atomen bilden, die noch durch ein Sauerstoff oder Stickstoffatom unterbrochen sein kann, steht,
(R³j) Reste der Formel -S(=O)-R und -S(=O)₂-R,
wobei in jedem der letztgenannten beiden Formeln R jeweils unabhängig voneinander für (C₁-C₆)Alkyl, (C₂-C₆)Alkenyl oder (C₂-C₆)Alkinyl oder für (C₁-C₆)Alkyl, das durch einen oder mehrere Reste aus der Gruppe Halogen, CN und (C₁-C₄)Alkoxy substituiert ist, steht,
(R³k) Reste der Formel -O-S(=O)₂-R,
wobei R für Wasserstoff, (C₁-C₆)Alkyl, (C₂-C₆)Alkenyl oder (C₂-C₆)Alkinyl oder für (C₁-C₆)Alkyl, das durch einen oder mehrere Reste aus der Gruppe Halogen, CN und (C₁-C₄)Alkoxy substituiert ist, oder für Phenyl, das durch einen oder mehrere Reste aus der Gruppe Halogen, CN, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Haloalkylsulfinyl, (C₁-C₄)Alkylsulfonyl und (C₁-C₄)Haloalkylsulfonyl substituiert ist, steht,
(R³L) Reste der Formel -N=C(OR^{a})R^{b},
wobei jeder der Reste R^{a} und R^{b} unabhängig voneinander für Wasserstoff oder (C₁-C₄)Alkyl stehen,
(R³m) Reste der Formel -N(OR^{a})COR^{b}
wobei jeder der Reste R^{a} und R^{b} unabhängig voneinander für Wasserstoff, (C₁-C₄)Alkyl oder Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, stehen,
(R³n) (C₁-C₆)Alkyl, (C₂-C₆)Alkenyl, (C₂-C₆)Alkinyl, (C₃-C₆)Cycloalkyl, (C₅-C₆)Cycloalkenyl, Heterocyclyl mit 3 bis 6 Ringatomen und einem bis 3 Heteroringatome aus der Gruppe N, O und S oder Phenyl,
wobei jeder der 7 letztgenannten Reste unsubstituiert oder durch einen oder mehrere Reste aus den Gruppen (G³a) bis (G³m) substituiert ist, enthaltend die Reste
(G³a) Halogen, Cyano und Isocyanato,
(G³b) (C₃-C₆)Cycloalkyl und einen gesättigten oder ungesättigten nicht-aromatischen heterocyclischen Rest mit 3 bis 6 Ringatomen und 1, 2 oder 3 Heteroringatomen aus der Gruppe N, O und S,
wobei jeder der letztgenannten 2 Reste unsubstituiert oder durch einen oder mehrere Reste aus der Gruppe Halogen und (C₁-C₃)Alkyl substituiert ist,
(G³c) Phenyl und einen aromatischen heterocyclischen Rest mit 5 oder 6 Ringatomen und 1, 2 oder 3 Heteroringatomen aus der Gruppe N, O und S,
wobei jeder der letztgenannten 2 Reste unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthlo, (C₁-C₄)Alkylsulfinyl und (C₁-C₄)Alkylsulfonyl substituiert ist,
(G³d) Reste der Formel -NR^{a}R^{b},
worin jeder der Reste R^{a} und R^{b} unabhängig voneinander für einen Rest aus der Gruppe, welche die Reste
(1) Wasserstoff, (C₁-C₆)Alkyl, (C₂-C₆)Alkenyl und (C₂-C₆)Alkinyl,
(2) Phenyl und Benzyl,
wobei jeder der letztgenannten beiden Reste im Ring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy und (C₁-C₄)Haloalkoxy substituiert ist,
(3) [(C₁-C₆)Alkanoyl und [(C₁-C₆)Haloalkanoyl,
(4) Phenyl-carbonyl und Benzyl-carbonyl, wobei jeder der letztgenannten beiden Reste im Phenylteil unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist,
(5) [(C₁-C₆)Alkoxy]-carbonyl, das unsubstituiert oder durch einen oder mehrere Reste aus der Gruppe Halogen, (C₁-C₄)Alkoxy und Phenyl, das unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist, substituiert ist,
(6) (C₁-C₄)Alkylsulfinyl, (C₁-C₄)Haloalkylsulfinyl, (C₁-C₄)Alkylsulfonyl und (C₁-C₄)Haloalkylsulfonyl
enthält, steht oder
worin R^{a} und R^{b} gemeinsam für eine (C₂-C₅)Alkylen-kette, welche ein Heteroatom aus der Gruppe O und S unterbrochen sein kann und welche unsubstituiert oder durch einen oder mehrere (C₁-C₃)Alkylreste substituiert ist, stehen oder
worin einer der Reste R^{a} und R^{b} wie oben definiert ist und der andere für Hydroxy oder (C₁-C₄)Alkoxy steht
oder
worin R^{a} und R^{b} gemeinsam für eine (C₂-C₅)Alkylen-kette, welche durch ein oder zwei Heteroatome aus der Gruppe O und S unterbrochen sein kann und welche unsubstituiert oder durch einen oder mehrere (C₁-C₃)Alkylreste substituiert ist, stehen, oder
(G³e) Reste der Formel -CO₂R,
worin R für Wasserstoff, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, (C₁-C₄)Alkoxy und Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfinyl und (C₁-C₄)Alkylsulfonyl substituiert ist, substituiert ist, steht,
(G³f) Reste der Formeln -CO-NR^{a}R^{b} und -CS-NR^{a}R^{b},
worin jeder der Reste R^{a} und R^{b} in den letztgenannten 2 Formeln unabhängig voneinander für einen Rest aus der Gruppe, welche die Reste
(1) Wasserstoff und (C₁-C₄)Alkyl, das unsubstituiert oder durch einen oder mehrere Reste aus der Gruppe (C₁-C₆)Alkoxy]-carbonyl und CN substituiert ist,
(2) (C₂-C₆)Alkenyl und (C₂-C₆)Alkinyl,
(3) Phenyl und Benzyl, wobei jeder der letztgenannten beiden letztgenannten Reste im Ring unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist,
enthält, steht oder
worin R^{a} und R^{b} gemeinsam für eine (C₂-C₅)Alkylen-kette, welche durch ein oder zwei Heteroatome aus der Gruppe O und S unterbrochen sein kann und welche unsubstituiert oder durch einen oder mehrere (C₁-C₄)Alkylreste substituiert ist, stehen,
(G³g) Reste der Formel -CO-R,
worin R für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, vorzugsweise für Wasserstoff oder (C₁-C₃)Alkyl steht,
(G³h) Reste der Formel -C(R^{a})=N-OR^{b}, worin
R^{a} für Wasserstoff, (C₁-C₄)Alkyl, (C₁-C₄)Haloalkyl oder (C₃-C₆)Cycloalkyl, wobei der Cycloalkylrest unsubstituiert oder durch einen oder mehrere (C₁-C₄)Alkylreste substituiert ist, und
R^{b} für Wasserstoff, (C₁-C₆)Alkyl, (C₁-C₆)Haloalkyl, (C₂-C₆)Alkenyl, (C₂-C₆)Haloalkenyl oder (C₂-C₆)Alkinyl stehen,
(G³i) Reste der Formel -C(SR^{a})=NR^{b}, worin
R^{a} für (C₁-C₆)Alkyl und
R^{b} für Wasserstoff oder (C₁-C₄)Alkyl stehen,
(G³j) Reste der Formel -OR, worin R für
(1) für Wasserstoff, (C₁-C₆)Alkyl, (C₂-C₆)Alkenyl oder (C₂-C₆)Alkinyl
(2) oder für (C₁-C₆)Alkyl, das unsubstituiert oder durch einen oder mehrere Reste aus der Gruppe Halogen, CN, (C₁-C₄)Alkoxy, (C₁-C₄)Haloalkoxy und (C₁-C₄)Alkoxy-(C₁-C₄)alkoxy substituiert ist,
(3) oder für [(C₁-C₆)Alkoxy]-carbonyl, das unsubstituiert oder durch einen oder mehrere Reste aus der Gruppe Halogen, (C₁-C₄)Alkoxy, (C₁-C₄)Haloalkoxy und (C₁-C₄)Alkoxy-(C₁-C₄)alkoxy substituiert ist,
(4) oder für Phenyl oder Phenyl-(C₁-C₄)alkyl, wobei jeder der letztgenannten beiden Reste im Phenylring unsubstituiert oder durch einen oder mehrere Reste aus der Gruppe Halogen, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy und (C₁-C₄)Alkylthio substituiert ist,
steht,
(G³k) Reste der Formel -SR, -S(=O)-R und -S(=O)₂-R,
wobei in jedem der letztgenannten 3 Formeln R jeweils unabhängig voneinander von den anderen Resten R für Wasserstoff, (C₁-C₆)Alkyl, (C₂-C₆)Alkenyl oder (C₂-C₆)Alkinyl oder für (C₁-C₆)Alkyl, das durch einen oder mehrere Reste aus der Gruppe Halogen, CN und (C₁-C₄)Alkoxy substituiert ist, steht,
(G³L) Reste der Formeln -Si[(C₁-C₃)alkyl]₃, -S⁺[(C₁-C₃)alkyl]₂, und -N⁺[(C₁-C₃)alkyl]₃, wobei in den Fällen der kationischen Reste ein Anionäquivalent als Gegenion vorliegt,
(G³m) und, nur für den Fall R³ = (C₃-C₆)Cycloalkyl, (C₅-C₆)Cycloalkenyl, Heterocyclyl oder Phenyl als Basisgruppe, auch die Reste (C₁-C₄)Alkyl, (C₁-C₄)Haloalkyl und (C₁-C₄)Alkoxy-(C₁-C₄)alkyl, oder
zwei benachbarte Reste R³ gemeinsam eine (C₂-C₅)Alkylen- oder (C₃-C₅)Alkenylengruppe, welche durch 1, 2 oder 3 Heteroatome aus der Gruppe O, S und N unterbrochen sein kann und welche unsubstituiert oder durch einen oder mehrere Reste aus der Gruppe CN und (C₁-C₄)Alkyl substituiert ist, bedeuten und
n eine ganze Zahl von 0 bis 5 bedeutet.

3. Verbindungen nach Anspruch 1 oder 2, **dadurch gekennzeichnet, dass**
(R¹)ₘ m Substituenten R¹ bedeutet, wobei R¹, im Fall dass m = 1 ist, oder jeder der Reste R¹ jeweils unabhängig voneinander, im Fall dass m größer als 1 ist, Halogen, Cyano, (C₁-C₆)Alkyl, das unsubstituiert oder durch einen oder mehrere Reste aus der Gruppe Halogen, CN, (C₁-C₄)Alkoxy, (C₁-C₄)Haloalkoxy und (C₁-C₄)Alkoxy-(C₁-C₄)alkoxy substituiert ist, oder (C₁-C₆)Alkoxy, das unsubstituiert oder durch einen oder mehrere Reste aus der Gruppe Halogen, CN, (C₁-C₄)Alkoxy, (C₁-C₄)Haloalkoxy und (C₁-C₄)Alkoxy-(C₁-C₄)alkoxy substituiert ist, oder Phenoxy, das unsubstituiert oder durch einen oder mehrere Reste aus der Gruppe Halogen, CN, (C₁-C₄)Alkyl, (C₁-C₄)Haloalkyl, (C₁-C₄)Alkoxy, (C₁-C₄)Haloalkoxy, (C₁-C₄)Alkoxy-(C₁-C₄)alkoxy, (C₁-C₄)Alkylthio substituiert ist, oder [(C₁-C₄)Alkoxy]-carbonyl, Mono- oder Di-[(C₁-C₄)Alkyl]-amino, Mono- oder Di-[(C₁-C₄)Alkyl]-aminocarbonyl, Mono- oder Di-[(C₁-C₄)Alkanoyl]-amino bedeutet,
R² Wasserstoff oder (C₁-C₄)Alkyl, das unsubstituiert oder durch einen oder mehrere Reste aus der Gruppe Halogen, CN, (C₁-C₄)Alkoxy, (C₁-C₄)Haloalkoxy und (C₁-C₄)Alkoxy-(C₁-C₄)alkoxy substituiert ist, oder Formyl, (C₁-C₄)Alkanoyl, (C₁-C₄)Haloalkanoyl, [(C₁-C₃)Alkoxy]-carbonyl, Phenoxy-carbonyl, das im Phenylteil unsubstituiert oder durch einen oder mehrere Reste aus der Gruppe Halogen, CN, Nitro, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl und (C₁-C₄)Alkylsulfonyl substituiert ist, oder (C₁-C₄)Alkylsulfinyl, (C₁-C₄)Haloalkylsulfinyl, (C₁-C₄)Alkylsulfonyl oder (C₁-C₄)Haloalkylsulfonyl bedeutet und
(R³)ₙ n Substituenten R³ bedeutet, wobei R³, im Fall dass n = 1 ist, oder jeder der Reste R³ jeweils unabhängig voneinander, im Fall dass n größer als 1 ist, Halogen, Cyano, (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, wie Fluor oder Chlor, CN, (C₁-C₄)Alkoxy, (C₁-C₄)Haloalkoxy, (C₁-C₄)Alkoxy-(C₁-C₄)alkoxy und Mono- und Di-[(C₁-C₄)Alkyl]-amino substituiert ist, oder (C₃-C₆)Cycloalkyl, wie Cyclopropyl, Cyclobutyl, Cyclopentyl, Cyclohexyl, oder (C₁-C₆)Alkoxy, (C₂-C₆)Alkenyloxy oder (C₂-C₆)Alkinyloxy, wobei jeder der letztgenannten 3 Reste unsubstituiert oder durch einen oder mehrere Reste aus der Gruppe Halogen, wie Fluor oder Chlor, CN, (C₁-C₄)Alkoxy, (C₁-C₄)Haloalkoxy und (C₁-C₄)Alkoxy-(C₁-C₄)alkoxy substituiert ist, oder (C₁-C₆)Alkylthio, (C₂-C₆)Alkenylthio, (C₁-C₄)Alkylsulfinyl, (C₁-C₄)Haloalkylsulfinyl, (C₁-C₄)Alkylsulfonyl, (C₁-C₄)Haloalkylsulfonyl, Aminosulfonyl, Mono- oder Di-[(C₁-C₄)Alkyl]-amino, Mono- oder Di-[(C₁-C₄)Alkyl]-aminosulfonyl, Mono- oder Di-[(C₁-C₄)Alkyl]-sulfonylamino, Mono- oder Di-[(C₁-C₄)Alkanoyl]-amino bedeutet.

4. Verbindungen nach einem der Ansprüche 1 bis 3, **dadurch gekennzeichnet, dass**
R⁴ H, (C₁-C₃)Alkyl, (C₁-C₃)Haloalkyl, (C₃-C₆)Cycloalkyl, das unsubstituiert oder durch einen oder mehrere Reste aus der Gruppe Halogen und (C₁-C₃)Alkyl substituiert ist, und
R⁵ H oder (C₁-C₃)Alkyl oder
R⁴ und R⁵ gemeinsam mit dem sie verbindenden C-Atom einen 3- bis 6-gliedrigen Ring, der carbocyclisch ist, wobei der Ring unsubstituiert oder durch einen oder mehrere Reste aus der Gruppe Halogen und (C₁-C₃)Alkyl substituiert ist,
R⁶ Wasserstoff, (C₁-C₄)Alkyl oder Cyclopropyl und
R⁷ Wasserstoff, (C₁-C₄)Alkyl, (C₁-C₄)Alkanoyl oder [(C₁-C₄)Alkoxy]-carbonyl
R⁸ Wasserstoff, Halogen, (C₁-C₄)Alkyl,
R⁹ Wasserstoff, Halogen, (C₁-C₄)Alkyl oder
R⁸ und R⁹ gemeinsam eine (C₂-C₅)Alkylen-Kette, welche durch ein oder mehrere
Heteroatome aus der Gruppe N, O und S unterbrochen sein kann und welche unsubstituiert oder durch einen oder mehrere Reste aus der Gruppe Halogen und (C₁-C₄)Alkyl substituiert ist
bedeuten.

5. Verbindungen nach einem der Ansprüche 1 bis 4, **dadurch gekennzeichnet, dass** Q einen Rest der Formeln (Q1) bis (Q12), 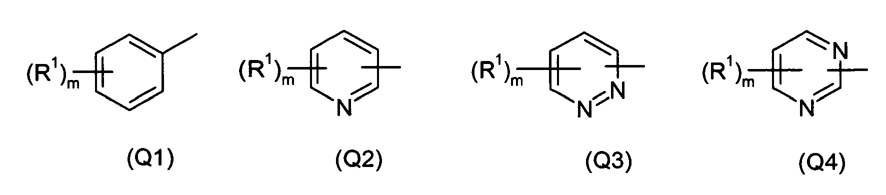 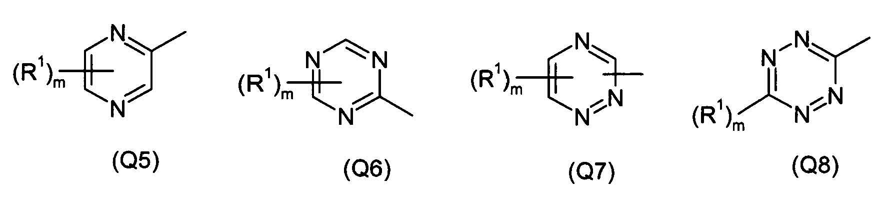 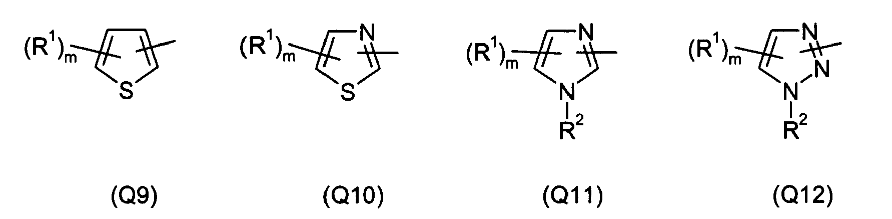 bedeutet, worin (R¹)ₘ und R² wie in Formel (I) definiert sind.

6. Verfahren zur Herstellung von Verbindungen der Formel (I) oder deren Salze, wie sie nach einem der Ansprüche 1 bis 5 definiert sind, **dadurch gekennzeichnet, dass** man
(a) eine Verbindung der Formel (II),
Q-A-H (II)
worin Q und A wie in der herzustellenden Verbindung der Formel (I) definiert sind, mit einer Verbindung der Formel (III), 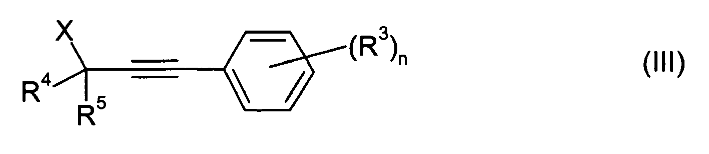 worin (R³)ₙ, R⁴ und R⁵ wie in der herzustellenden Verbindung der Formel (I) definiert sind und X eine Abgangsgruppe bedeutet, zur Verbindung der Formel (I) oder deren Salz umsetzt oder
(b) eine Verbindung der Formel (IV), 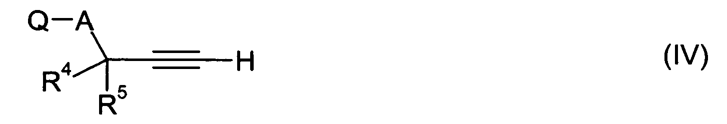 worin Q, A, R⁴ und R⁵ wie in der herzustellenden Verbindung der Formel (I) definiert sind, mit einer Verbindung der Formel (V), 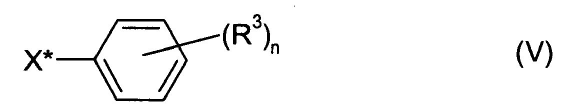 worin (R³)ₙ wie in der herzustellenden Verbindung der Formel (I) definiert ist und X* eine Abgangsgruppe bedeutet, zur Verbindung der Formel (I) oder deren Salz umsetzt oder
(c) für den Fall, dass A eine Gruppe der Formel (A1) bedeutet, eine Verbindung der Formel (VI), 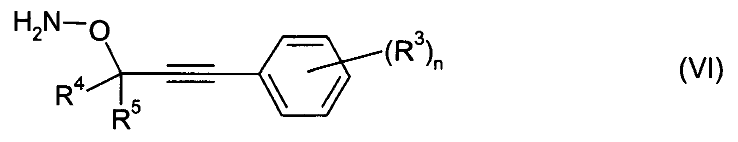 worin (R³)ₙ, R⁴ und R⁵ wie in der herzustellenden Verbindung der Formel (I) definiert sind, mit einer Verbindung der Formel (VII)
Q-CO-R⁶ (VII)
worin Q und R⁶ wie in der herzustellenden Verbindung der Formel (I) definiert sind, zur Verbindung der Formel (I) oder deren Salz, worin A eine Gruppe der Formel (A1) 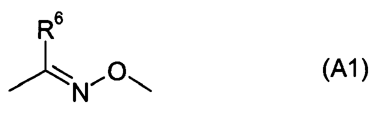 umsetzt oder
(d) für den Fall, dass A eine Gruppe der Formel (A2) bedeutet, eine Verbindung der Formel (VIII), 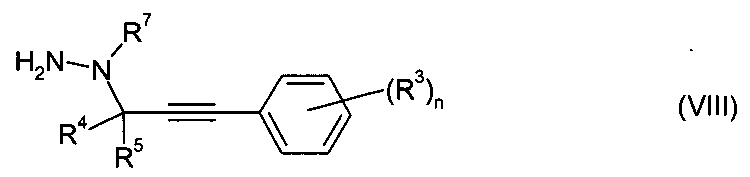 worin (R³)ₙ, R⁴, R⁵ und R⁷ wie in der herzustellenden Verbindung der Formel (I) definiert sind, mit einer Verbindung der Formel (VII)
Q-CO-R⁶ (VII)
worin Q und R⁶ wie in der herzustellenden Verbindung der Formel (I) definiert sind, zur Verbindung der Formel (I) oder deren Salz, worin A eine Gruppe der Formel (A2) 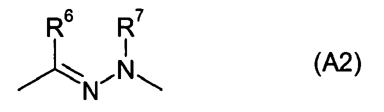 umsetzt oder
(e) für den Fall, dass A eine Gruppe der Formel A = (A3), (A4) oder (A5) bedeutet, eine Verbindung der Formel (IX), 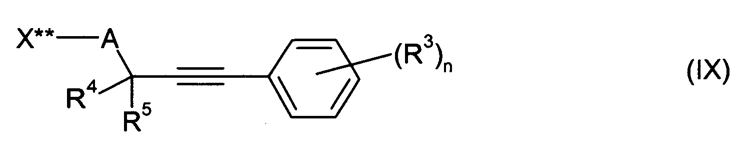 worin A, (R³)ₙ, R⁴ und R⁵ wie in der herzustellenden Verbindung der Formel (I) definiert sind und X** eine Abgangsgruppe bedeutet, mit einer Verbindung der Formel (X)
Q-B(OH)₂ (X)
worin Q wie in der herzustellenden Verbindung der Formel (I) definiert ist, unter Bedingungen einer Kreuzkupplung zur Verbindung der Formel (I) oder deren Salz, worin A die Gruppe der Formel (A3), (A4) oder (A5) bedeutet, 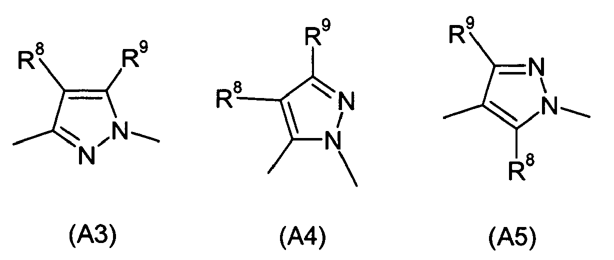 wobei R⁸ und R⁹ wie in der herzustellenden Verbindung der Formel (I) definiert sind, umsetzt oder
(f) für den Fall, dass A eine Gruppe der Formel A = (A3) oder (A4) bedeutet, eine Verbindung der Formel (VIII'), 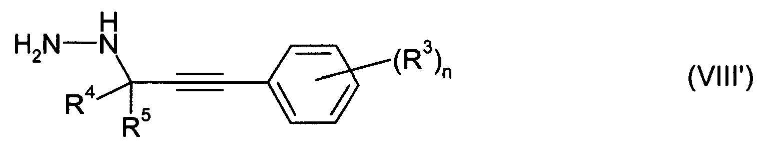 worin (R³)ₙ, R⁴ und R⁵ wie in der herzustellenden Verbindung der Formel (I) definiert sind, mit einer Verbindung der Formel (XI), 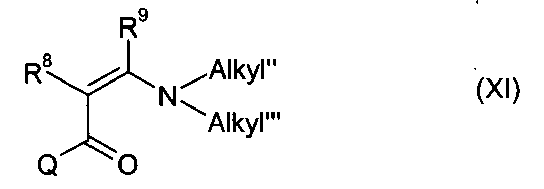 worin Q, R⁸ und R⁹ wie in der herzustellenden Verbindung der Formel (I) definiert sind und Alkyl" und Alkyl"' Alkylreste mit 1 bis 10 C-Atomen bedeuten, zur Verbindung der Formel (I) oder deren Salz, worin A die Gruppe der Formel (A3) oder (A4) bedeutet, 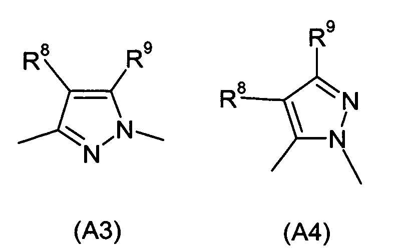 wobei R⁸ und R⁹ wie in der herzustellenden Verbindung der Formel (I) definiert sind, umsetzt oder
(g) für den Fall, dass A eine Gruppe der Formel A = (A5) bedeutet, eine Verbindung der Formel (VIII'), 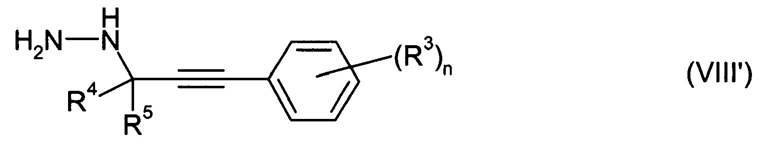 worin (R³)ₙ, R⁴ und R⁵ wie in der herzustellenden Verbindung der Formel (I) definiert sind, mit einer Verbindung der Formel (XI), 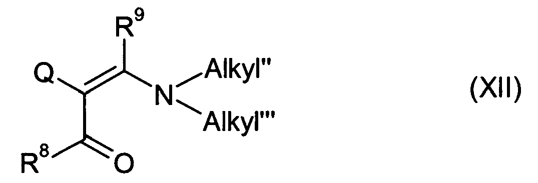 worin Q, R⁸ und R⁹ wie in der herzustellenden Verbindung der Formel (I) definiert sind und Alkyl" und Alkyl'" Alkylreste mit 1 bis 10 C-Atomen bedeuten, zur Verbindung der Formel (I) oder deren Salz, worin A die Gruppe der Formel (A5) bedeutet, 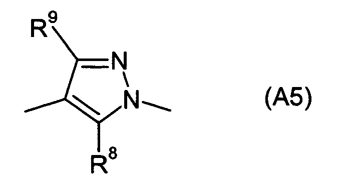 wobei R⁸ und R⁹ wie in der herzustellenden Verbindung der Formel (I) definiert sind, umsetzt.

7. Herbizides oder pflanzenwachstumsregulierendes Mittel, **dadurch gekennzeichnet, dass** es eine oder mehrere Verbindungen der Formel (I) oder deren Salze, wie sie nach einem der Ansprüche 1 bis 5 definiert sind, und im Pflanzenschutz übliche Formulierungshilfsmittel enthält.

8. Verfahren zur Bekämpfung von Schadpflanzen oder zur Wachstumsregulierung von Pflanzen, **dadurch gekennzeichnet, dass** man eine wirksame Menge von einer oder mehreren Verbindungen der Formel (I) oder deren Salzen, wie sie nach einem der Ansprüche 1 bis 5 definiert sind, auf die Pflanzen, Pflanzensamen oder die Anbaufläche appliziert.

9. Verfahren nach Anspruch 8, **dadurch gekennzeichnet, dass** die Verbindungen der Formel (I) oder deren Salze zur Bekämpfung von Schadpflanzen oder zur Wachstumsregulierung in Kulturen von Nutz- oder Zierpflanzen eingesetzt werden.

10. Verwendung der Verbindungen nach einem der Ansprüche 1 bis 5 als Herbizide oder Pflanzenwachstumsregulatoren.
